# EUROPEAN PATENT APPLICATION

(11) **EP 4 803 519 A1**
(43) Date of publication of application: **09.09.2026**
(21) Application number: 24899959.1
(22) Date of filing: 06.12.2024
(51) Int. Cl.: C07D 471/04, C07D 495/04, C07D 471/14, C07D 491/048, C07D 491/147, C07D 491/052, C07D 498/04, C07D 519/00

(54) **NOVEL PRMT5 INHIBITOR AND USE THEREOF**

(30) Priority: 07.12.2023 CN 202311675469; 26.01.2024 CN 202410117756; 07.03.2024 CN 202410259295; 29.04.2024 CN 202410536408
(71) Applicant: Shanghai Apeiron Therapeutics Company Limited, Shanghai 201210 (CN)
(72) Inventor: YAO, Bing, Shanghai 201210 (CN); WANG, Minghua, Shanghai 201210 (CN); ZHANG, Junqing, Shanghai 201210 (CN); GU, Xiaohui, Shanghai 201210 (CN); XIE, Shanshan, Shanghai 201210 (CN); CHENG, Kai, Shanghai 201210 (CN)
(74) Representative: Bayramoglu et al.
(86) International application number: PCT/CN2024/137400
(87) International publication number: WO 2025/119326

(57) **Abstract**

The present disclosure describes a novel molecule having protein arginine methyltransferase 5 inhibitory activity, and synthetic and use methods of the compound. Specifically, the present disclosure describes the compound of formula (I) or pharmaceutically acceptable salts, hydrates or solvates thereof and synthetic and use methods of the compound.

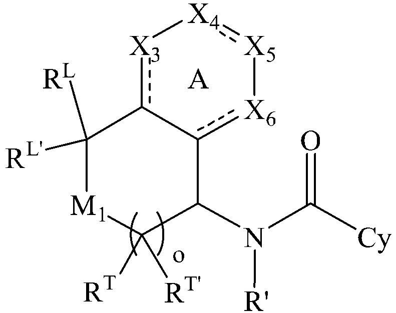

## Description

### TECHNICAL FIELD

The present disclosure belongs to the field of drug synthesis, specifically to a PRMT5 inhibitor and use thereof.

### BACKGROUND ART

Epigenetic alterations are key mediators that drive and maintain the malignant phenotype of tumors. Changes in DNA methylation, histone acetylation and methylation, noncoding RNA, and post-translational modifications are all epigenetic driving forces of cancer development, independent of changes in the DNA sequence. Arginine methylation is an important kind of post-translational modifications that affect cell growth and proliferation, apoptosis, angiogenesis and metastasis by regulating transcription and post-transcriptional RNA processing. There are three types of methylarginine, ω-NG, N'G-asymmetric dimethylarginine (ADMA) and ω-NG, N'G-symmetric dimethylarginine (SDMA). This modification is catalyzed by the protein arginine methyltransferase (PRMT) family, which transfers methyl from S-adenosylmethionine (AdoMet) to histone and nonhistone arginine side chains. Nine PRMT genes are annotated in the human genome, and classified as Type I (PRMT1, 2, 3, 4, 6 and 8), Type II (PRMT5 and PRMT9), and Type III enzyme (PRMT7) based on the type of methylarginine produced. PRMT5 is primarily a type II enzyme that catalyzes the symmetric dimethylation of arginine. PRMT5 was first identified in a two-hybrid assay designed to detect proteins interacting with Janus kinase 2 (Jak2).

PRMT5 is a universal transcriptional repressor that forms complexes with other transcription factors, including BRG1, Hbrm, Blimpl, and Snail. PRMT5 participates in different cell biological processes through methylation of substrates in the cytoplasm and nucleus, including histone H4 residue Arg3 (H4R3) and H3 residue Arg8 (H3R8). H4R3 methylation is associated with transcriptional repression, while H3R8 methylation is considered to be associated with both transcriptional activation and transcriptional repression. In addition to direct induction of inhibitory histone labeling by PRMT5, the role of this enzyme in gene silencing is mediated through the formation of multiple inhibitory protein complexes including NuRD fractions, HDACs, MDB proteins and DNA methyltransferase. PRMT5 affects its substrate specificity by interacting with some binding proteins. The core component of this protein complex is MEP50. MEP50 is required for the enzymatic activity of PRMT5. Studies have found that PRMT5 can methylate proteins participating RNA splicing, such as SmD3, which can be used to track the chemical activity of PRMT5 in cellular organism.

PRMT5 plays an important role in tumorigenesis. Studies have found that PRMT5 expression is up-regulated in a variety of tumors, including lymphoma, lung cancer, breast cancer and colorectal cancer. In addition, PRMT5 expression is increased in the samples of mantle cell lymphoma (MCL) patients, and PRMT5 knockout can inhibit MCL cell proliferation, indicating that PRMT5 plays an important role in MCL. Overexpression of PRMT5 promotes cell proliferation which is inhibited by PRMT5 knockout in the cell lines of melanoma, breast and lung cancer. Therefore, PRMT5 is a potential target for cancer therapy.

Loss of methylthioadenosine phosphorylase (MTAP) makes the cells selectively dependent on PRMT5 and its binding protein of WDR77. MTAP is often lost due to its proximity to the tumor suppressor gene of CDKN2A which is generally missing. The concentration of methionine adenosine (MTA, the metabolite cleaved by MTAP) is increased in the cells with MTAP deficiency. MTA has a similar structure to S-adenosylmethionine (SAM). With increase of concentration, MTA acts as an intrinsic selective inhibitor to inhibit the binding of SAM to PRMT5, thus inhibiting the methyltransferase activity of PRMT5.

The most significant structural difference between MTAP-deficient cancer cells and MTAP wild-type cancer cells is the PRMT5-MTA complex produced in MTAP-deficient cancer cells due to accumulation of MTA concentration. The inhibitor developed for the PRMT5-MTA complex can selectively target cancer cells with MTAP deficiency, with little effect on normal cells, which greatly improves the therapeutic index.

Thus, the identification and development of small molecules that inhibit PRMT5 activity would be useful as a therapeutic method for treating various PRMT5-related diseases or disorders (e.g., cancer).

### CONTENT OF THE INVENTION

In order to solve the technical problem of the present disclosure, the present disclosure provides a type of novel structural compounds with excellent inhibitory activity against PRMT5.

Specifically, the present disclosure provides a compound of formula (I), or a pharmaceutically acceptable salt, an ester, a prodrug, a stereoisomer or an isotopic derivative thereof. 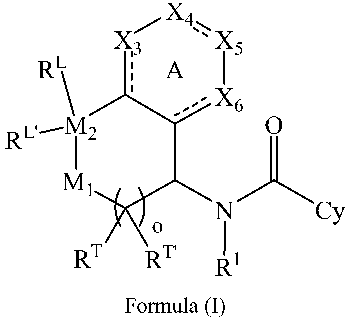
Wherein, Cy represents the following structure: 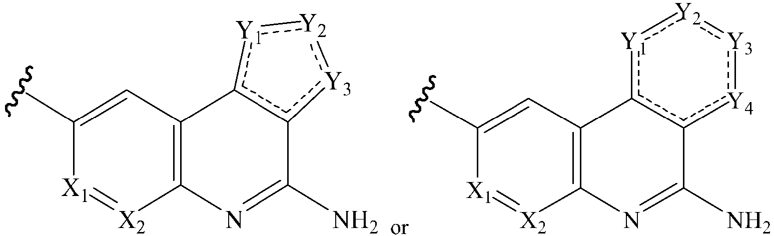
Wherein, the dashed line represents a single bond or double bond;
Wherein, Y₁ independently represents O, S, Se, N or CR^{Y1};
Wherein, Y₂ independently represents O, S, Se, N or CR^{Y2};
Wherein, Y₃ independently represents O, S, Se, N or CR^{Y3};
Wherein, Y₄ independently represents O, S, Se, N or CR^{Y4};
Wherein, X₁ represents N or CR^{X1};
Wherein, X₂ represents N or CR^{X2};
Wherein, X₃ represents N or CR^{X3};
Wherein, X₄ represents N or CR^{X4};
Wherein, X₅ represents N or CR^{X5};
Wherein, X₆ represents N or CR^{X6};
Wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, - OC(O)R^{a}, -OCONR^{a}R^{b}, halogen, -OSO₃R^{a}, -NR^{a}R^{b}, -SF₅, -C(O)OR^{a}, and -CO₂NR^{a}R^{b};
Wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, -C(O)OR^{a}, -CO₂NR^{a}R^{b}, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, -NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
Wherein, R^{X4} represents -L-R^{X4-1};
Wherein, L represents absent or CR^{a}R^{b}, SiR^{a}R^{b}, O, S, Se, or NR^{a};
Wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -SF₅, -NR^{a}R^{b}, -C(O)OR^{a}, -OC(O)R^{a}, -OCONR^{a}R^{b}, -NR^{a}COR^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, - C(O)R^{a}, -C(O)OR^{a}, -OC(O)R^{a}, -OC(O)NR^{a}R^{b}, -NR^{a}COR^{b} and -CONR^{a}R^{b};
wherein R^{Y1}, R^{Y2}, R^{Y3} and R^{Y4} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, -OC(O)R^{a}, -OCONR^{a}R^{b}, halogen, -OSO₃R^{a}, -NR^{a}R^{b}, -SF₅, -C(O)OR^{a}, and -CO₂NR^{a}R^{b};
Wherein, said ring A may also be optionally fused with a 5- to 6-membered saturated or unsaturated ring at the chemical bond between X₃ and X₄, which may contain 0-3 heteroatoms selected from O, N and S; wherein, the ring may also optionally be substituted with 0, 1, 2, or 3 substituents selected from the group consisting of: deuterium, C₁-C₆ alkyl, C₁-C₆ deuterated alkyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
Wherein, the ring A may also optionally be fused with the chemical bond between X₄ and X₅ to form a 5-6-membered saturated or unsaturated ring which may contain 0-3 heteroatoms selected from O, N and S; wherein, the ring may also optionally be substituted with 0, 1, 2, or 3 substituents selected from the group consisting of: deuterium, C₁-C₆ alkyl, C₁-C₆ deuterated alkyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
Wherein, R¹ represents hydrogen or C₁-C₆ alkyl, C₃-C₁₀ cycloalkyl, 4- to 10-membered heterocycloalkyl, C₆-C₁₀ aryl, and 5- to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
Preferably, R¹ represents -CHR^{s}R^{t} or -CDR^{s}R^{t};
Wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, - OC(O)R^{a}, -OC(O)NR^{a}R^{b}, -NR^{a}COR^{b} and -CONR^{a}R^{b};
Wherein, R² and R³ each independently represent hydrogen, deuterium, C₁-C₆ alkyl, halogen, hydroxyl, carboxyl, C₁-C₆ haloalkyl, hydroxy (C₁-C₆) alkyl, or mercapto (C₁-C₆) alkyl;
Wherein, M₁ represents CR^{a}R^{b}, -SiR^{a}R^{b}, NR^{a}, O, S or Se;
Wherein, M₂ represents C or Si;
Wherein, R^{L} and R^{L'} each independently represent C₁-C₆ alkyl; or R^{L} and R^{L'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -C(O)OR^{a}, -OC(O)R^{a}, -OCONR^{a}R^{b}, halogen, -OSO₃R^{a}, -NR^{a}R^{b}, and -SF₅;
Wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -C(O)OR^{a}, -OC(O)R^{a}, -OCONR^{a}R^{b}, halogen, -OSO₃R^{a}, - NR^{a}R^{b}, and -SF₅;
wherein, o represents 0, 1 or 2;
Wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S, Se, N and Si.

In addition, the present disclosure provides a compound of formula (I-1), or a pharmaceutically acceptable salt, ester, prodrug, stereoisomer or isotopic derivative thereof, 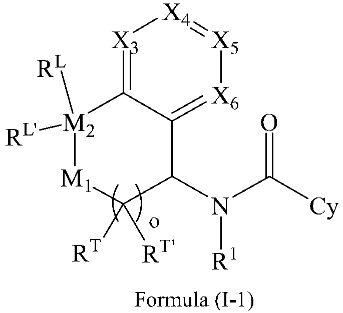
Wherein, Cy represents the following structure: 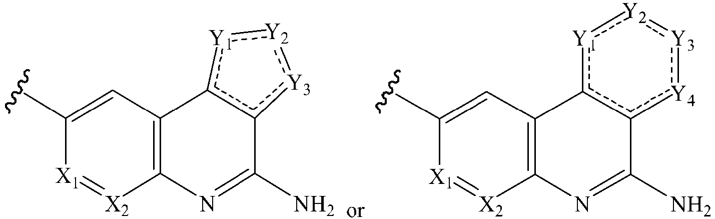
Wherein, the dashed line represents a single bond or double bond;
Wherein, Y₁ independently represents O, S, Se, N or CR^{Y1};
Wherein, Y₂ independently represents O, S, Se, N or CR^{Y2};
Wherein, Y₃ independently represents O, S, Se, N or CR^{Y3};
Wherein, Y₄ independently represents O, S, Se, N or CR^{Y4};
Wherein, X₁ represents N or CR^{X1};
Wherein, X₂ represents N or CR^{X2};
Wherein, X₃ represents N or CR^{X3};
Wherein, X₄ represents N or CR^{X4};
Wherein, X₅ represents N or CR^{X5};
Wherein, X₆ represents N or CR^{X6};
Wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, - OC(O)R^{a}, -OCONR^{a}R^{b}, halogen, -OSO₃R^{a}, -NR^{a}R^{b}, -SF₅, -C(O)OR^{a}, and -CO₂NR^{a}R^{b};
Wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, -C(O)OR^{a}, -CO₂NR^{a}R^{b}, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, -NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
Wherein, R^{X4} represents -L-R^{X4-1};
Wherein, L represents absent or CR^{a}R^{b}, SiR^{a}R^{b}, O, S, Se, or NR^{a};
Wherein, R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O)R^{a}R^{b}, -CN, -S(O)₂R^{a}, -S(O)R^{a}, -SF₅, -NR^{a}R^{b}, -C(O)OR^{a}, -OC(O)R^{a}, -OCONR^{a}R^{b}, -NR^{a}COR^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, - C(O)R^{a}, -C(O)OR^{a}, -OC(O)R^{a}, -OC(O)NR^{a}R^{b}, -NR^{a}COR^{b} and -CONR^{a}R^{b};
wherein R^{Y1}, R^{Y2}, R^{Y3} and R^{Y4} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, -OC(O)R^{a}, -OCONR^{a}R^{b}, halogen, -OSO₃R^{a}, -NR^{a}R^{b}, -SF₅, -C(O)OR^{a}, and -CO₂NR^{a}R^{b};
Wherein, R¹ represents hydrogen or C₁-C₆ alkyl, C₃-C₁₀ cycloalkyl, 4- to 10-membered heterocycloalkyl, C₆-C₁₀ aryl, and 5- to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
Preferably, R¹ represents -CHR^{s}R^{t} or -CDR^{s}R^{t};
Wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, - OC(O)R^{a}, -OC(O)NR^{a}R^{b}, -NR^{a}COR^{b} and -CONR^{a}R^{b};
Wherein, R² and R³ each independently represent hydrogen, deuterium, C₁-C₆ alkyl, halogen, hydroxyl, carboxyl, C₁-C₆ haloalkyl, hydroxy (C₁-C₆) alkyl, or mercapto (C₁-C₆) alkyl;
Wherein, M₁ represents CR^{a}R^{b}, -SiR^{a}R^{b}, NR^{a}, O, S or Se;
Wherein, M₂ represents C or Si;
Wherein, R^{L} and R^{L'} each independently represent C₁-C₆ alkyl; or R^{L} and R^{L'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -C(O)OR^{a}, -OC(O)R^{a}, -OCONR^{a}R^{b}, halogen, -OSO₃R^{a}, -NR^{a}R^{b}, and -SF₅;
Wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -C(O)OR^{a}, -OC(O)R^{a}, -OCONR^{a}R^{b}, halogen, -OSO₃R^{a}, - NR^{a}R^{b}, and -SF₅;
wherein, o represents 0, 1 or 2;
Wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

In a preferred embodiment of Formula (I) or Formula (I-1), Cy represents the following structure:

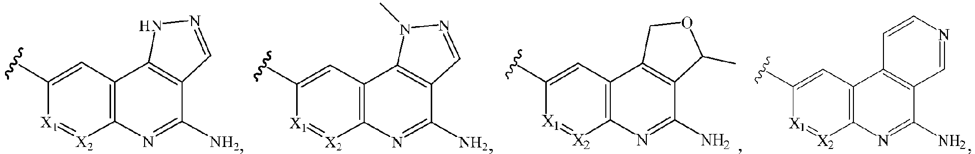

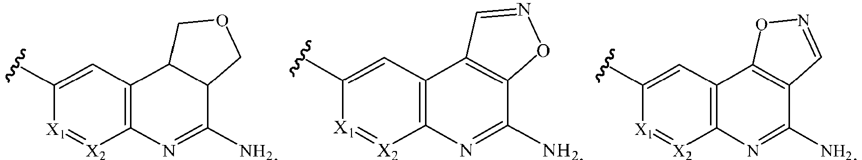

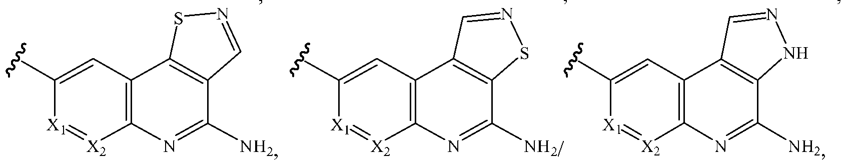

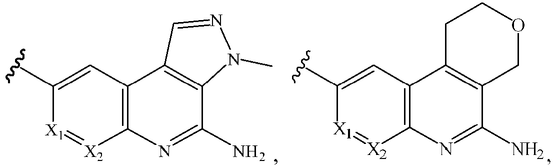

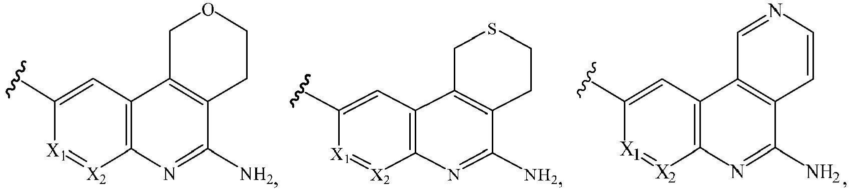

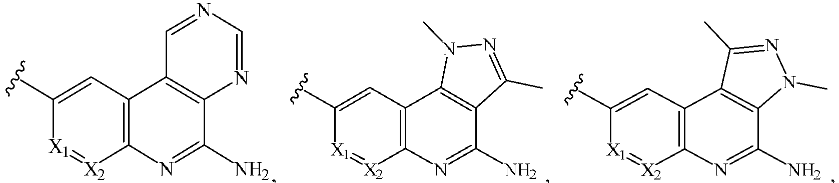

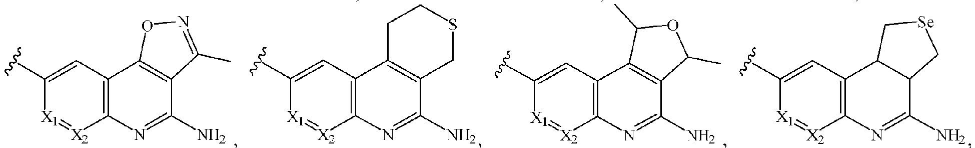

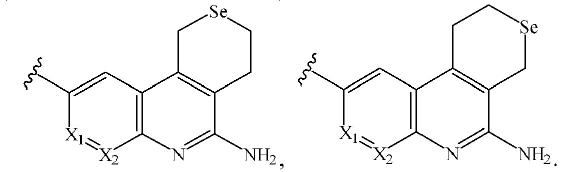

In a preferred embodiment of Formula (I) or Formula (I-1), wherein X₁ represents CR^{X1} or N, wherein R^{X1} represents hydrogen, deuterium, halogen, -CN, C₁-C₆ alkyl, C₁-C₆ deuterated alkyl, C₁-C₆ alkoxy, C₁-C₆ haloalkyl.

In a preferred embodiment of Formula (I) or Formula (I-1), X₁ represents CH, CF, or N.

In a preferred embodiment of Formula (I) or Formula (I-1), X₂ represents CH or CD.

In a preferred embodiment of Formula (I) or Formula (I-1), X₂ represents CH.

In a preferred embodiment of Formula (I) or Formula (I-1), X₃ represents CH, CD, or N.

In a preferred embodiment of Formula (I) or Formula (I-1), X₃ represents CH.

In a preferred embodiment of formula (I) or formula (I-1), X⁴ represents C-L-R^{X4-1}, wherein L represents absence, and R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0 to 4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b}.

In a preferred embodiment of formula (I) or formula (I-1), X⁴ represents C-L-R^{X4-1}, wherein L represents absence, and R^{X4-1} represents hydrogen, deuterium, C₁-C₆ alkyl, halogen, C₁-C₆ haloalkyl, -CN, -NH₂, -NHCH₃, -N(CH₃)₂, -OH, -OCH₃, C₁-C₆ alkoxy, C₁-C₆ haloalkoxy, -SF₅, or -P(O)(CH₃)₂.

In a preferred embodiment of formula (I) or formula (I-1), X⁴ represents C-L-R^{X4-1}, wherein L represents absence, and R^{X4-1} represents hydrogen, halogen (preferably F), -CF₃, -NH₂, -NHCH₃, -N(CH₃)₂, -OCH₃, -OCF₃, -SF₅, or -P(O)(CH₃)₂.

In a preferred embodiment of formula (I) or formula (I-1), X⁴ represents C-L-R^{X4-1}, wherein L represents absence, and R^{X4-1} represents C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b}.

In a preferred embodiment of formula (I) or formula (I-1), X⁴ represents C-L-R^{X4-1}, wherein L represents absence, and R^{X4-1} represents the following groups:

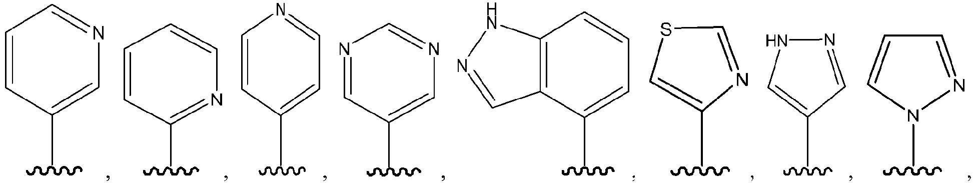

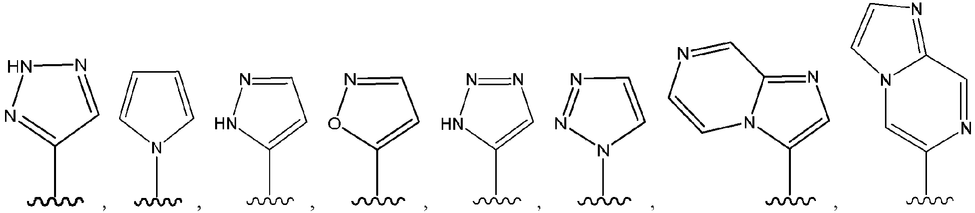

Furthermore, R^{X4} may optionally be substituted with 0, 1 or 2 substituents selected from the group consisting of: halogen, hydroxyl, C₁-C₆ alkyl, C₁-C₆ haloalkyl, and C₁-C₆ haloalkoxy.

In a preferred embodiment of formula (I) or formula (I-1), X⁴ represents C-L-R^{X4-1}, wherein L represents absence, and R^{X4-1} represents C₃-C₁₀ cycloalkyl, C₆-C₁₀ cycloalkenyl or 4-10-membered saturated or unsaturated heterocycle substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, - C(O)R^{a}, -C(O)OR^{a}, -OC(O)R^{a}, -OC(O)NR^{a}R^{b}, -NR^{a}COR^{b} and -CONR^{a}R^{b}.

In a preferred embodiment of formula (I) or formula (I-1), X⁴ represents C-L-R^{X4-1}, wherein L represents O, S, NH, or N(CH₃), and RX⁴⁻¹ represents the following groups:

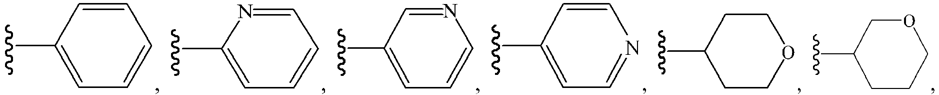

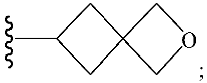

Furthermore, R^{X4-1} may optionally be substituted with 0, 1 or 2 substituents selected from the group consisting of: halogen, hydroxyl, -CN, C₁-C₆ alkyl, C₁-C₆ haloalkyl, and C₁-C₆ haloalkoxy.

In a preferred embodiment of formula (I) or formula (I-1), X⁴ represents C-L-R^{X4-1}, wherein L represents absence, and R^{X4-1} represents the following groups: 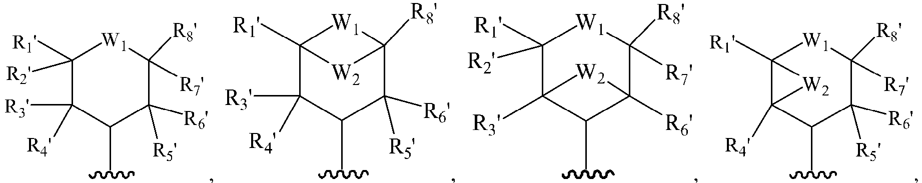 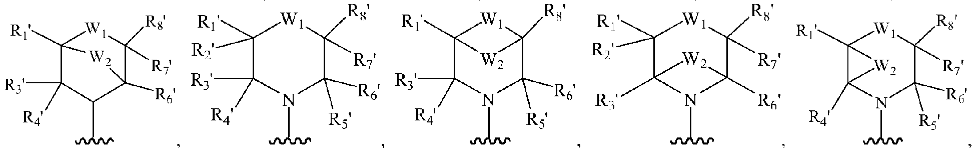 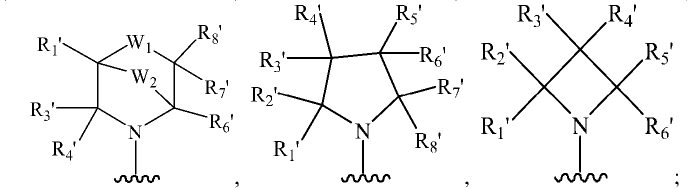
Wherein, W₁ represents CR^{C}R^{D}, NR^{C}, O, S, or SiR^{C}R^{D};
Wherein, W₂ represents -(CR^{M}R^{N})ᵢ-;
Wherein, R₁', R₂', R₃', R₄', R₅', R₆', R₇' and R₈' each independently represent hydrogen, deuterium, halogen, C₁-C₆ alkyl or hydroxyl; or pairs R₁' and R₂', pairs R₃' and R₄', pairs R₅' and R₆' or pairs R₇' and R₈', together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl, and hydroxyl;
Wherein, R^{C} and R^{D} each independently represent hydrogen, deuterium, halogen, C₁-C₆ alkyl or hydroxyl; or R^{S} and R^{T}, together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl, and hydroxyl;
Wherein, R^{M} and R^{N} each independently represent hydrogen or C₁-C₆ alkyl;
Wherein, i represents an integer of 1 or 2.

Furthermore, R^{X4-1} may optionally be substituted with 0, 1 or 2 substituents selected from the group consisting of: halogen, hydroxyl, -CN, C₁-C₆ alkyl, C₁-C₆ haloalkyl, and C₁-C₆ haloalkoxy.

In a preferred embodiment of formula (I) or formula (I-1), X⁴ represents C-L-R^{X4-1}, wherein L represents absence, and R^{X4-1} represents the following groups:

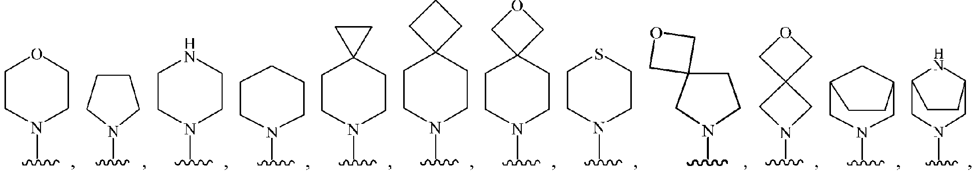

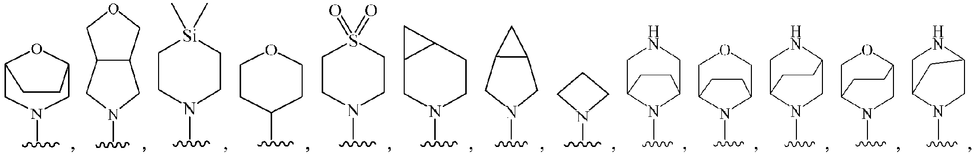

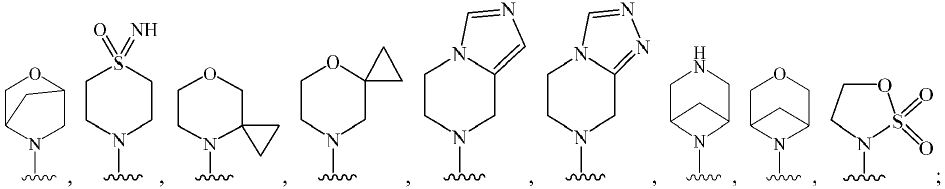

Wherein, the R^{X4-1} may optionally be substituted with 0, 1, or 2 substituents selected from the group consisting of: halogen, hydroxyl, cyano, C₁-C₆ alkyl, and C₁-C₆ haloalkyl.

In a preferred embodiment of formula (I) or formula (I-1), X⁴ represents C-L-R^{X4-1}, wherein L represents absence, and R^{X4-1} represents the following groups:

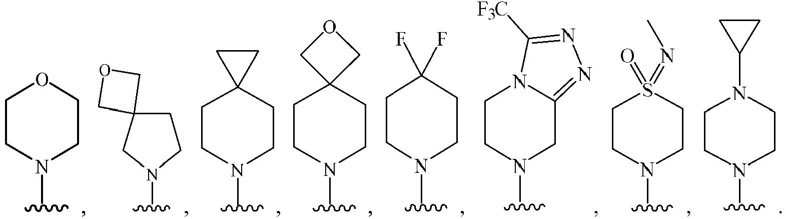

In a preferred embodiment of formula (I) or formula (I-1), X₄ represents CR^{X4}, wherein R^{X4} represents C₁-C₆ alkoxy, C₁-C₆ alkylthio, C₁-C₆ haloalkoxy, C₁-C₆ haloalkylthio, C₁-C₆ haloalkyl, or -SF₅.

In a preferred embodiment of formula (I) or formula (I-1), X₄ represents CR^{X4}, wherein R^{X4} represents C₁-C₆ haloalkoxy, C₁-C₆ haloalkylthio, C₁-C₆ haloalkyl, or -SF₅.

In a preferred embodiment of Formula (I) or Formula (I-1), wherein X₅ represents CR^{X5} or N, wherein R^{X5} represents hydrogen, deuterium, C₁-C₆ alkyl, C₁-C₆alkoxy, C₁-C₆ haloalkyl, C₁-C₆ haloalkoxy, halogen, SF₅, or cyano.

In a preferred embodiment of formula (I) or formula (I-1), X₅ represents CH.

In a preferred embodiment of formula (I) or formula (I-1), X₆ represents CH, CD, or N.

In a preferred embodiment of formula (I) or formula (I-1), X₆ represents CH.

In a preferred embodiment of Formula (I) or Formula (I-1), wherein R¹ represents -CHR²R³ or -CDR²R³, wherein R² and R³ each independently represent hydrogen, deuterium, C₁-C₆ alkyl, or C₃-C₁₀ cycloalkyl, 4- to 10-membered heterocycloalkyl, C₆-C₁₀ aryl, 5- to 10-membered heteroaryl, wherein the said C₃-C₁₀ cycloalkyl, 4- to 10-membered heterocycloalkyl, C₆-C₁₀ aryl, 5- to 10-membered heteroaryl can be furtherly substituted with 0-3 substituents optionally selected from the group consisting of: halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, - C(O)OR^{a}, -OC(O)R^{a}, -OC(O)NR^{a}R^{b}, -NR^{a}COR^{b} or -CONR^{a}R^{b}.

In a preferred embodiment of Formula (I) or Formula (I-1), wherein R¹ represents -CHR²R³ or -CDR²R³, wherein R² represents hydrogen, deuterium, C₁-C₆ alkyl; and R³ represents hydrogen, deuterium, C₁-C₆ alkyl, or C₃-C₁₀ cycloalkyl, 4- to 10-membered heterocycloalkyl, C₆-C₁₀ aryl, 5- to 10-membered heteroaryl, wherein the said C₃-C₁₀ cycloalkyl, 4- to 10-membered heterocycloalkyl, C₆-C₁₀ aryl, 5- to 10-membered heteroaryl can be furtherly substituted with 0-3 substituents optionally selected from the group consisting of: halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b}.

In a preferred embodiment of Formula (I) or Formula (I-1), wherein R¹ represents C₃-C₁₀ cycloalkyl substituted with 0-3 substituents selected from the group consisting of: deuterated, halogen, C₁-C₆ alkyl, hydroxy (C₁-C₆) alkyl, -OR^{a,} -CN, NR^{a}R^{b}, C₁-C₆ haloalkyl, C₁-C₆ haloalkoxy.

In a preferred embodiment of formula (I) or formula (I-1), R¹ represents C₁-C₆ alkyl (preferably methyl or ethyl) or C₁-C₆ deuterated alkyl (preferably deuterated methyl or deuterated ethyl) or C₃-C₆ cycloalkyl (preferably cyclopropyl).

In a preferred embodiment of formula (I) or formula (I-1), M₁ represents O or S.

In a preferred embodiment of formula (I) or formula (I-1), o represents 1 or 2.

In a preferred embodiment of Formula (I) or Formula (I-1), o is 1.

In a preferred embodiment of formula (I) or formula (I-1), R^{L} and R^{L'} each independently represent C₁-C₆ alkyl.

In a preferred embodiment of formula (I) or formula (I-1), R^{L} and R^{L'}, together with the atom attached thereto, form a 3-6-membered ring.

In a preferred embodiment of formula (I) or formula (I-1), R^{L} and R^{L'}, together with the atom attached thereto, form a 3- or 4-membered ring.

In a preferred embodiment of formula (I) or formula (I-1), R^{L} and R^{L'}, together with the atom attached thereto, form a ring of the following structure:

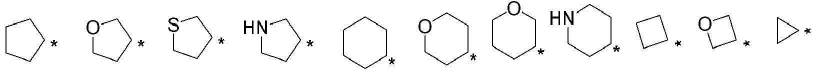

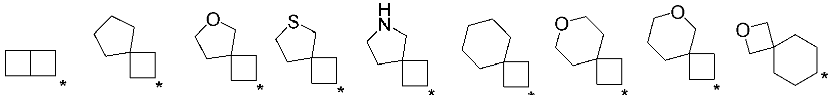

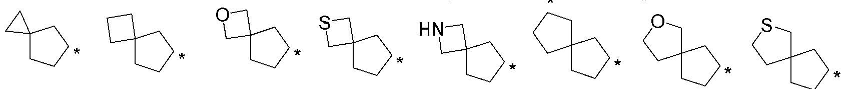

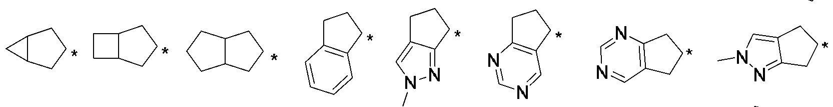

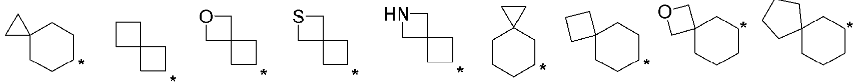

Wherein, * represents an atomic site where R^{L} and R^{L'} are attached; further, the above cyclic structures may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)_{z}R^{a}, -S(O)R^{a}, -CN, -C(O)OR^{a}, -OC(O)R^{a}, -OCONR^{a}R^{b}, halogen, -OSO₃R^{a}, -NR^{a}R^{b} and -SF₅.

In a preferred embodiment of formula (I) or formula (I-1), R^{T} and R^{T'}, together with the atom attached thereto, form a ring of the following structure:

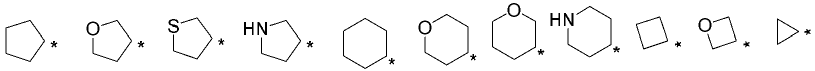

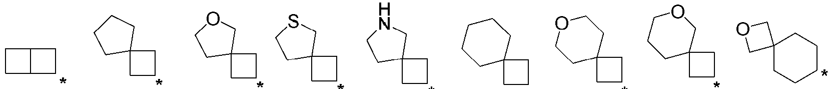

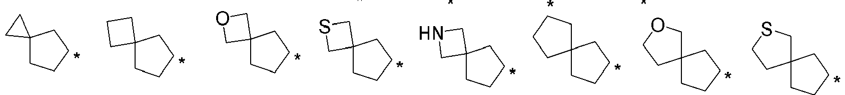

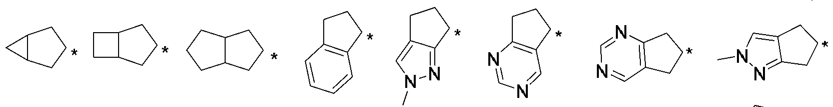

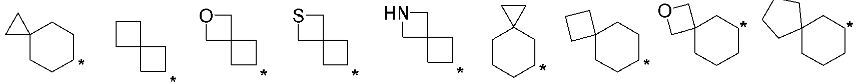

Wherein, * represents an atomic site where R^{T} and R^{T'} are attached; further, the above cyclic structures may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)zR^{a}, -S(O)R^{a}, -CN, -C(O)OR^{a}, -OC(O)R^{a}, -OCONR^{a}R^{b}, halogen, -OSO₃R^{a}, -NR^{a}R^{b} and -SF₅.

In addition, the present disclosure provides a compound of formula (I-2), or a pharmaceutically acceptable salt, ester, prodrug, stereoisomer or isotopic derivative thereof, 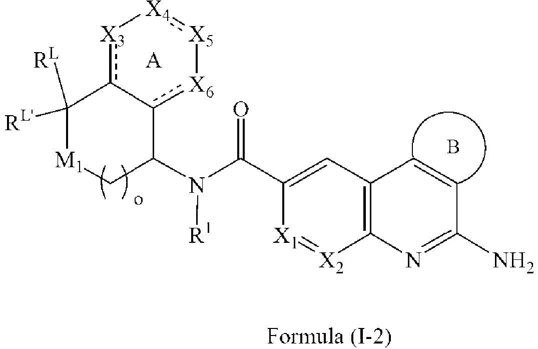
Wherein, X₃ represents N or CR^{X3}; X₄ represents N or CR^{X4}; X₅ represents N or CR^{X5}; and X₆ represents N or CR^{X6};
Wherein, when X₃ represents CR^{X3}, R^{X3} represents hydrogen, deuterium, C₁-C₆ alkyl, C₁-C₆ deuterated alkyl, halogen, -OR^{a}, -SR^{a}, -P(O)R^{a}R^{b}, -CN, -S(O)zR^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Wherein, when X₄ represents CR^{X4}, R^{X4} represents hydrogen, deuterium, C₁-C₆ alkyl, C₁-C₆ deuterated alkyl, halogen, -OR^{a}, -SR^{a}, -P(O)R^{a}R^{b}, -CN, -S(O)zR^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Wherein, when X₅ represents CR^{X5}, R^{X5} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Wherein, when X₆ represents CR^{X6}, R^{X6} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Wherein, said ring A may also be optionally fused with a 5- to 6-membered saturated or unsaturated ring at the chemical bond between X₃ and X₄, which may contain 0-3 heteroatoms selected from O, N and S;
Wherein, the ring A may also optionally be fused with the chemical bond between X₄ and X₅ to form a 5-6-membered saturated or unsaturated ring which may contain 0-3 heteroatoms selected from O, N and S;
Wherein, the ring A may also optionally be fused with the chemical bond between X₅ and X₆ to form a 5-6-membered saturated or unsaturated ring which may contain 0-3 heteroatoms selected from O, N and S;
Wherein, the ring A may also optionally be substituted with 0, 1, 2 or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -CONR^{a}R^{b};
Wherein, R' represents C₁-C₆ alkyl, C₃-C₁₀ cycloalkyl, 4- to 10-membered heterocycloalkyl, C₆-C₁₀ aryl, and 5- to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
Preferably, R¹ represents -CHR²R³ or -CDR²R³;
Wherein, R² and R³ each independently represent hydrogen, deuterium, -OR^{a}, halogen, -CN, -C₁-C₆ alkyl, C₁-C₆ haloalkyl, C₁-C₆ haloalkoxy, hydroxy (C₁-C₆) alkyl, C₃-C₁₀ cycloalkyl, 4- to 10-membered heterocycloalkyl, C₆-C₁₀ aryl, or 5- to 10-membered heteroaryl, wherein the said 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)zR^{a}, -S(O)R^{a}, -SF₅, -C(O)R^{a}, -C(O)OR^{a}, -OC(O)R^{a}, -OC(O)NR^{a}R^{b}, - NR^{a}COR^{b} or -CONR^{a}R^{b};
Wherein, M¹ represents CR^{a}R^{b}, NR^{a}, O, S or Se;
Wherein, R^{L} and R^{L'} each independently represent C₁-C₆ alkyl; or R^{L} and R^{L'}, together with the atom attached thereto, form a 3-6-membered ring;
wherein, o represents 0, 1 or 2;
Wherein, m represents an integer of 0 to 10;
Wherein, ring B represents a 3-10-membered carbocycle, a 6-10-membered unsaturated carbocycle, a 4-10-membered heterocycle, a 6-10-membered unsaturated heterocycle, a C₆-C₁₀ aromatic ring, or a 5-10-membered heteroaromatic ring; the 4-10-membered heterocycle, the 6-10-membered unsaturated heterocycle, and the 5-10-membered heteroaromatic ring may contain 0-3 heteroatoms selected from O, S, and N.
Wherein, the ring B may also optionally be substituted with 0, 1, 2 or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -CONR^{a}R^{b};
Wherein, X₁ represents N or CR^{X1};
Wherein, X₂ represents N or CR^{X2};
Wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, - OC(O)R^{a}, -OCONR^{a}R^{b}, halogen, -OSO₃R^{a}, -NR^{a}R^{b} and -SF₅;
Wherein, -̅ -̅ -̅ represents a single bond or double bond;

In addition, the present disclosure provides a compound of formula (I-3), or a pharmaceutically acceptable salt, ester, prodrug, stereoisomer or isotopic derivative thereof, 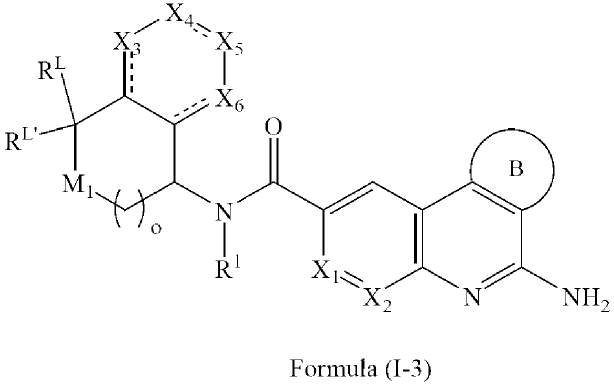
Wherein, X₃ represents N or CR^{X3}; X₄ represents N or CR^{X4}; X₅ represents N or CR^{X5}; and X₆ represents N or CR^{X6};
Wherein, when X₃ represents CR^{X3}, R^{X3} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Wherein, when X₄ represents CR^{X4}, R^{X4} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Wherein, when X₅ represents CR^{X5}, R^{X5} represents hydrogen, deuterium, C₁-C₆ alkyl, C₁-C₆ deuterated alkyl, halogen, -OR^{a}, -SR^{a}, -P(O)R^{a}R^{b}, -CN, -S(O)zR^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Wherein, when X₆ represents CR^{X6}, R^{X6} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
Wherein, said ring A may also be optionally fused with a 5- to 6-membered saturated or unsaturated ring at the chemical bond between X₃ and X₄, which may contain 0-3 heteroatoms selected from O, N and S;
Wherein, the ring A may also optionally be fused with the chemical bond between X₄ and X₅ to form a 5-6-membered saturated or unsaturated ring which may contain 0-3 heteroatoms selected from O, N and S;
Wherein, the ring A may also optionally be fused with the chemical bond between X₅ and X₆ to form a 5-6-membered saturated or unsaturated ring which may contain 0-3 heteroatoms selected from O, N and S;
Wherein, the ring A may also optionally be substituted with 0, 1, 2 or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -CONR^{a}R^{b};
Wherein, R¹ represents C₁-C₆ alkyl, C₃-C₁₀ cycloalkyl, 4- to 10-membered heterocycloalkyl, C₆-C₁₀ aryl, and 5- to 10-membered heteroaryl, wherein the said C₁-C₆ alkyl, C₃-C₁₀ cycloalkyl, 4- to 10-membered heterocycloalkyl, C₆-C₁₀ aryl, and 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
Preferably, R¹ represents -CHR²R³ or -CDR²R³;
Wherein, R² and R³ each independently represent hydrogen, deuterium, -OR^{a}, halogen, -CN, -C₁-C₆ alkyl, C₁-C₆ haloalkyl, C₁-C₆ haloalkoxy, hydroxy (C₁-C₆) alkyl, C₃-C₁₀ cycloalkyl, 4- to 10-membered heterocycloalkyl, C₆-C₁₀ aryl, or 5- to 10-membered heteroaryl, wherein the said 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, - NR^{a}COR^{b} or -CONR^{a}R^{b};
Wherein, M¹ represents CR^{a}R^{b}, NR^{a}, O, S or Se;
Wherein, R^{L} and R^{L'} each independently represent C₁-C₆ alkyl; or R^{L} and R^{L'}, together with the atom attached thereto, form a 3-6-membered ring;
wherein, o represents 0, 1 or 2;
Wherein, m represents an integer of 0 to 10;
Wherein, ring B represents a 3-10-membered carbocycle, a 6-10-membered unsaturated carbocycle, a 4-10-membered heterocycle, a 6-10-membered unsaturated heterocycle, a C₆-C₁₀ aromatic ring, or a 5-10-membered heteroaromatic ring; the 4-10-membered heterocycle, the 6-10-membered unsaturated heterocycle, and the 5-10-membered heteroaromatic ring may contain 0-3 heteroatoms selected from O, S, and N.
Wherein, the ring B may also optionally be substituted with 0, 1, 2 or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -CONR^{a}R^{b};
Wherein, X₁ represents N or CR^{X1},
Wherein, X₂ represents N or CR^{X2};
Wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, - OC(O)R^{a}, -OCONR^{a}R^{b}, halogen, -OSO₃R^{a}, -NR^{a}R^{b} and -SF₅;
Wherein, -̅ -̅ -̅ represents a single bond or double bond.

In a preferred embodiment of formula (I-2) or formula (I-3), ring B represents the following structure: 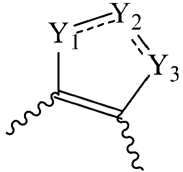
Wherein, Y₁ represents CR^{Y1}R^{Y1'}, NR^{Y1}, O, S;
Wherein, Y₂ represents CR^{Y2}R^{Y2'}, NR^{Y2}, O or S;
Wherein, Y₃ represents CR^{Y3}R^{Y3'}, NR^{Y3}, O or S;
Wherein, R^{Y1}, R^{Y1'}, R^{Y2}, R^{Y2'}, R^{Y3}, and R^{Y3'} each independently represent absent, hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -OSO₃R^{a}, -NR^{a}R^{b}, -SF₅;
Wherein, R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom attached thereto, form a 3- to 14-membered saturated or unsaturated ring which may optionally contain 0-2 heteroatoms selected from O, S, and N.

In a preferred embodiment of formula (I-2) or formula (I-3), ring B represents the following structure:

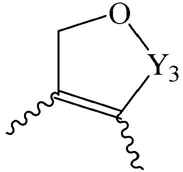

Wherein, Y₃ represents CR^{Y3}R^{Y3'}, and R^{Y3} and R^{Y3'} each independently represent hydrogen, deuterium, or C₁-C₆ alkyl.

In a preferred embodiment of formula (I-2) or formula (I-3), ring B represents the following structure: 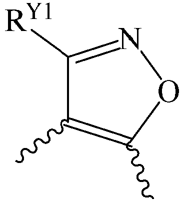
R^{Y1} represents hydrogen, deuterium, or C₁-C₆ alkyl;
In a preferred embodiment of formula (I-2) or formula (I-3), ring B represents the following structure:

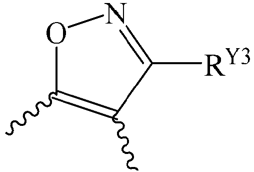

R^{Y3} represents hydrogen, deuterium, or C₁-C₆ alkyl.

In a preferred embodiment of formula (I-2) or formula (I-3), ring B represents the following structure:

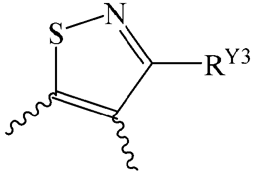

R^{Y3} represents hydrogen, deuterium, or C₁-C₆ alkyl.

In a preferred embodiment of formula (I-2) or formula (I-3), ring B represents the following structure:

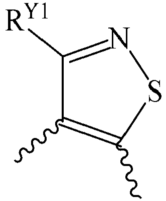

R^{Y1} represents hydrogen, deuterium, or C₁-C₆ alkyl.

In a preferred embodiment of formula (I-2) or formula (I-3), ring B represents the following structure:

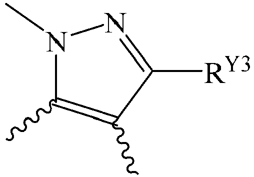

R^{Y3} represents hydrogen, deuterium, or C₁-C₆ alkyl.

In a preferred embodiment of formula (I-2) or formula (I-3), ring B represents the following structure:

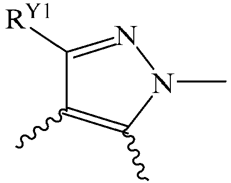

R^{Y1} represents hydrogen, deuterium, or C₁-C₆ alkyl.

In a preferred embodiment of formula (I-2) or formula (I-3), ring B represents the following structure: 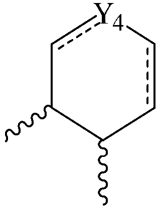
Wherein, Y₄ represents CR^{Y4}R^{Y4'}, NR^{Y4}, O or S;
Wherein, R^{Y4} and R^{Y4'} each independently represent absence, hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, - S(O)₂R^{a}, -S(O)R^{a}, -CN, -OC(O)R^{a}, -OCONR^{a}R^{b}, halogen, -OSO₃R^{a}, -NR^{a}R^{b}, or -SF₅; wherein, R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom attached thereto, form a 3- to 14-membered saturated or unsaturated ring which may optionally contain 0-2 heteroatoms selected from O, S, and N.

In a preferred embodiment of formula (I-2) or formula (I-3), ring B represents the following structure: 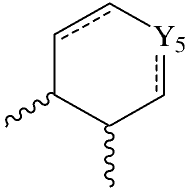
Wherein, Y₅ represents CR^{Y5}R^{Y5'}, NR^{Y5}, O, or S;
Wherein, R^{Y5} and R^{Y5'} each independently represent absence, hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, - S(O)₂R^{a}, -S(O)R^{a}, -CN, -OC(O)R^{a}, -OCONR^{a}R^{b}, halogen, -OSO₃R^{a}, -NR^{a}R^{b}, or -SF₅; wherein, R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom attached thereto, form a 3- to 14-membered saturated or unsaturated ring which may optionally contain 0-2 heteroatoms selected from O, S, and N.

In a preferred embodiment of formula (I-2) or formula (I-3), -̅ -̅ -̅ represents a double bond.

In a preferred embodiment of Formula (I-2) or Formula (I-3), wherein X₁ represents CR^{X1} or N, wherein R^{X1} represents hydrogen, deuterium, halogen, -CN, C₁-C₆ alkyl, C₁-C₆ deuterated alkyl, C₁-C₆ alkoxy, C₁-C₆ haloalkyl.

In a preferred embodiment of formula (I-2) or formula (I-3), X₁ represents CH, CF or N.

In a preferred embodiment of Formula (I-2) or Formula (I-3), X₂ represents CH or CD.

In a preferred embodiment of formula (I-2) or formula (I-3), X₂ represents CH.

In a preferred embodiment of formula (I-2) or formula (I-3), X₃ represents CH, CD or N.

In a preferred embodiment of Formula (I-2) or Formula (I-3), X₃ represents CH.

In a preferred embodiment of formula (I-2) or formula (I-3), X₄ represents CR^{X4}, wherein R^{X4} represents C₁-C₆ alkoxy, C₁-C₆ alkylthio, C₁-C₆ haloalkoxy, C₁-C₆ haloalkylthio, C₁-C₆ haloalkyl, or -SF₅.

In a preferred embodiment of formula (I-2) or formula (I-3), X₄ represents CR^{X4}, wherein R^{X4} represents C₁-C₆ haloalkoxy, C₁-C₆ haloalkylthio, C₁-C₆ haloalkyl, or -SF₅.

In a preferred embodiment of Formula (I-2) or Formula (I-3), wherein X₅ represents CR^{X5} or N, wherein R^{X5} represents hydrogen, deuterium, C₁-C₆ alkyl, C₁-C₆ alkoxy, C₁-C₆ haloalkyl, C₁-C₆ haloalkoxy, halogen, SF₅, or cyano.

In a preferred embodiment of formula (I-2) or formula (I-3), X₅ represents CH.

In a preferred embodiment of formula (I-2) or formula (I-3), X₆ represents CH, CD, or N.

In a preferred embodiment of formula (I-2) or formula (I-3), X₆ represents CH.

In a preferred embodiment of Formula (I-2) or Formula (I-3), wherein R¹ represents -CHR²R³ or -CDR²R³, wherein R² and R³ each independently represent hydrogen, deuterium, C₁-C₆ alkyl, C₃-C₁₀ cycloalkyl, 4- to 10-membered heterocycloalkyl, C₆-C₁₀ aryl, 5- to 10-membered heteroaryl, wherein the said C₃-C₁₀ cycloalkyl, 4- to 10-membered heterocycloalkyl, C₆-C₁₀ aryl, 5- to 10-membered heteroaryl can be furtherly substituted with 0-3 substituents optionally selected from the group consisting of: halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, - C(O)OR^{a}, -OC(O)R^{a}, -OC(O)NR^{a}R^{b}, -NR^{a}COR^{b} or -CONR^{a}R^{b}.

In a preferred embodiment of Formula (I-2) or Formula (I-3), wherein R¹ represents -CHR²R³ or -CDR²R³, wherein R² represents hydrogen, deuterium, C₁-C₆ alkyl; and R³ represent hydrogen, C₁-C₆ alkyl, C₃-C₁₀ cycloalkyl, 4- to 10-membered heterocycloalkyl, C₆-C₁₀ aryl, 5- to 10-membered heteroaryl, wherein the said C₃-C₁₀ cycloalkyl, 4- to 10-membered heterocycloalkyl, C₆-C₁₀ aryl, 5- to 10-membered heteroaryl can be furtherly substituted with 0-3 substituents optionally selected from the group consisting of: halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, - C(O)OR^{a}, -OC(O)R^{a}, -OC(O)NR^{a}R^{b}, -NR^{a}COR^{b} or -CONR^{a}R^{b}.

In a preferred embodiment of Formula (I-2) or Formula (I-3), wherein R¹ represents C₃-C₁₀ cycloalkyl substituted with 0-3 substituents selected from the group consisting of: deuterated, halogen, C₁-C₆ alkyl, hydroxy (C₁-C₆) alkyl, -OR^{a}, -CN, NR^{a}R^{b}, C₁-C₆ haloalkyl, C₁-C₆ haloalkoxy.

In a preferred embodiment of formula (I-2) or formula (1-3), R¹ represents C₁-C₆ alkyl (preferably methyl or ethyl) or C₁-C₆ deuterated alkyl (preferably deuterated methyl or deuterated ethyl) or C₃-C₆ cycloalkyl (preferably cyclopropyl).

In a preferred embodiment of formula (I-2) or formula (I-3), M₁ represents O or S.

In a preferred embodiment of formula (I-2) or formula (I-3), o represents 1 or 2.

In a preferred embodiment of formula (I-2) or formula (I-3), o represents 1.

In a preferred embodiment of formula (I-2) or formula (I-3), R^{L} and R^{L'} each independently represent C₁-C₆ alkyl.

In a preferred embodiment of formula (I-2) or formula (I-3), R^{L} and R^{L'}, together with the atom attached thereto, form a 3-6-membered ring.

In a preferred embodiment of formula (I-2) or formula (I-3), R^{L} and R^{L'}, together with the atom attached thereto, form a 3- or 4-membered ring.

Specifically, the present disclosure provides the following compounds:

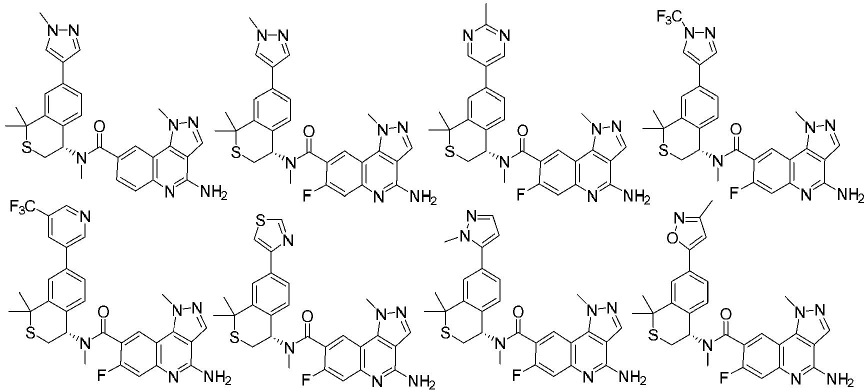

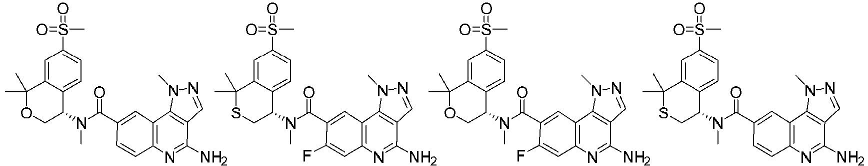

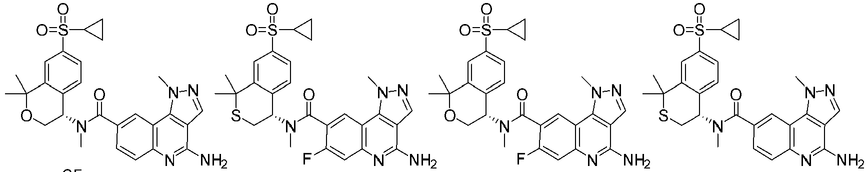

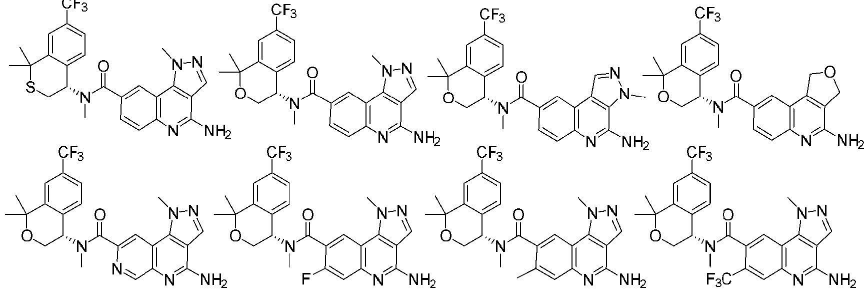

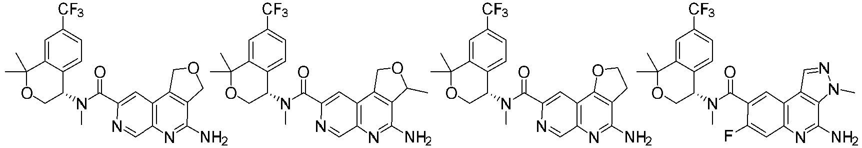

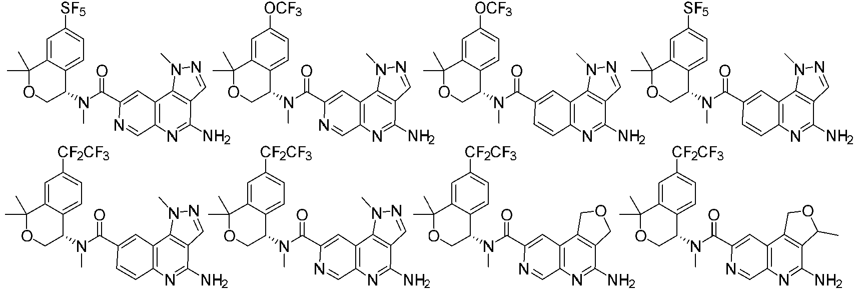

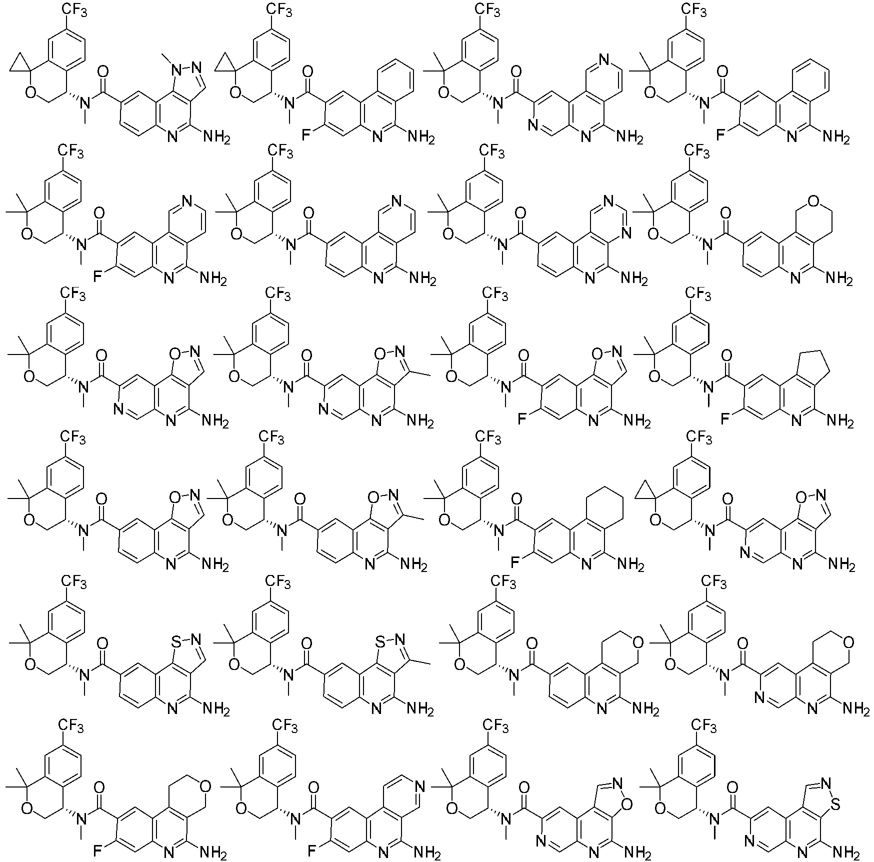

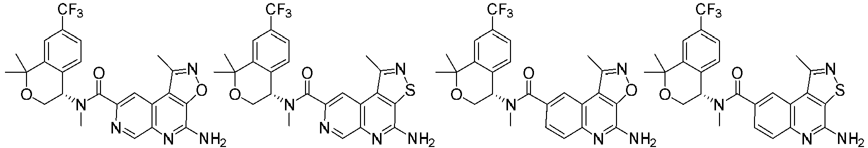

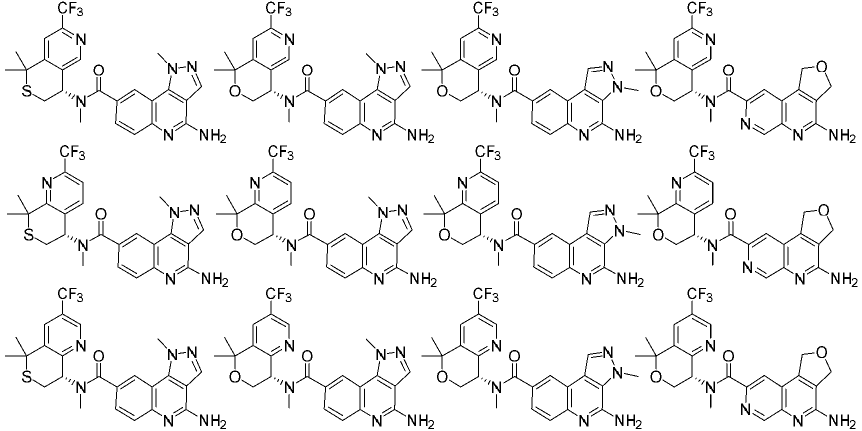

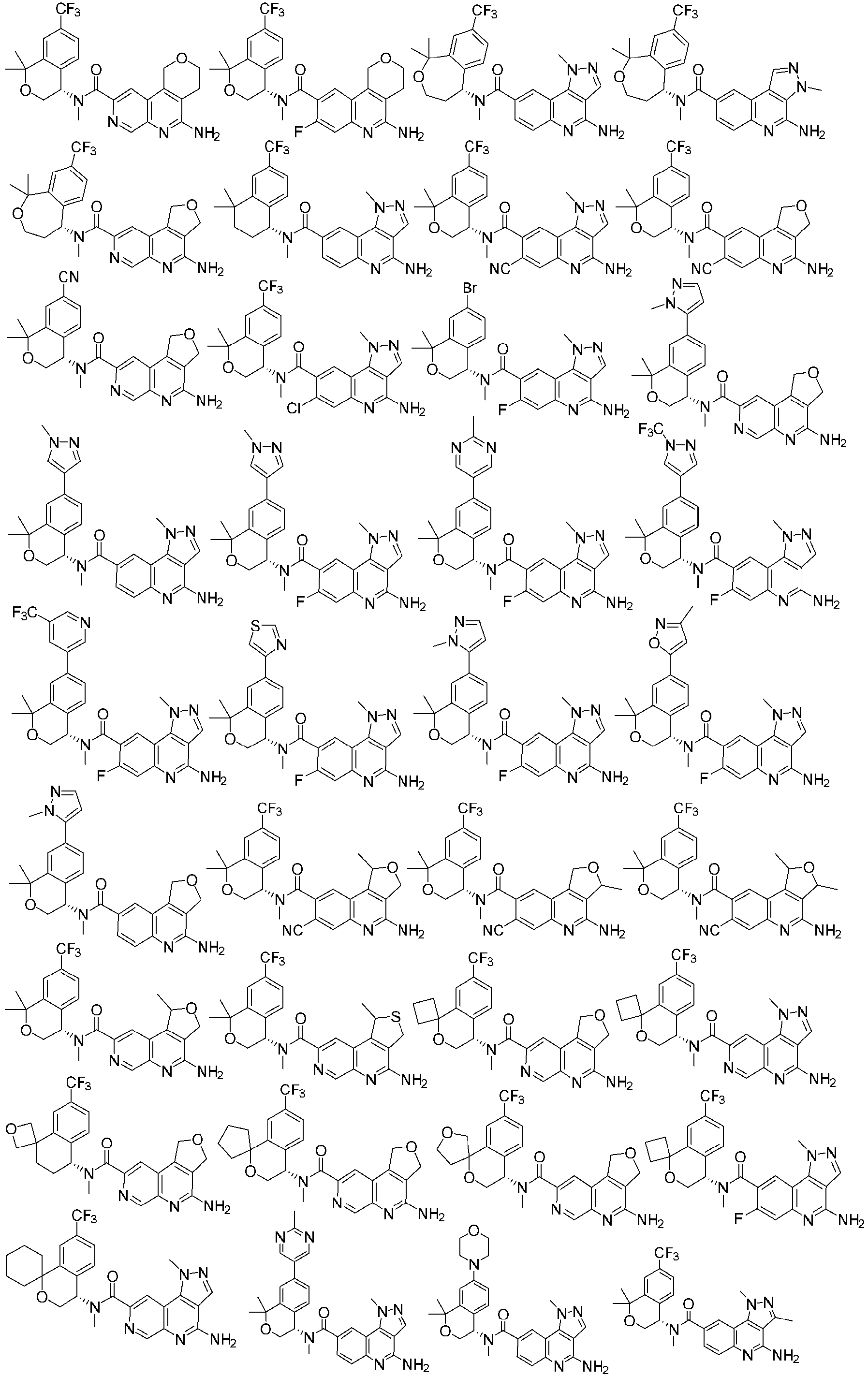

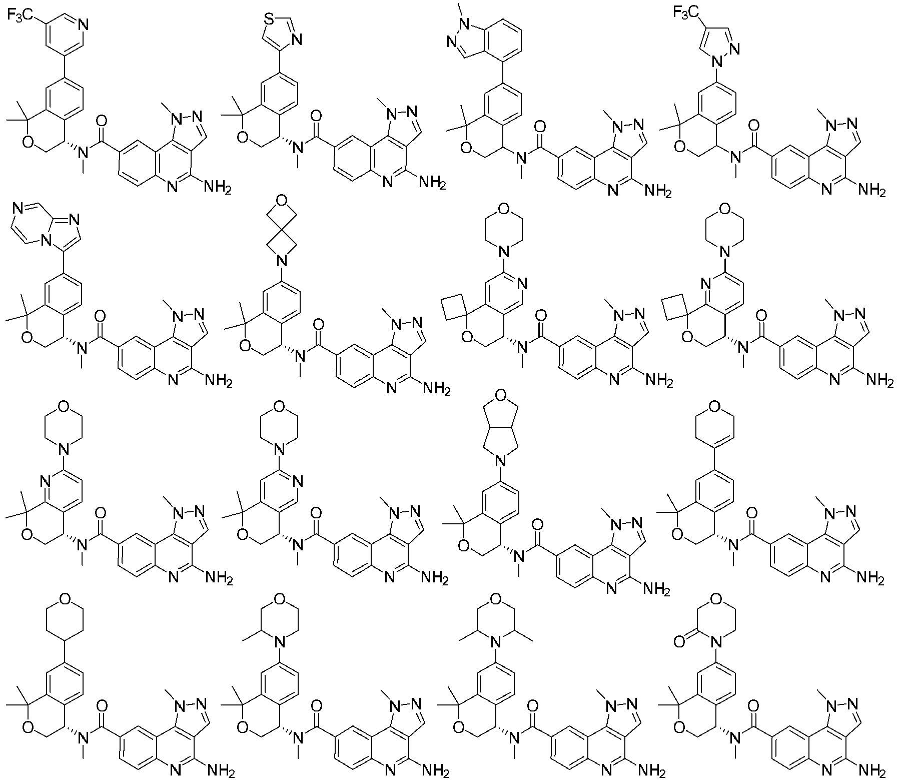

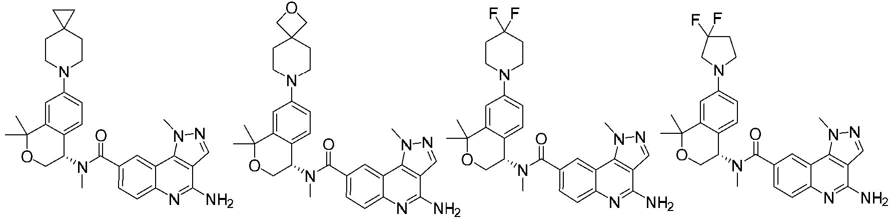

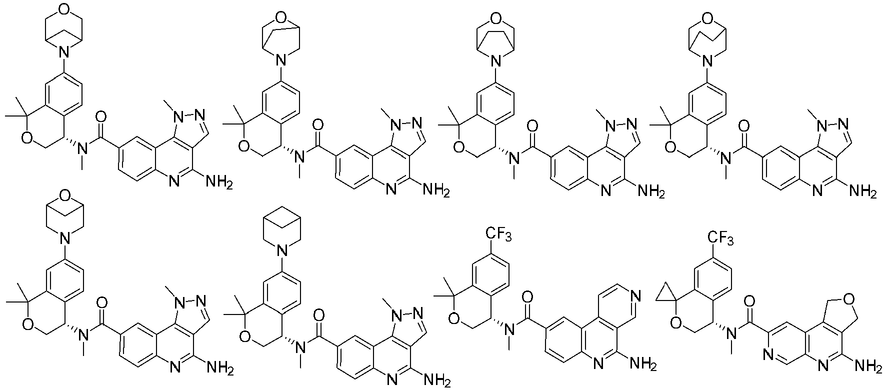

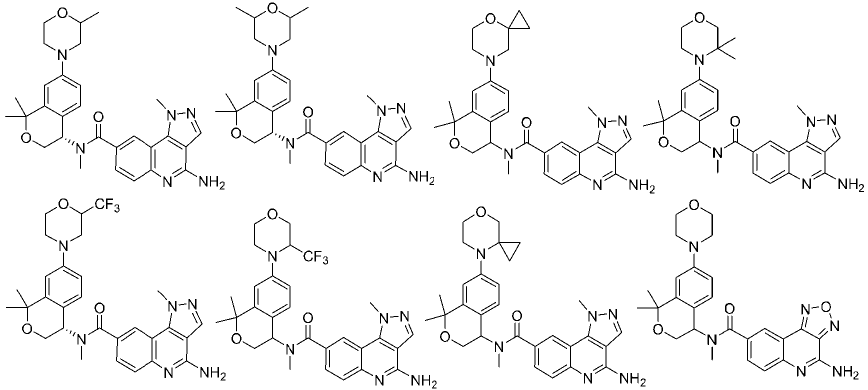

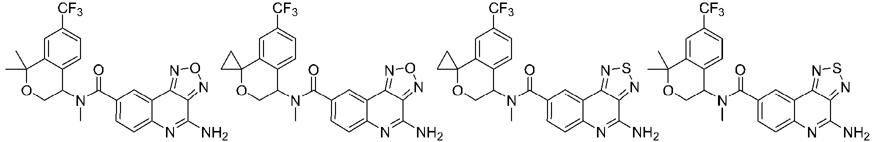

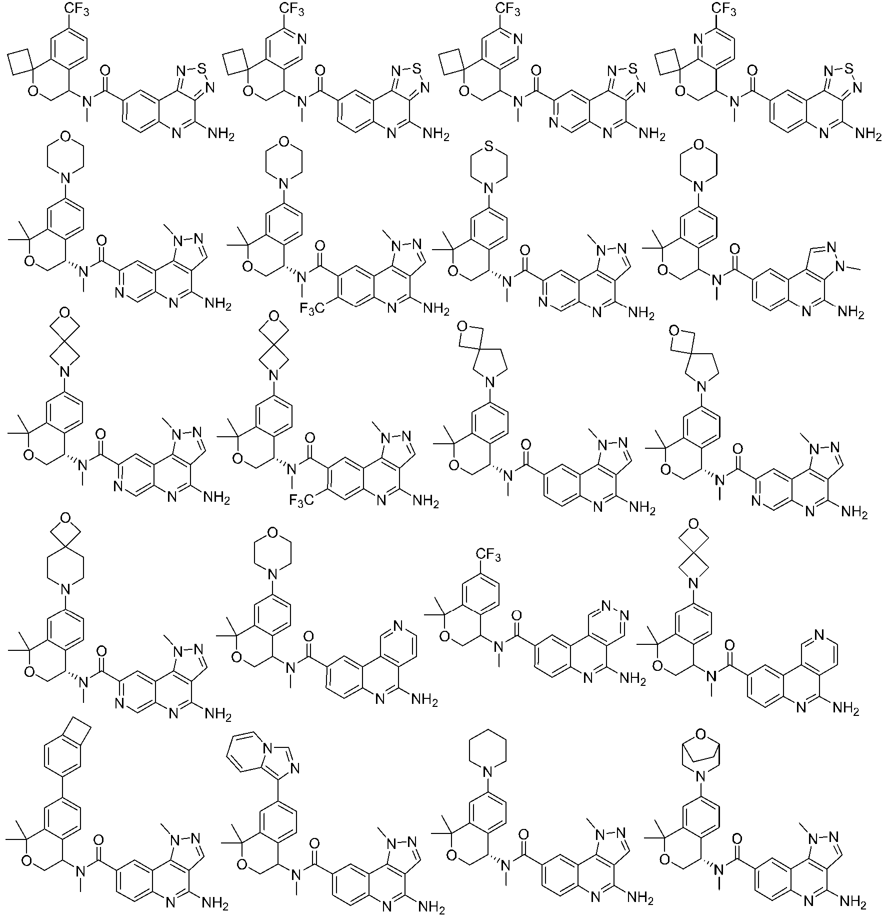

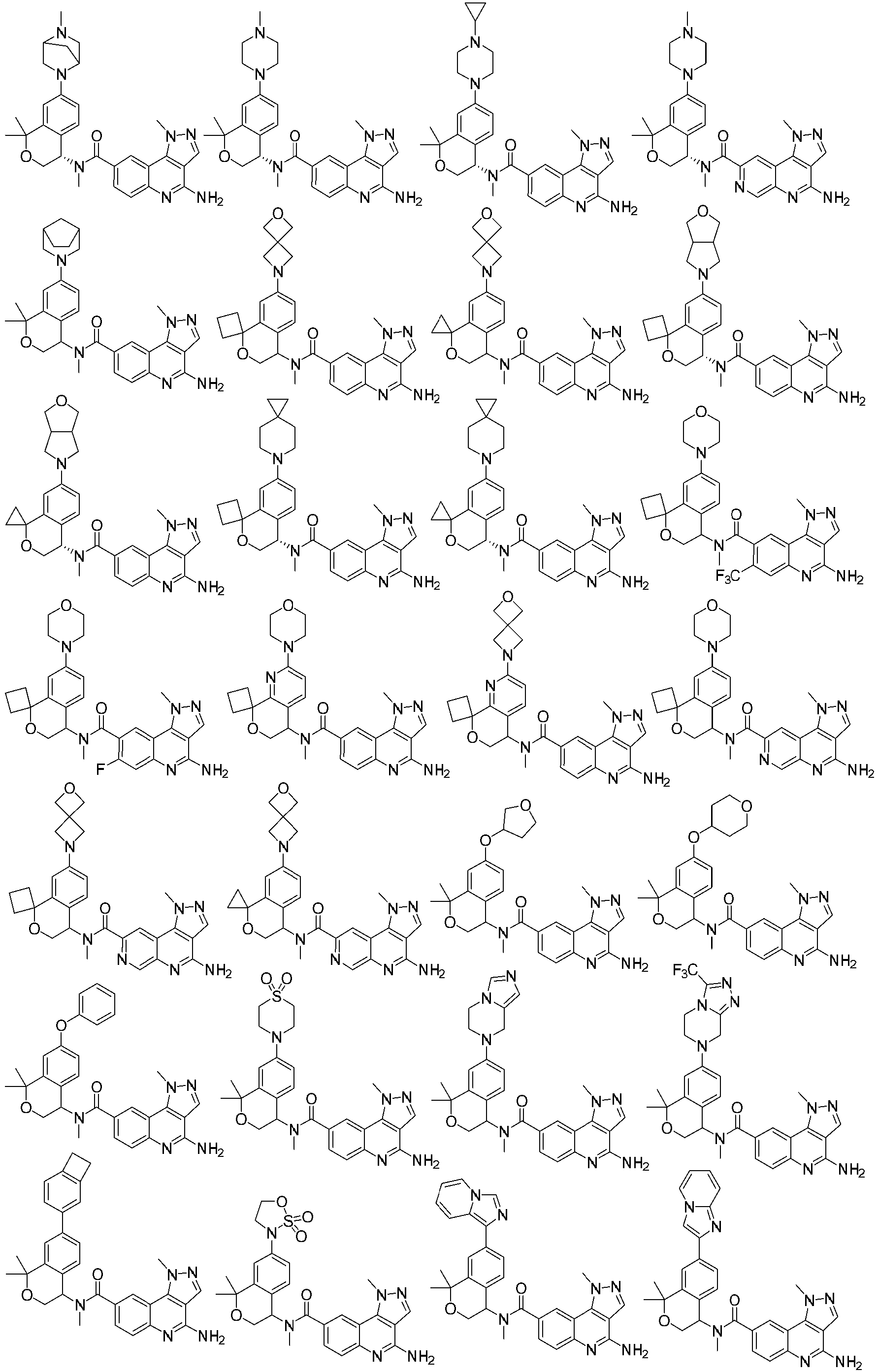

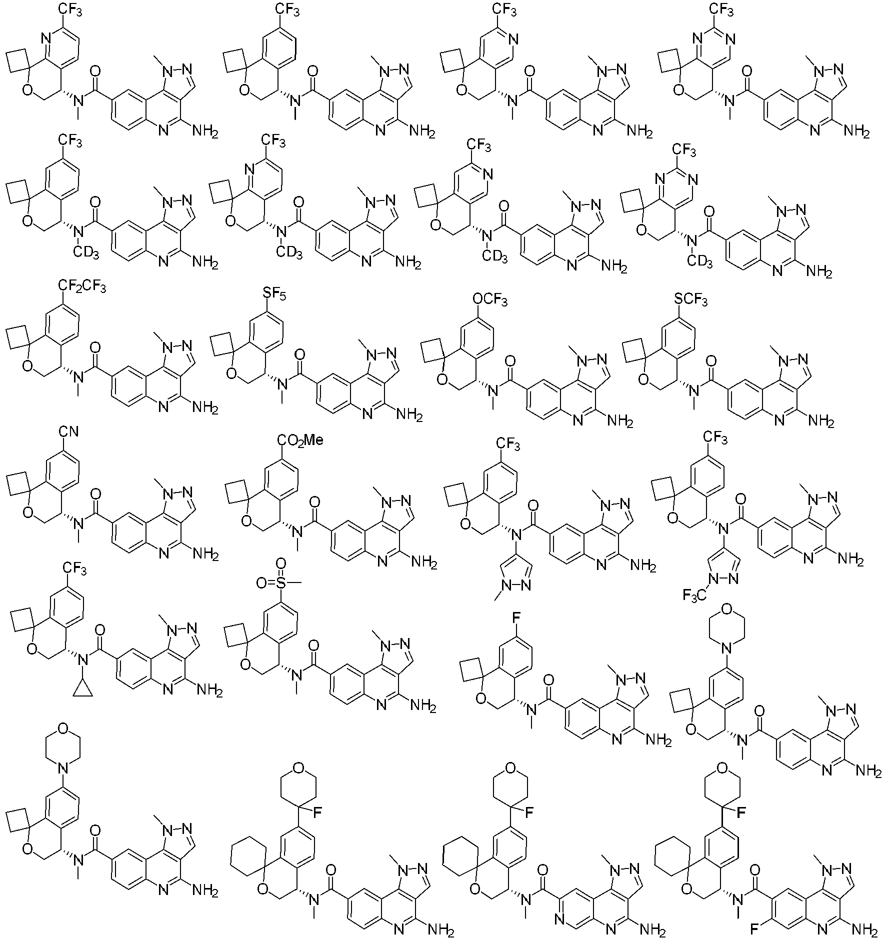

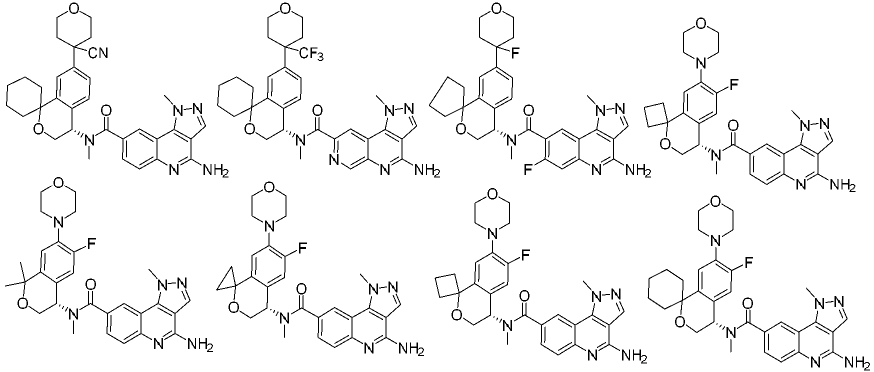

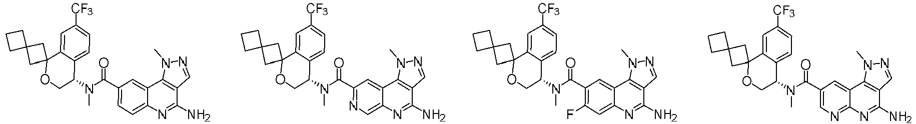

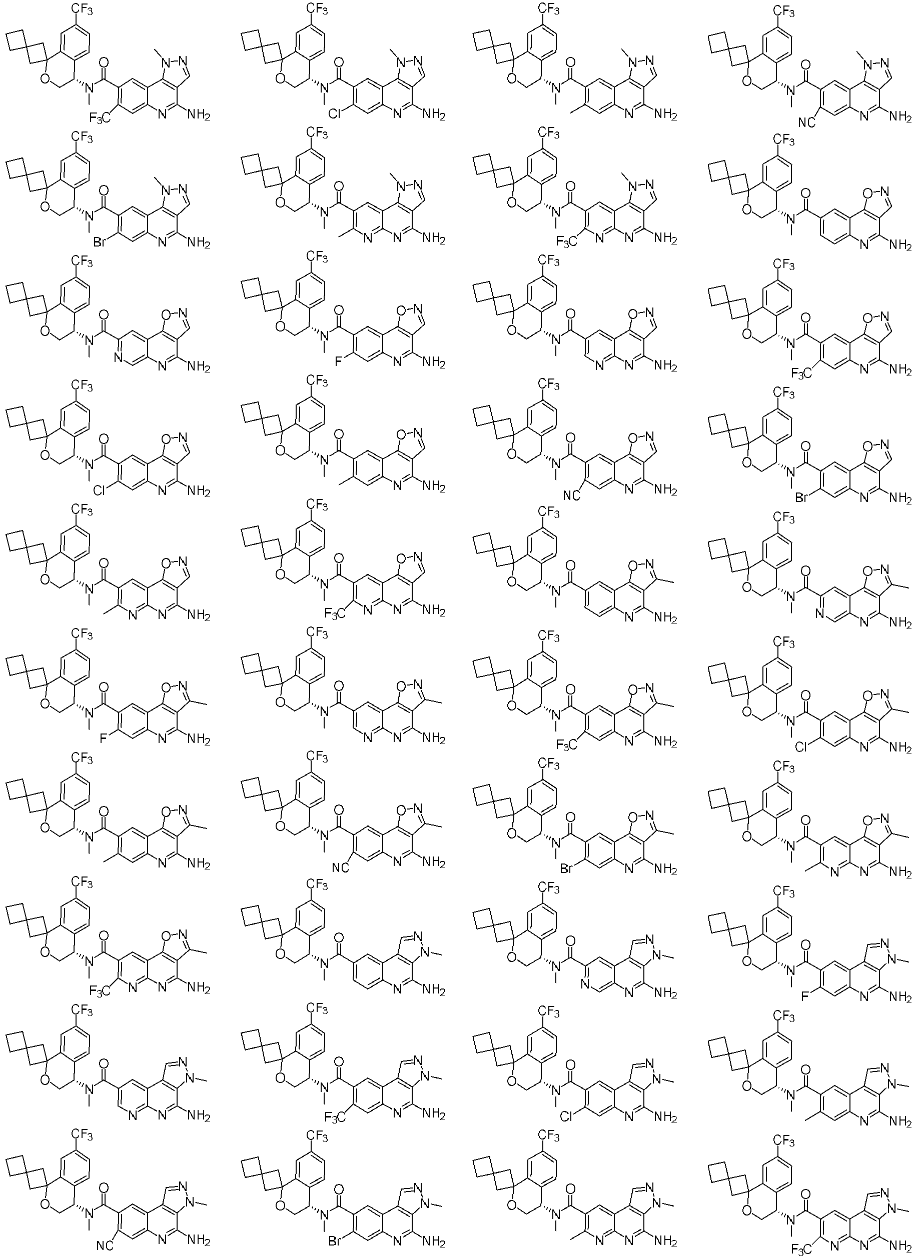

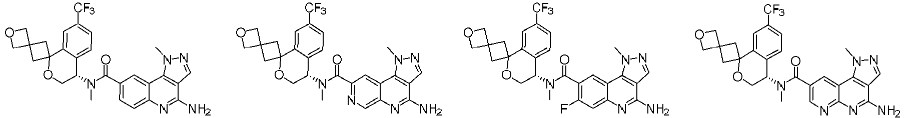

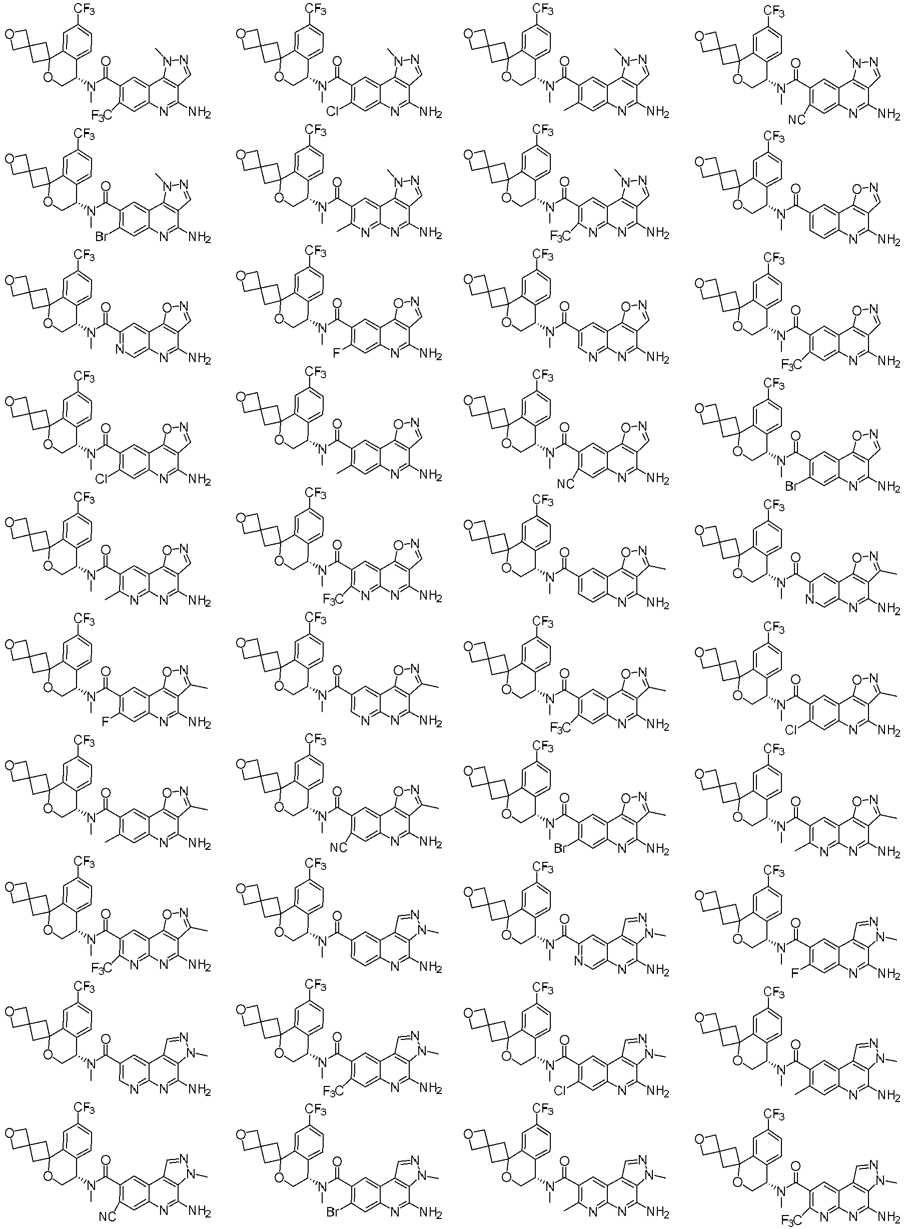

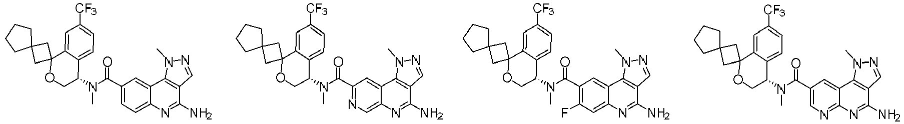

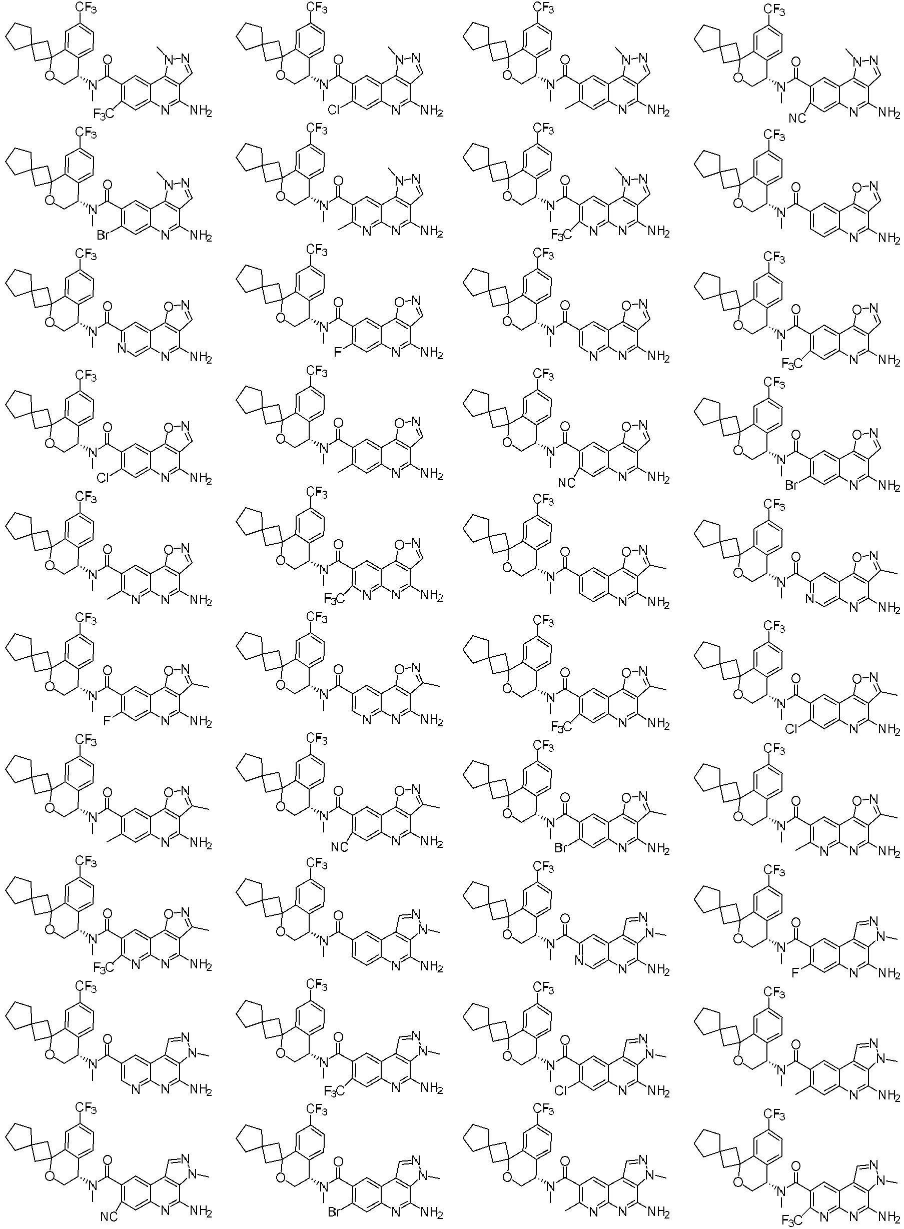

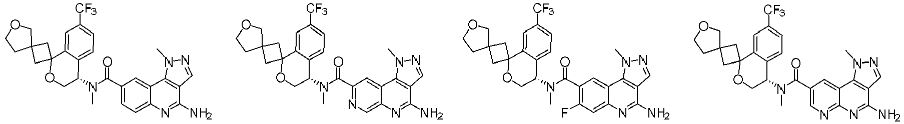

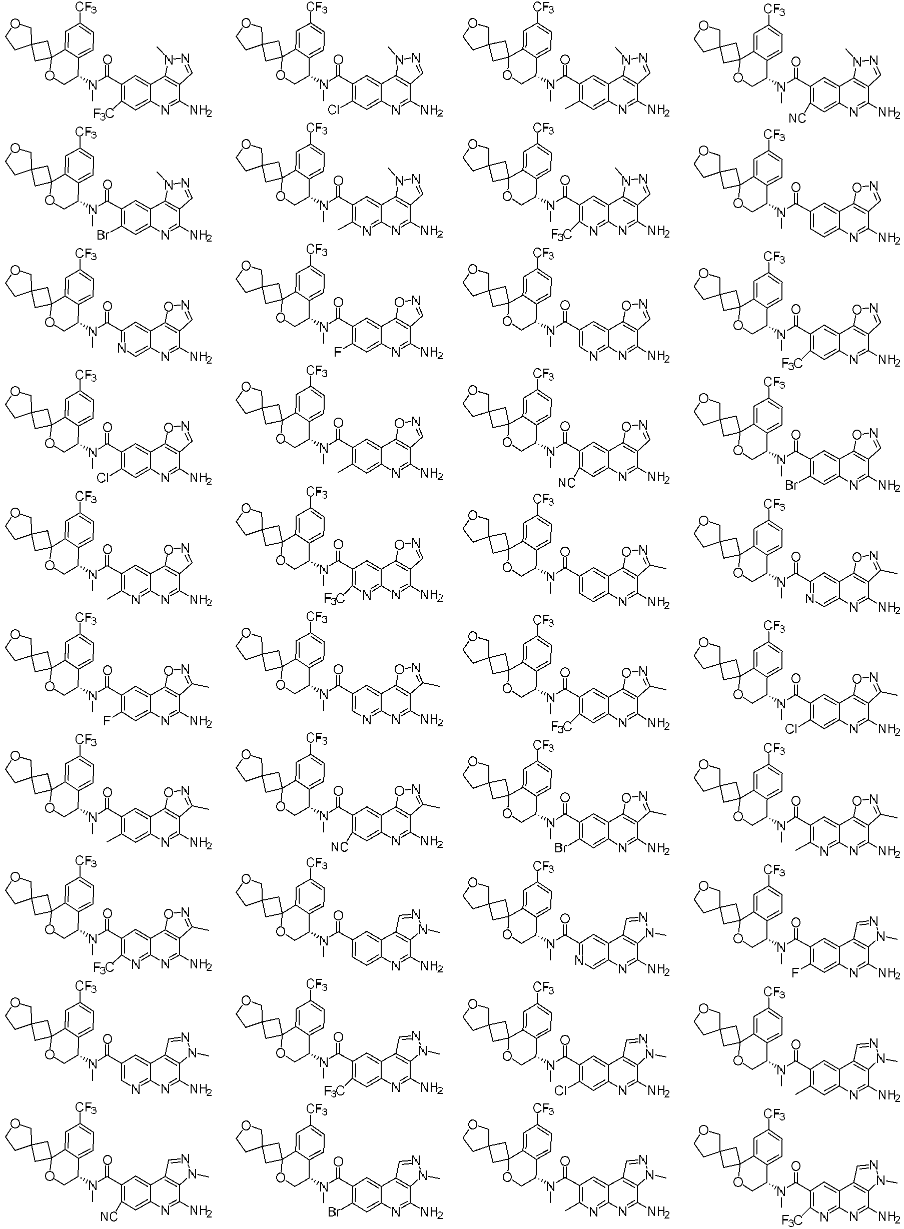

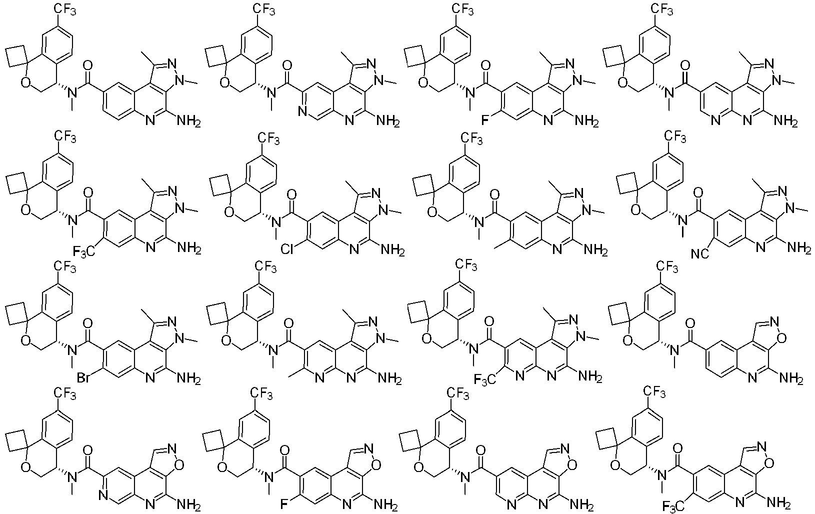

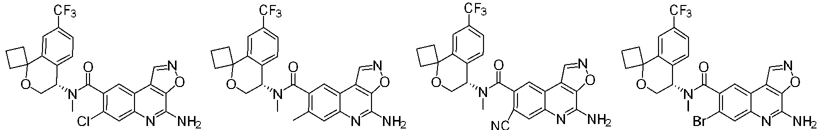

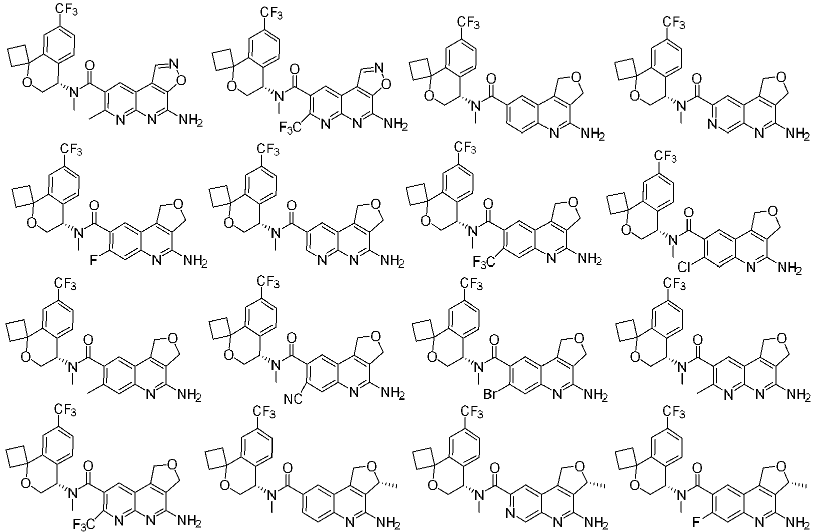

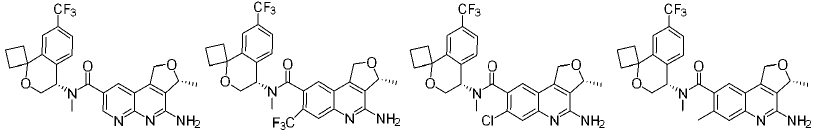

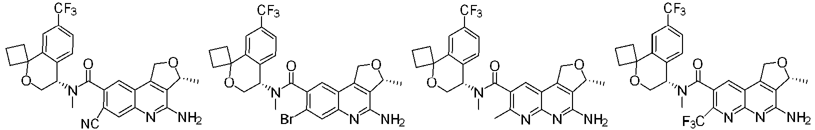

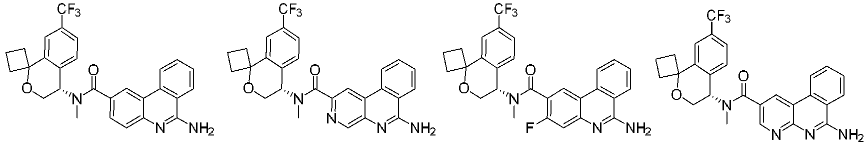

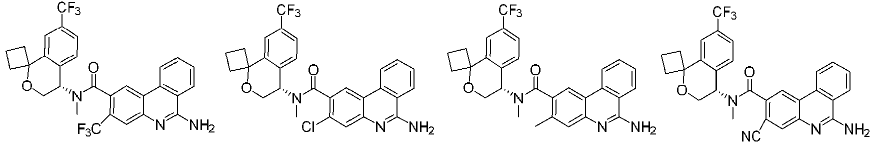

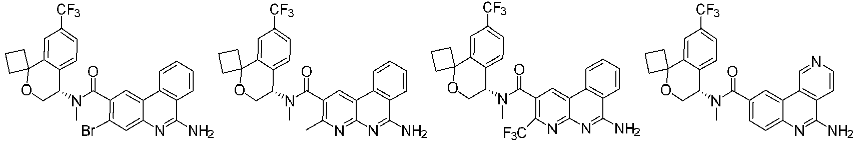

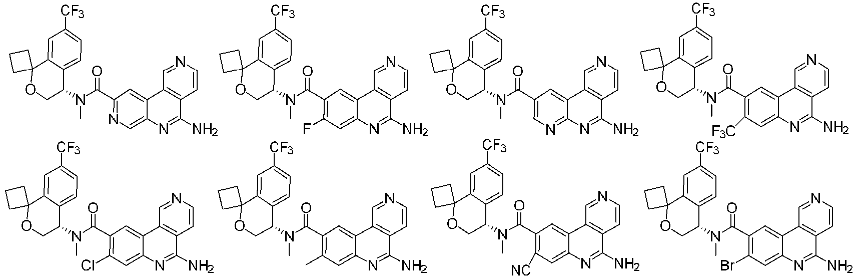

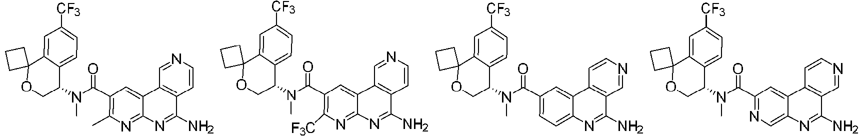

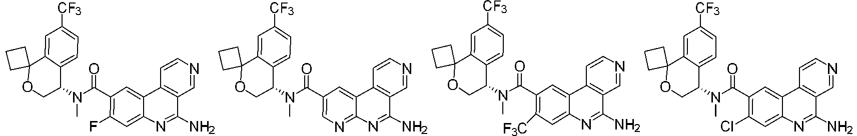

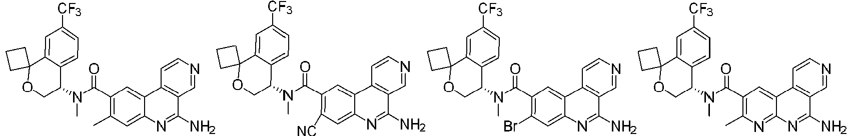

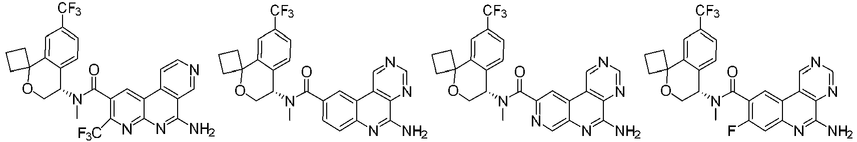

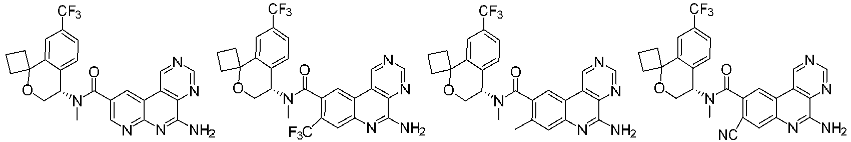

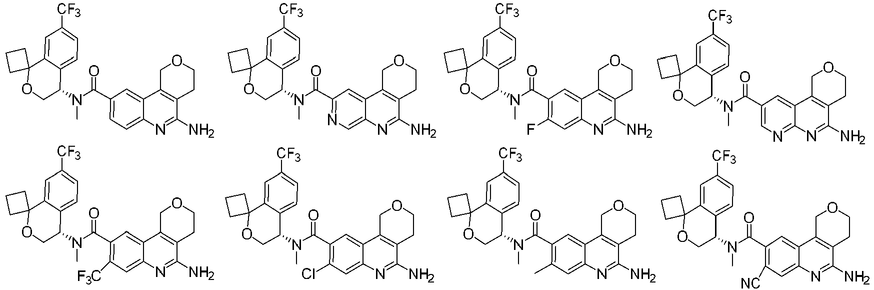

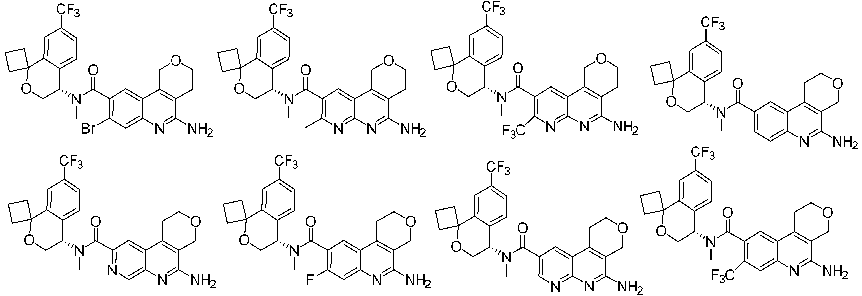

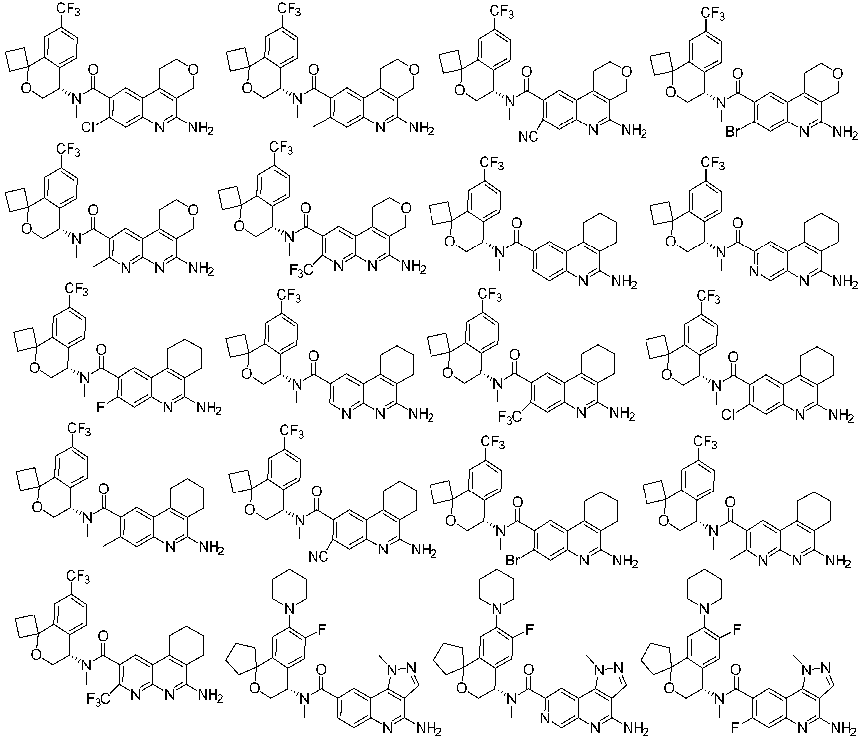

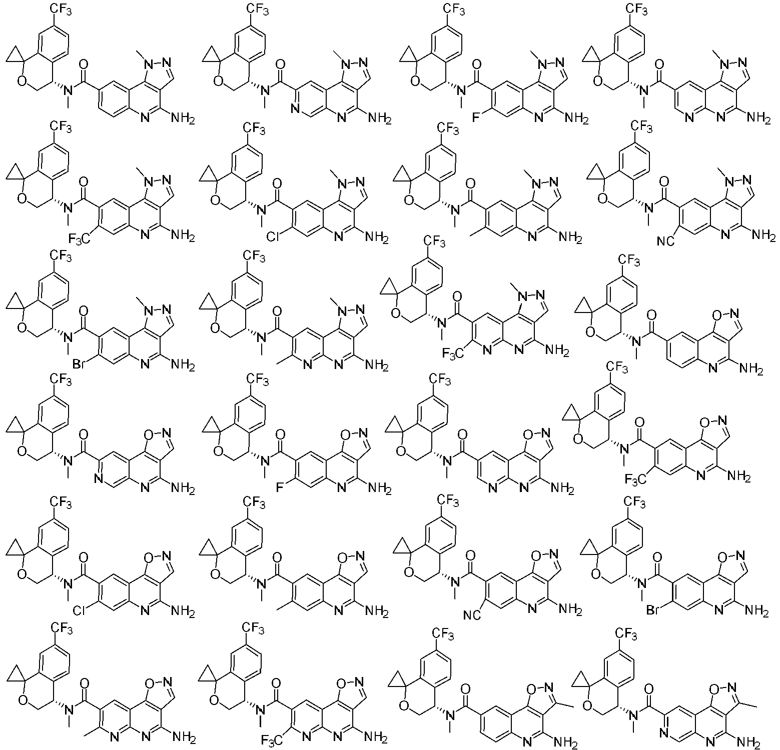

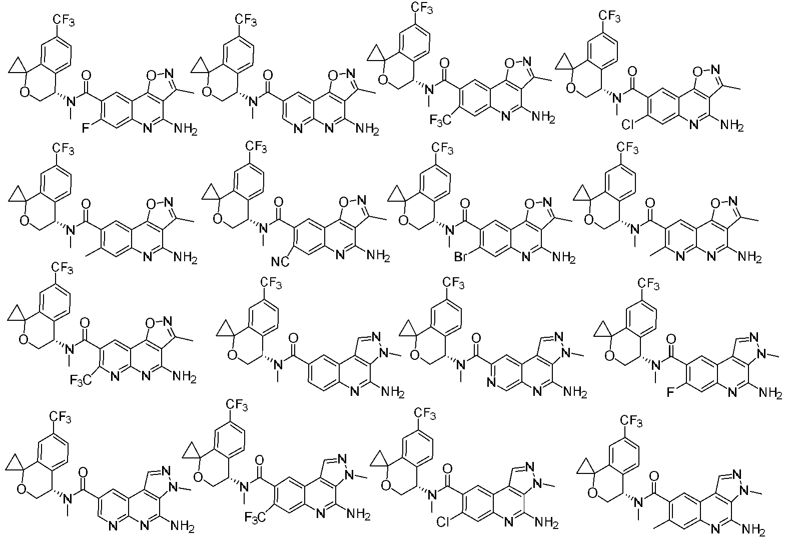

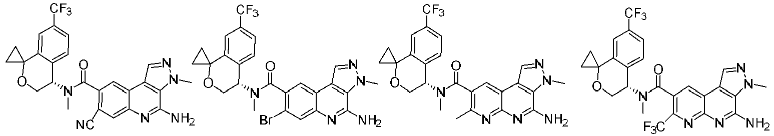

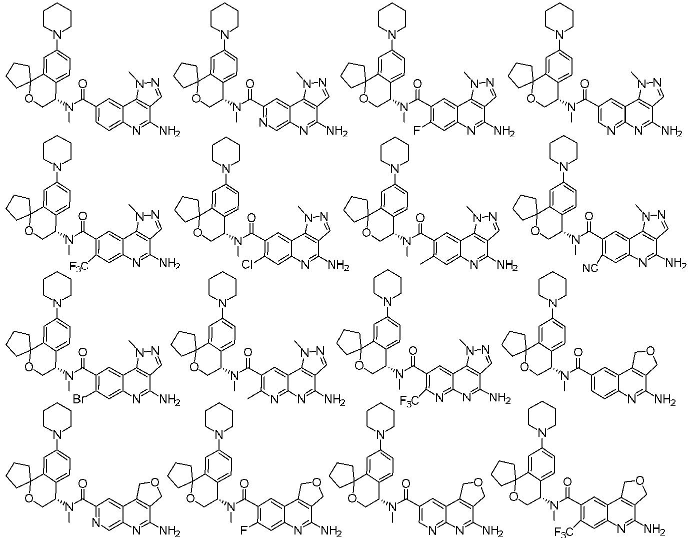

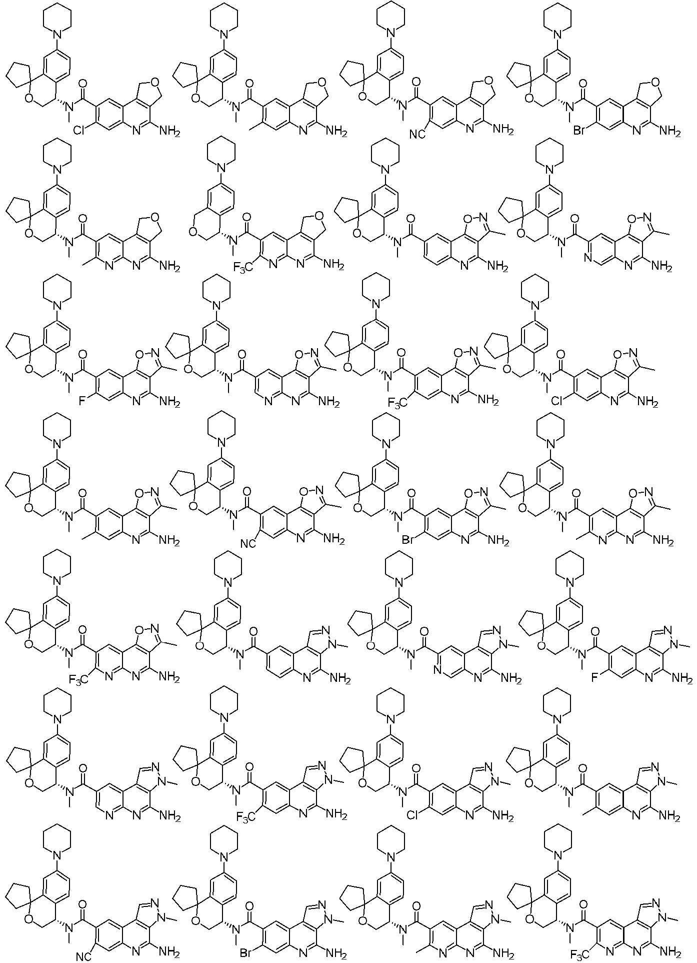

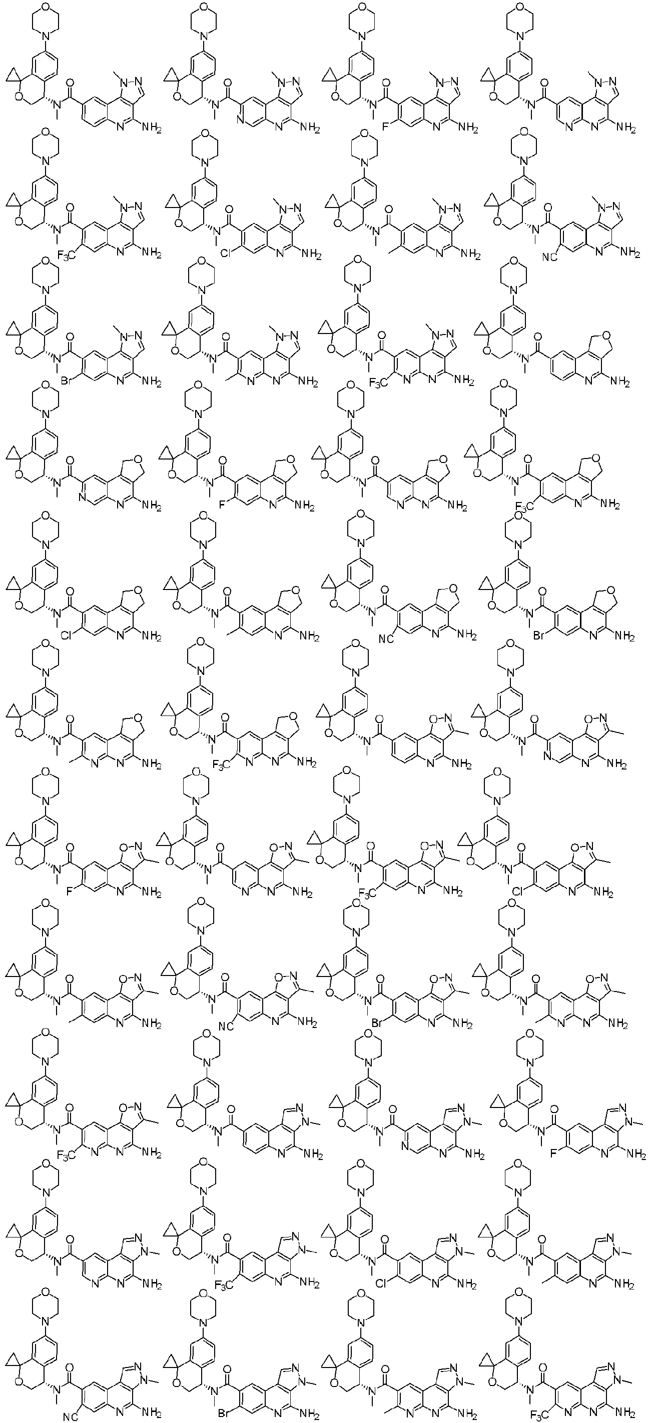

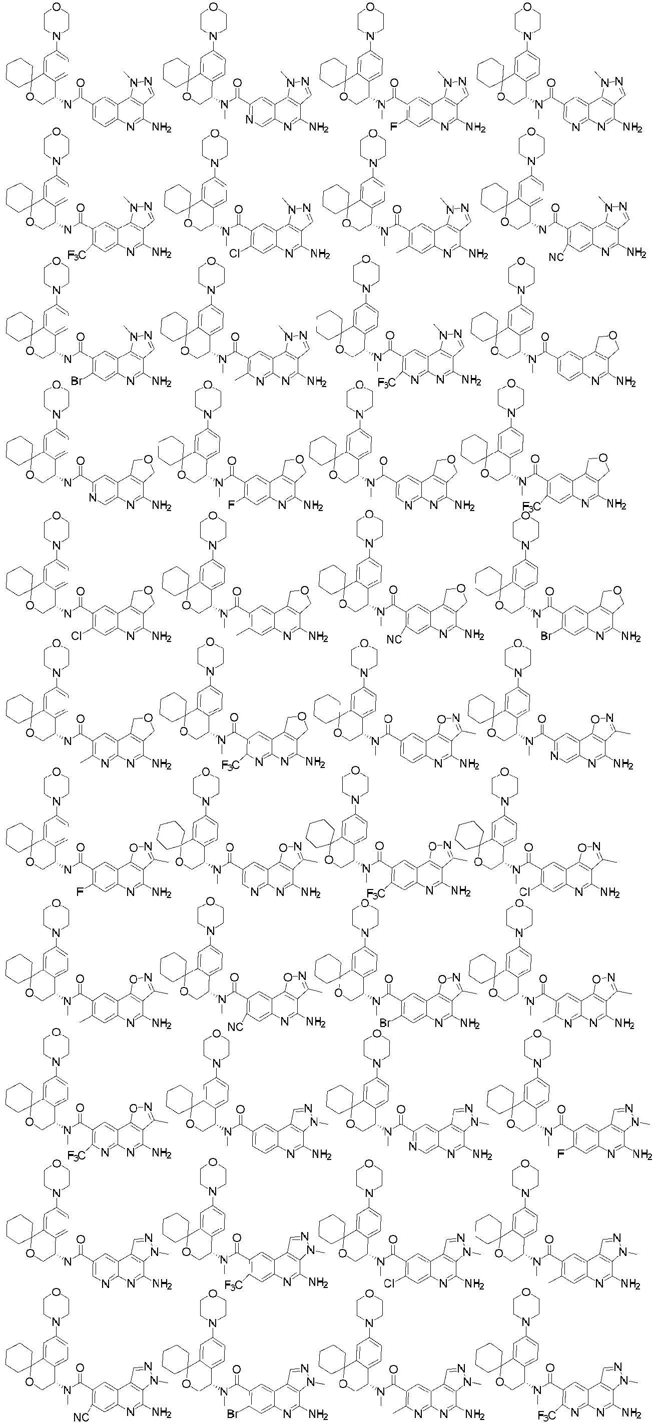

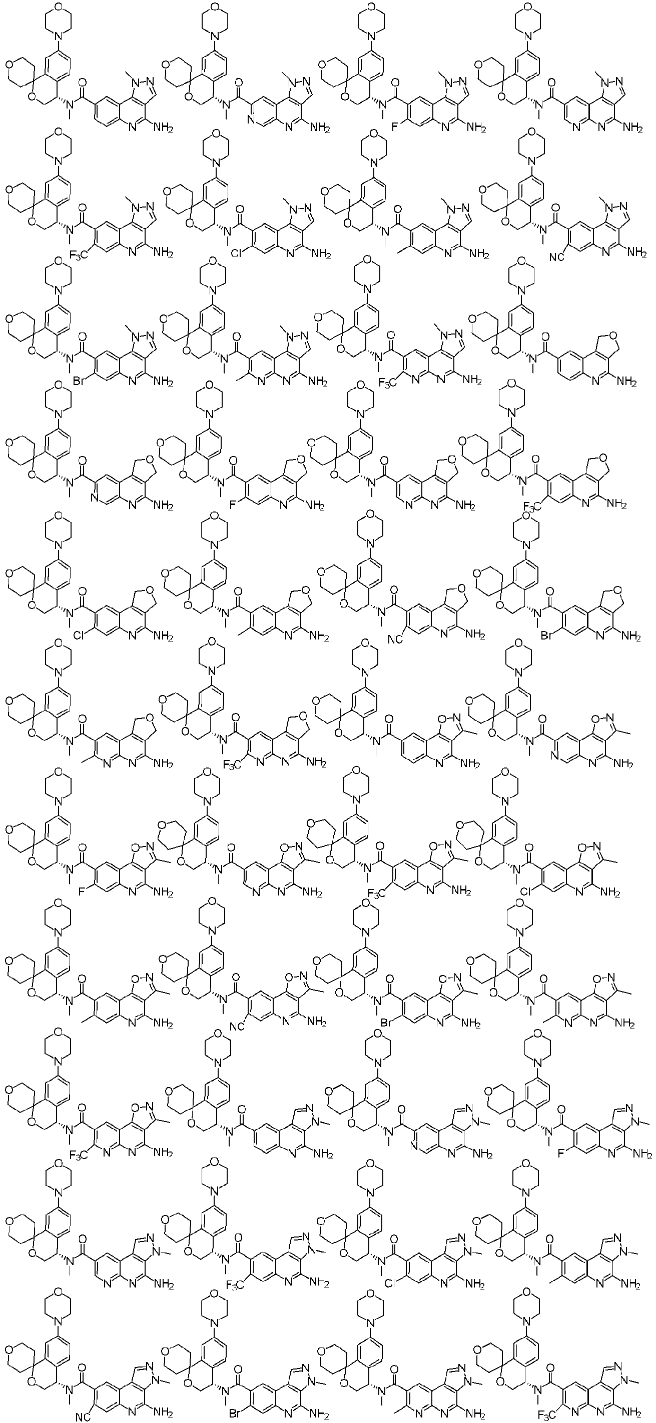

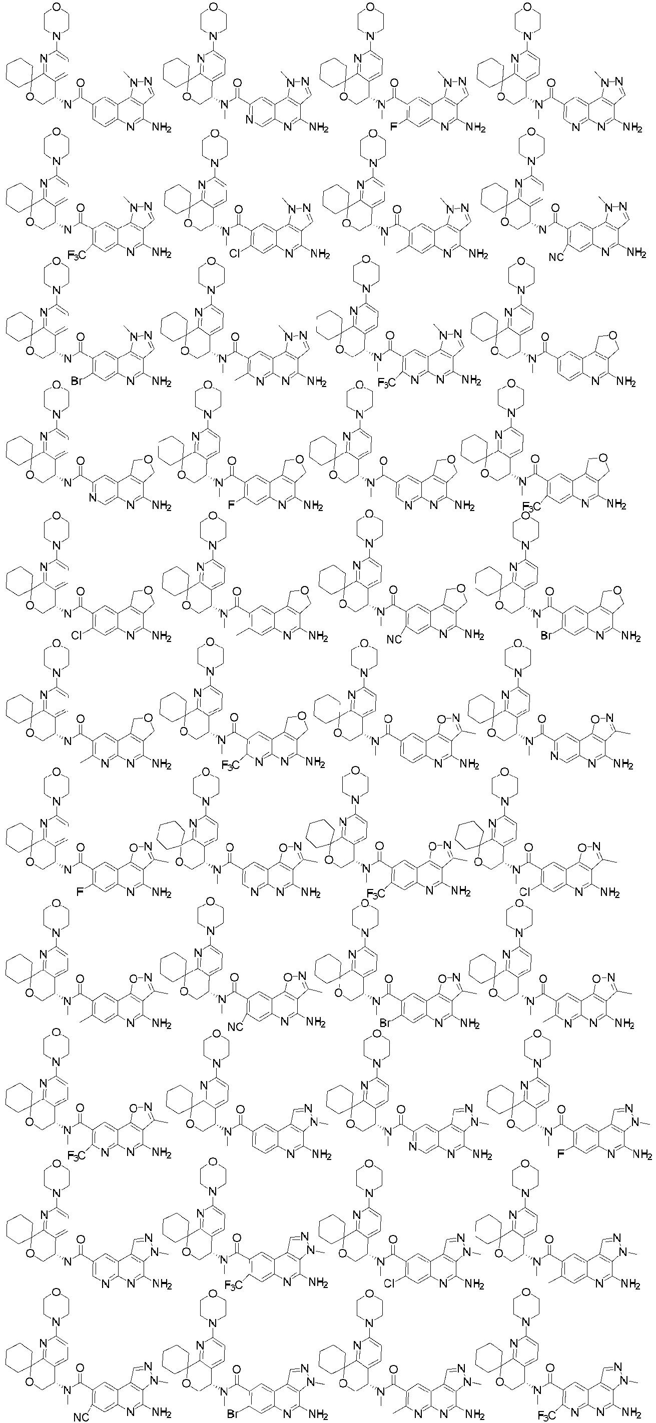

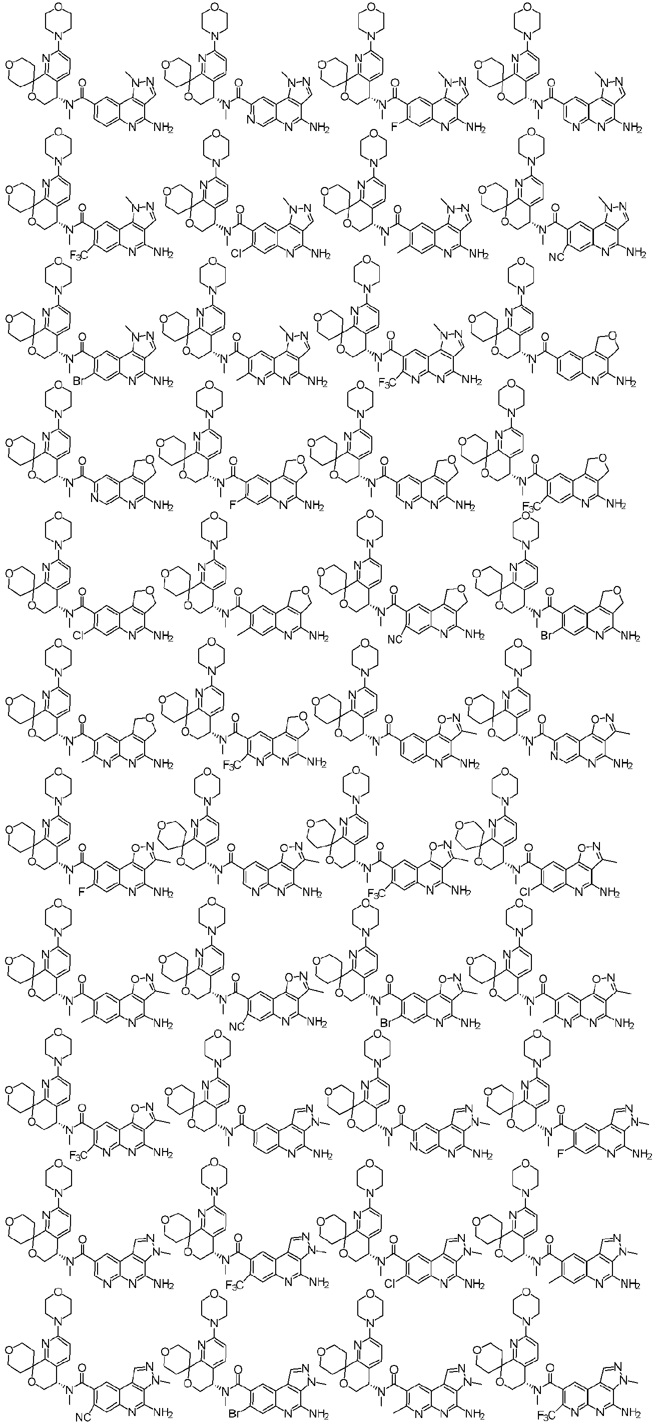

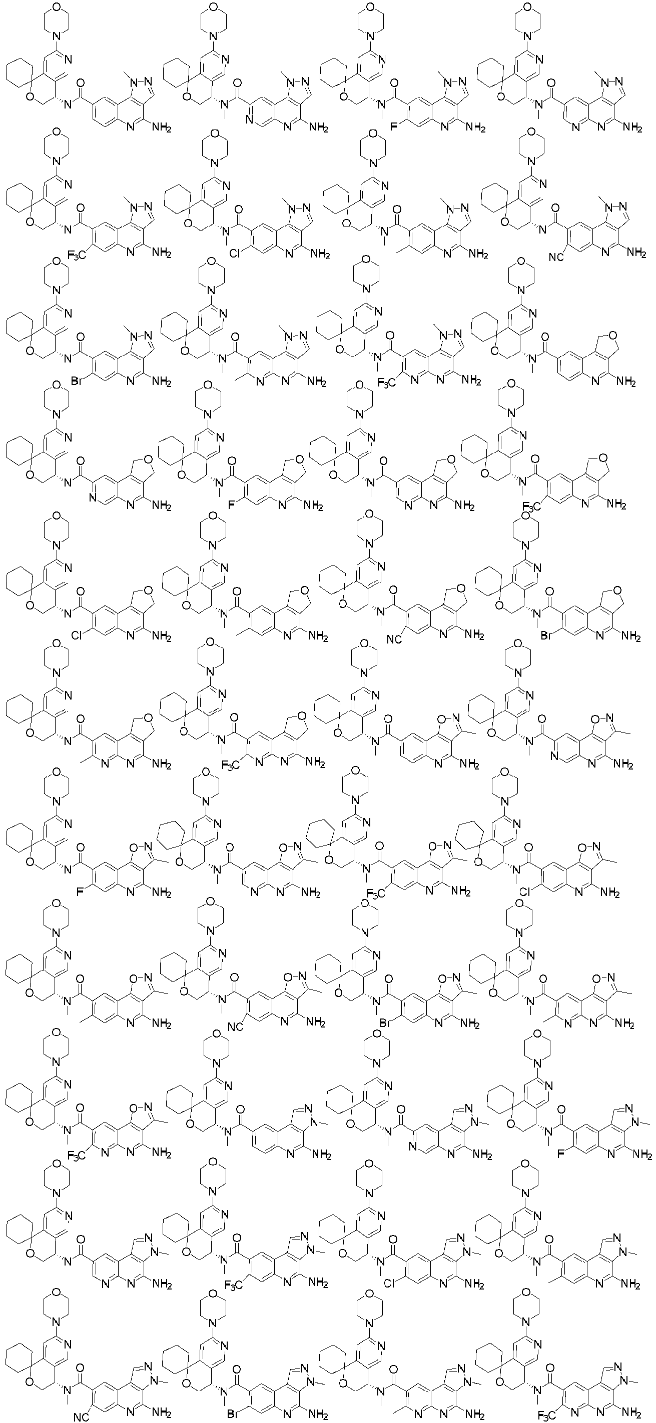

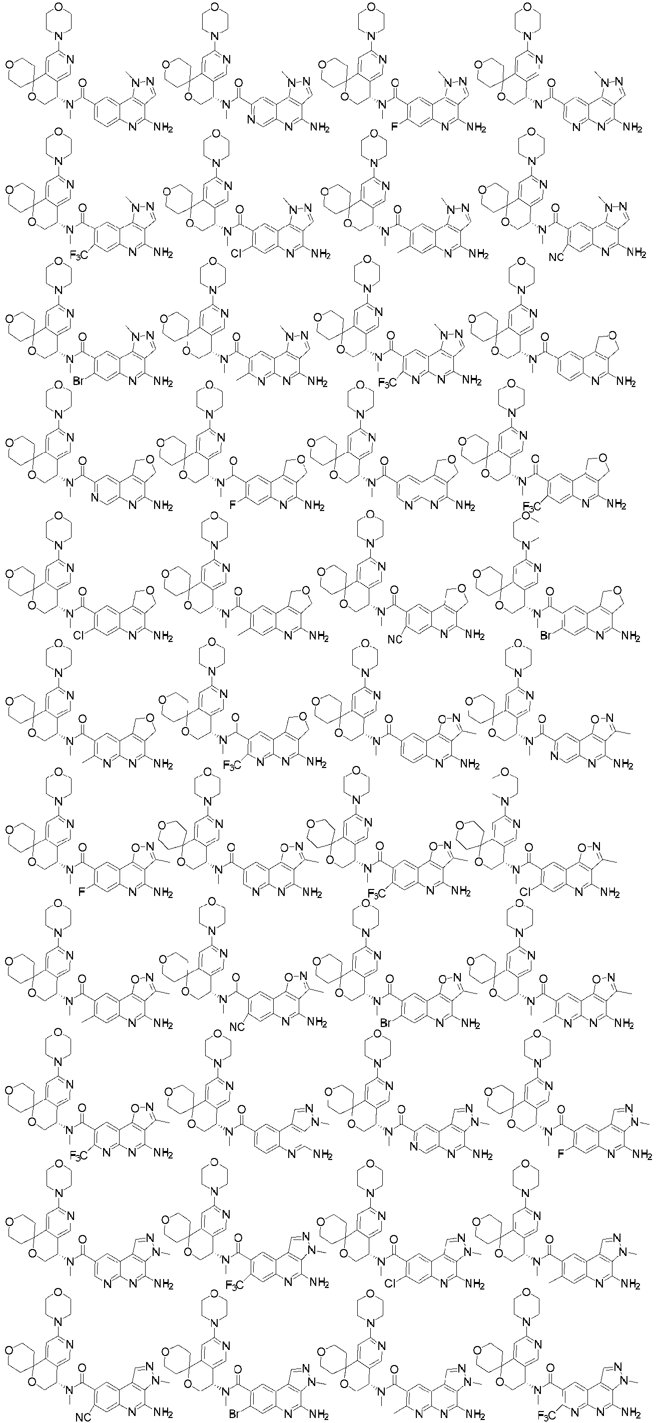

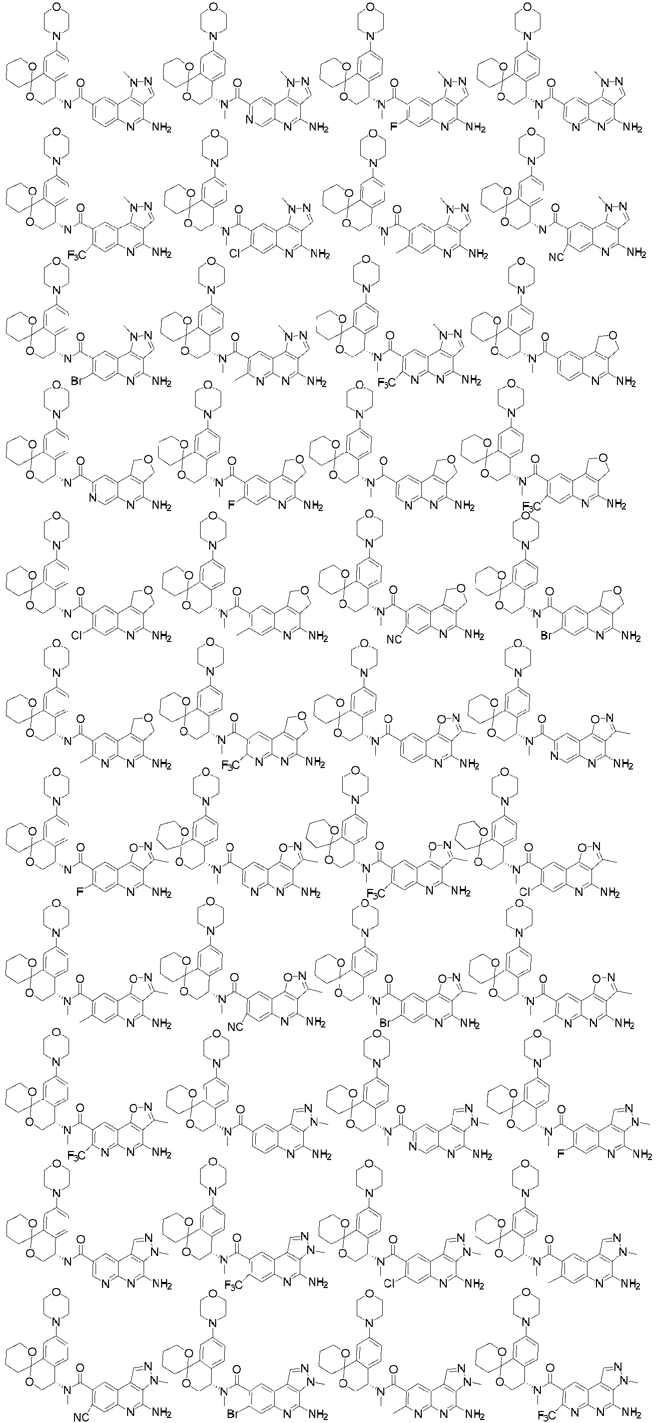

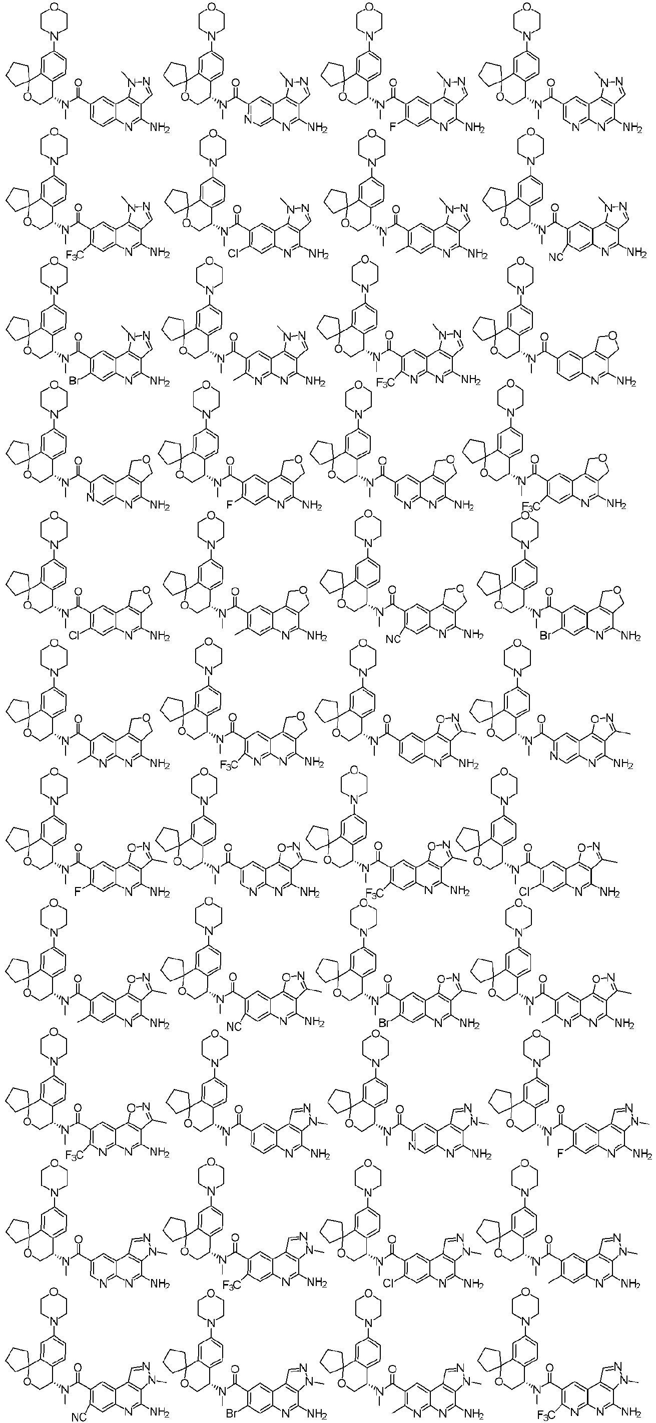

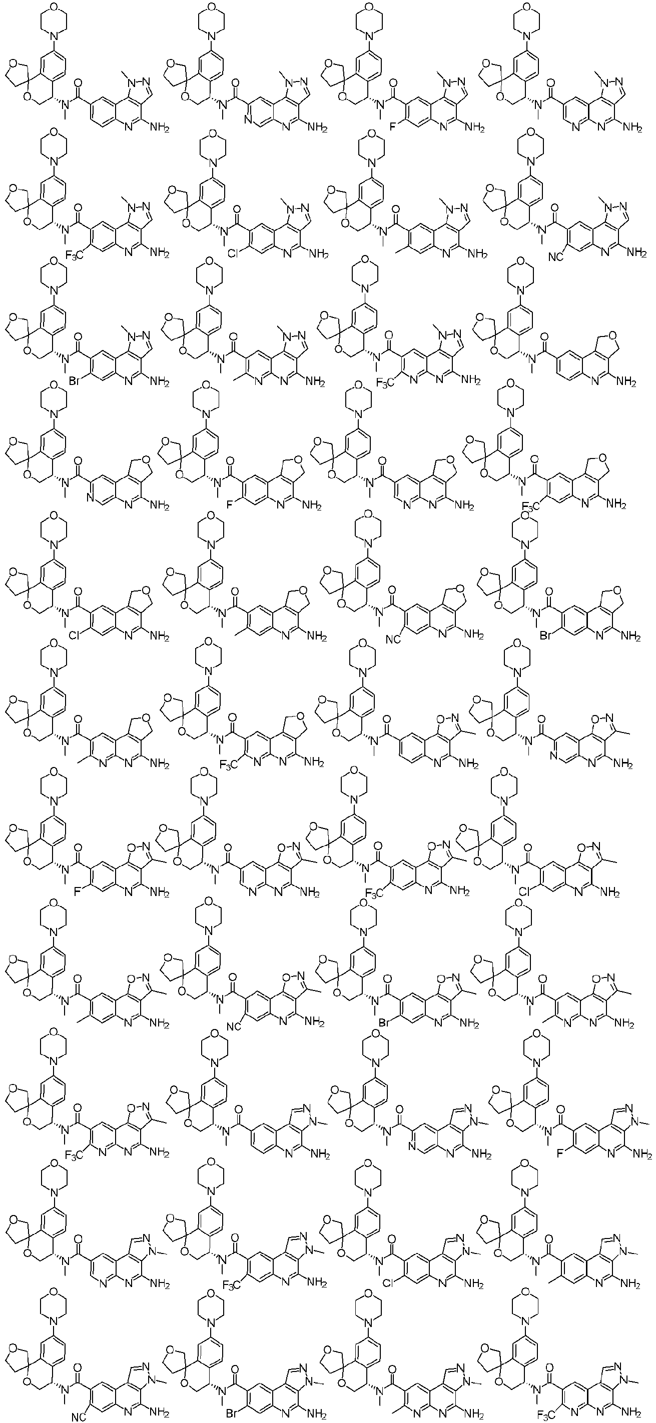

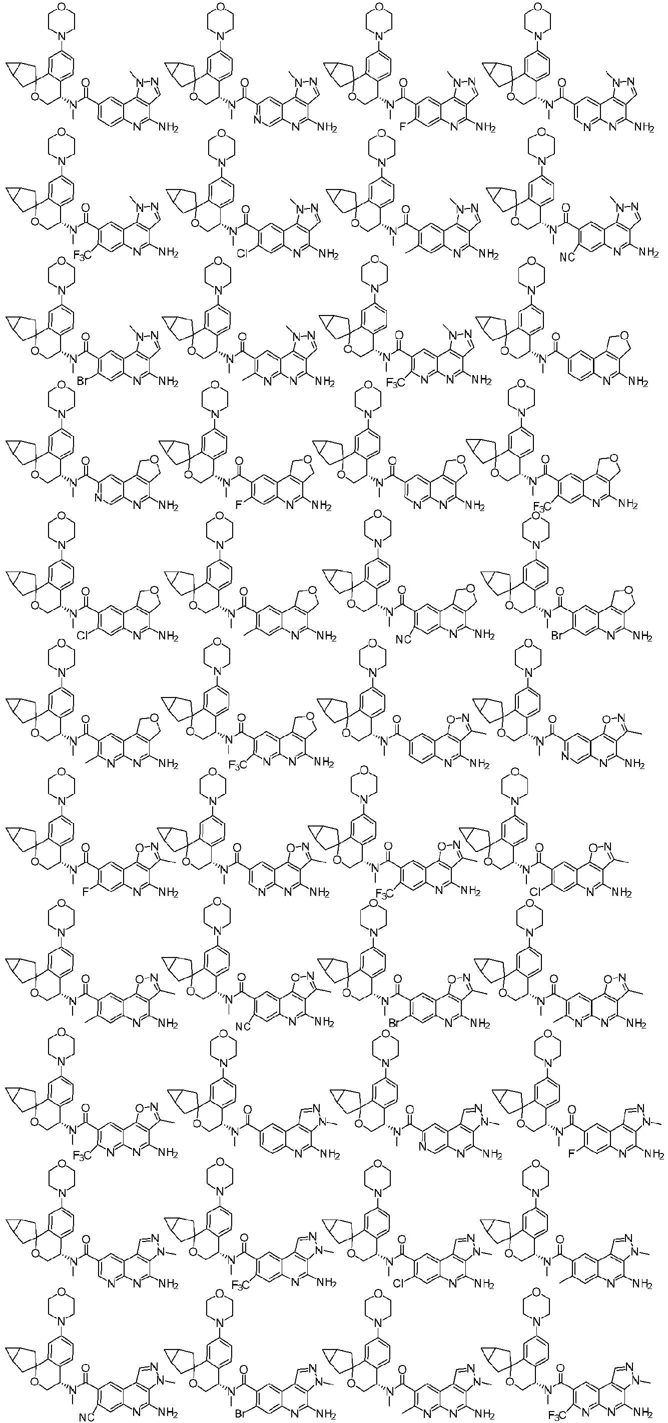

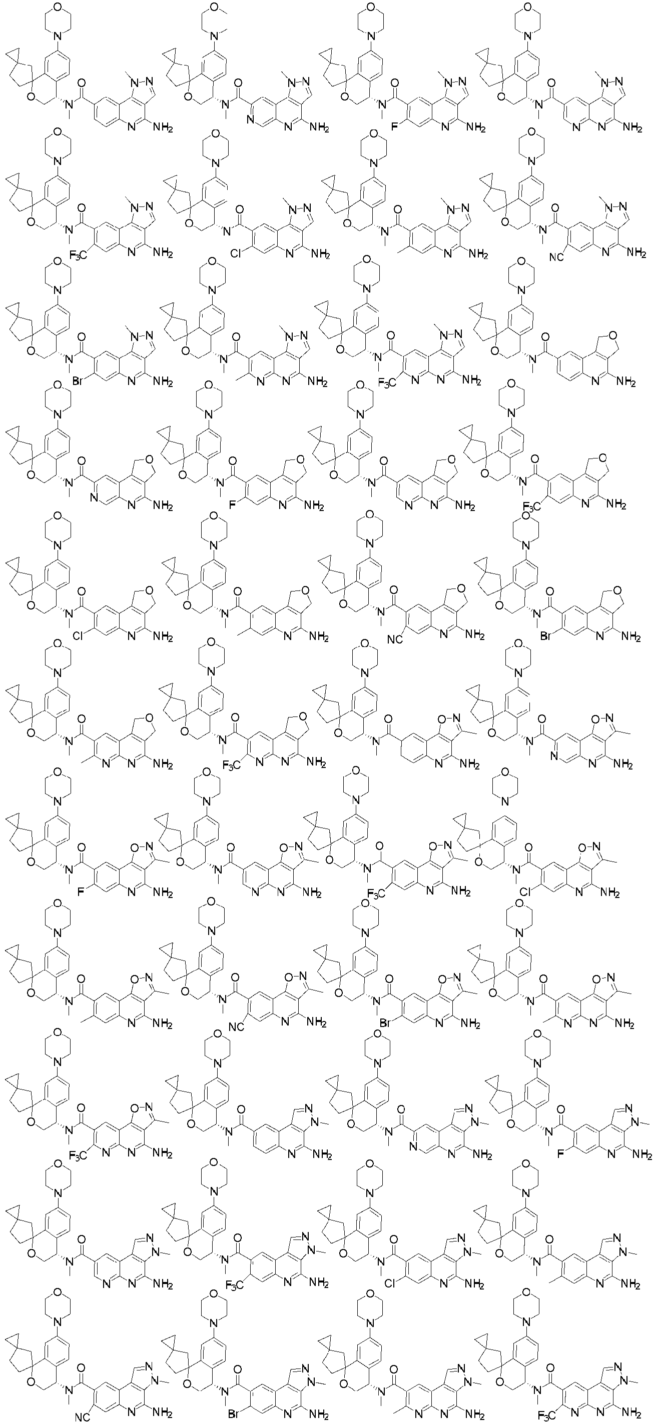

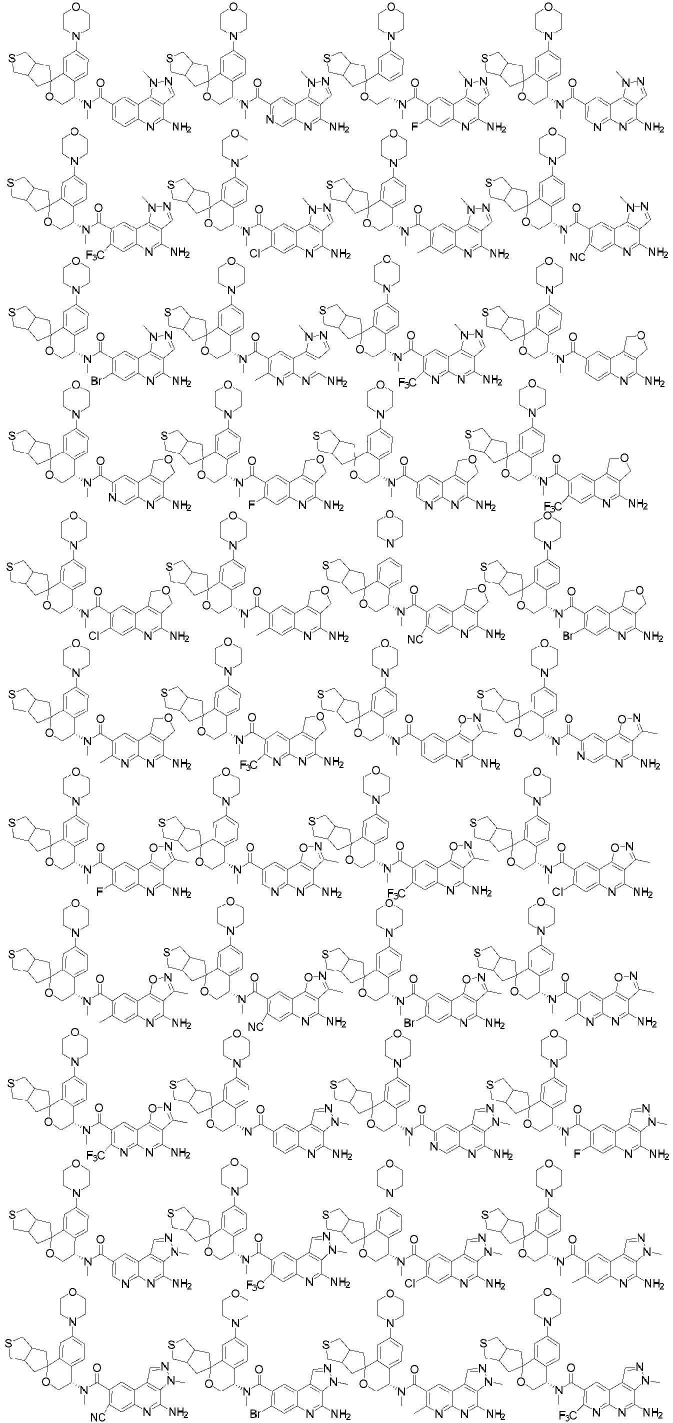

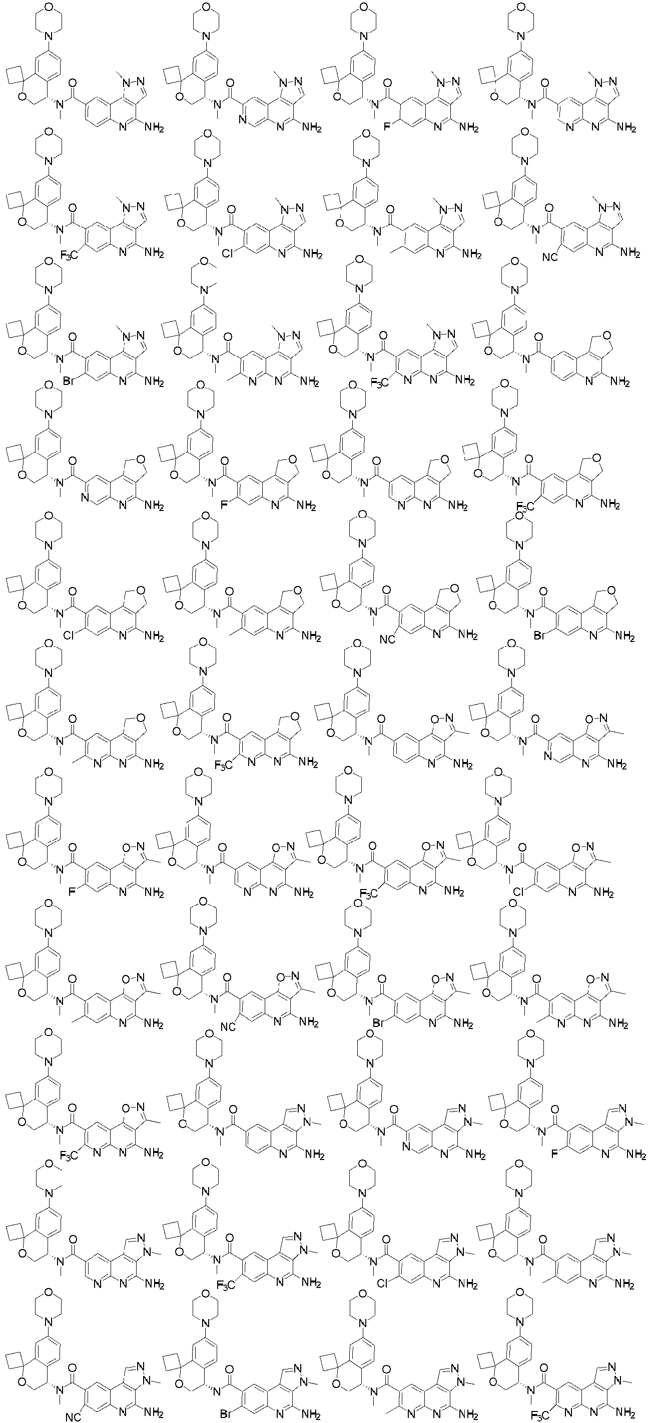

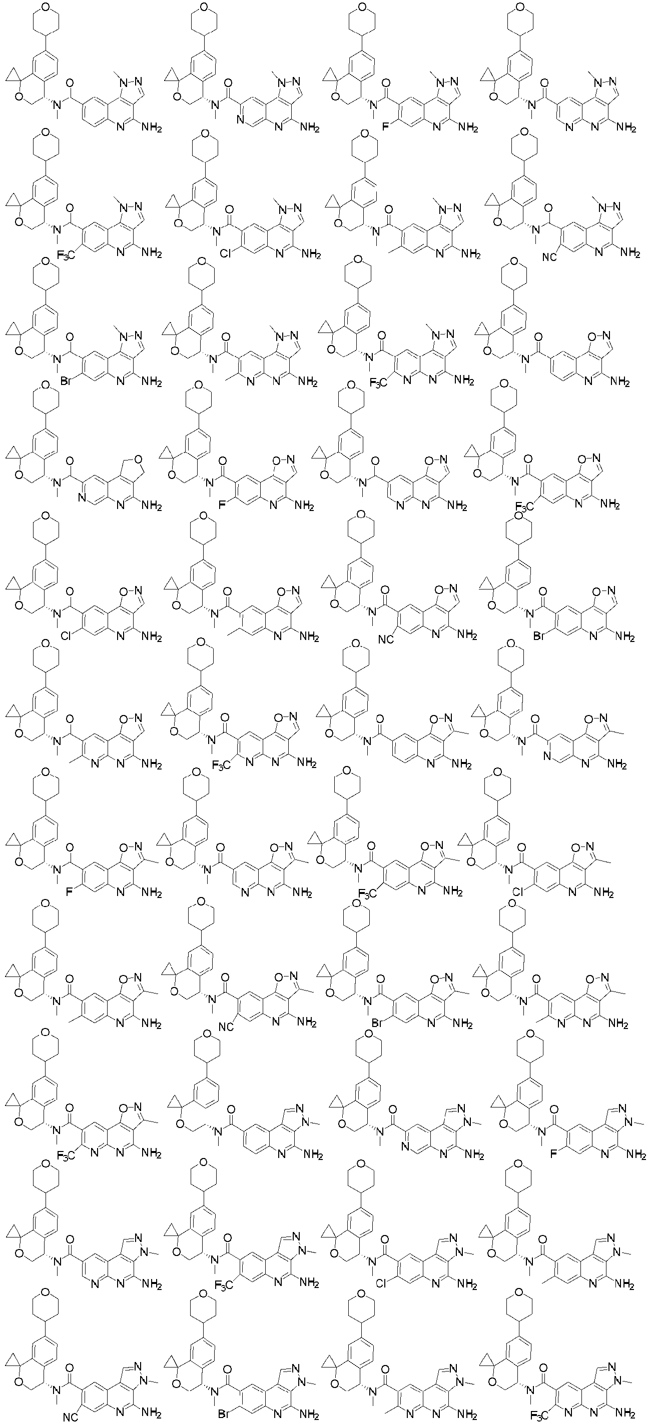

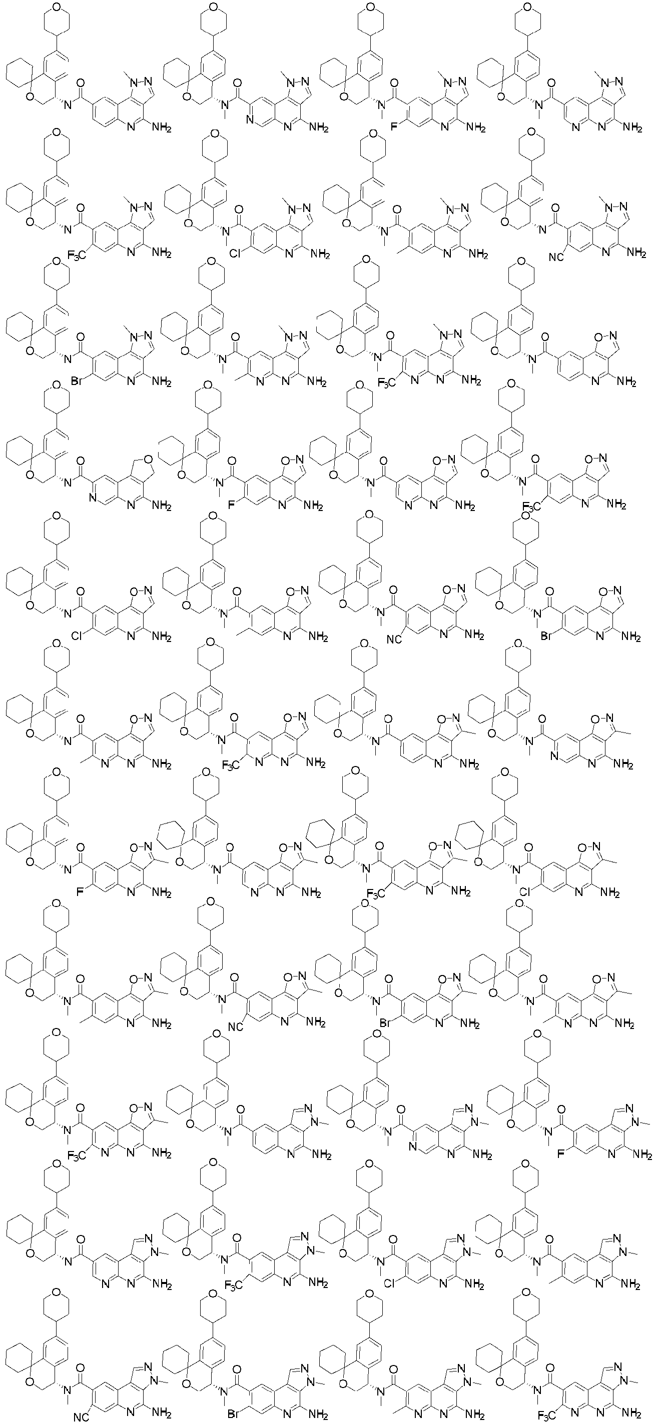

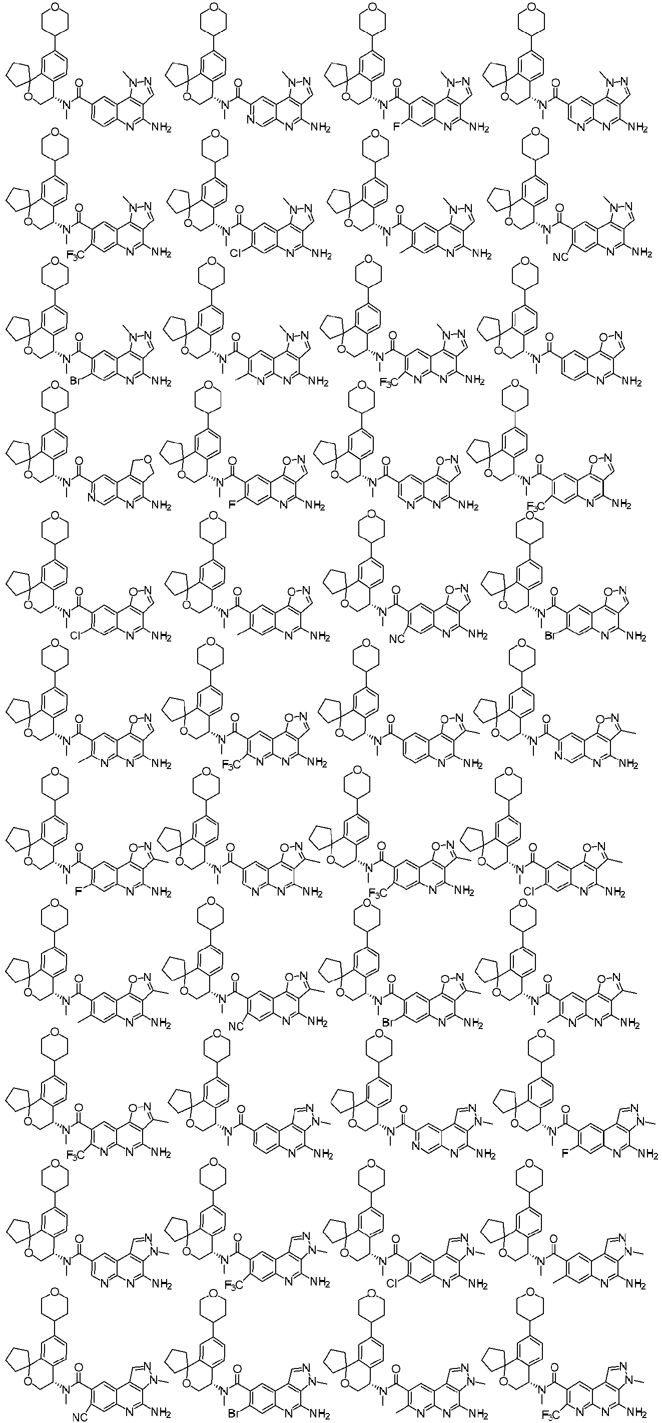

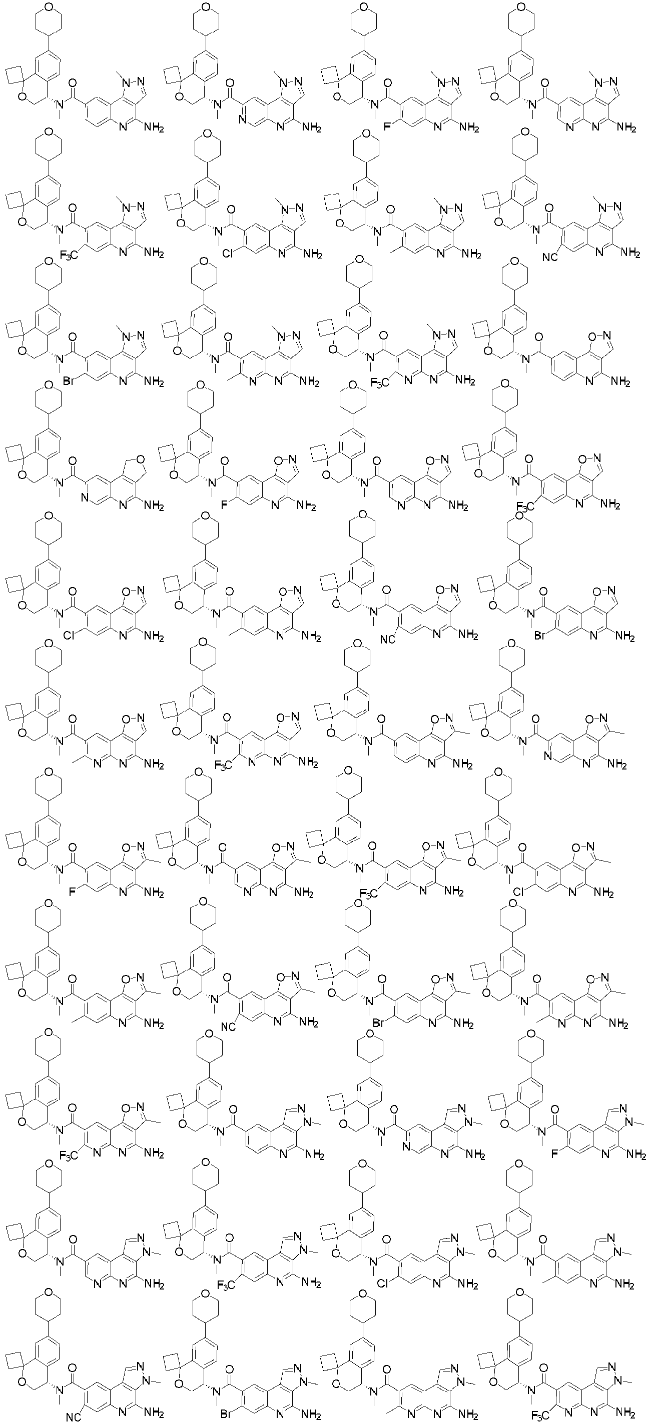

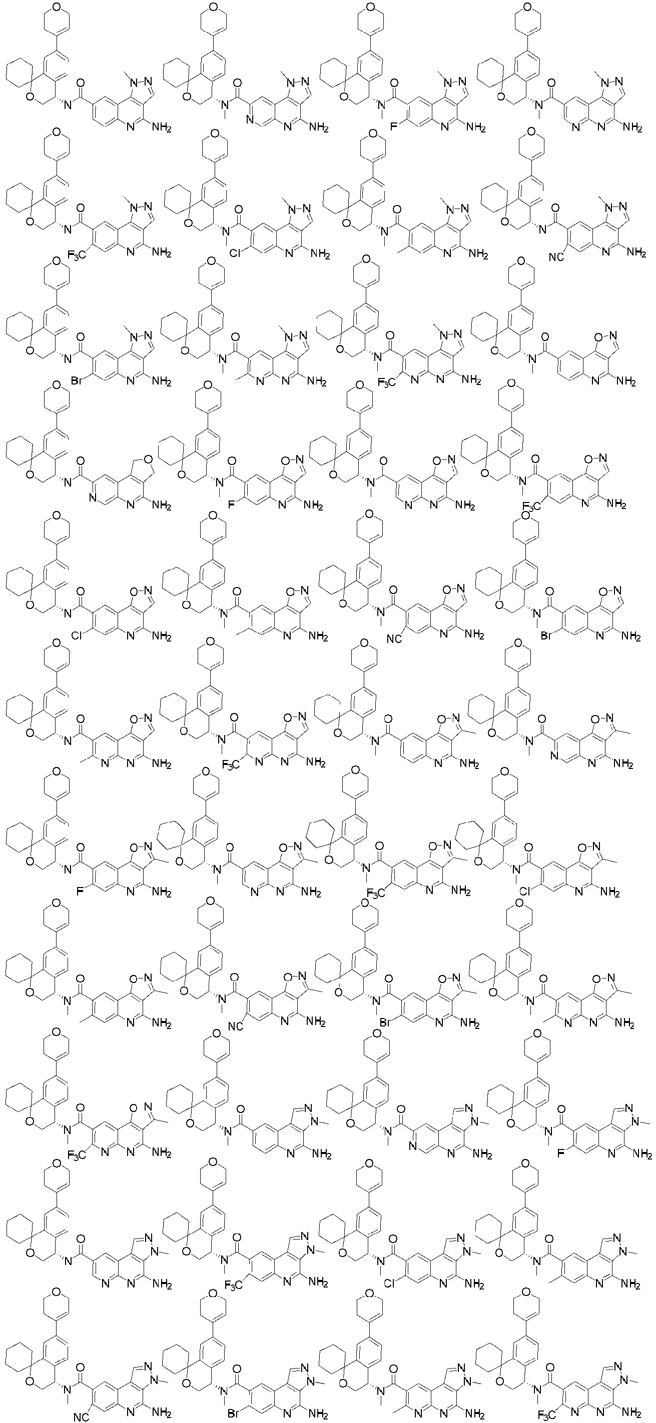

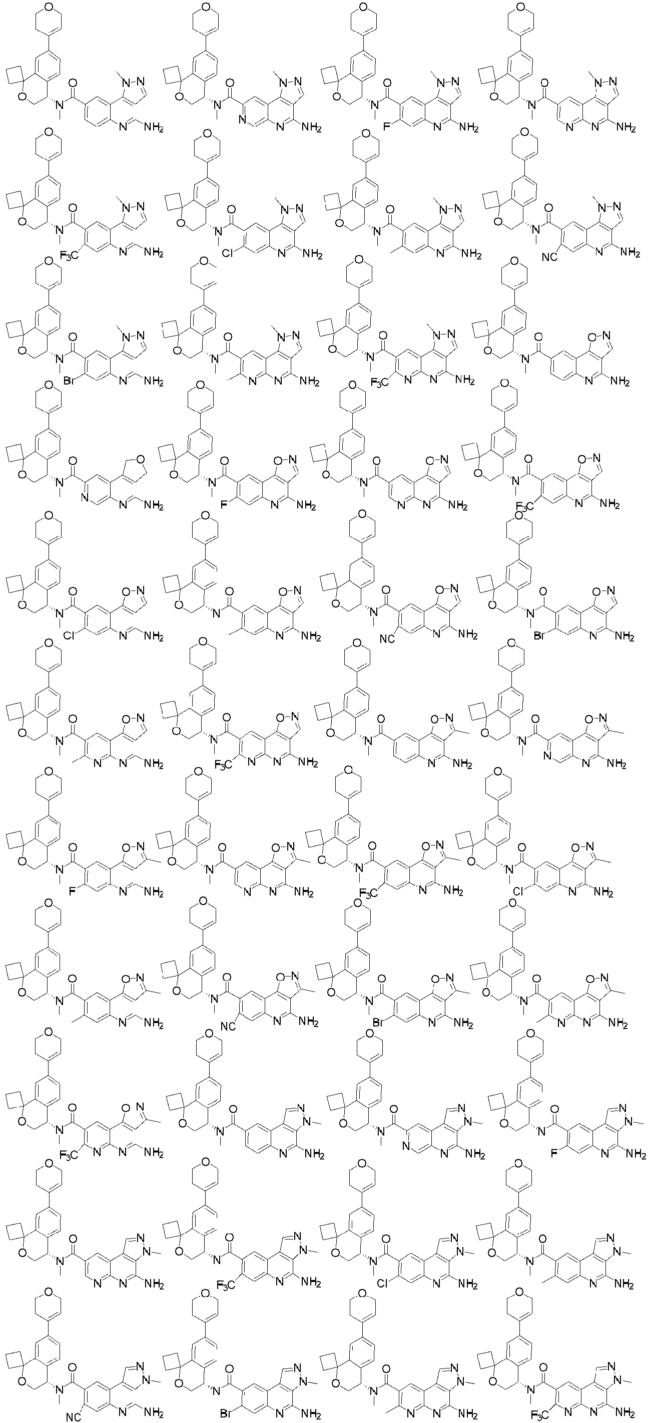

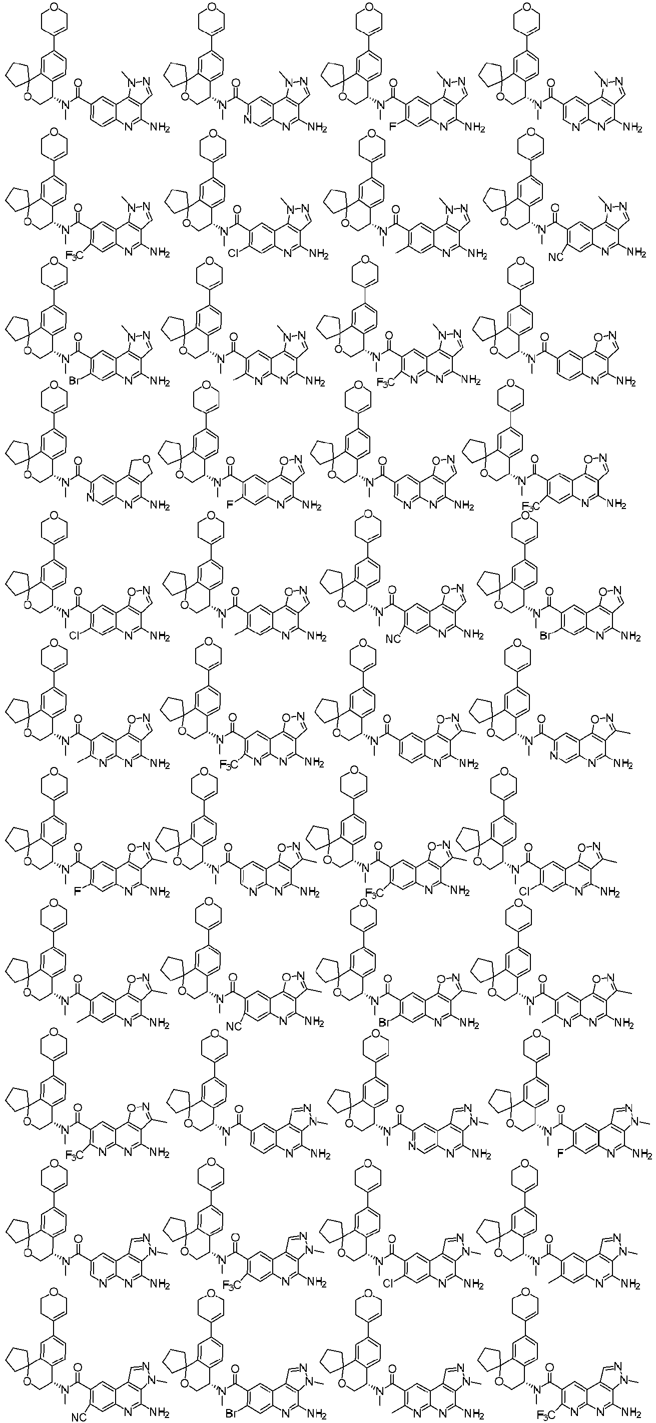

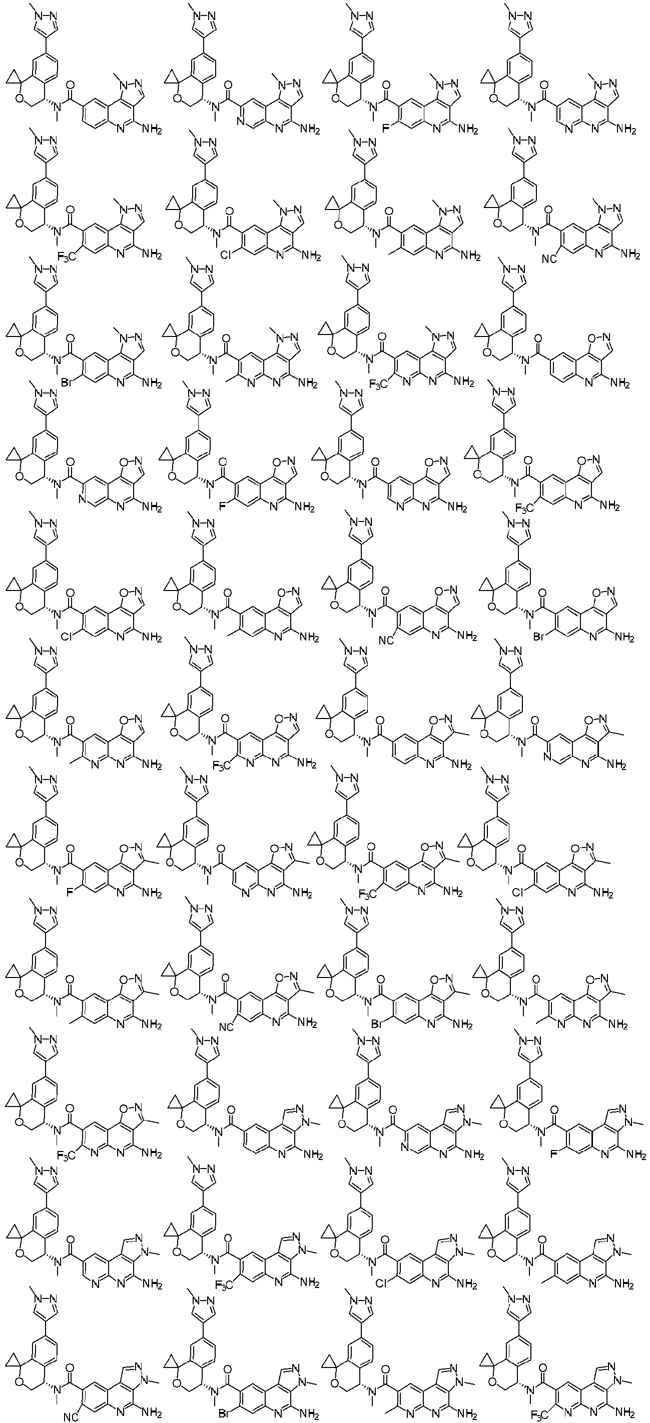

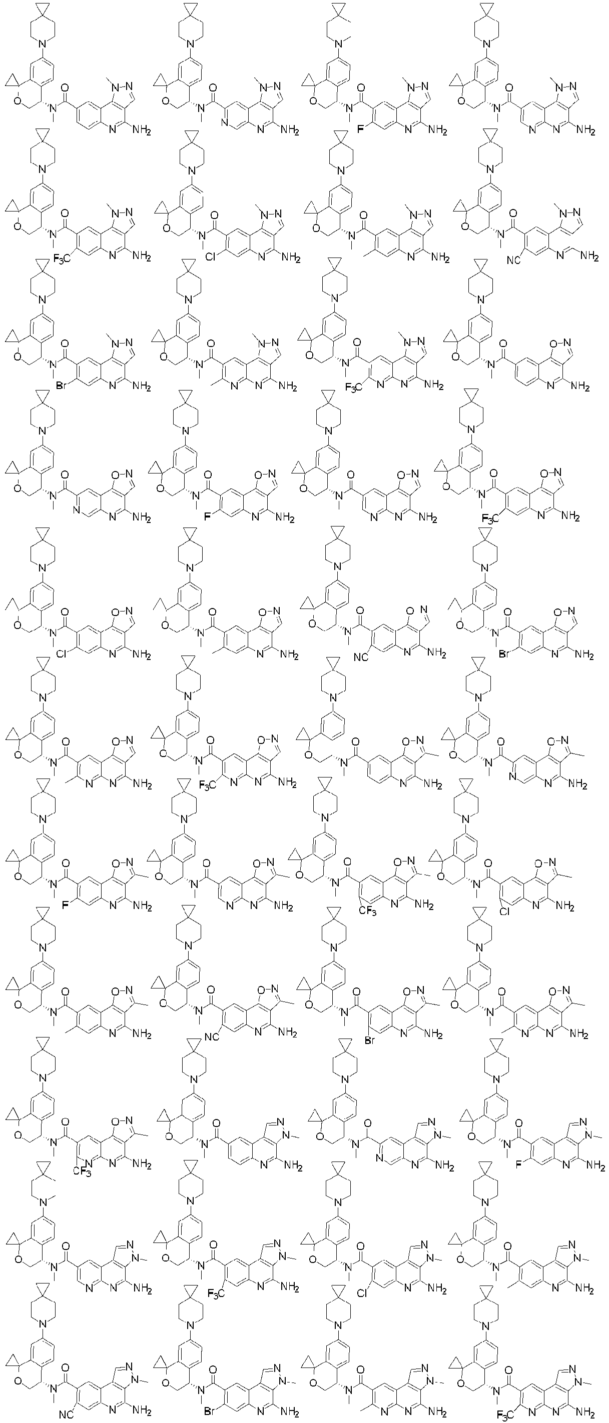

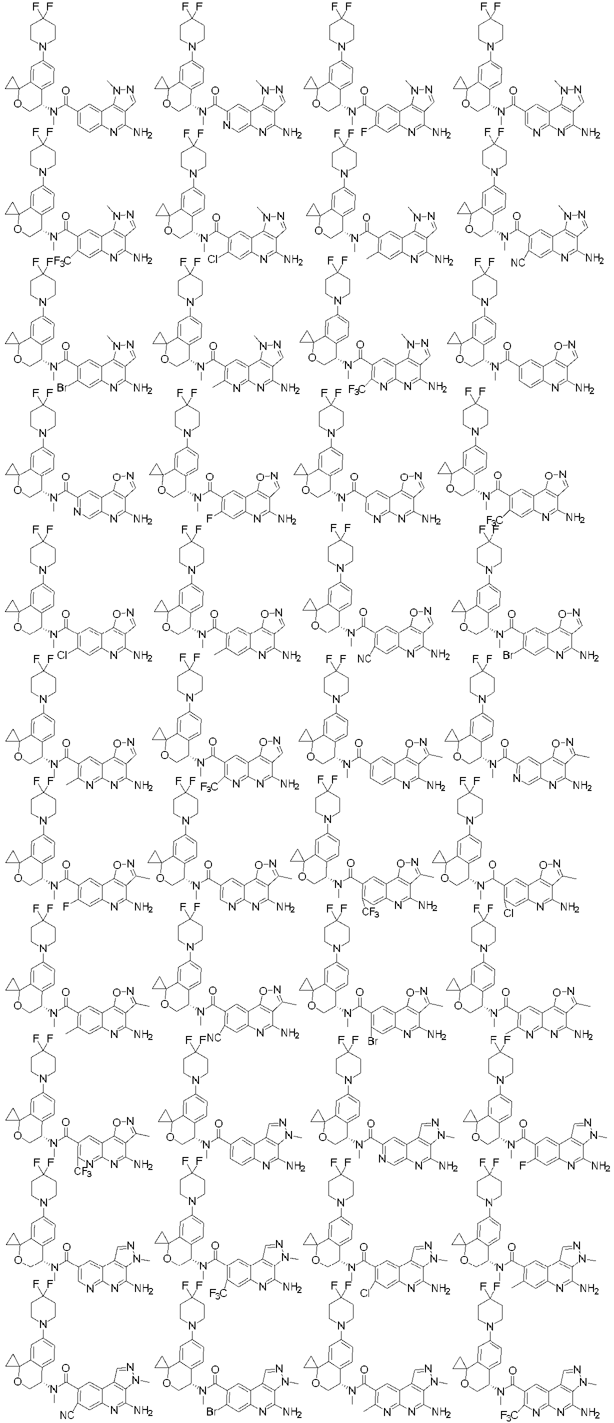

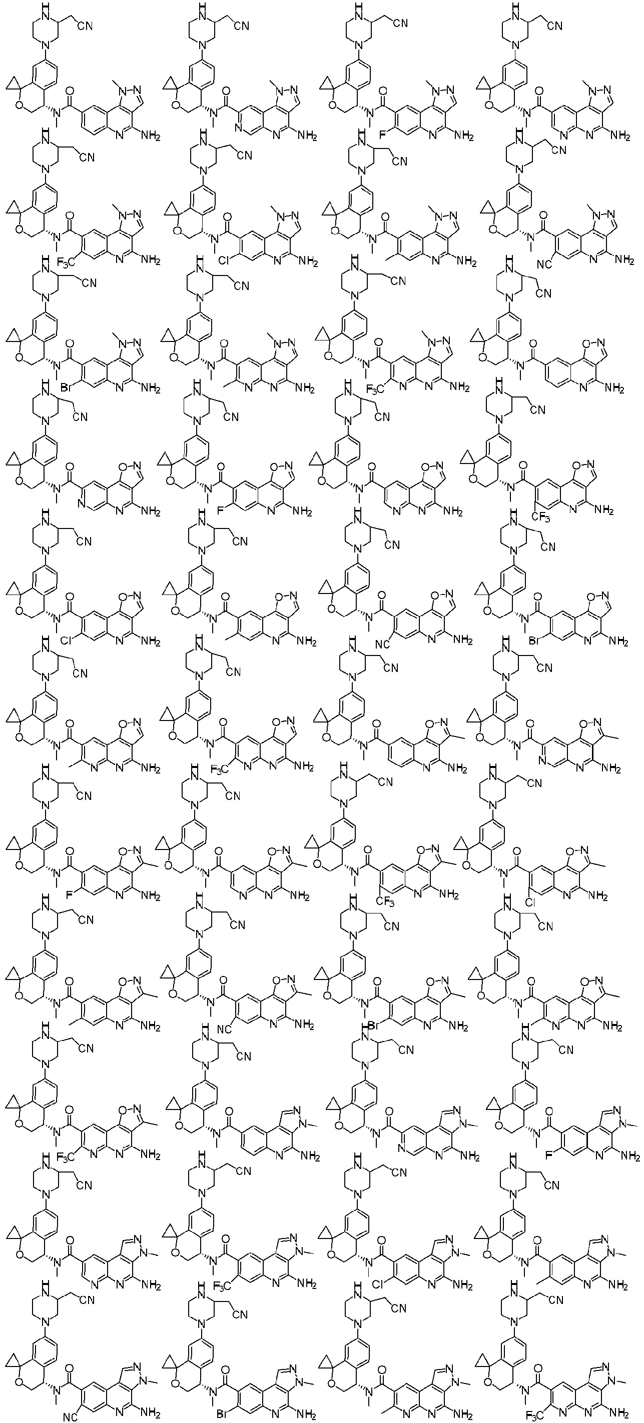

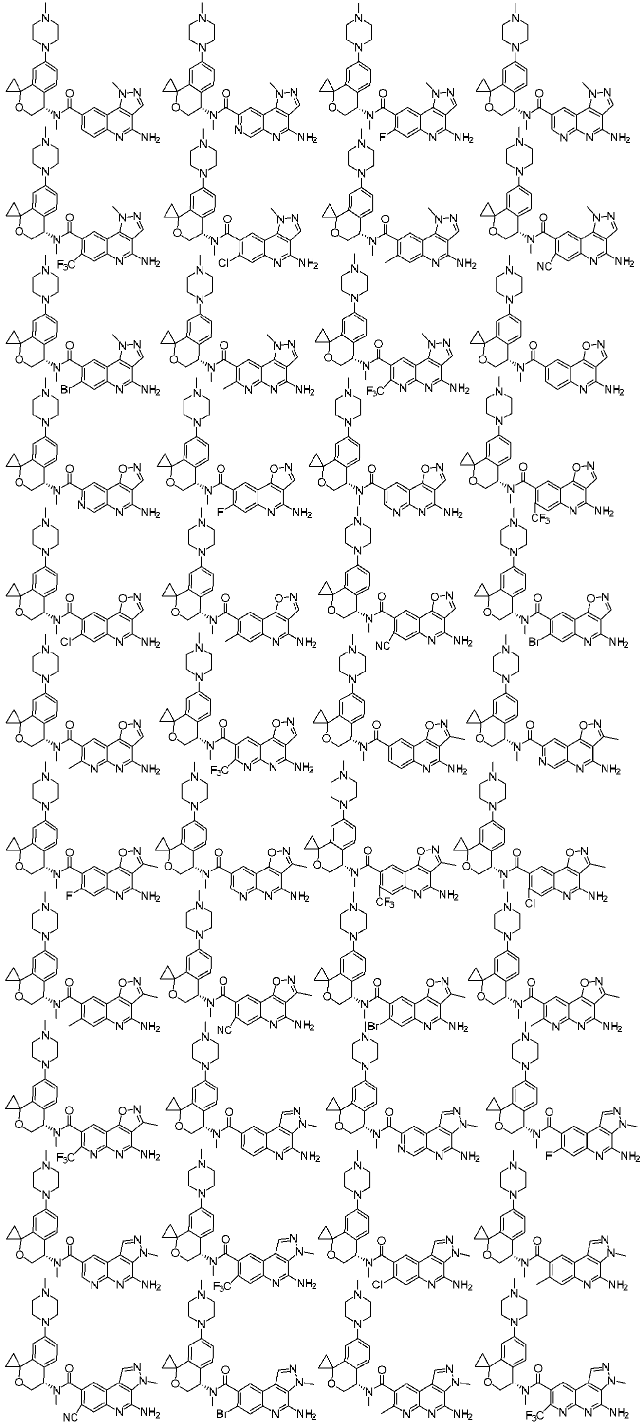

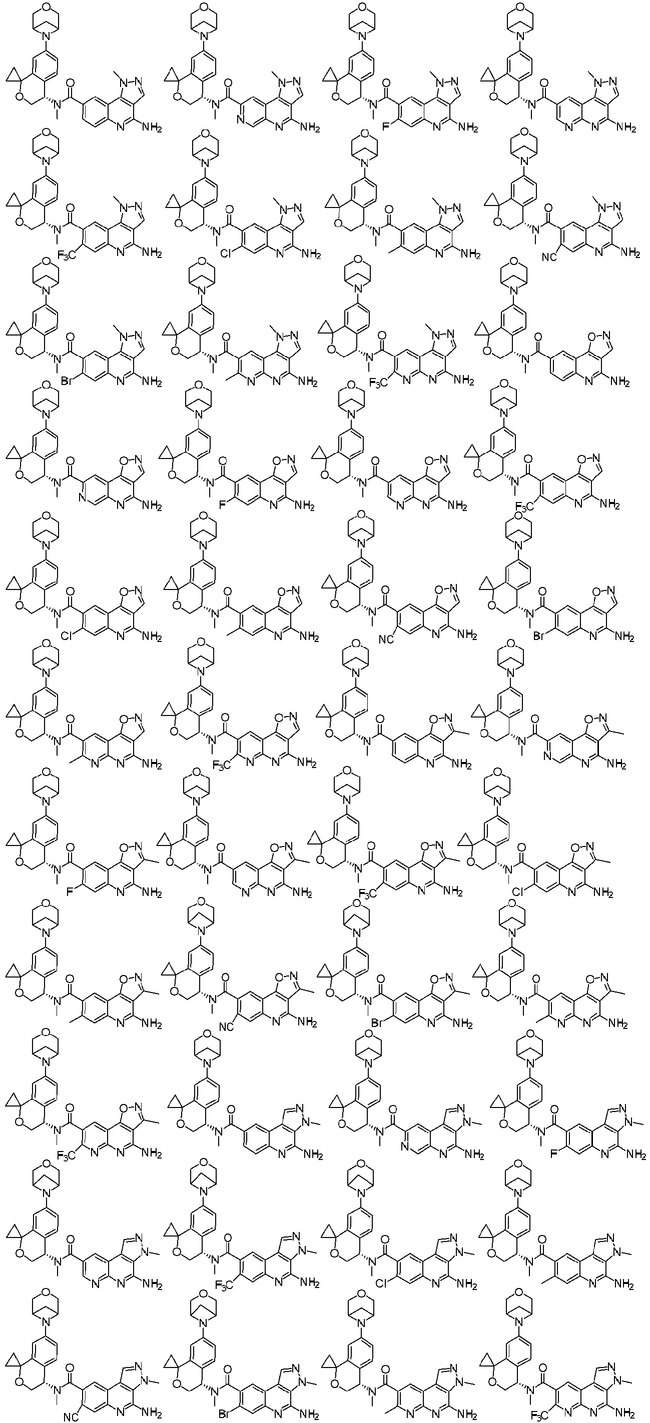

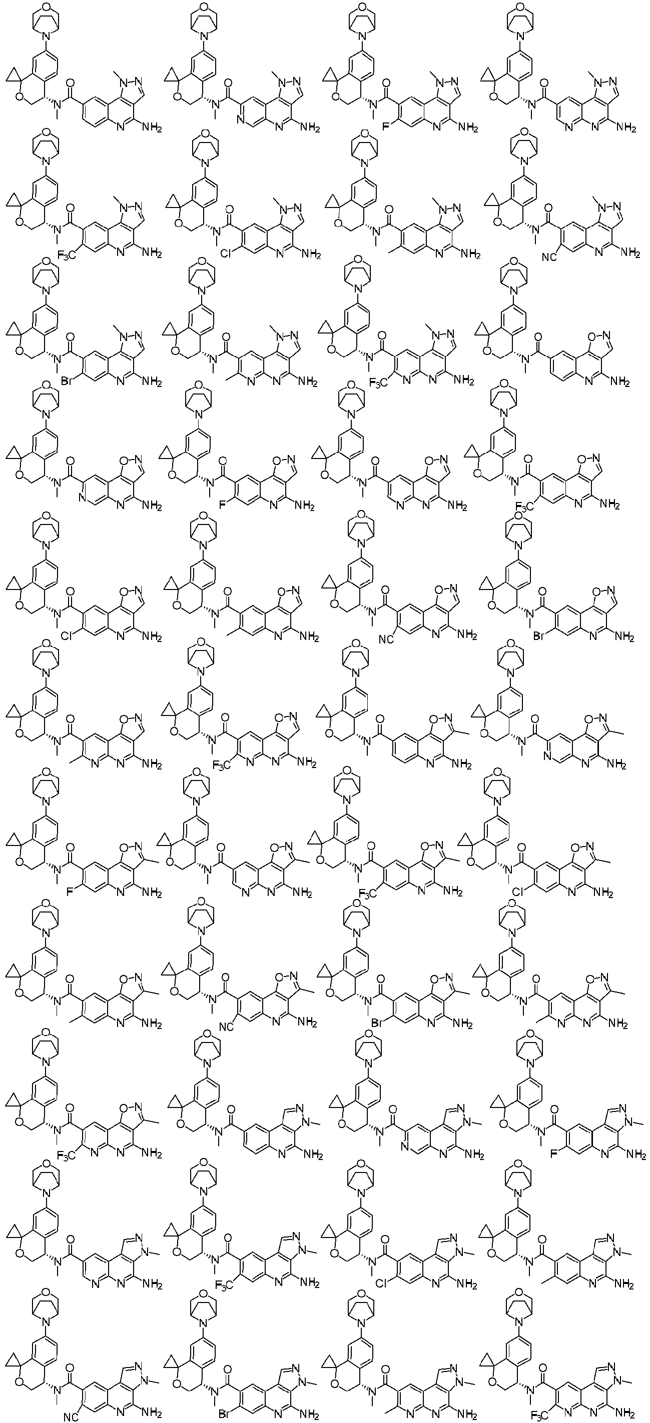

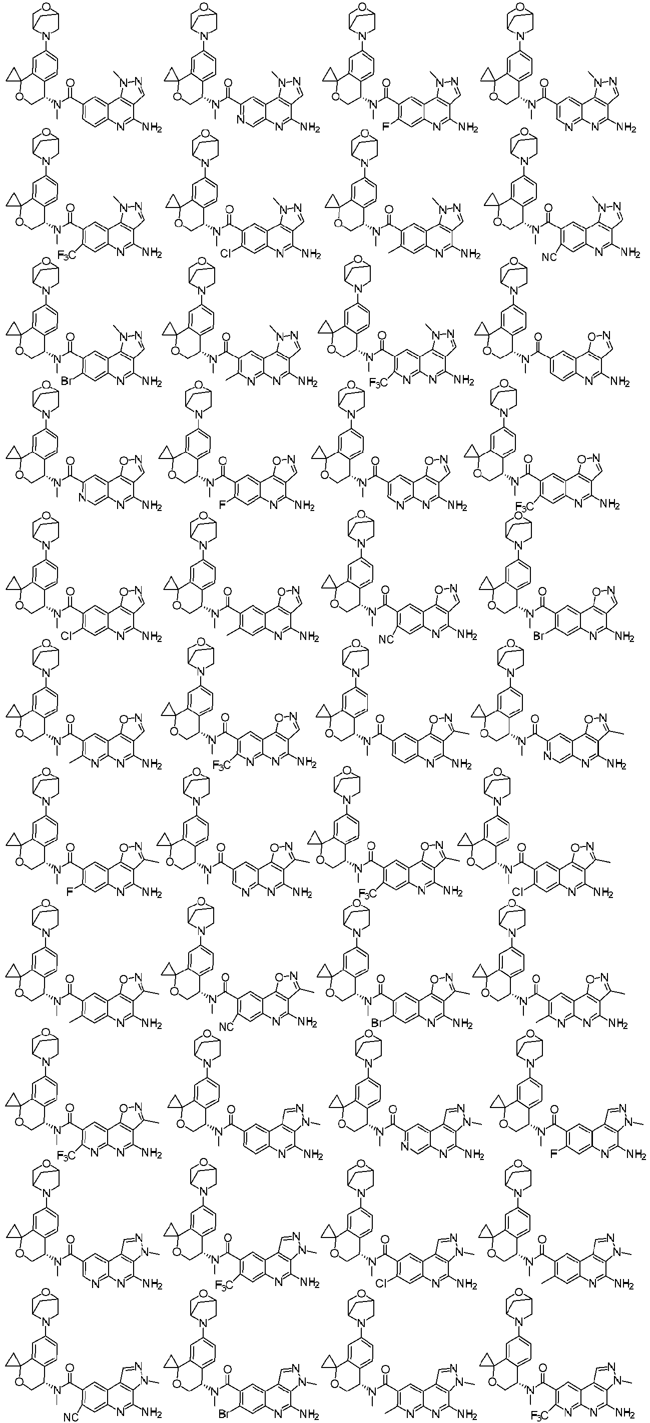

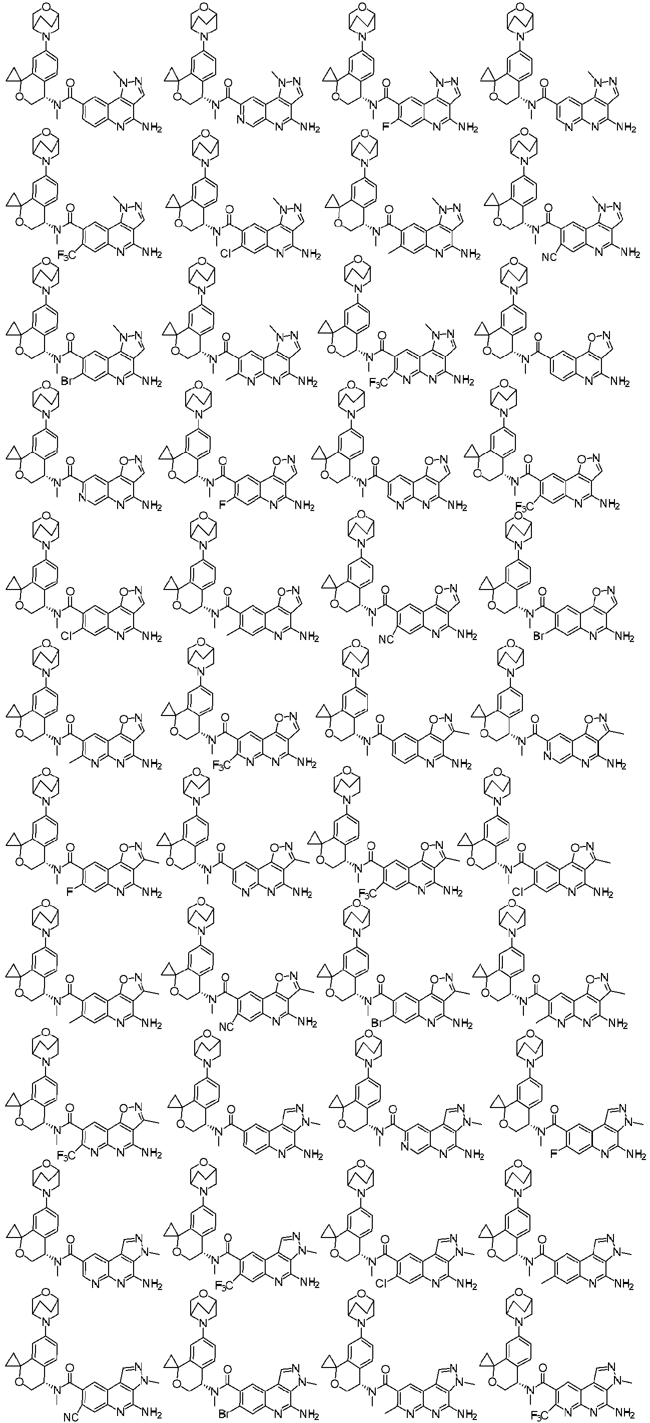

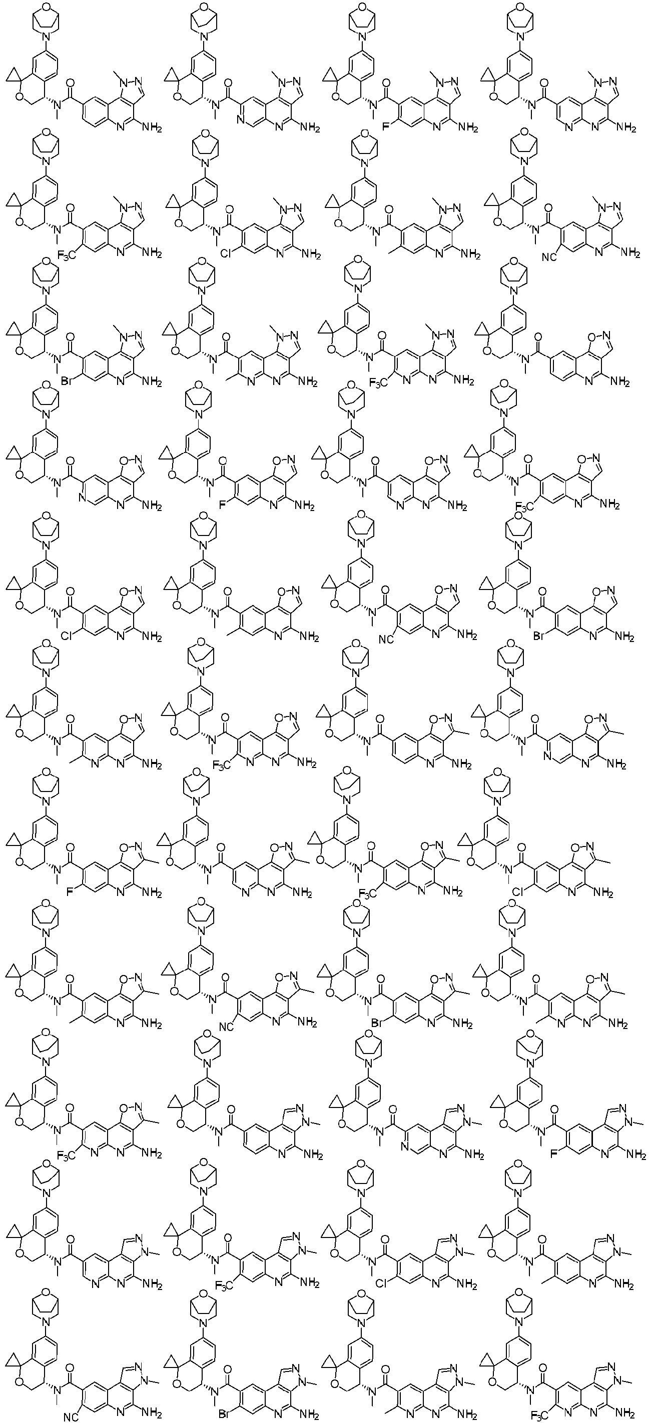

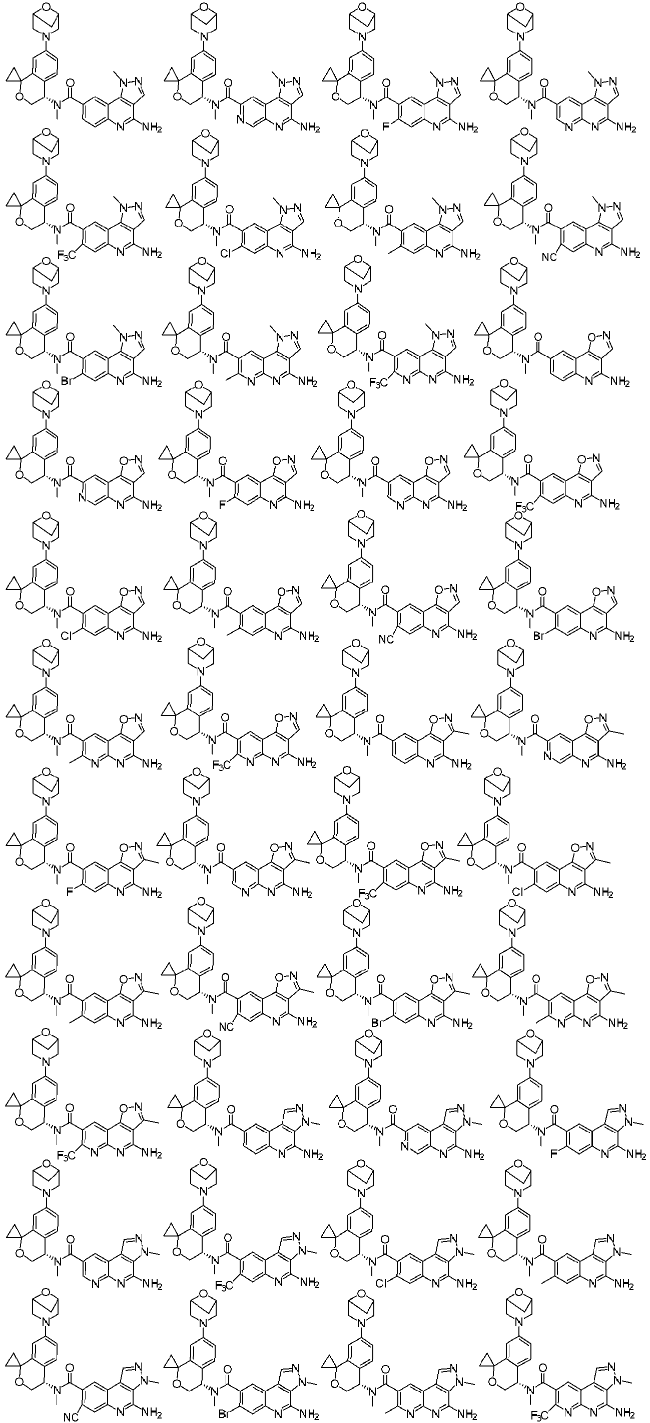

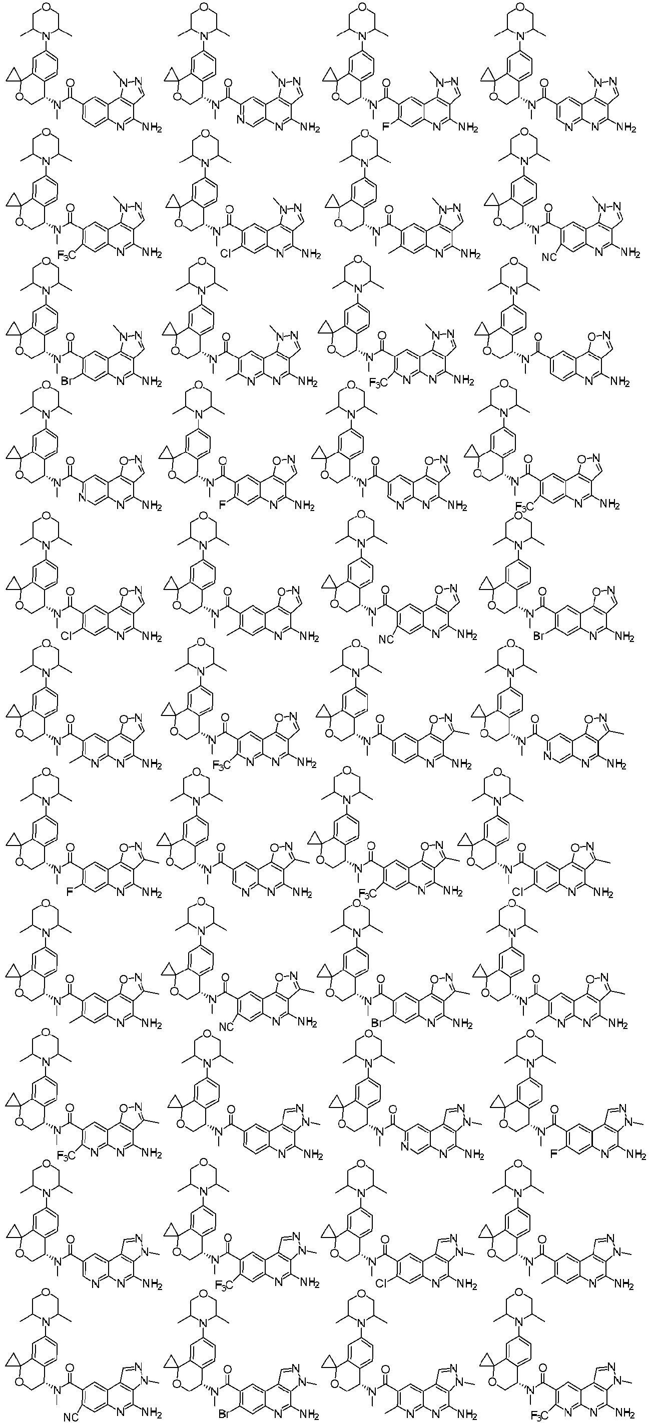

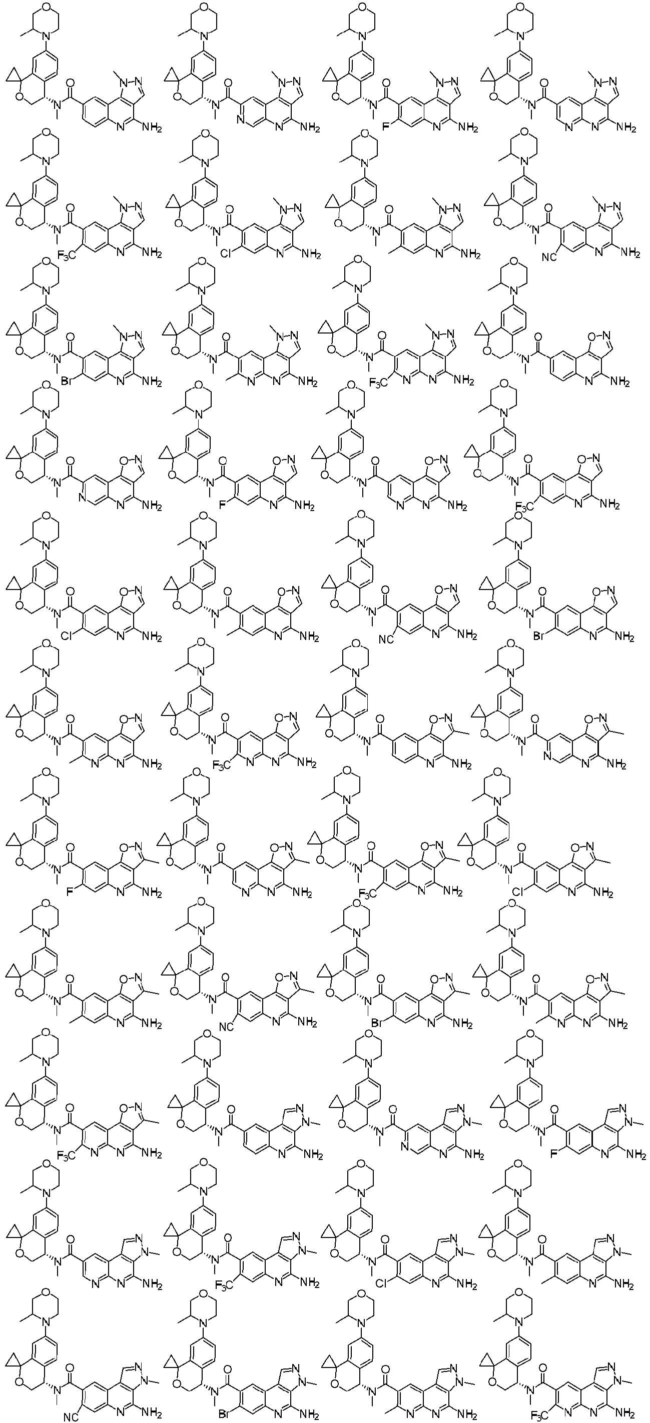

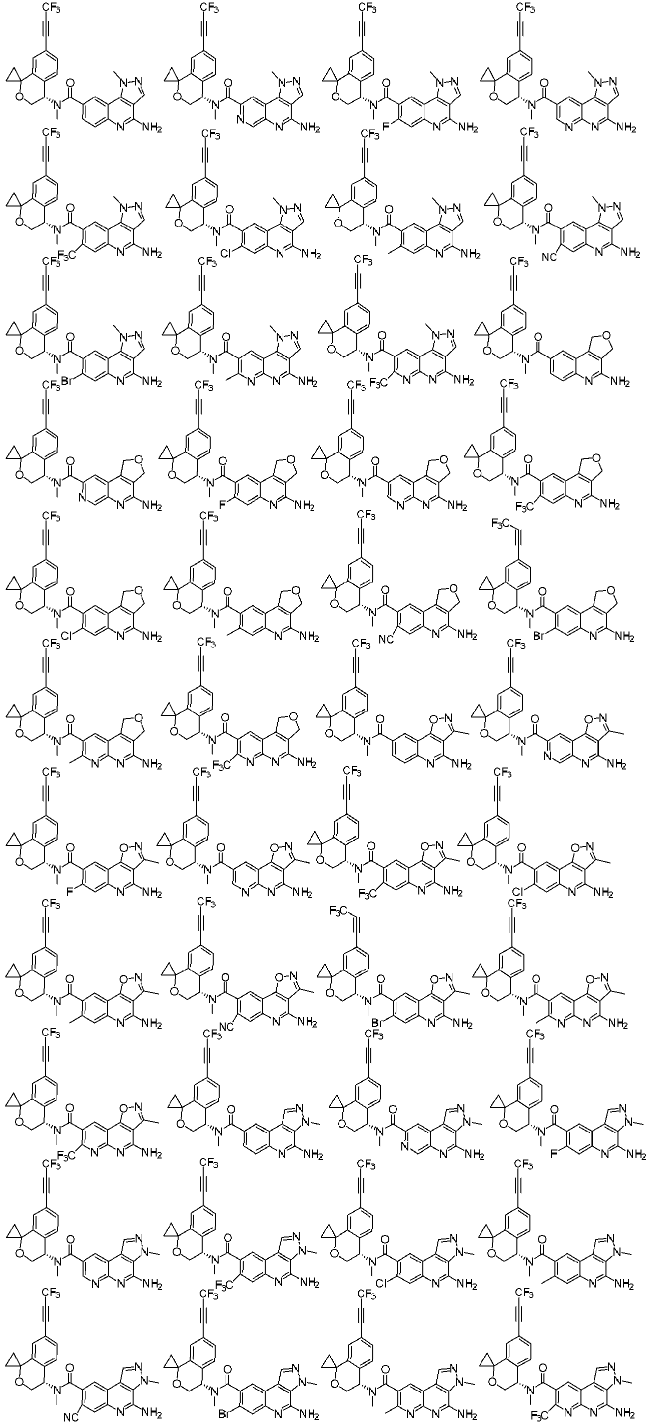

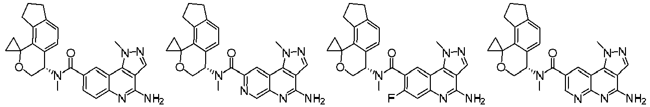

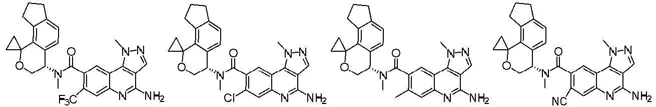

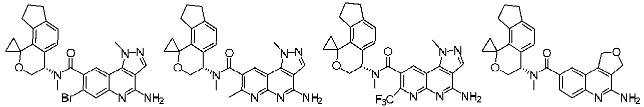

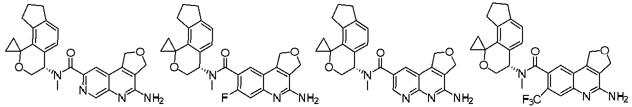

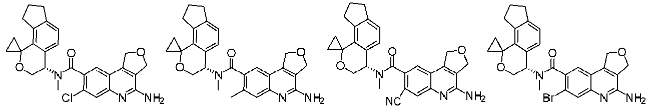

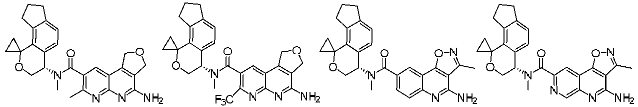

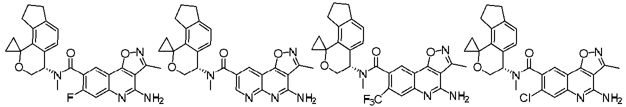

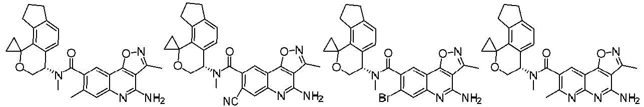

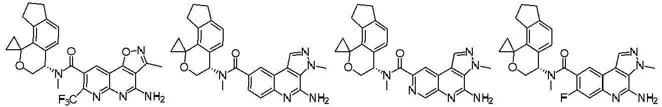

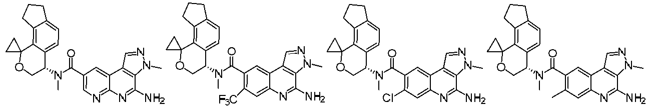

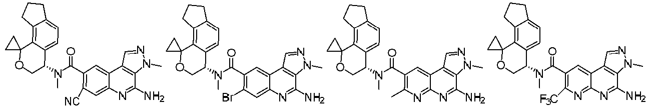

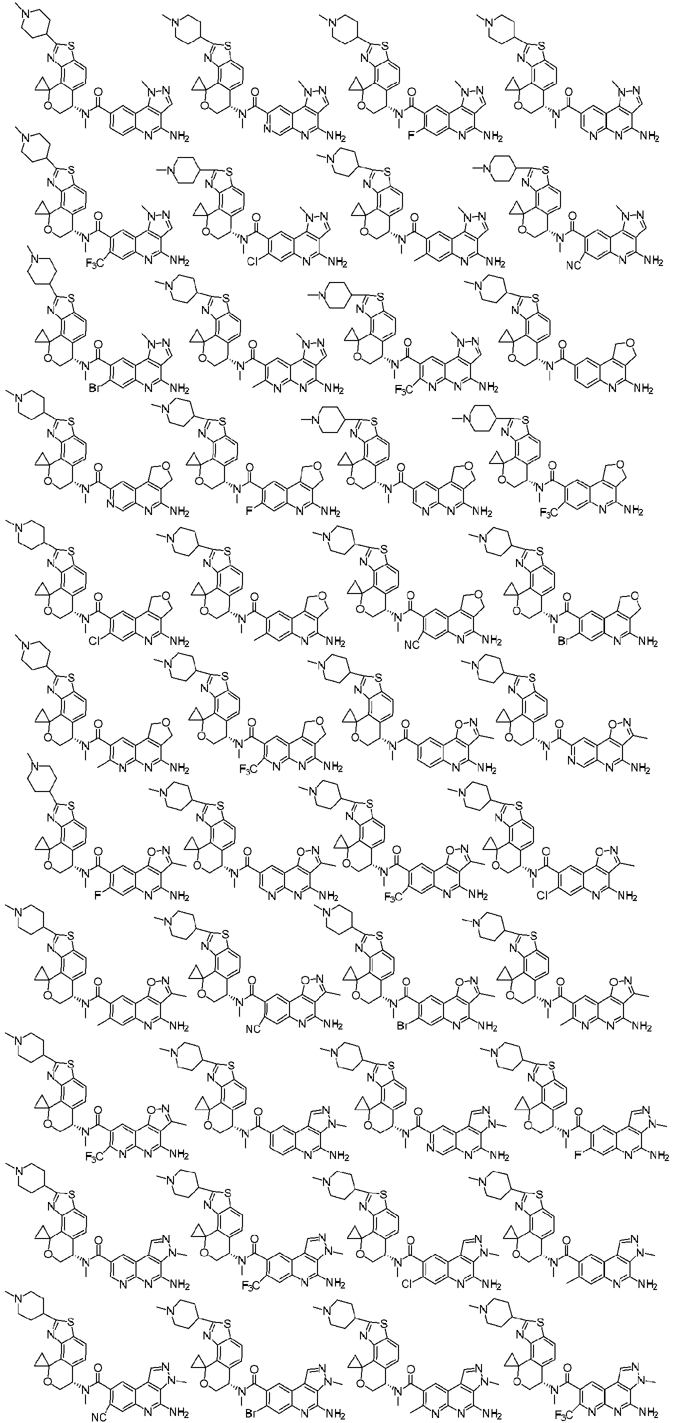

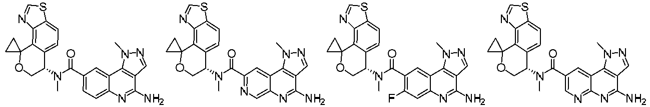

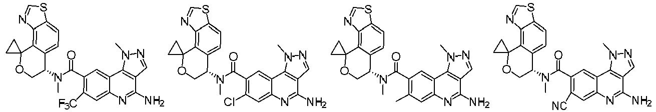

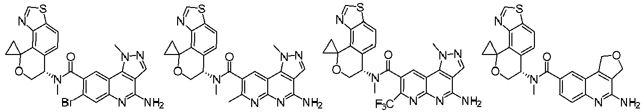

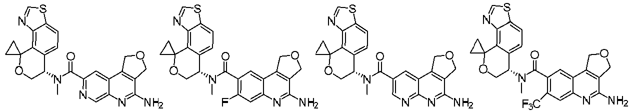

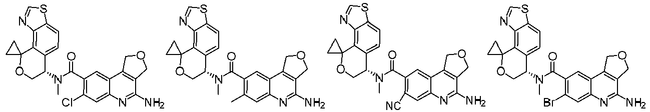

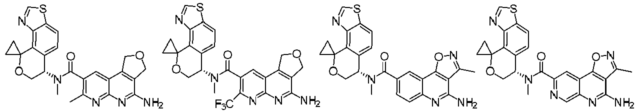

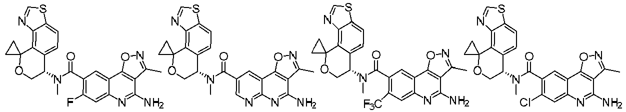

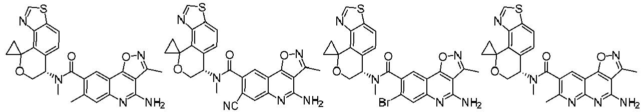

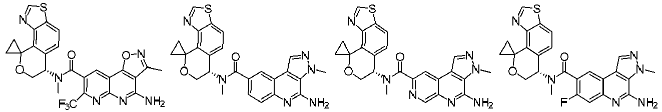

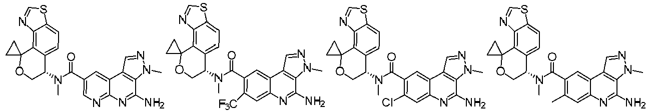

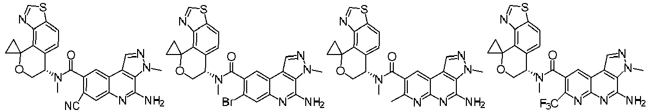

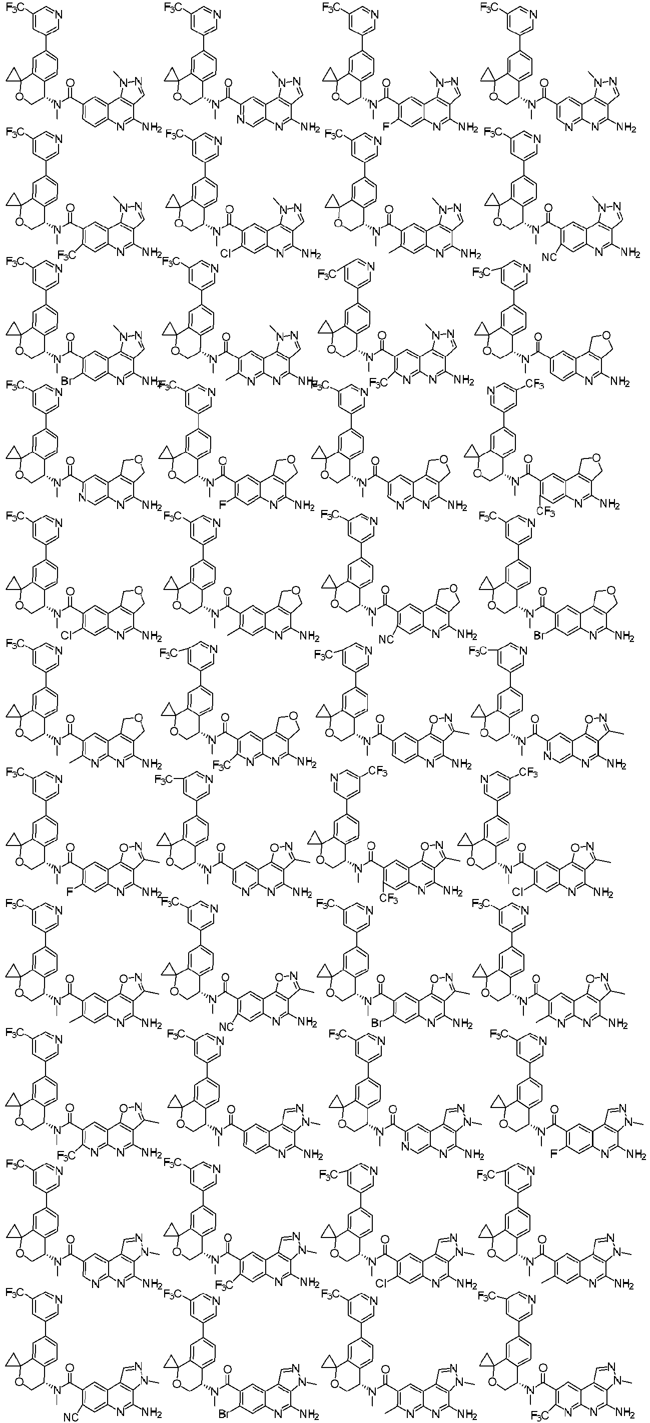

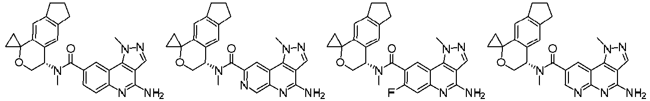

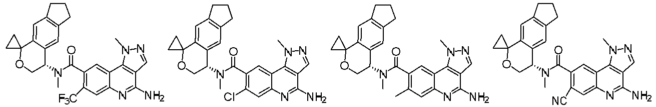

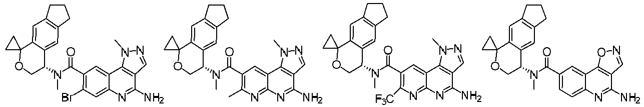

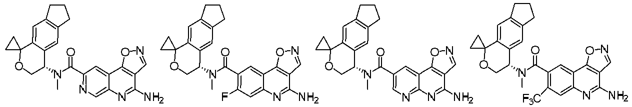

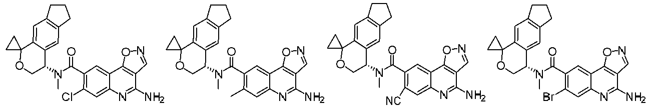

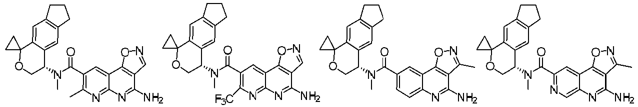

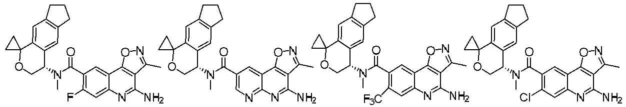

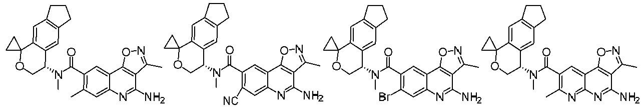

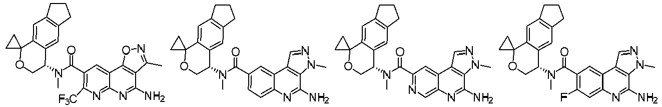

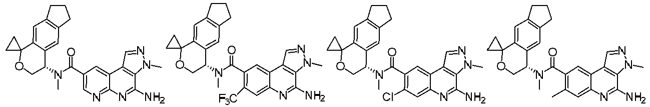

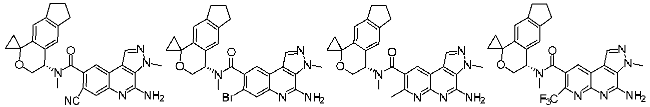

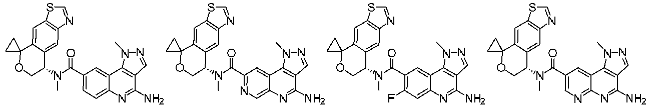

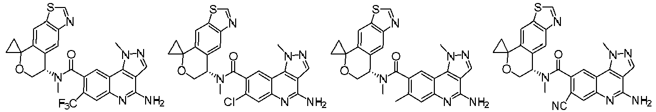

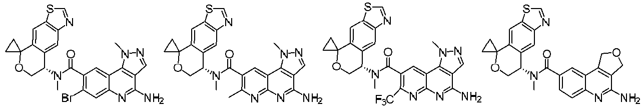

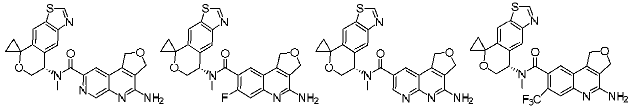

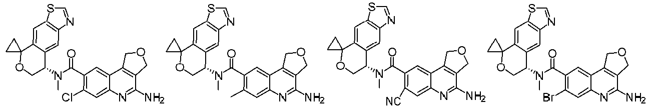

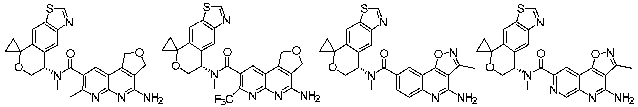

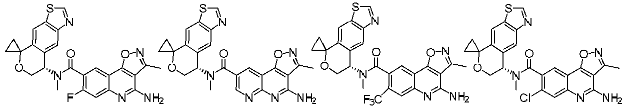

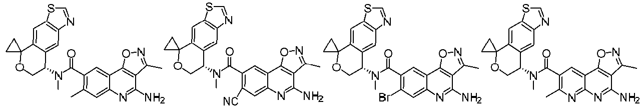

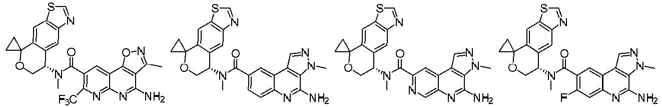

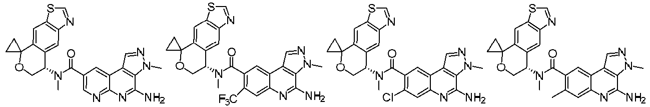

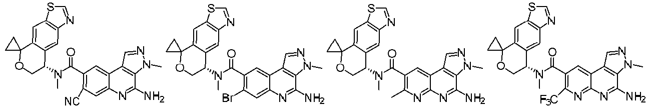

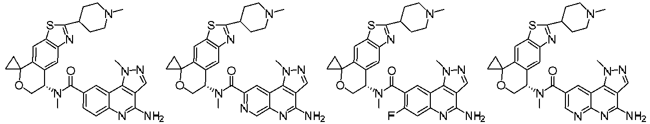

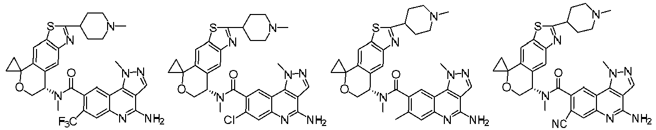

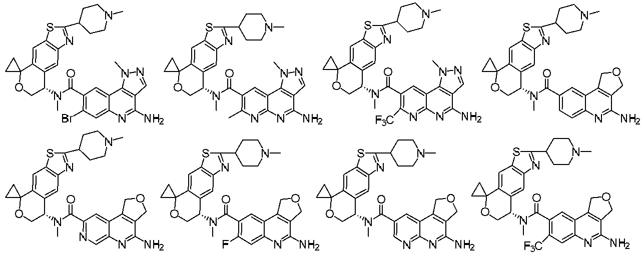

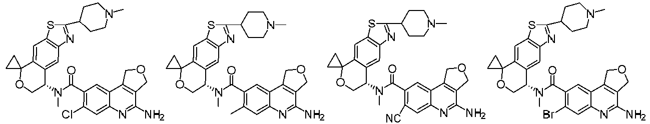

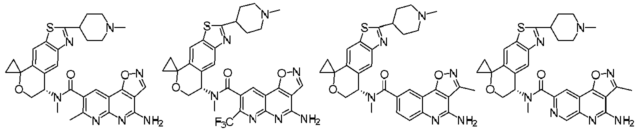

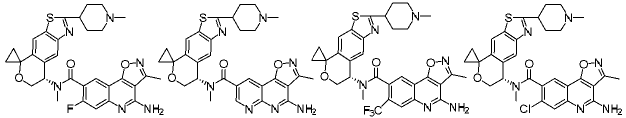

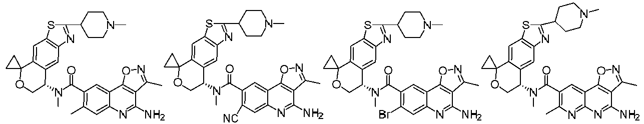

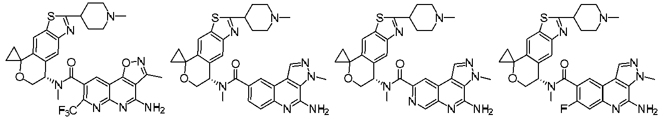

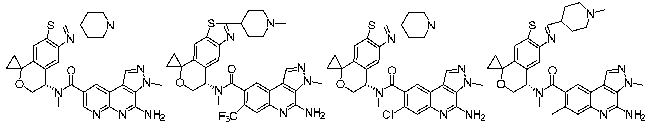

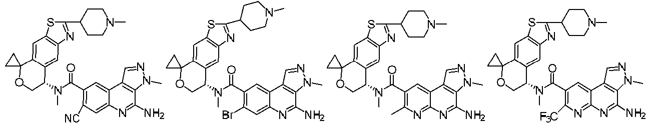

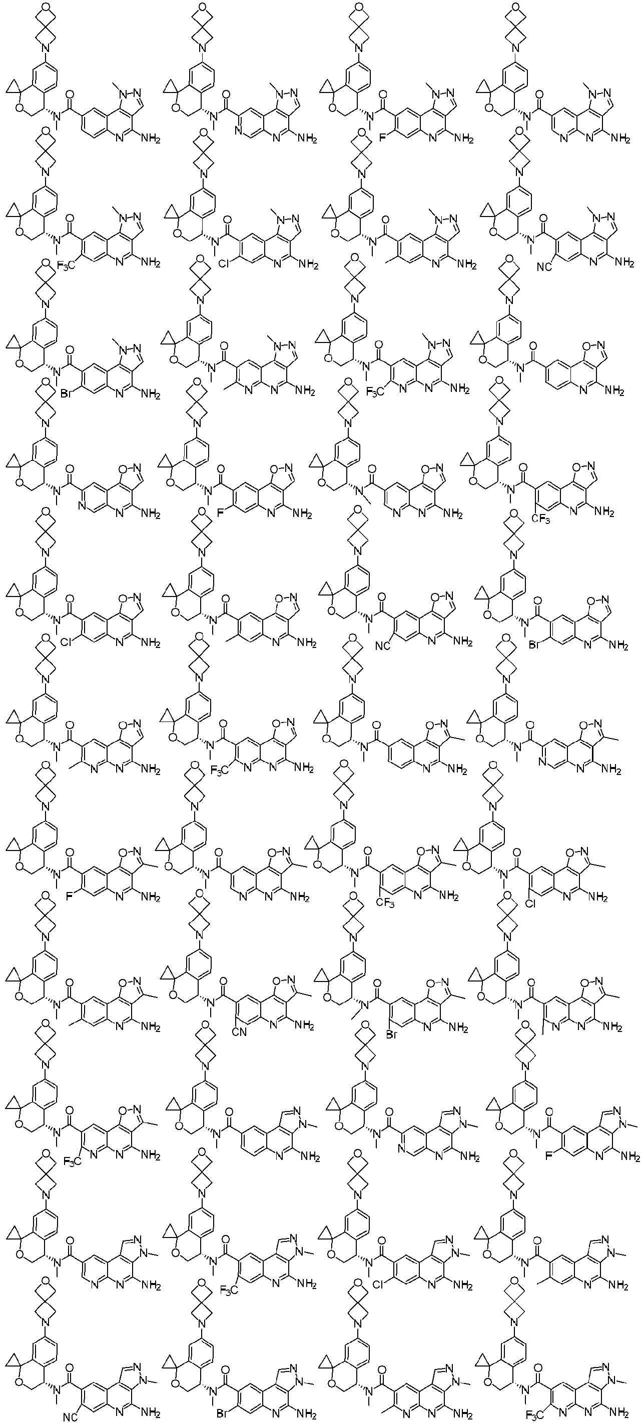

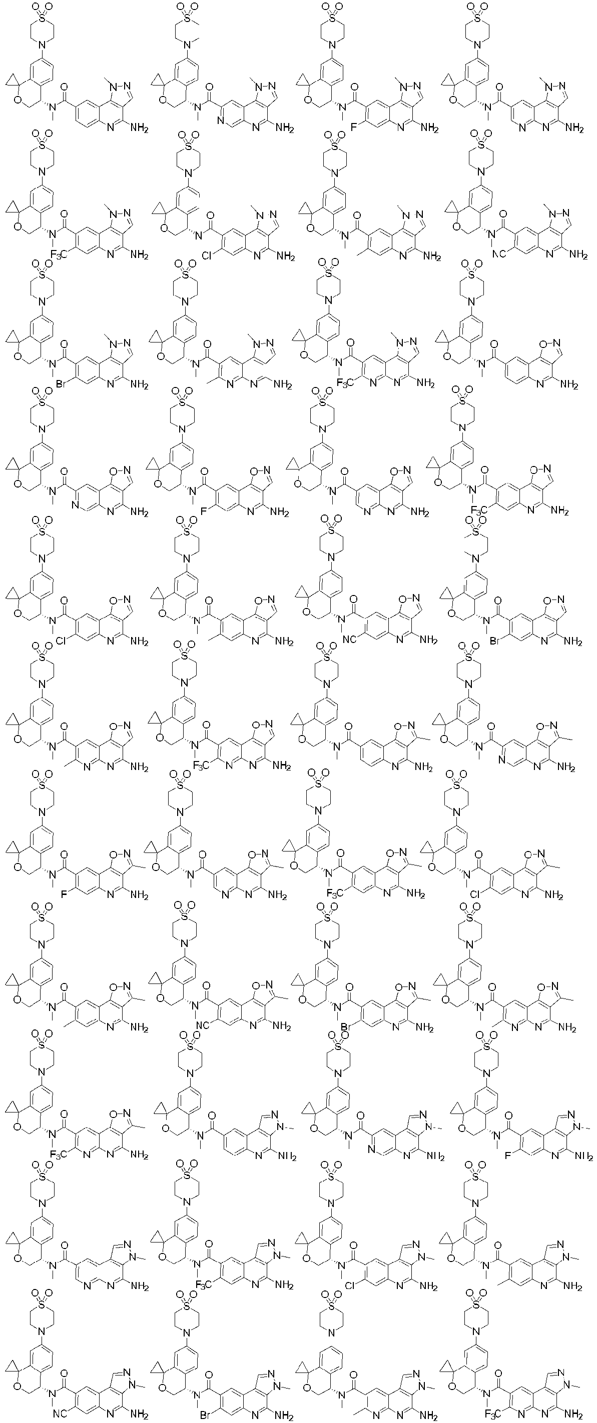

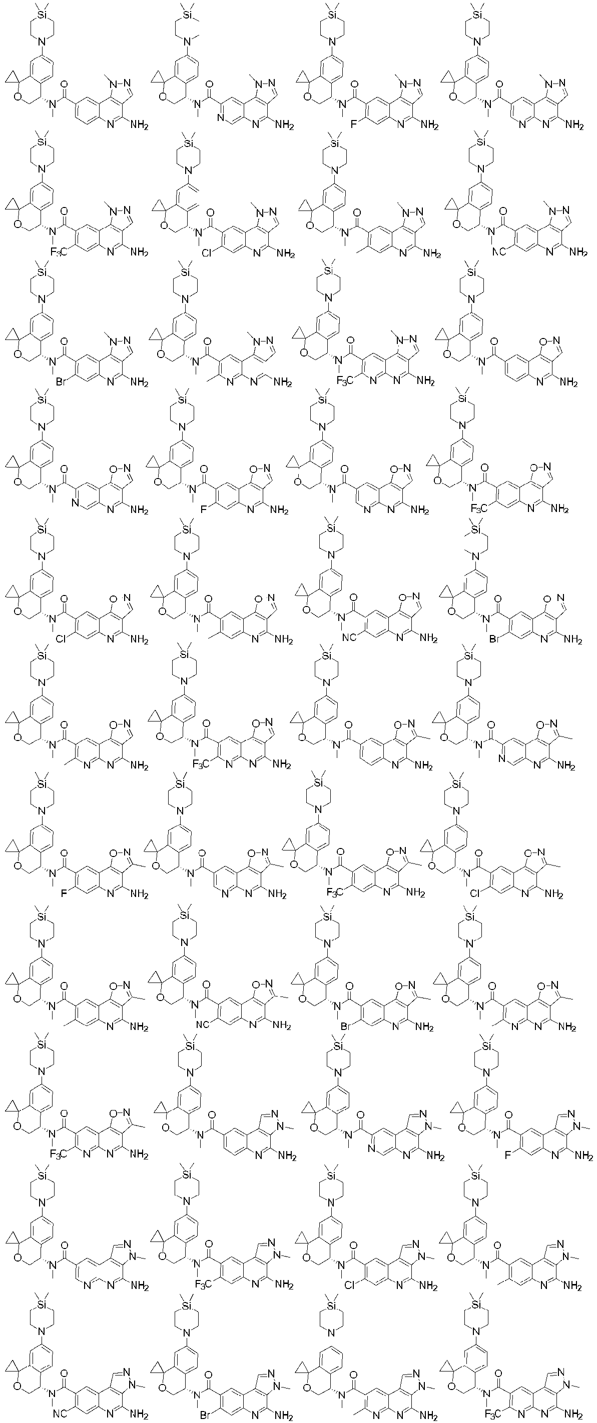

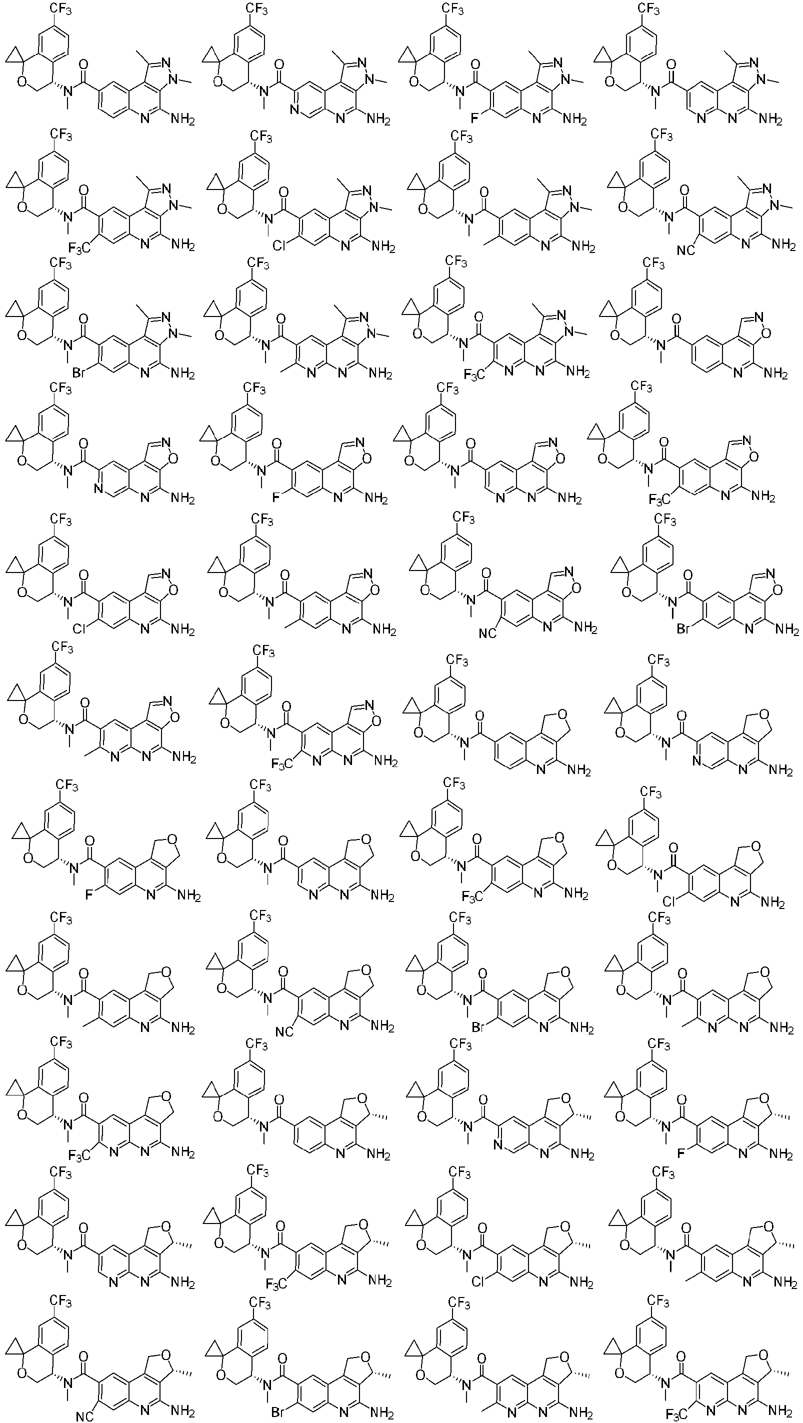

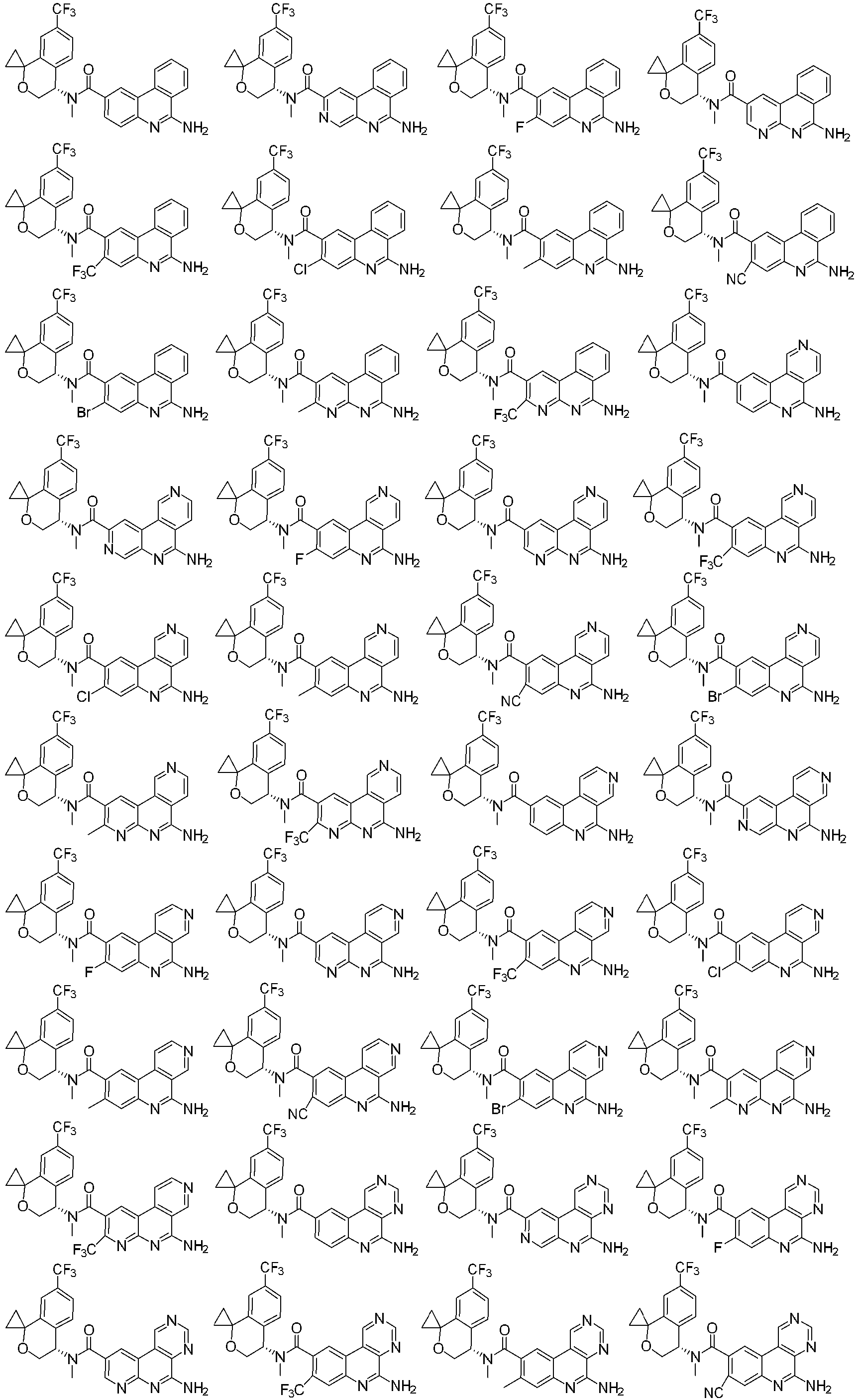

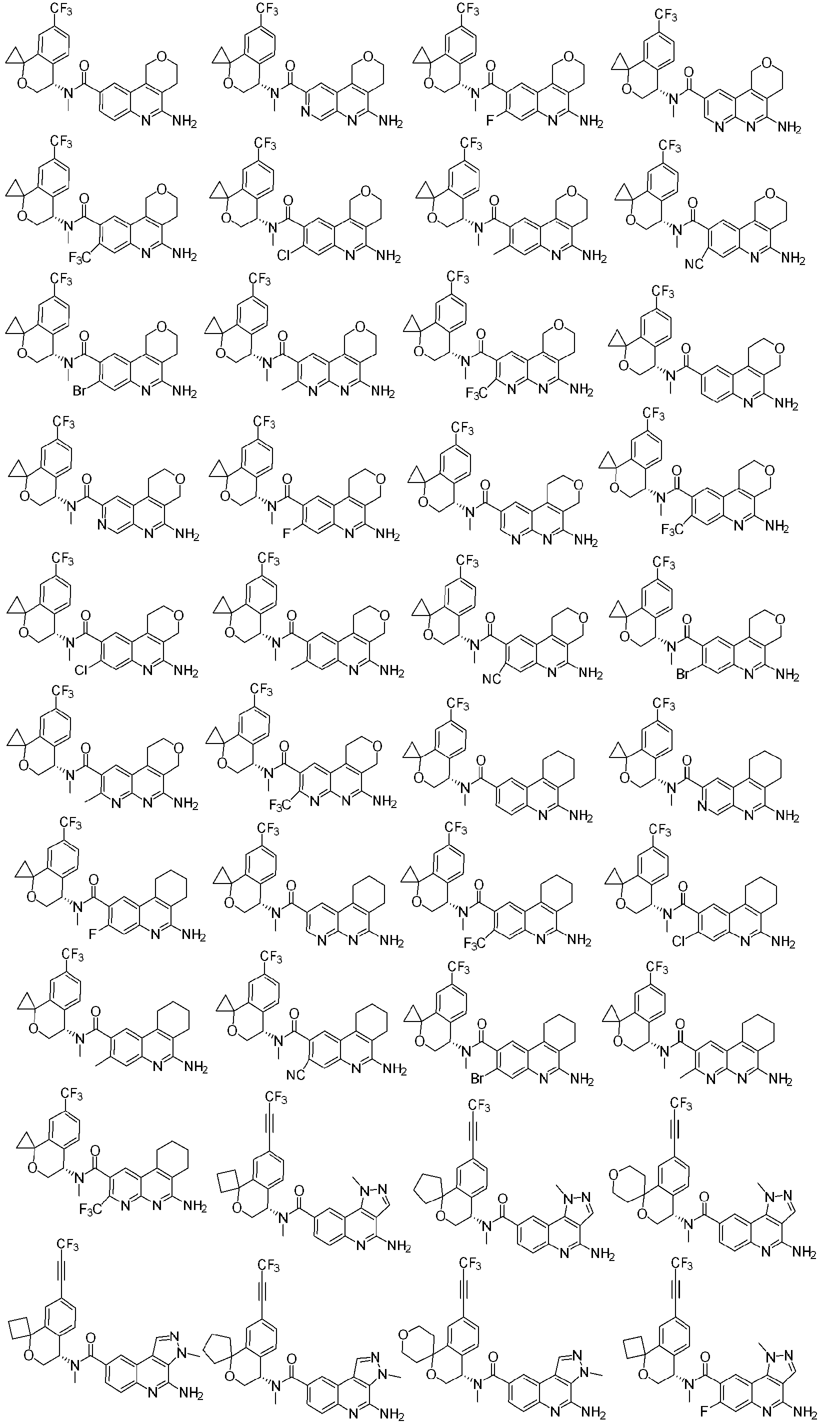

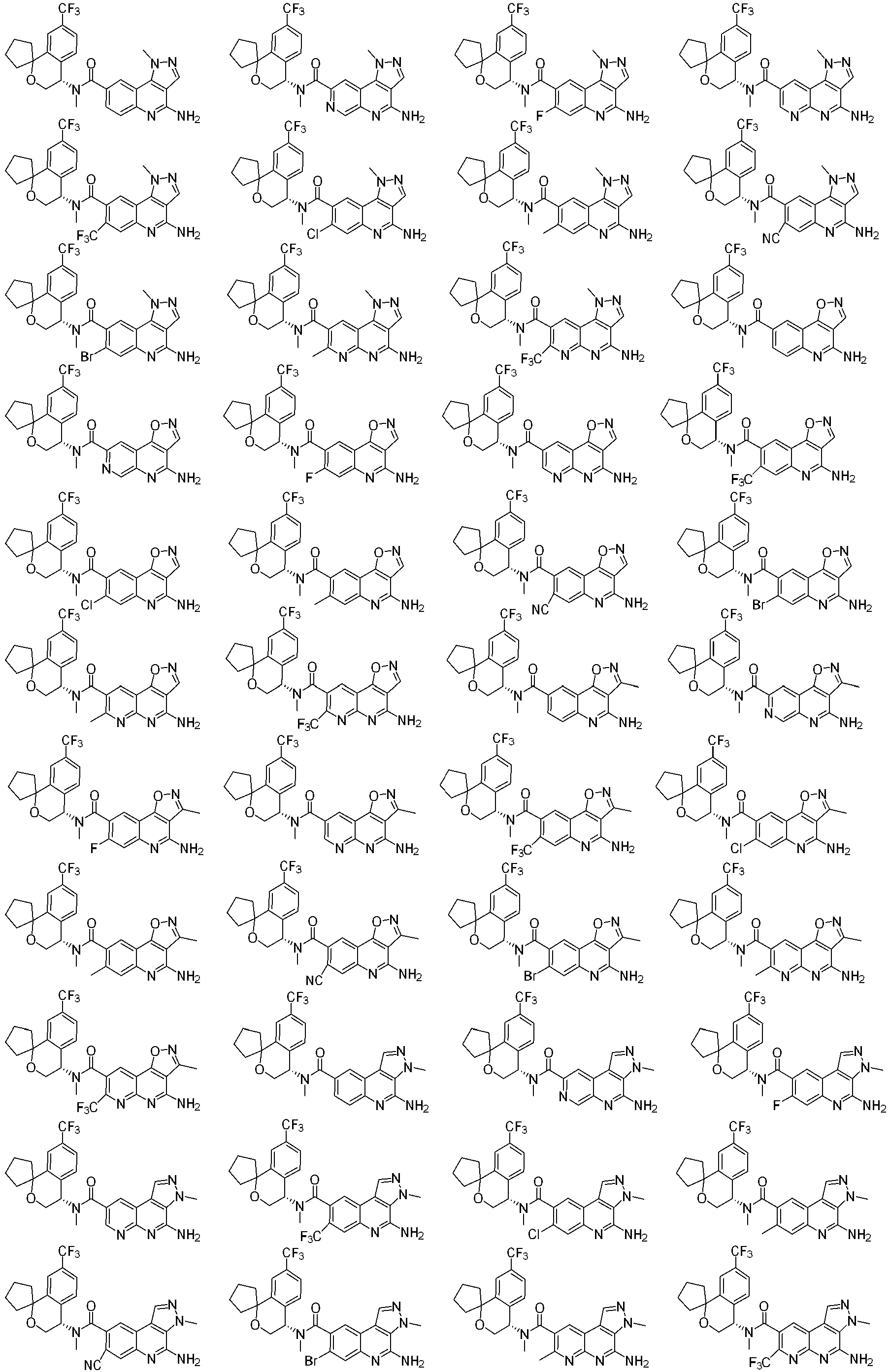

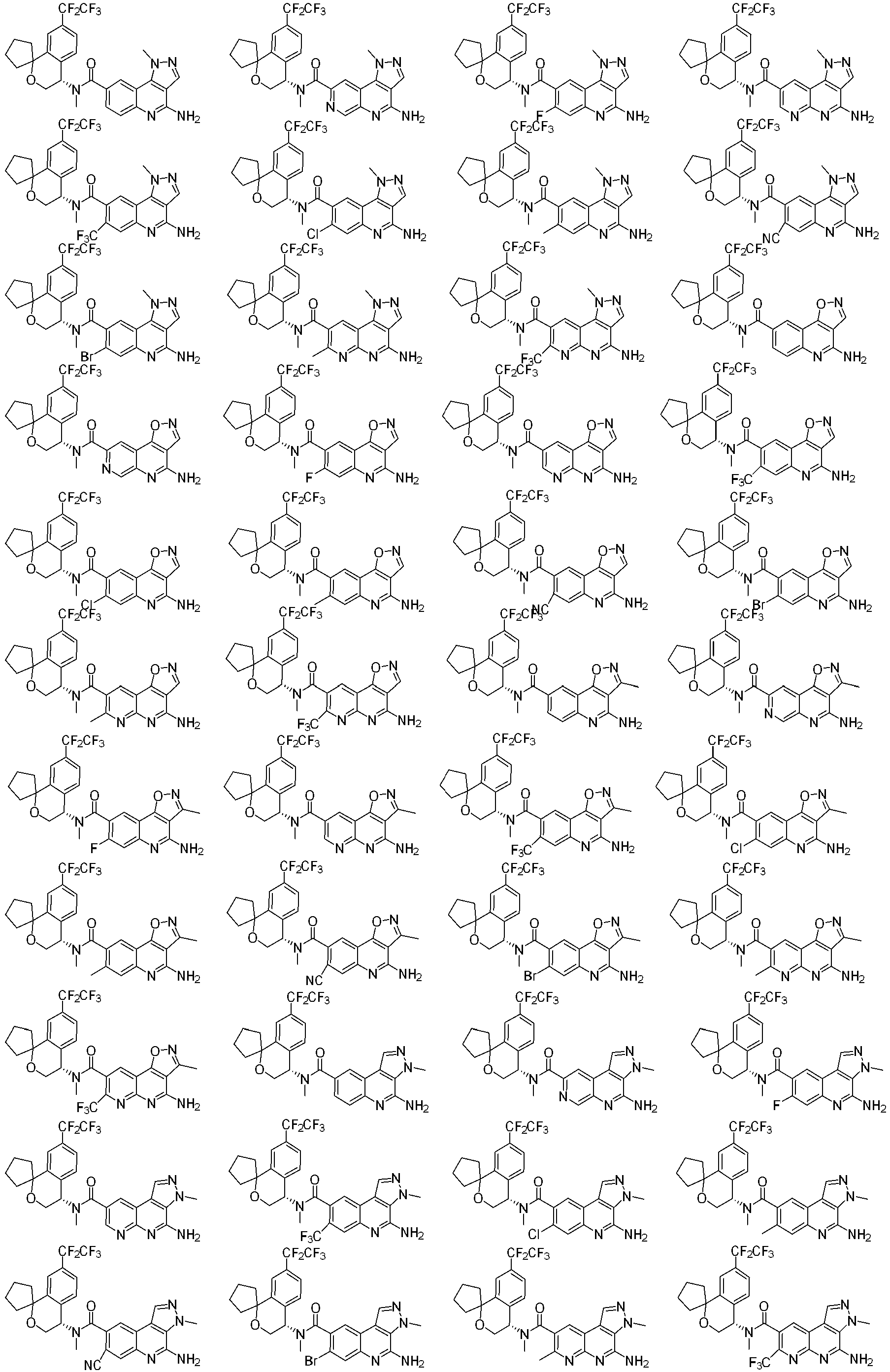

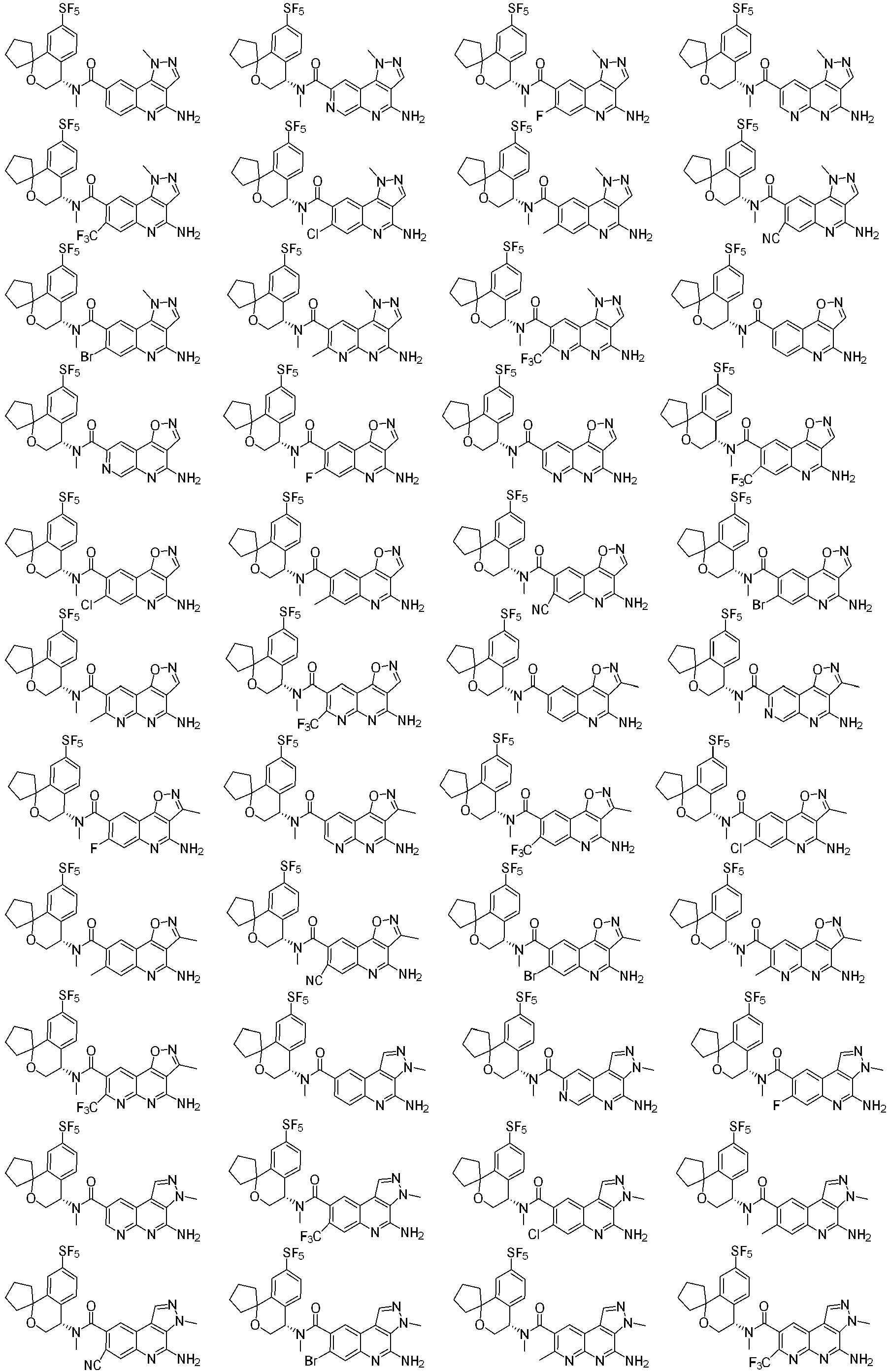

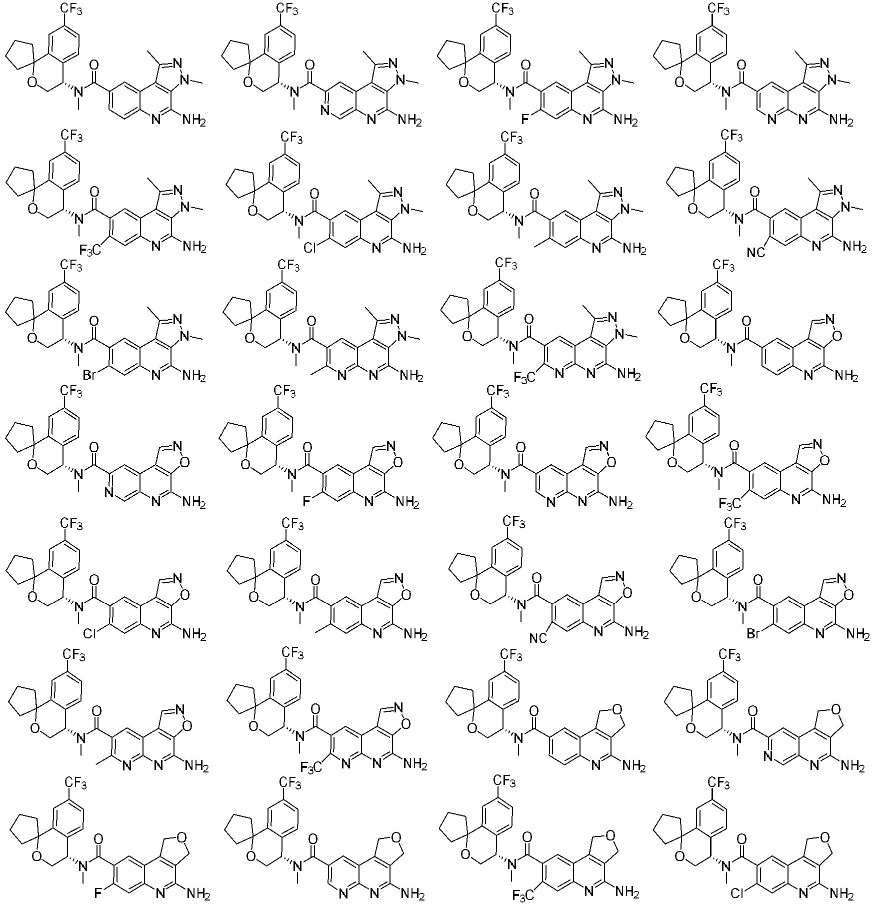

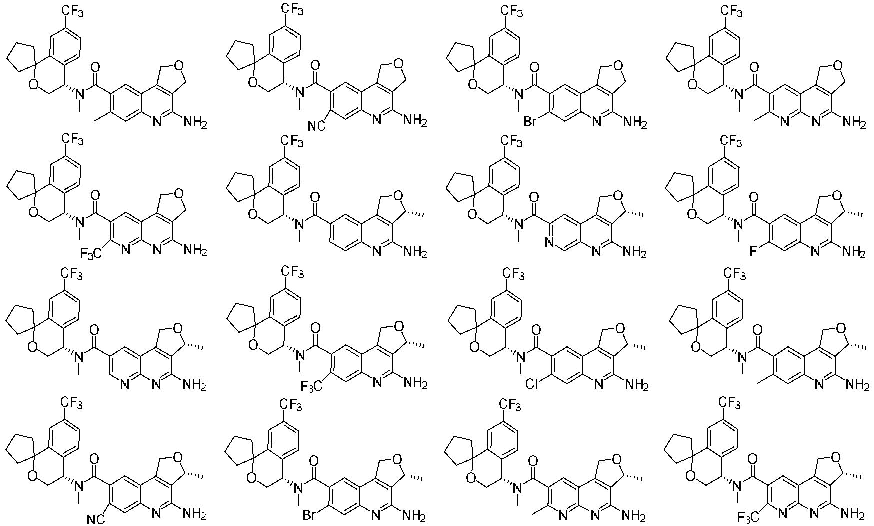

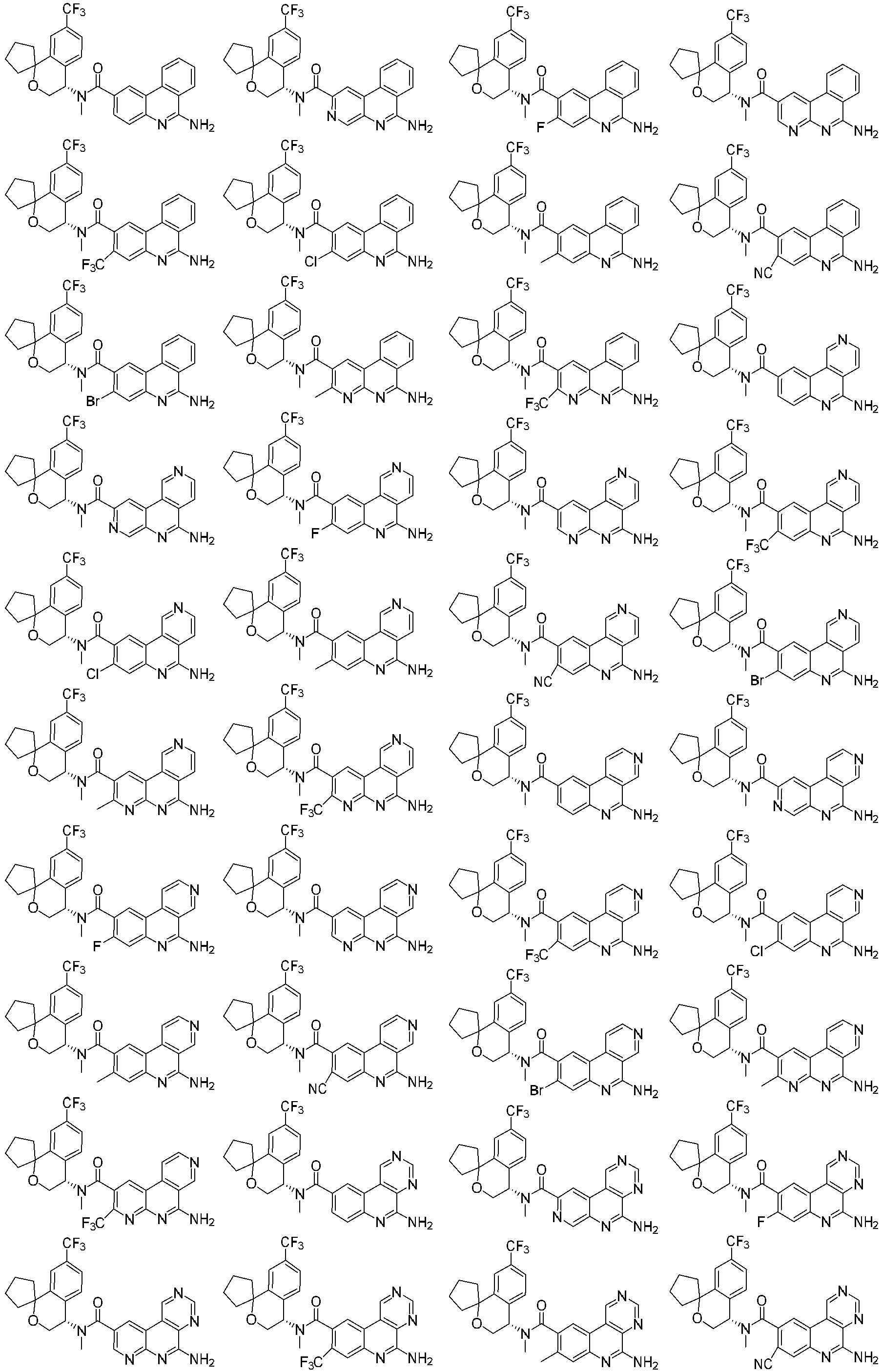

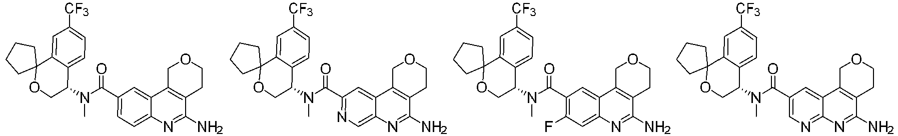

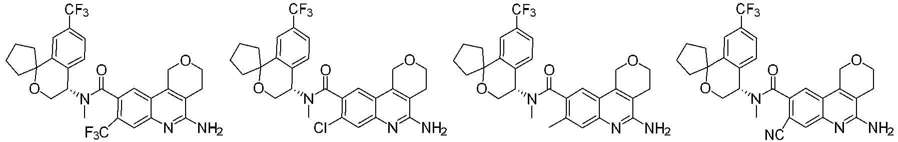

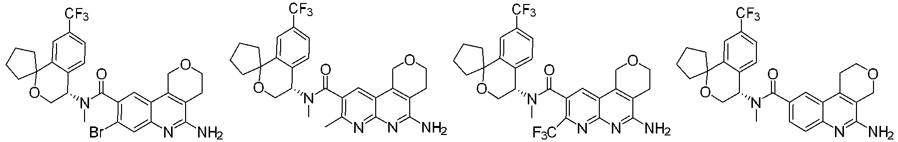

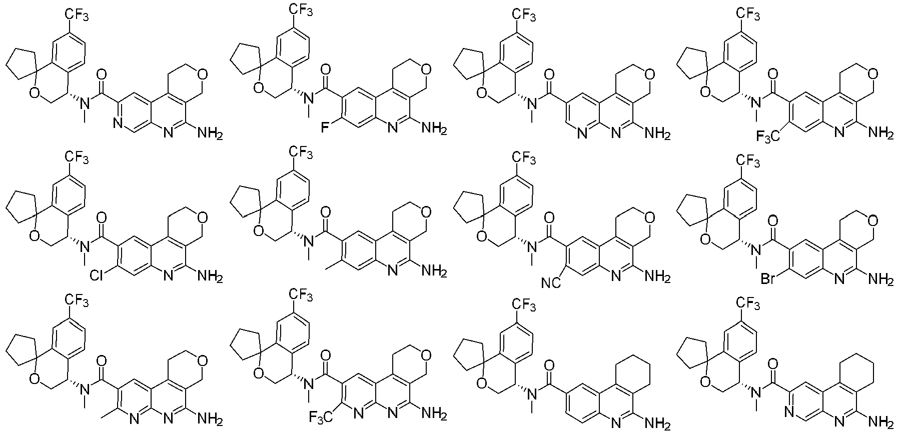

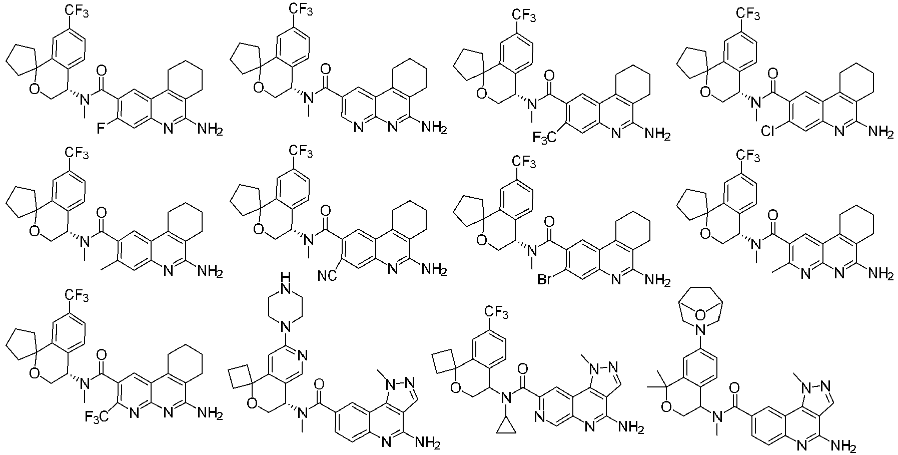

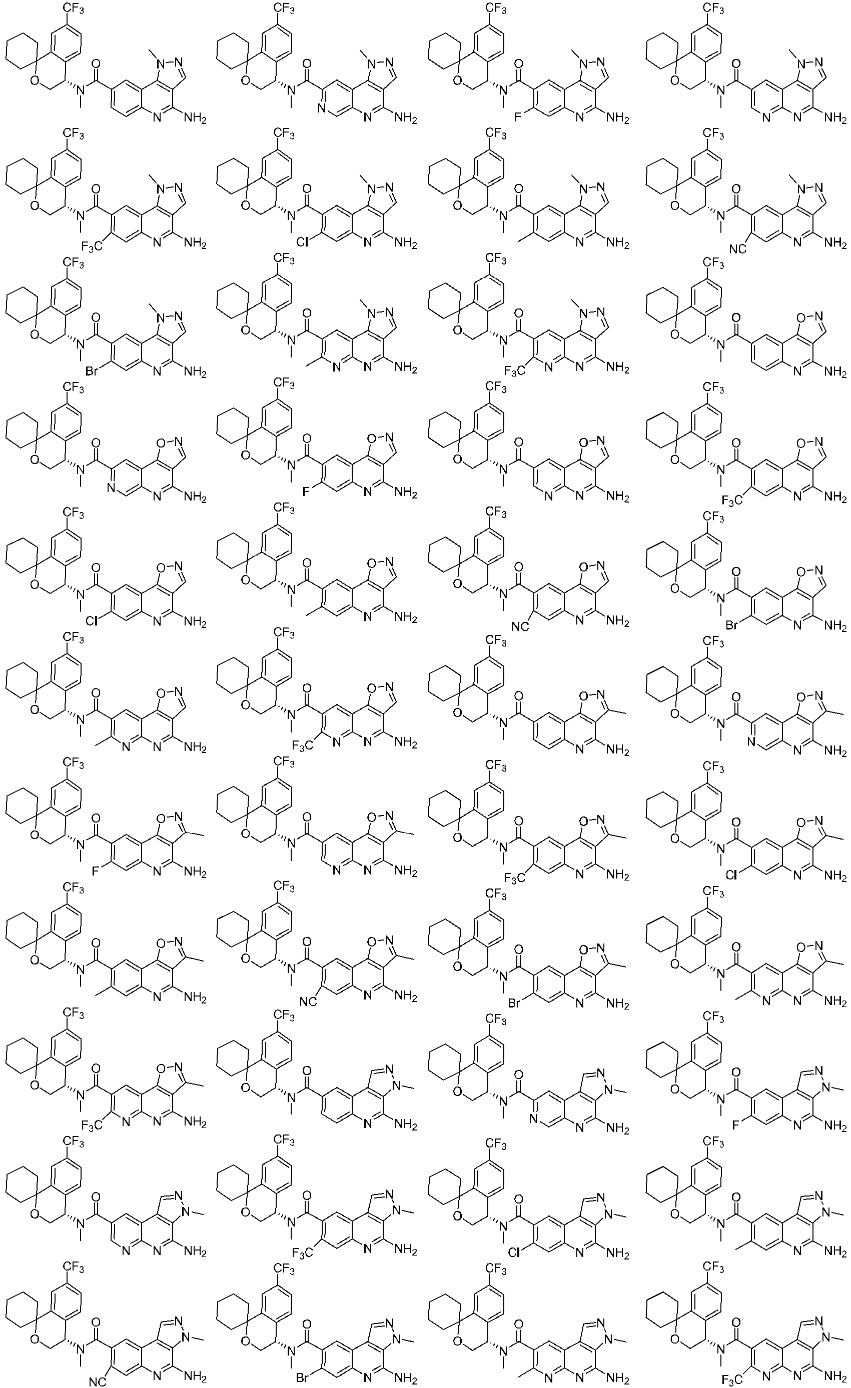

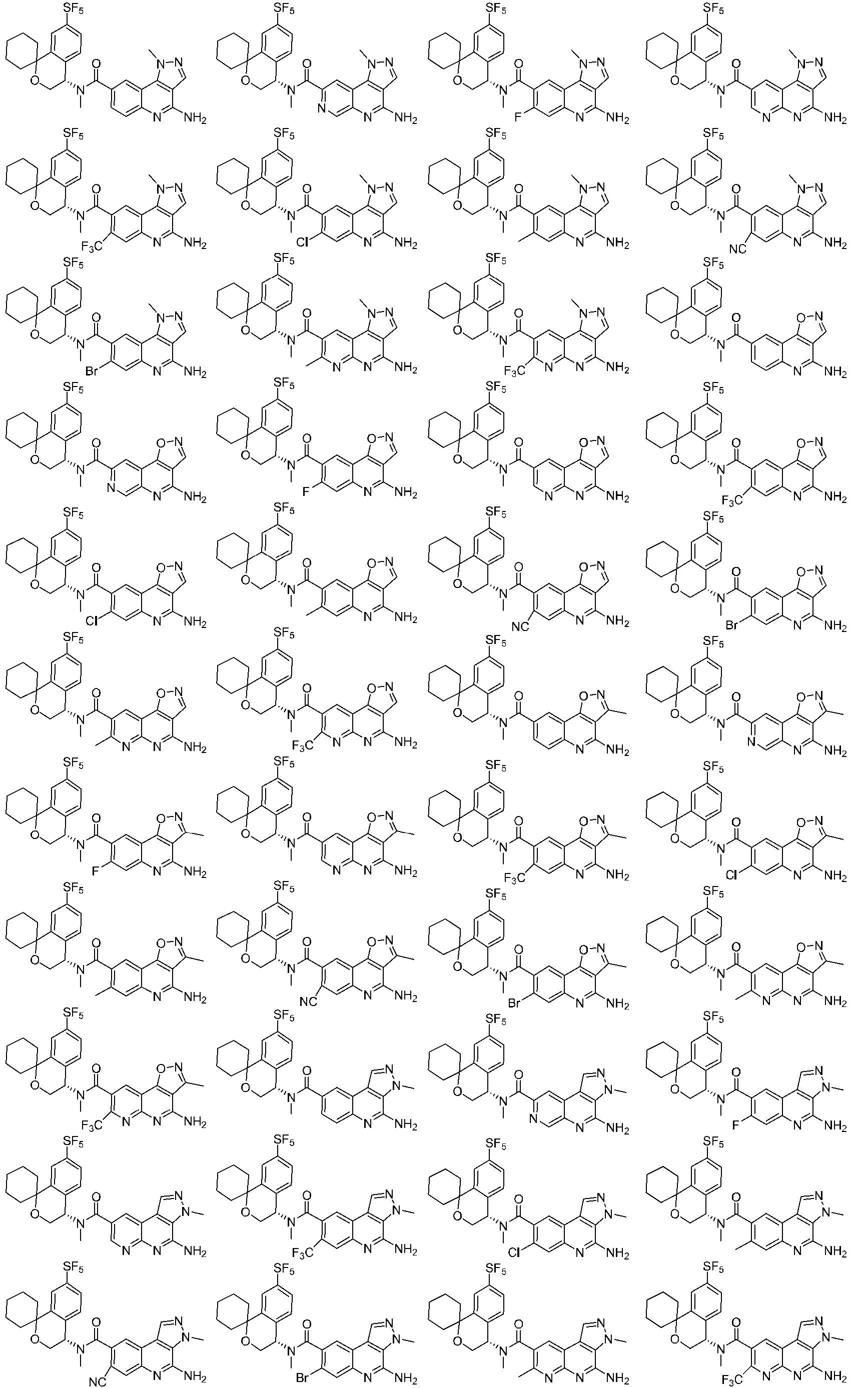

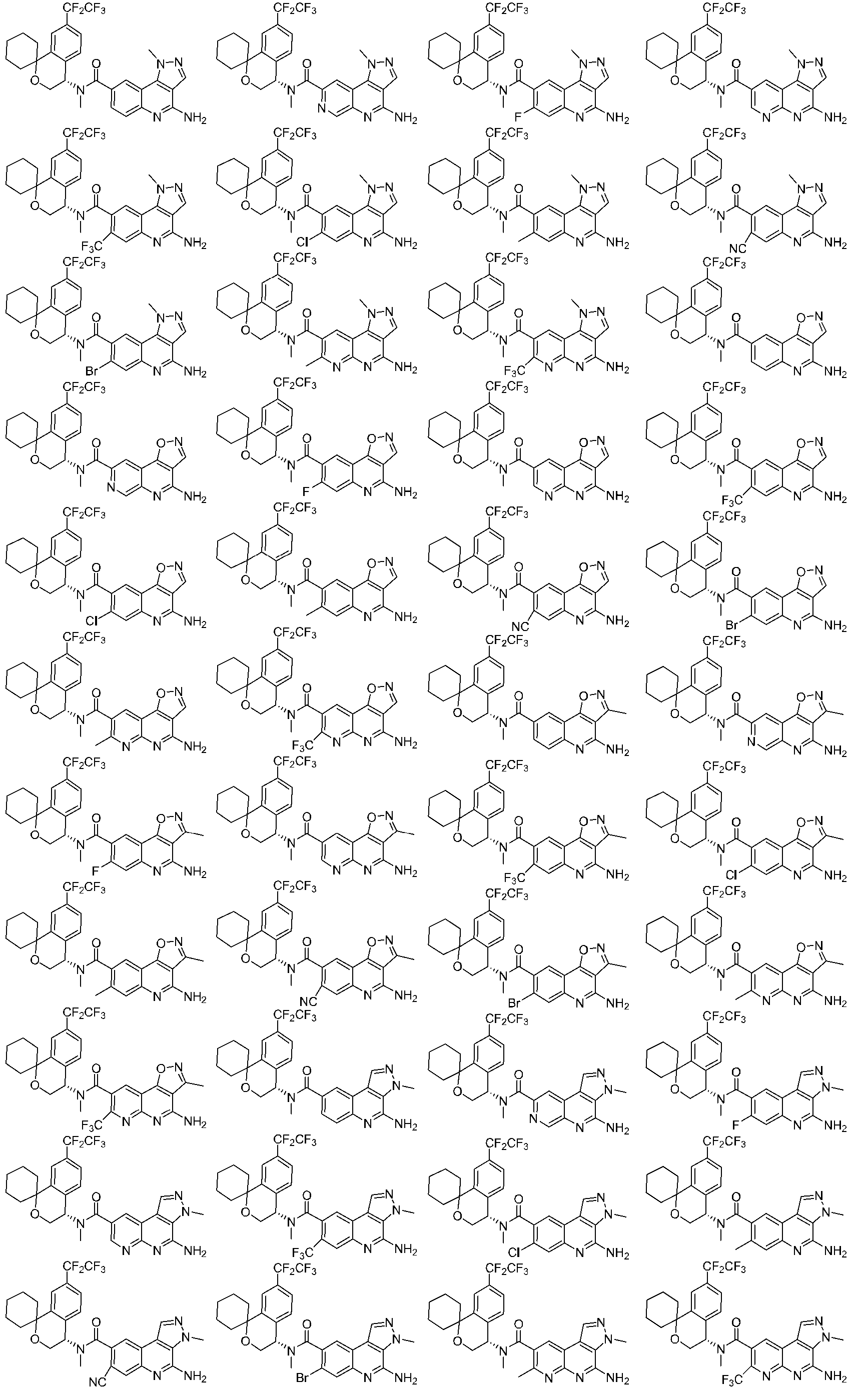

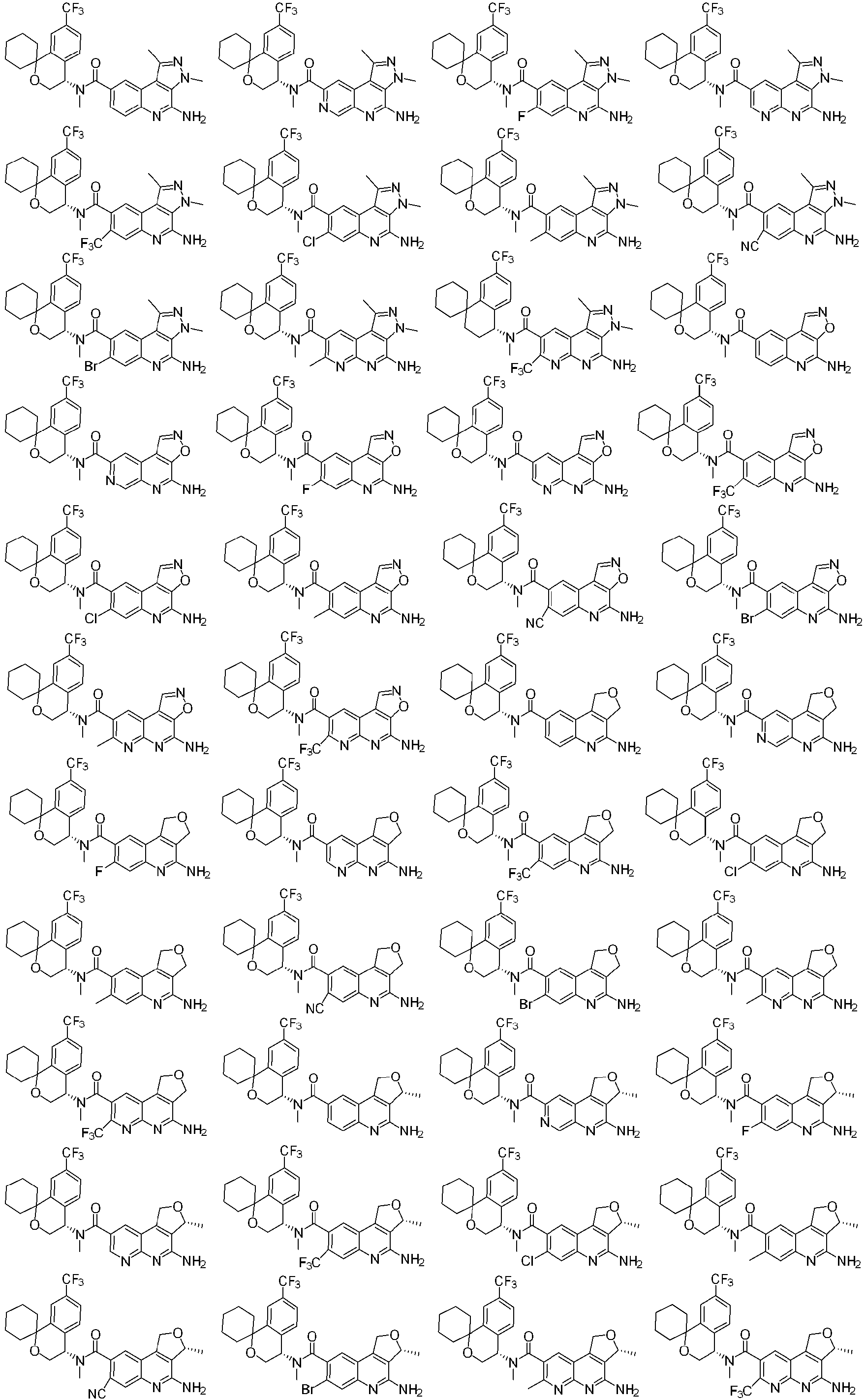

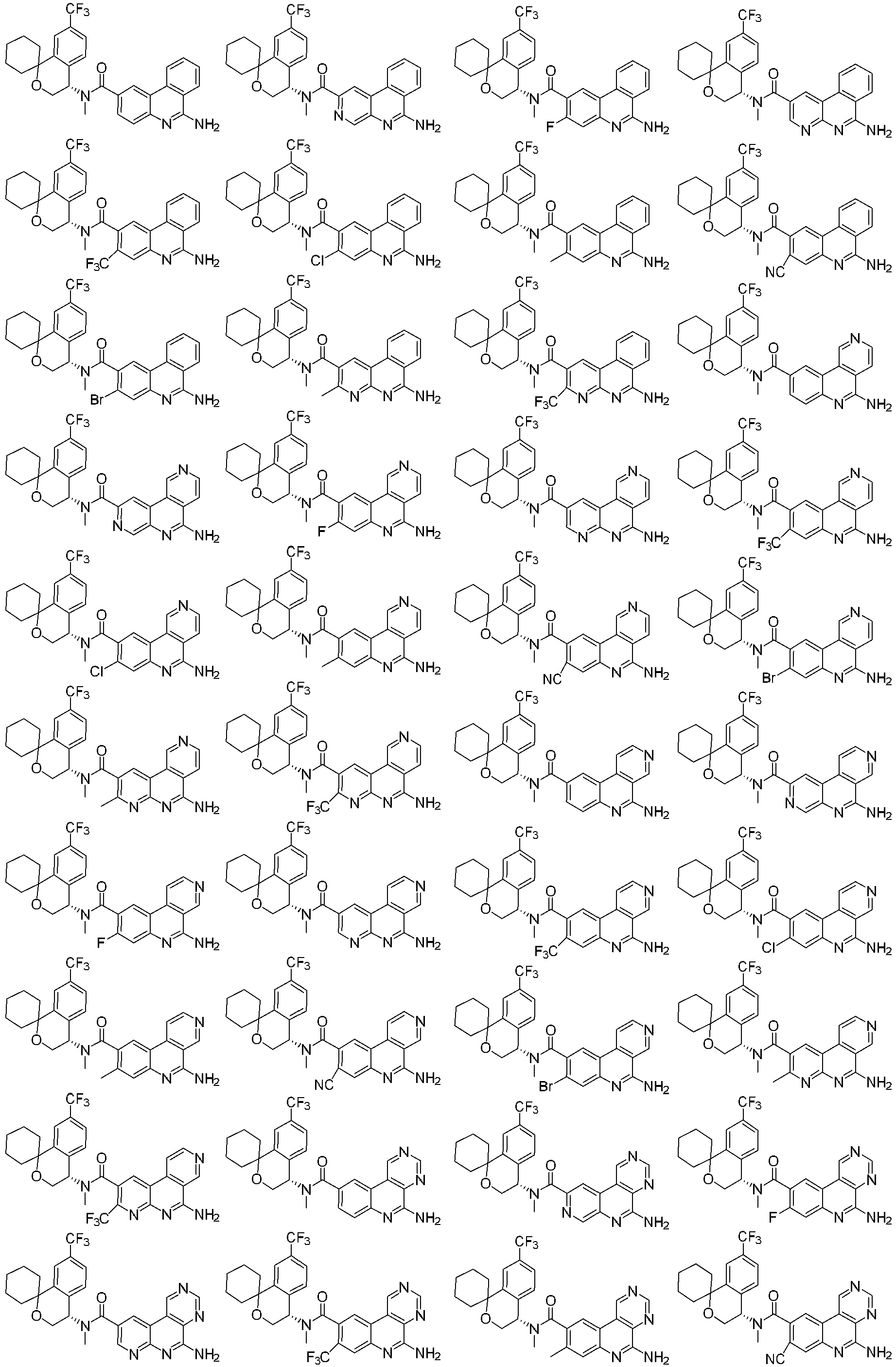

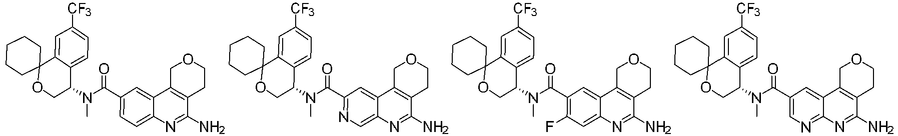

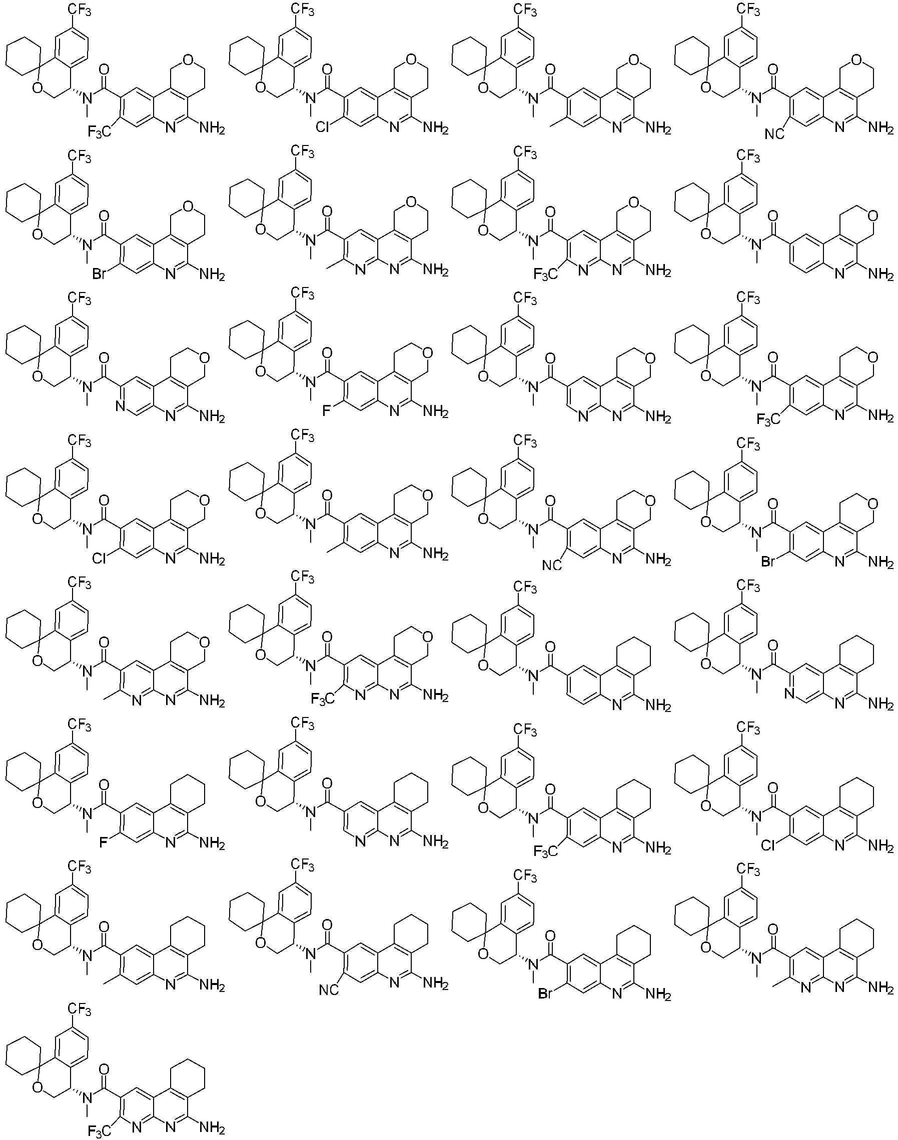

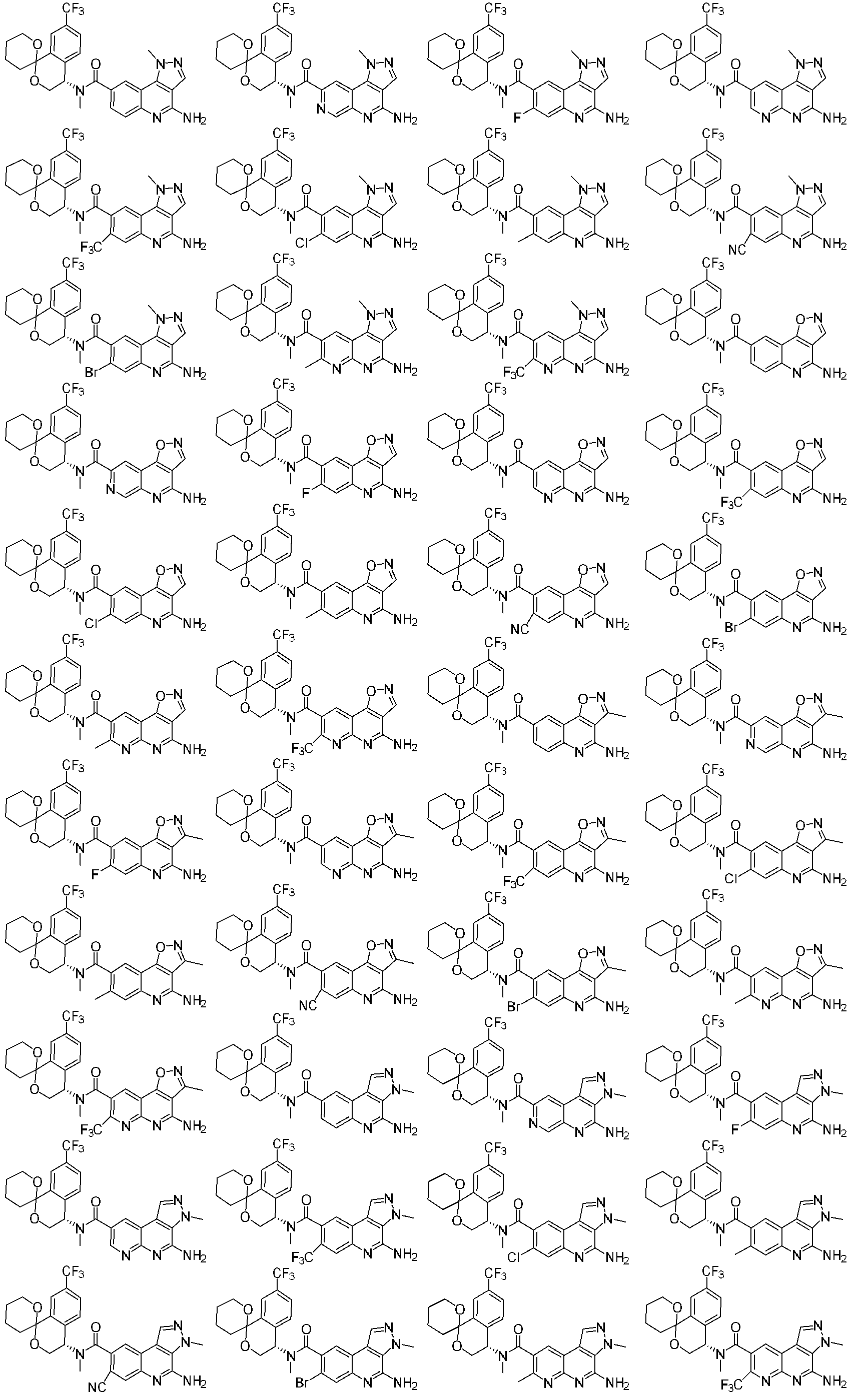

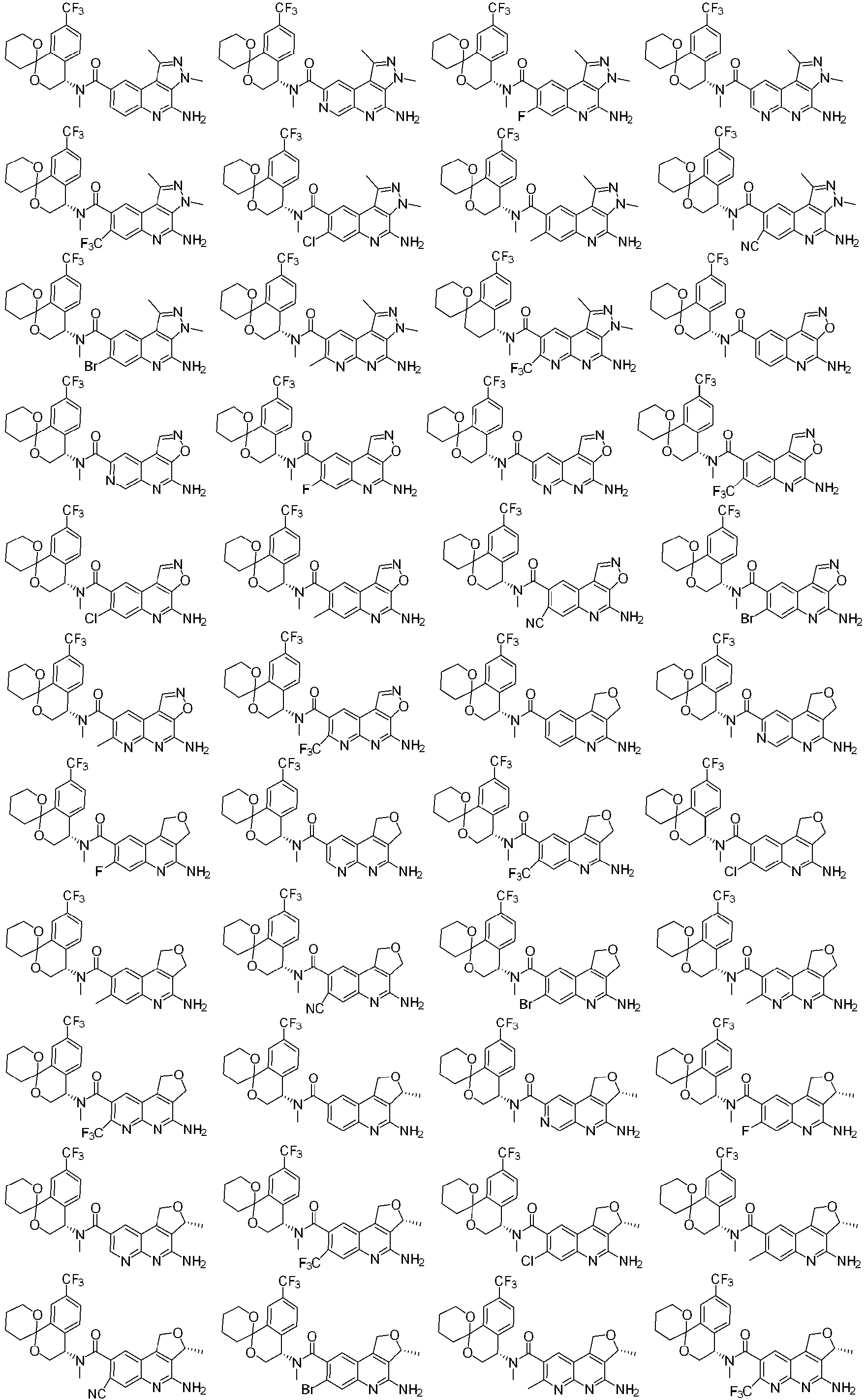

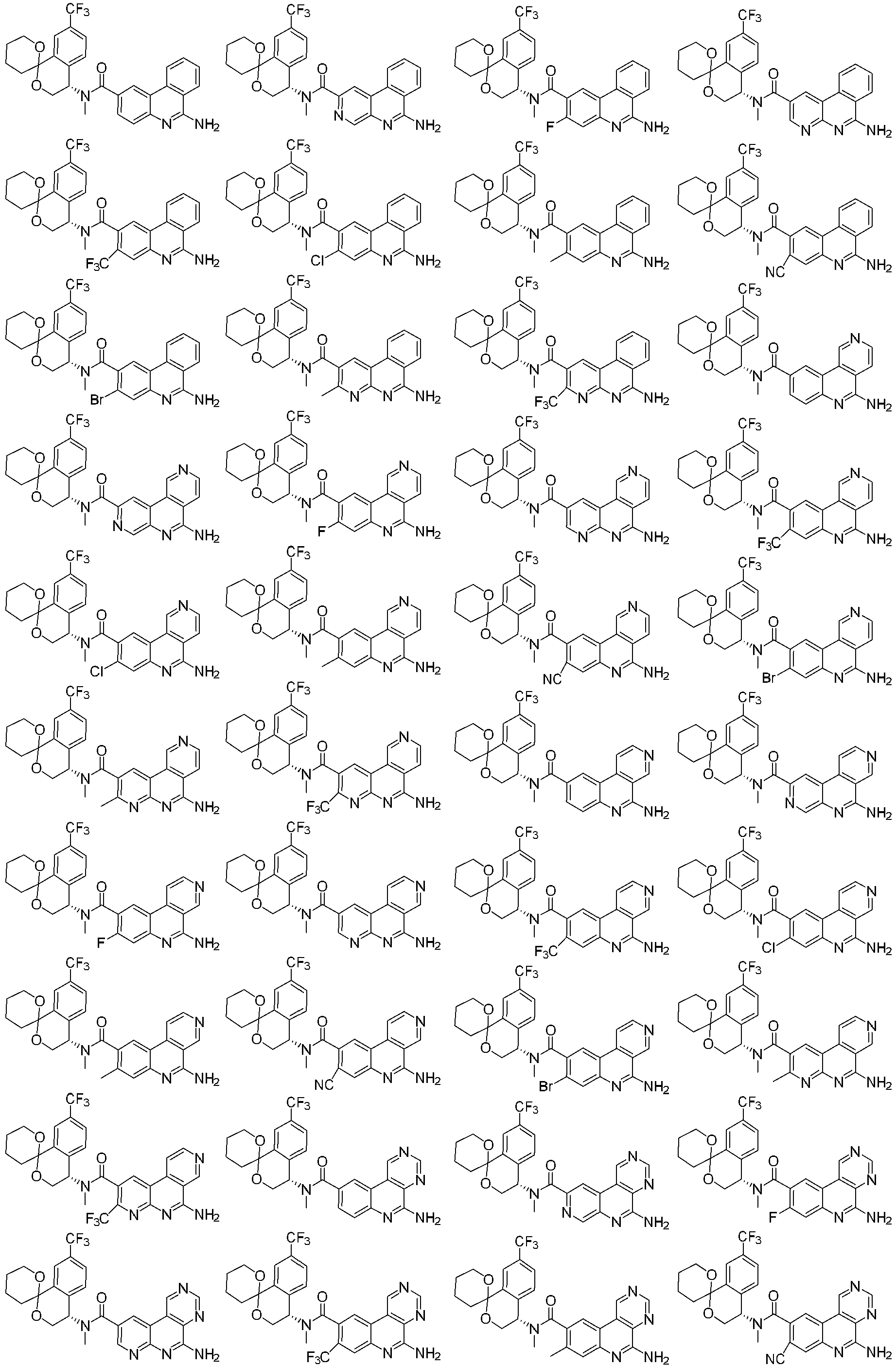

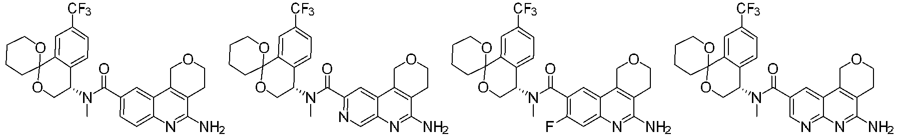

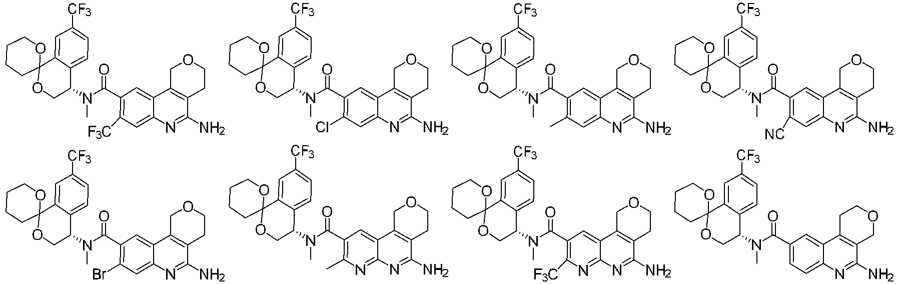

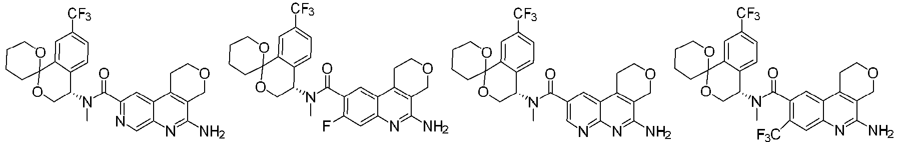

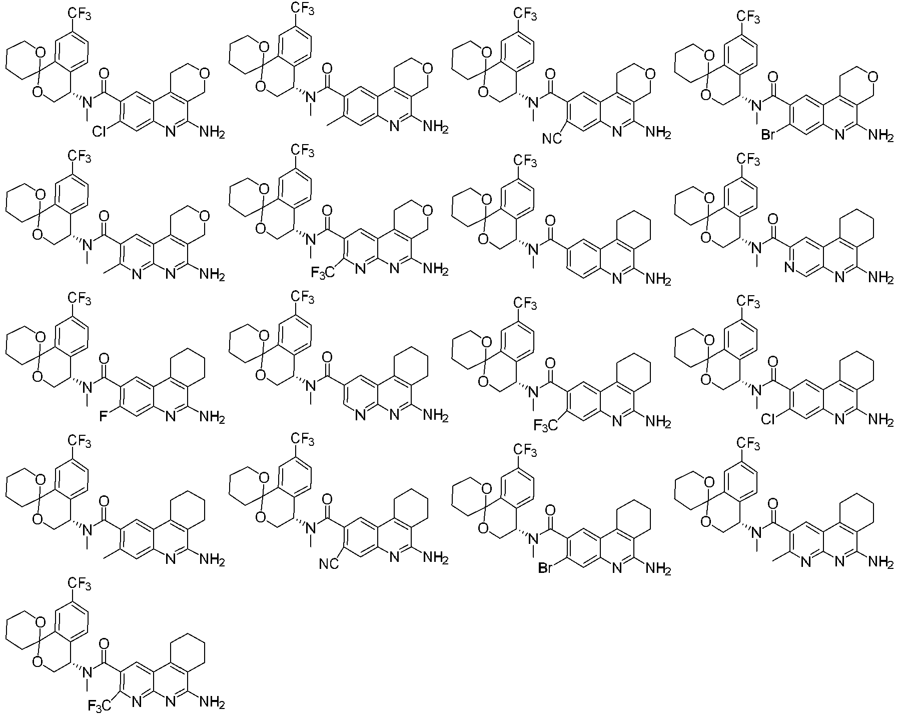

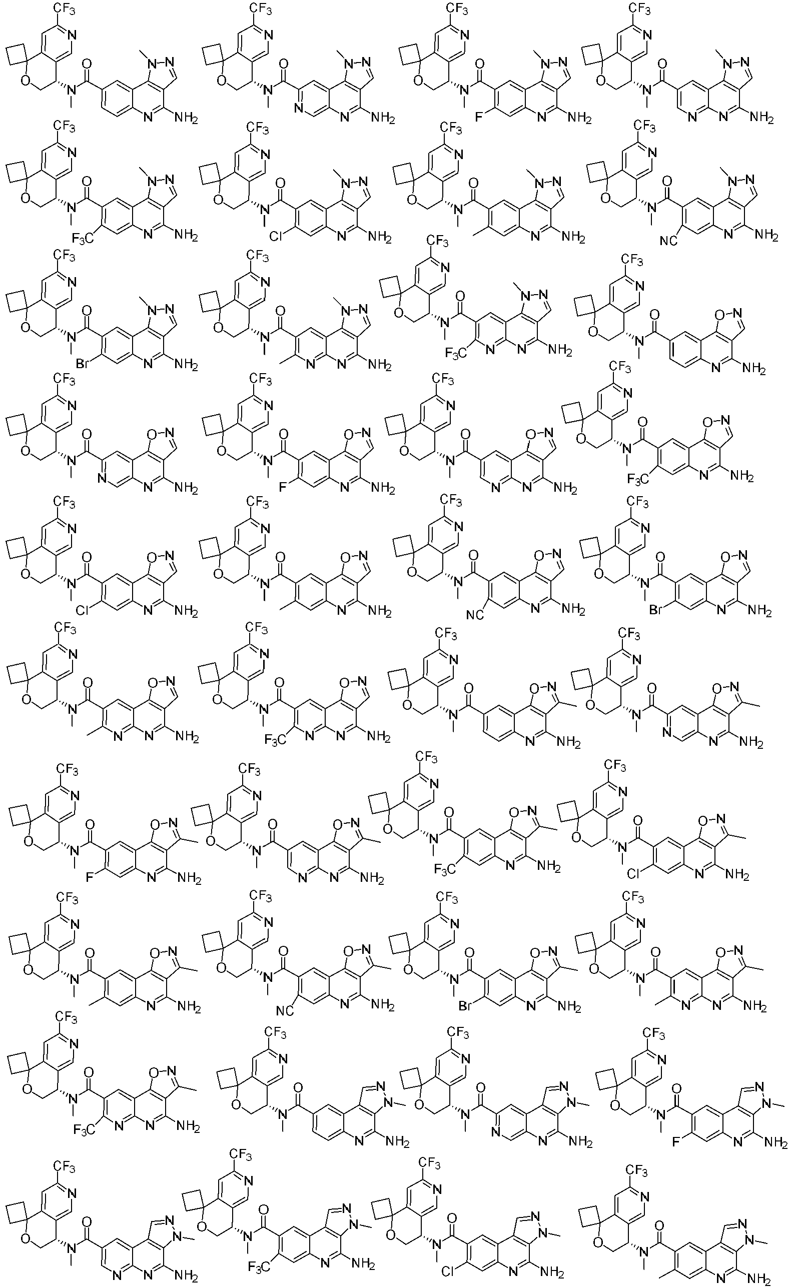

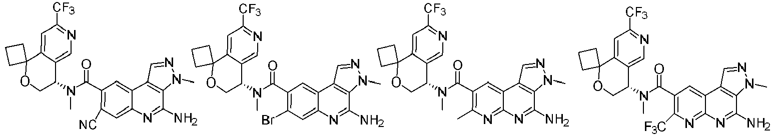

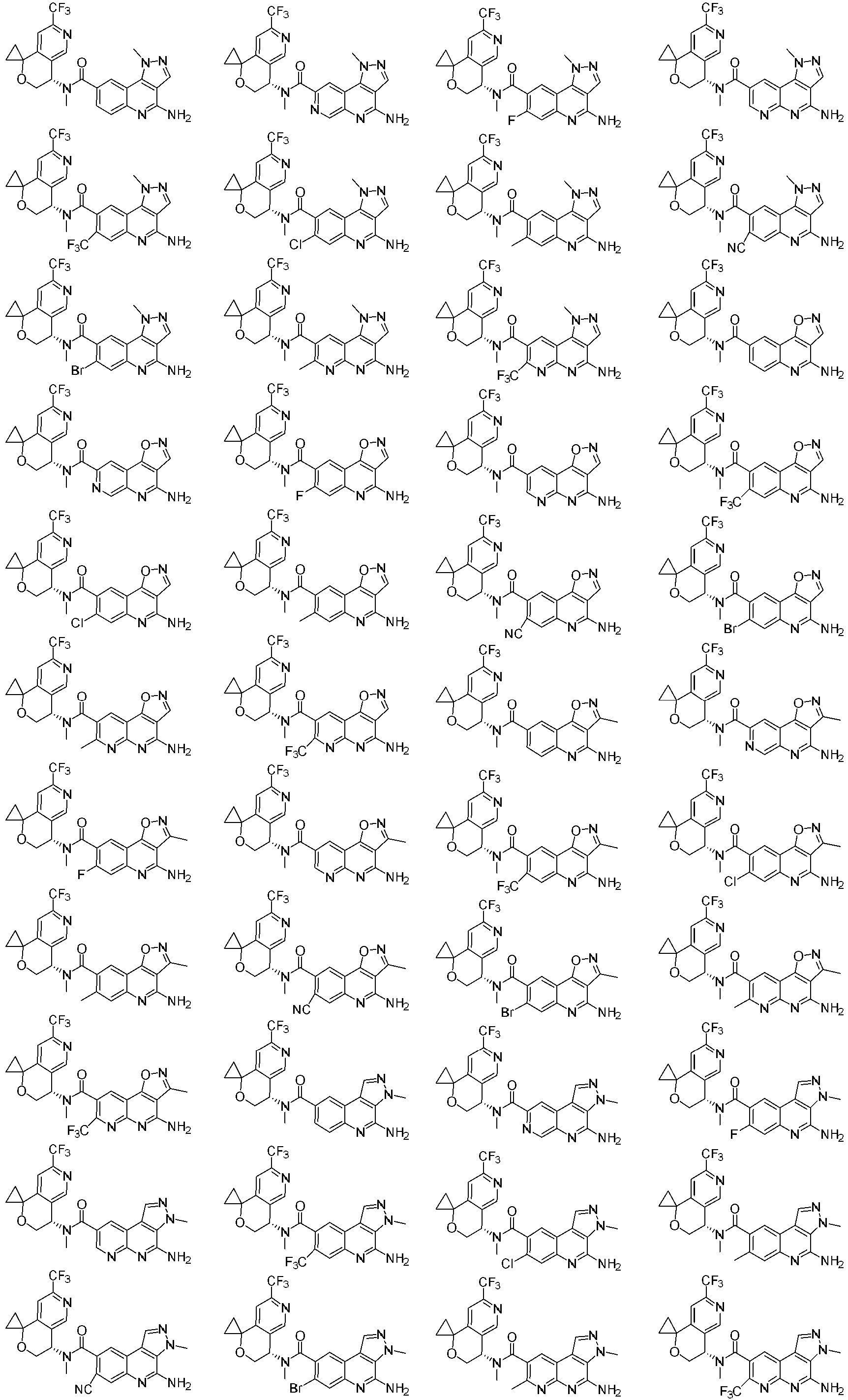

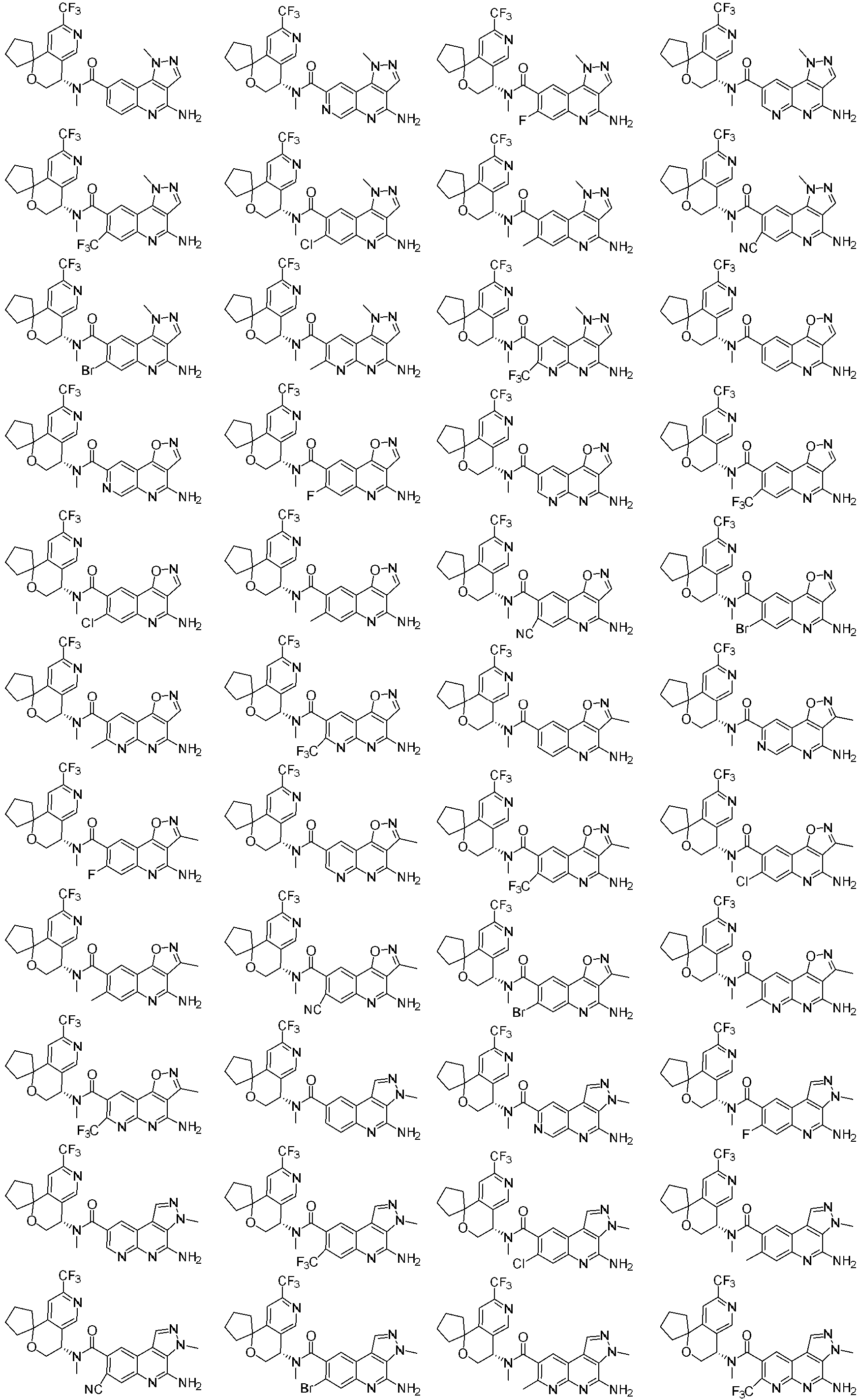

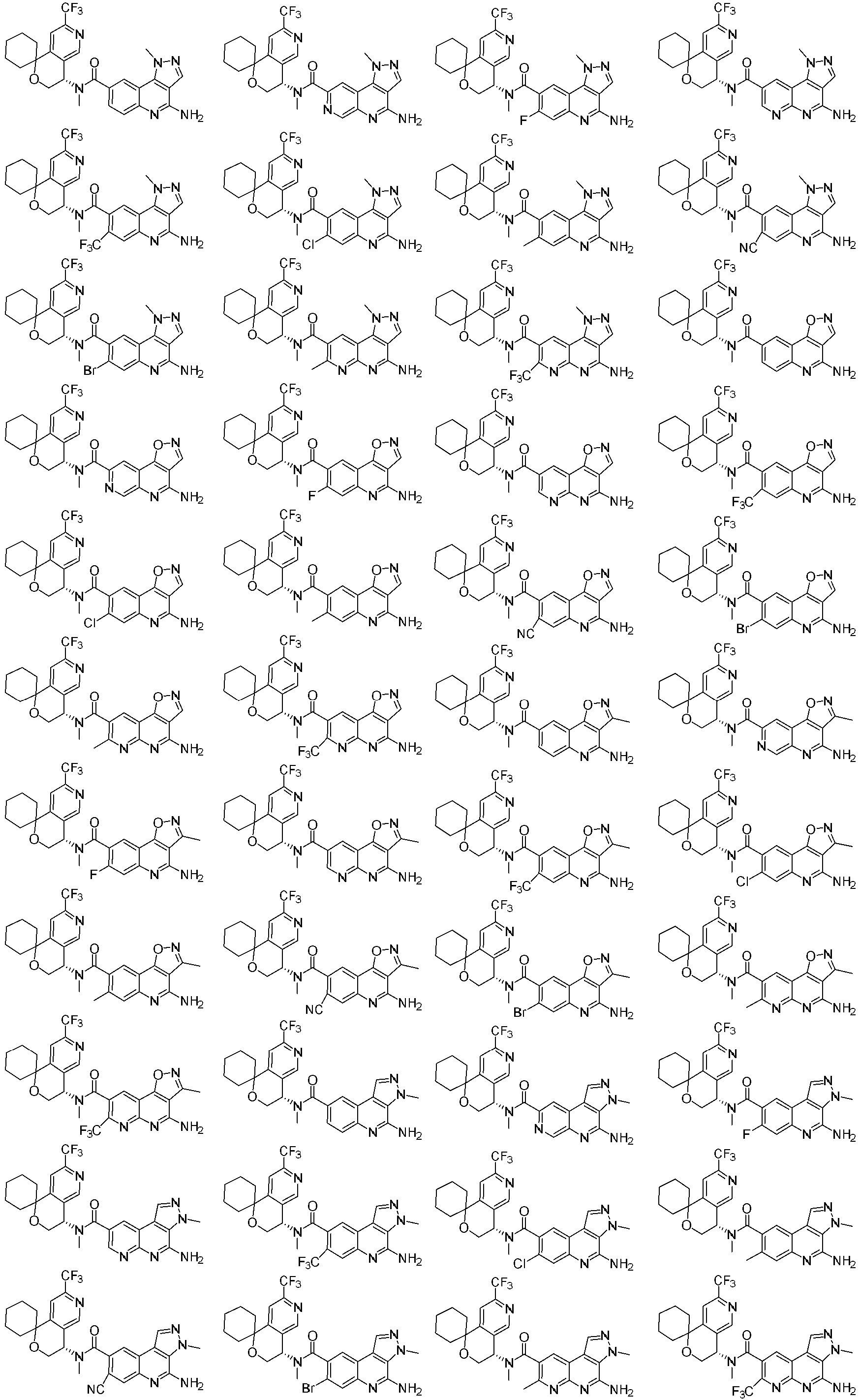

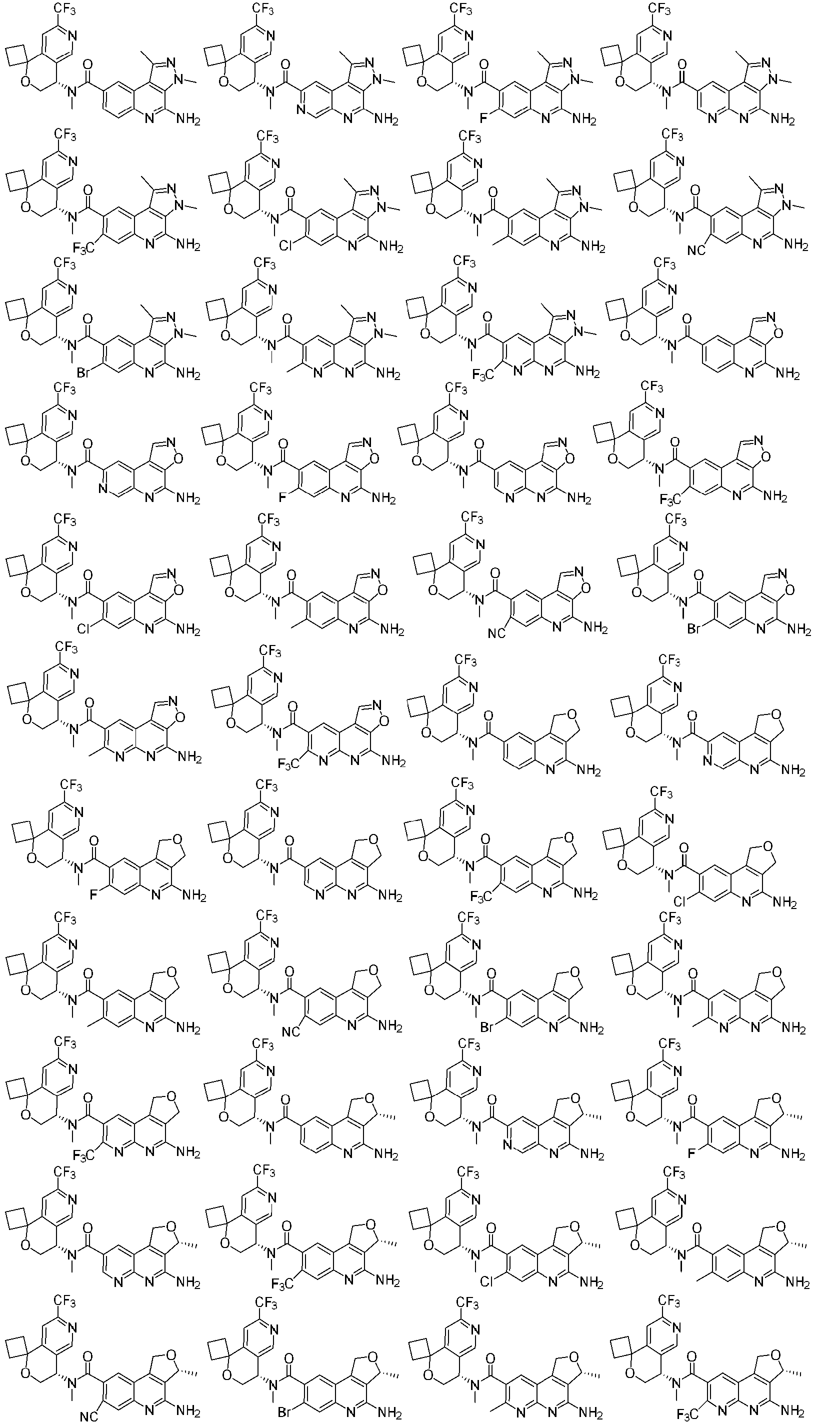

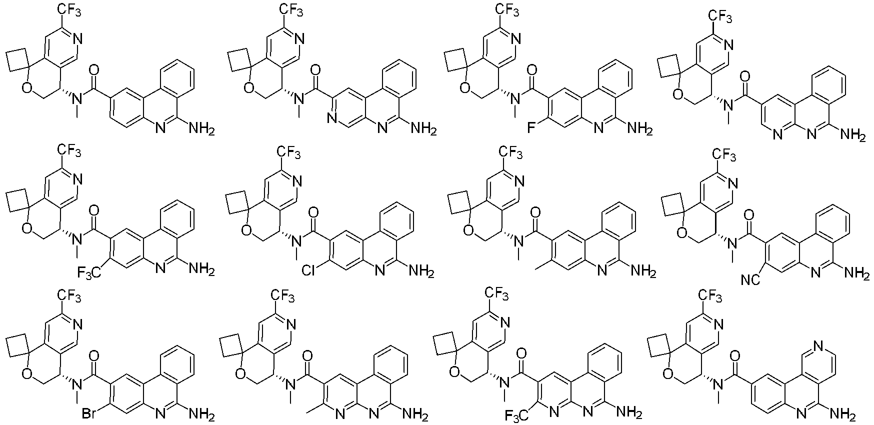

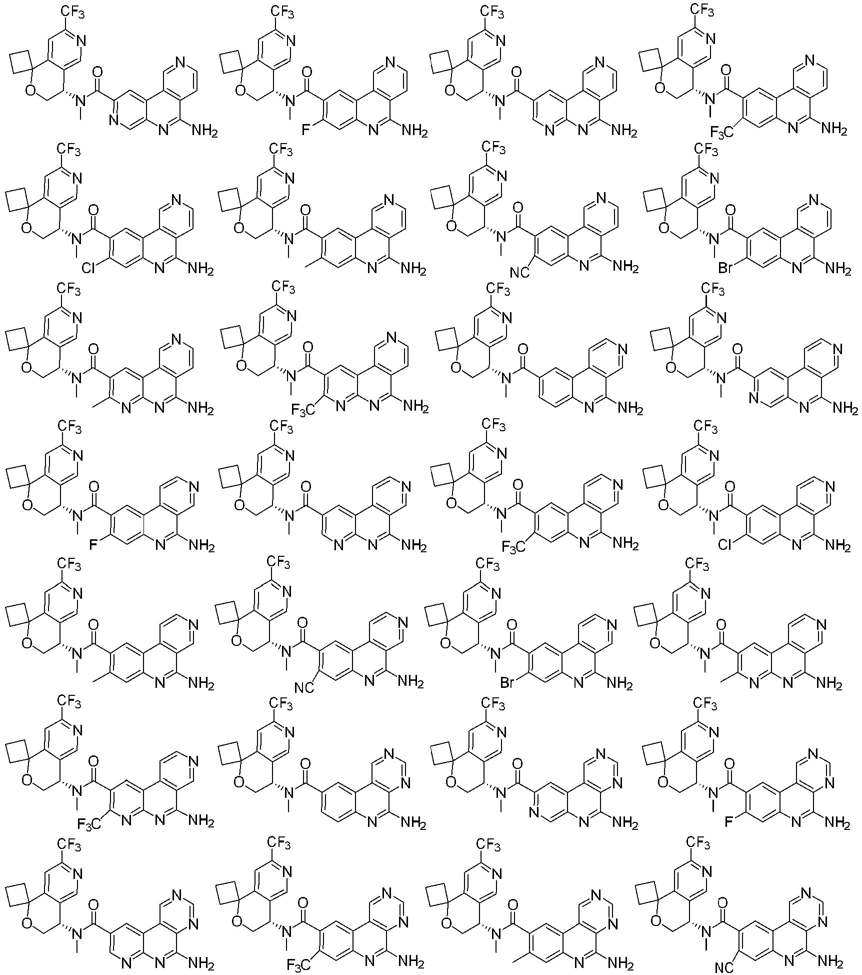

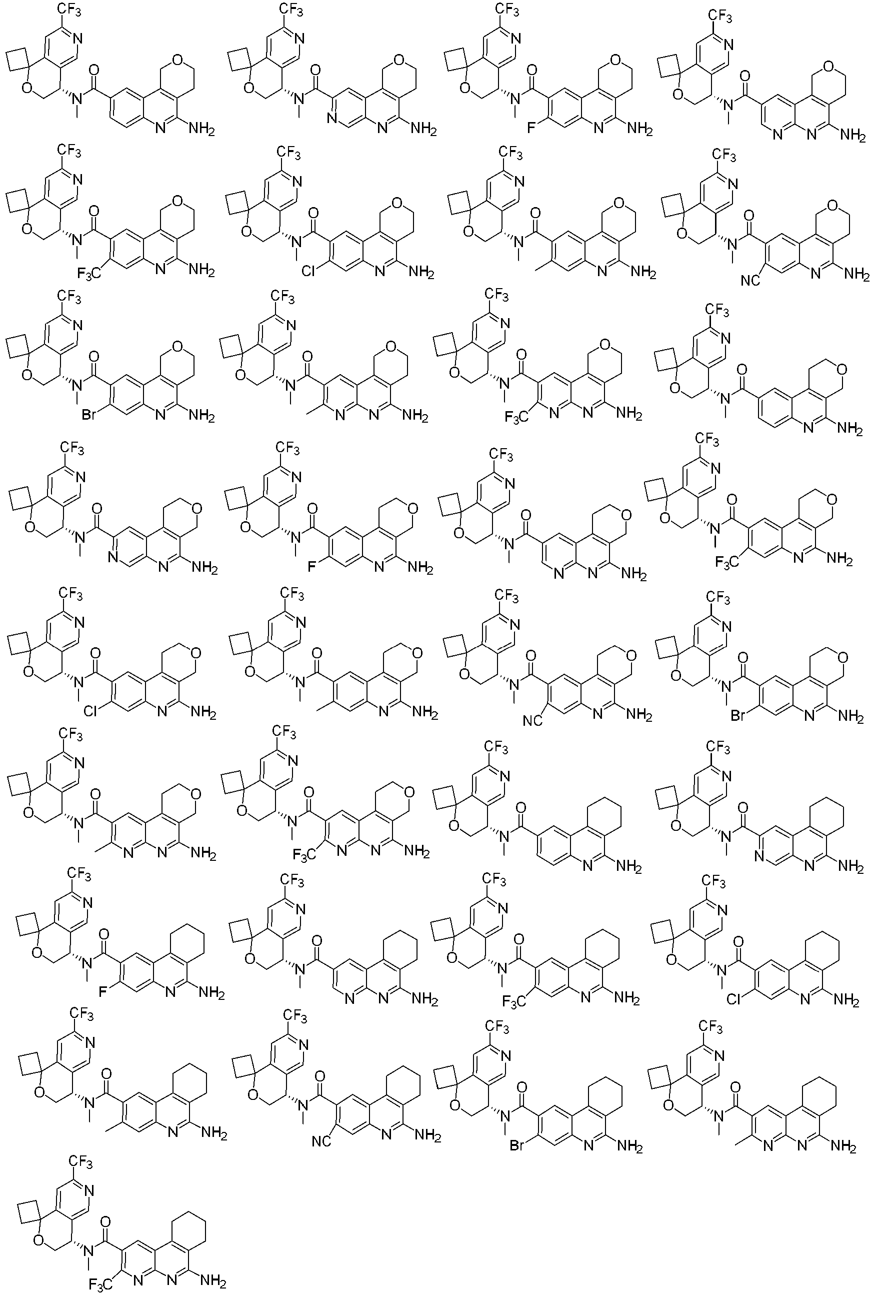

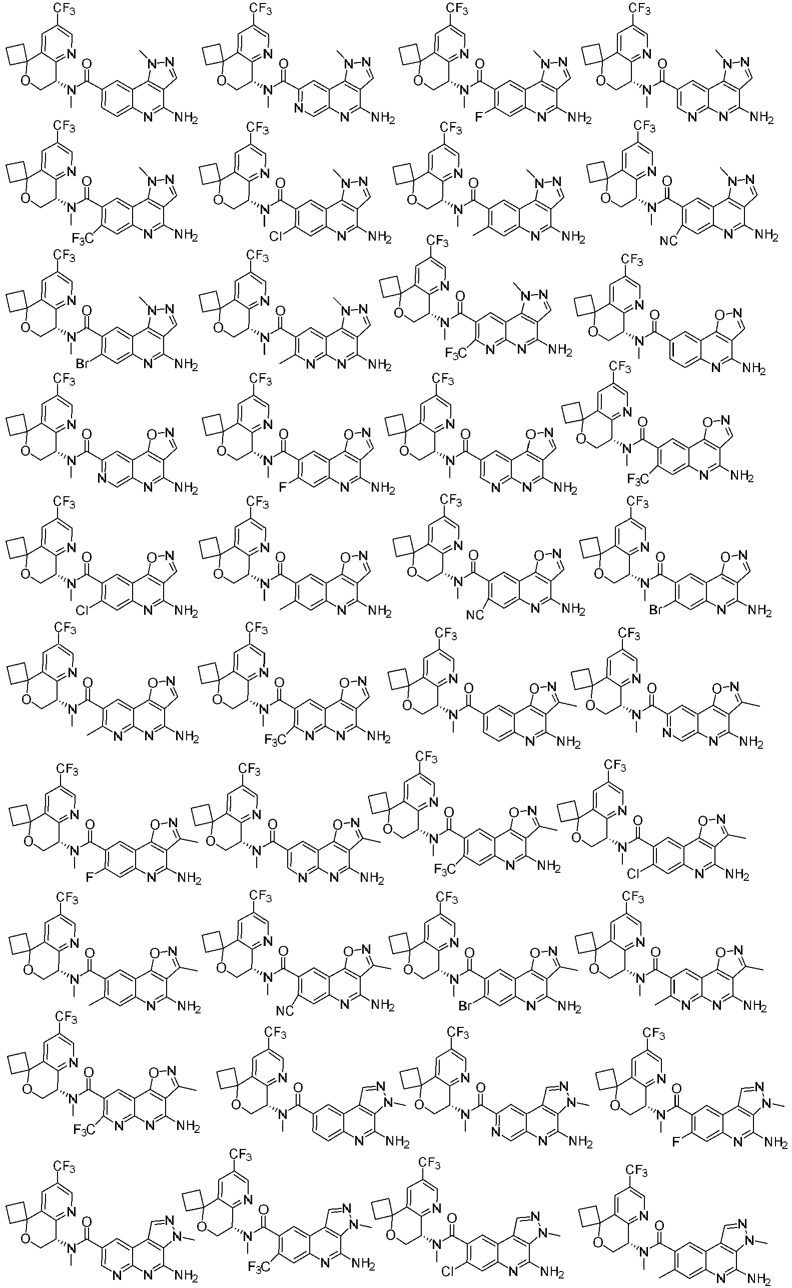

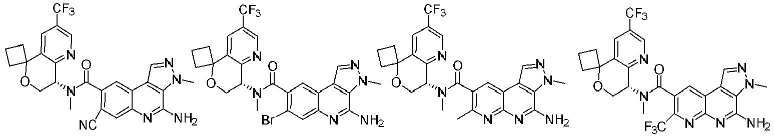

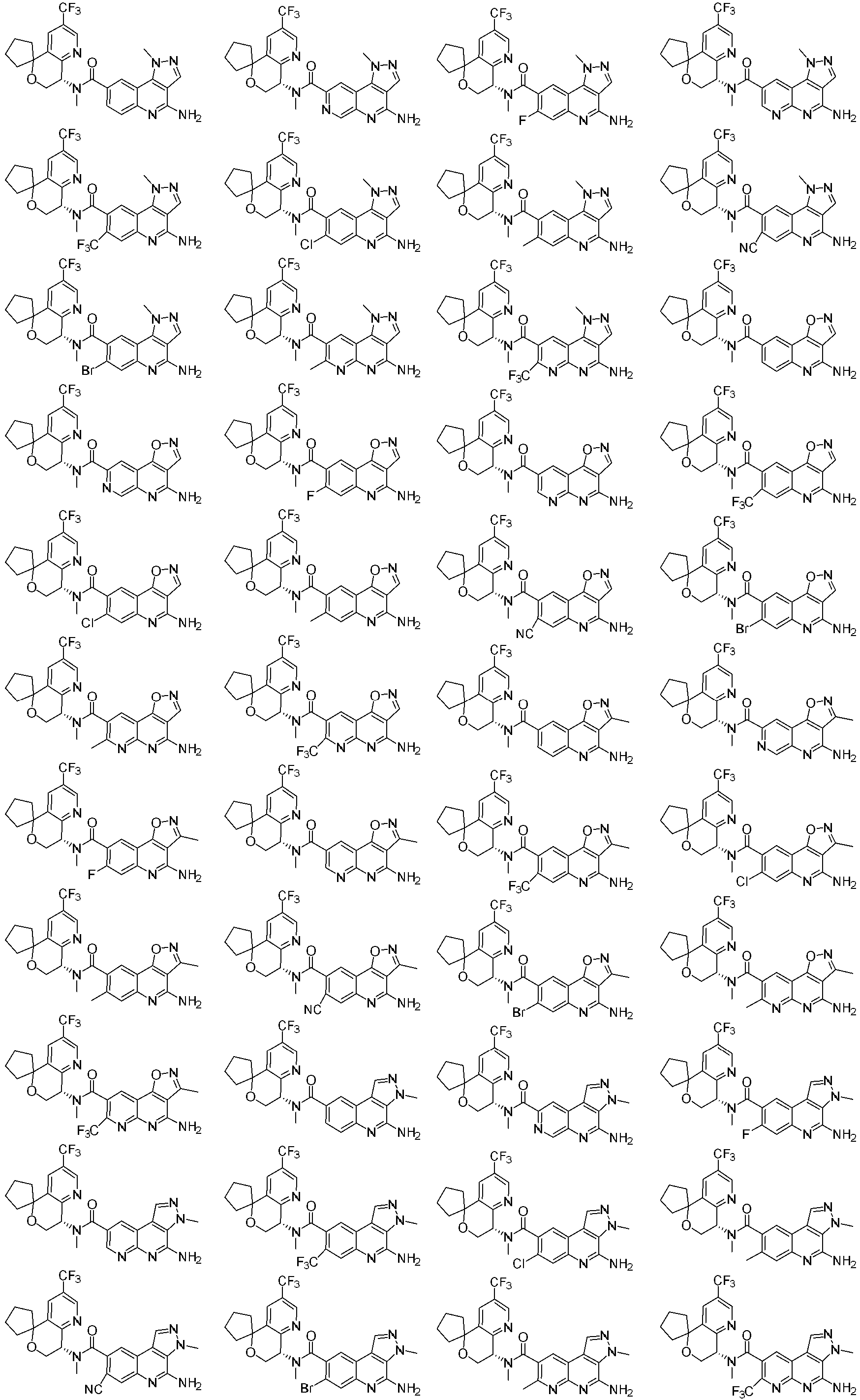

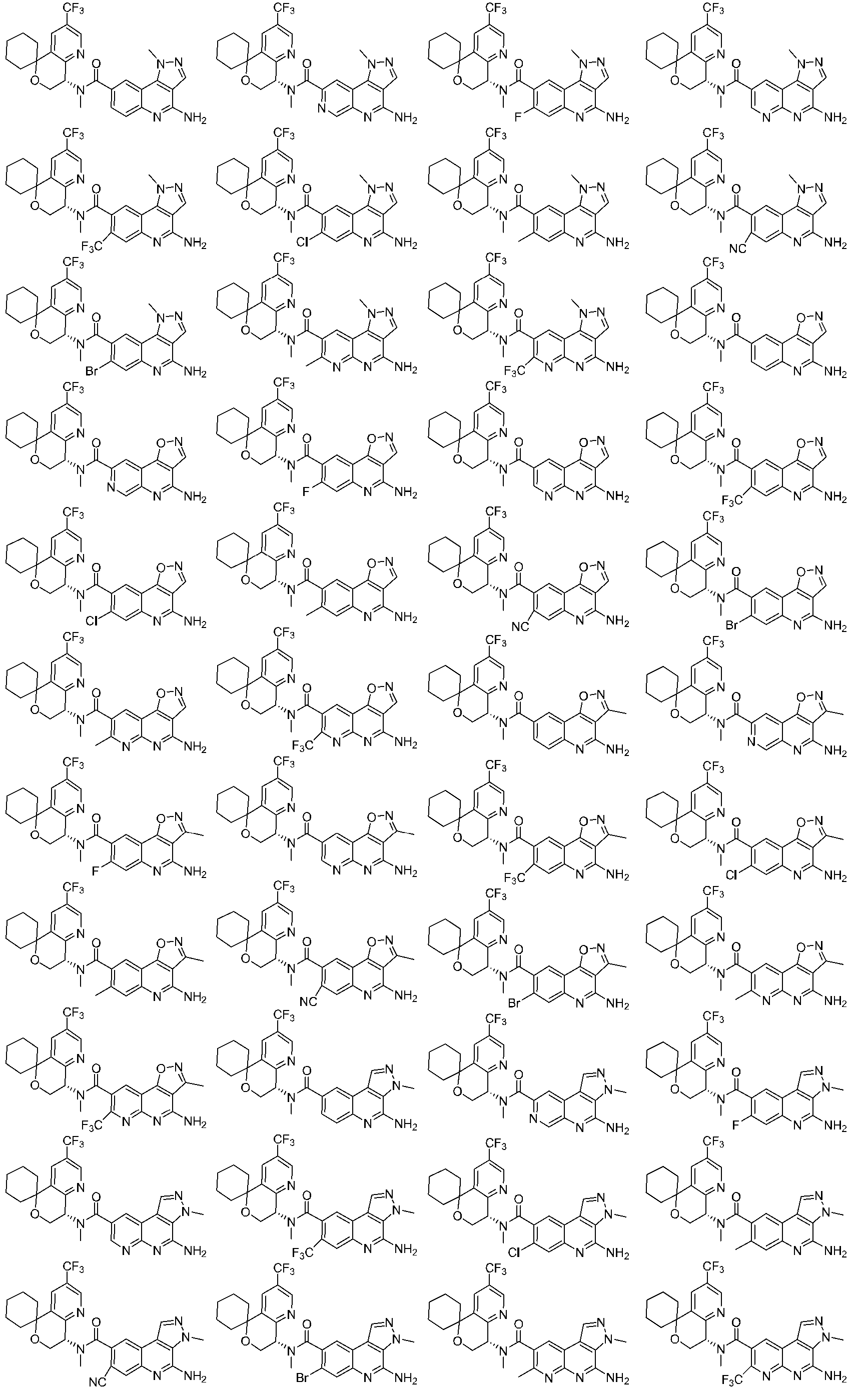

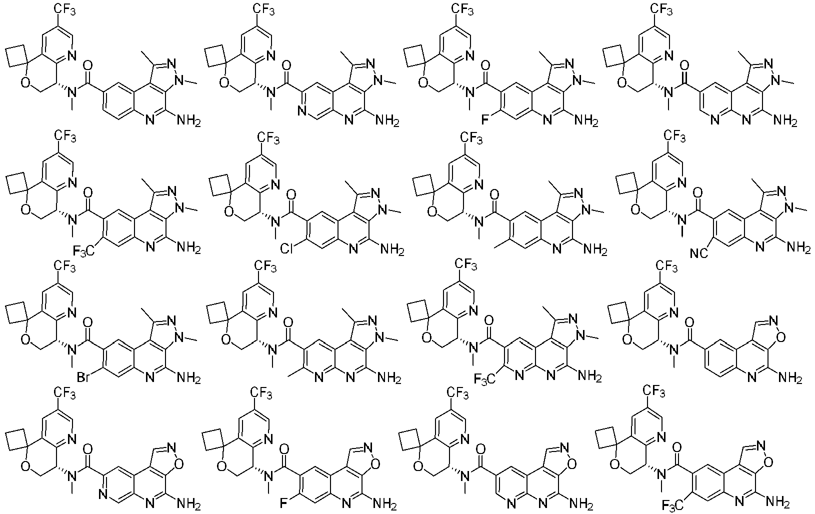

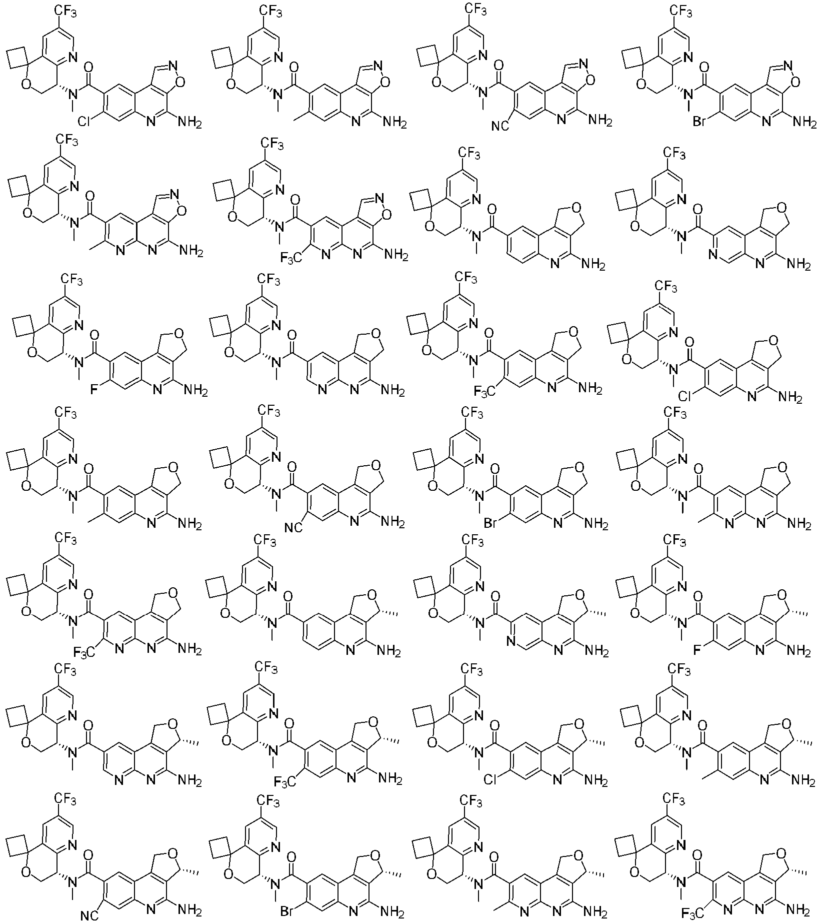

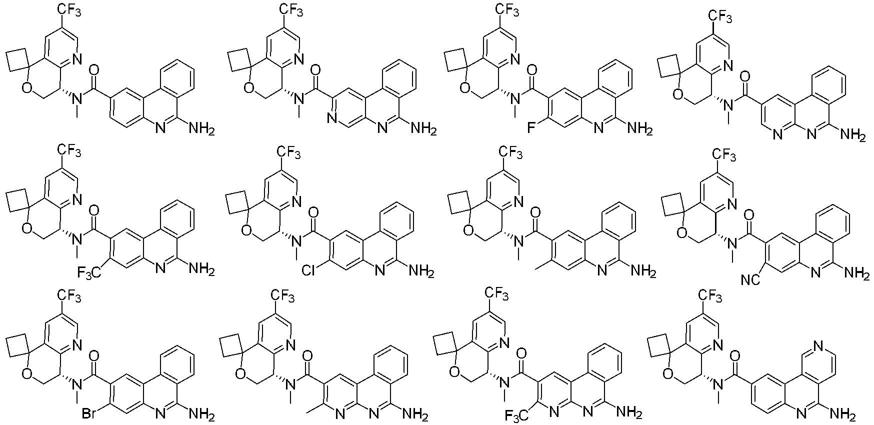

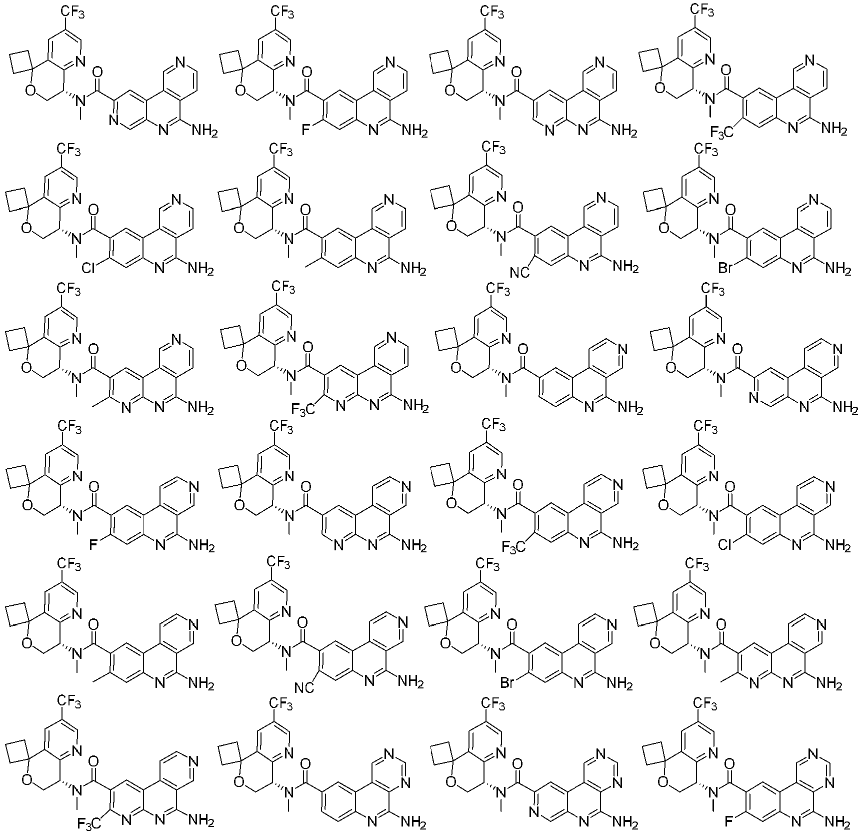

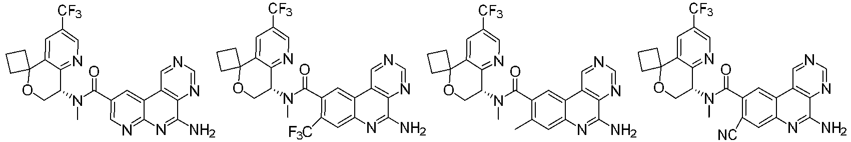

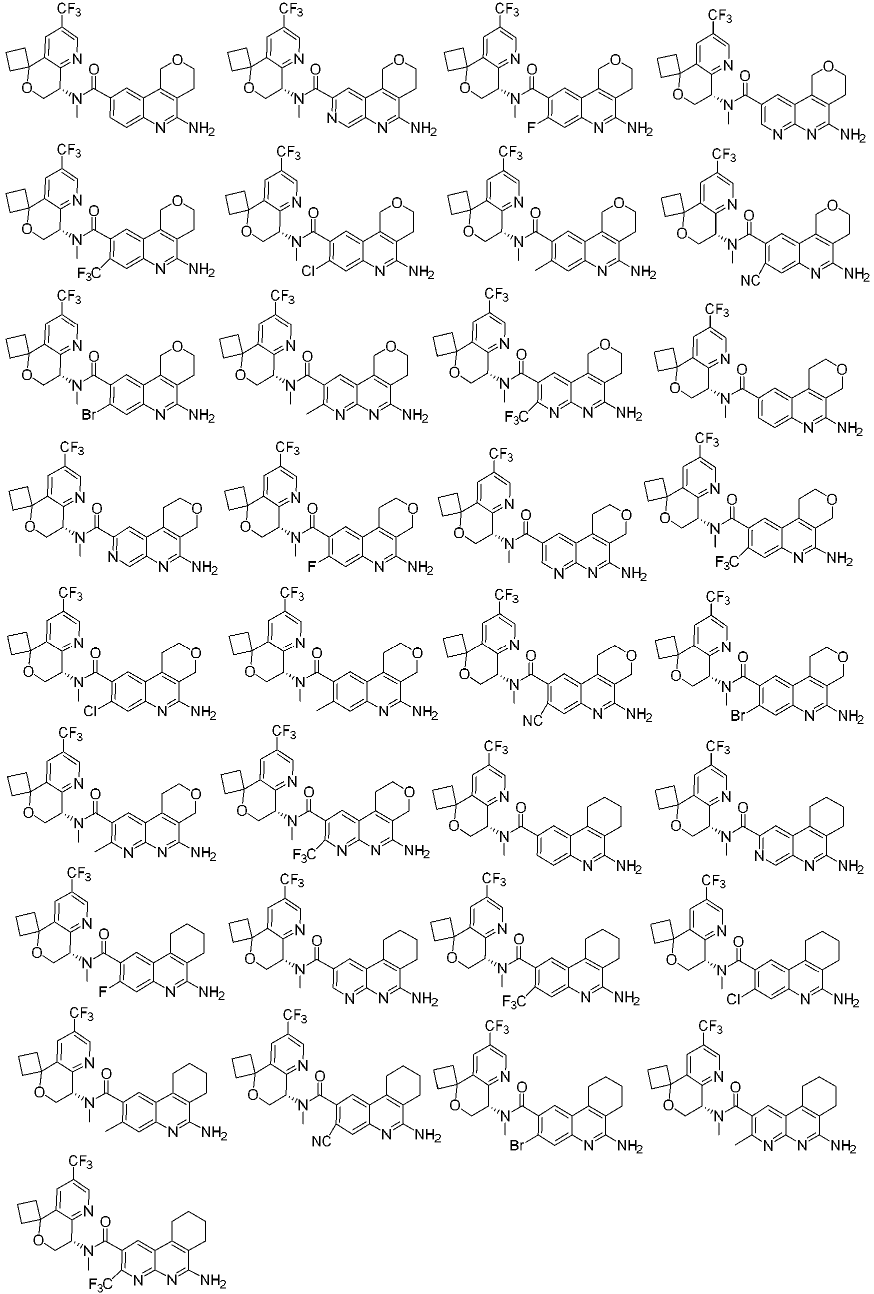

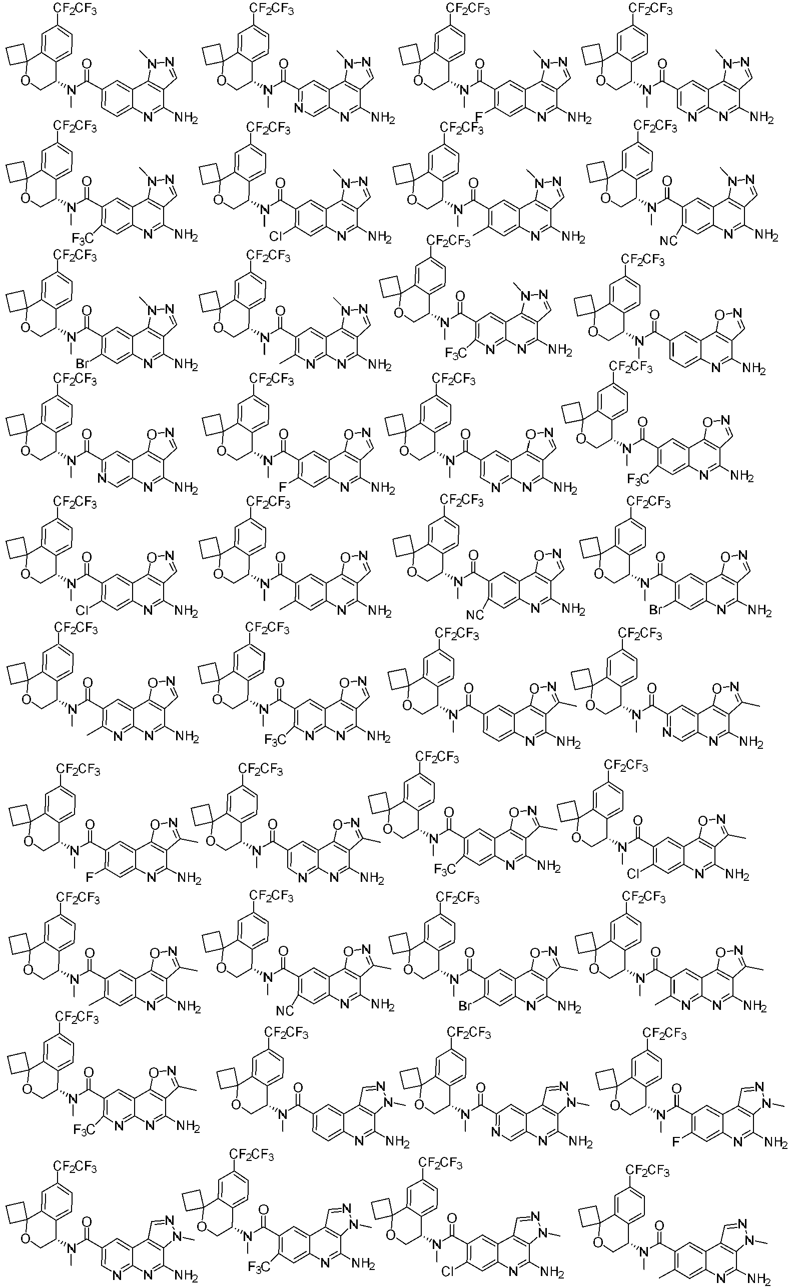

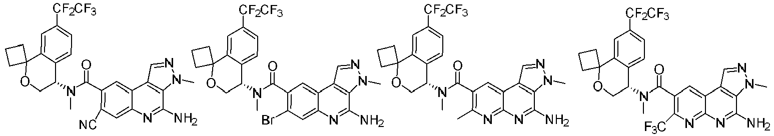

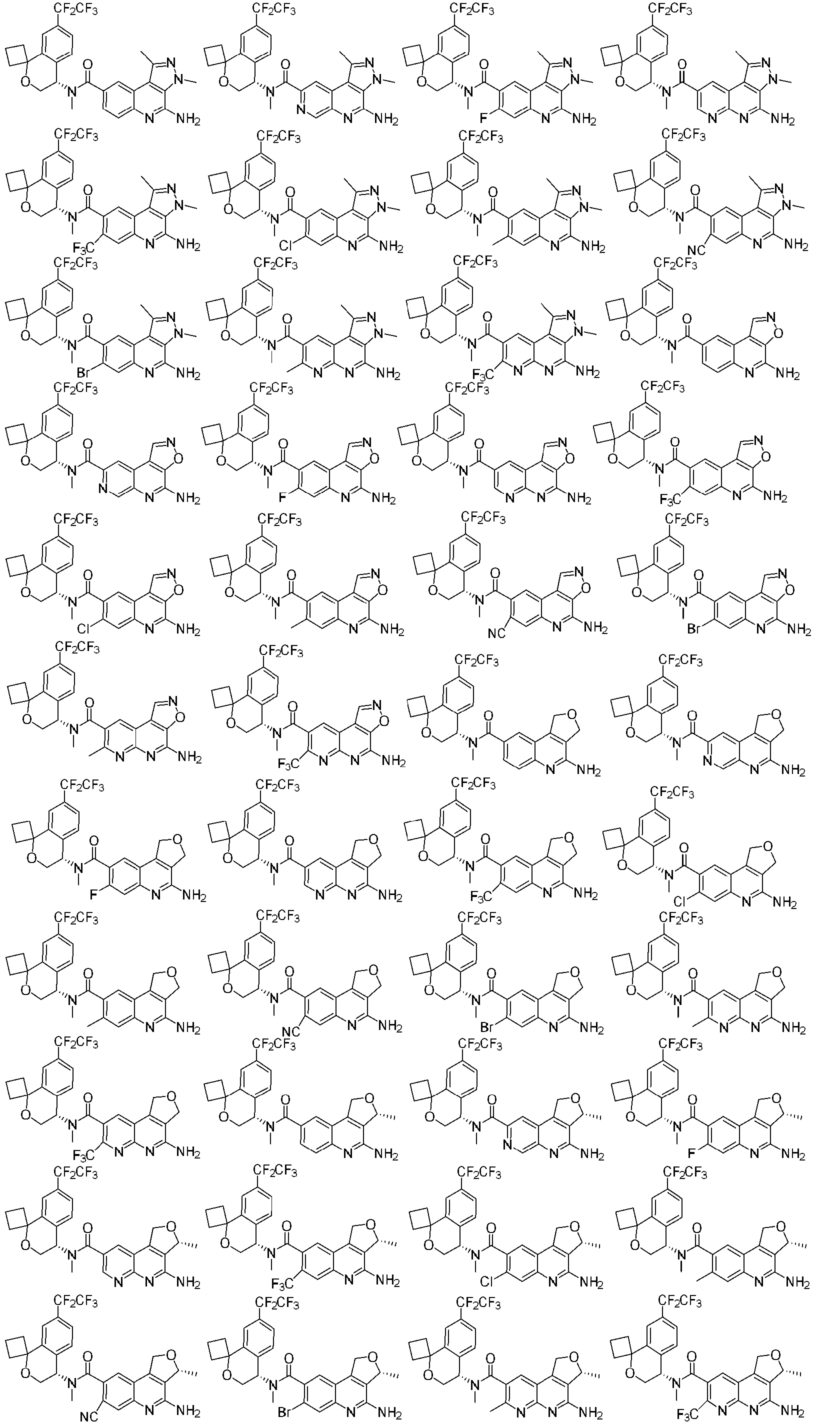

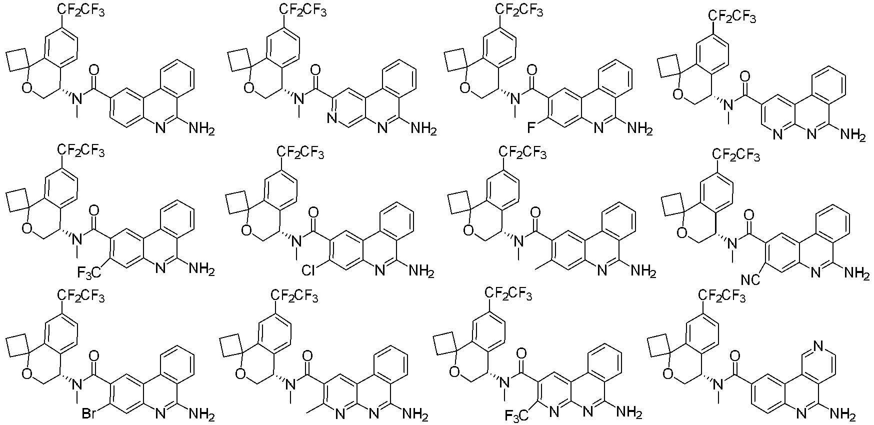

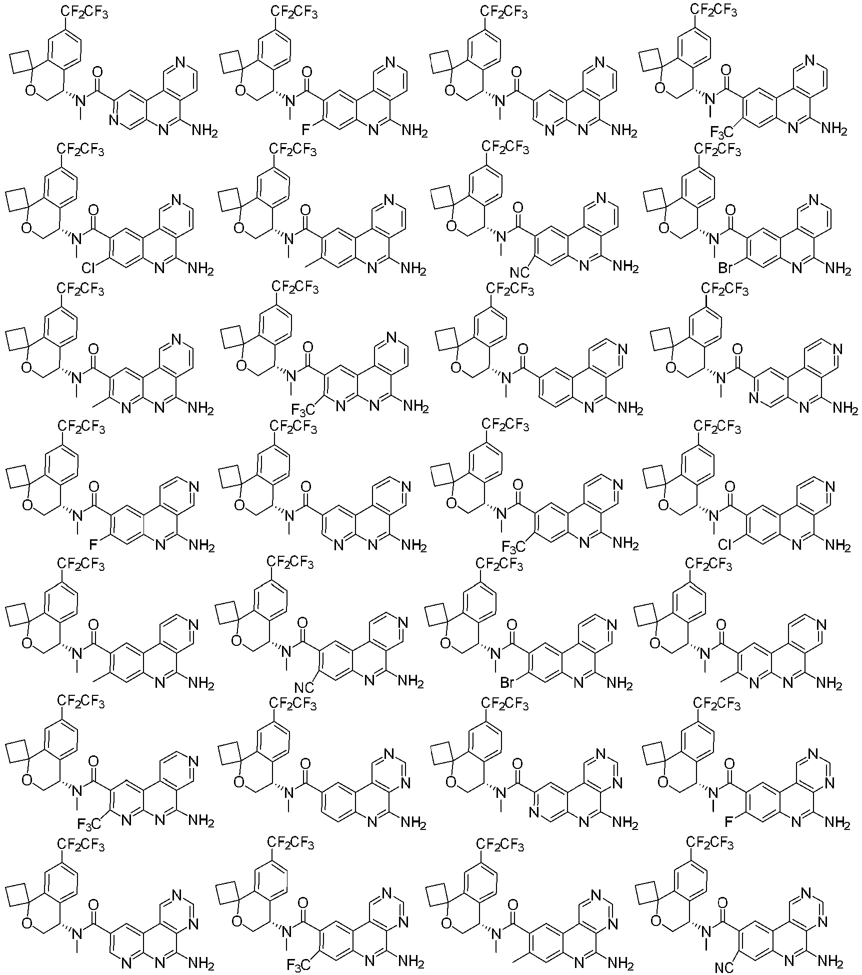

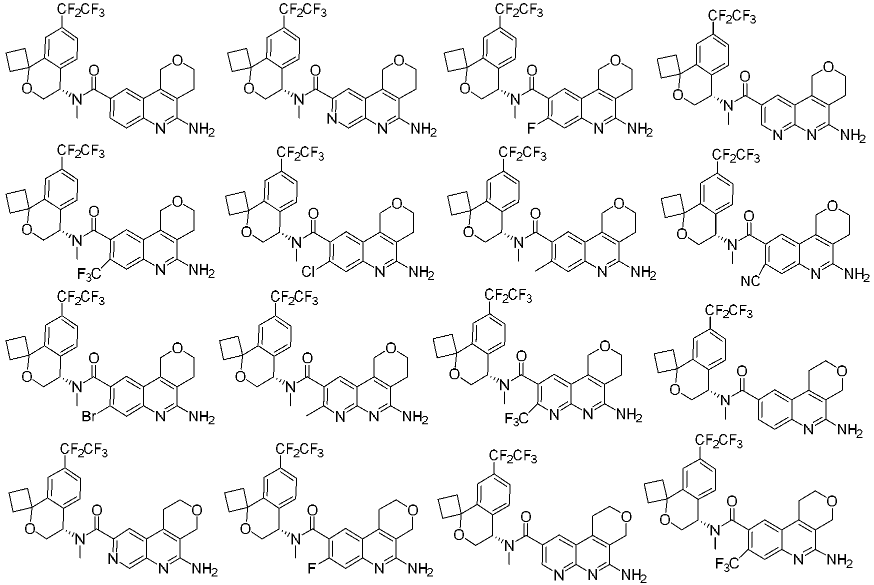

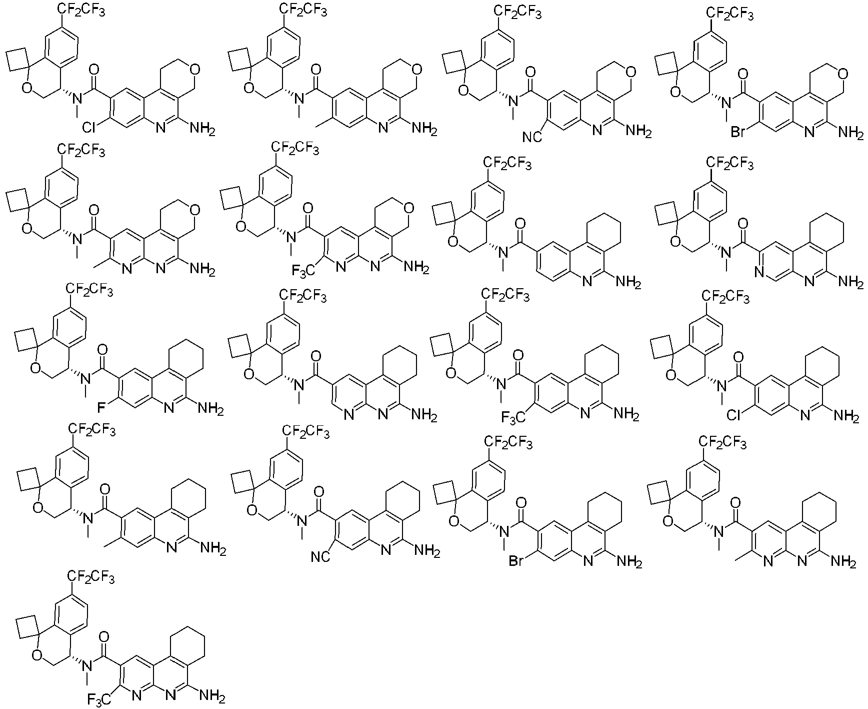

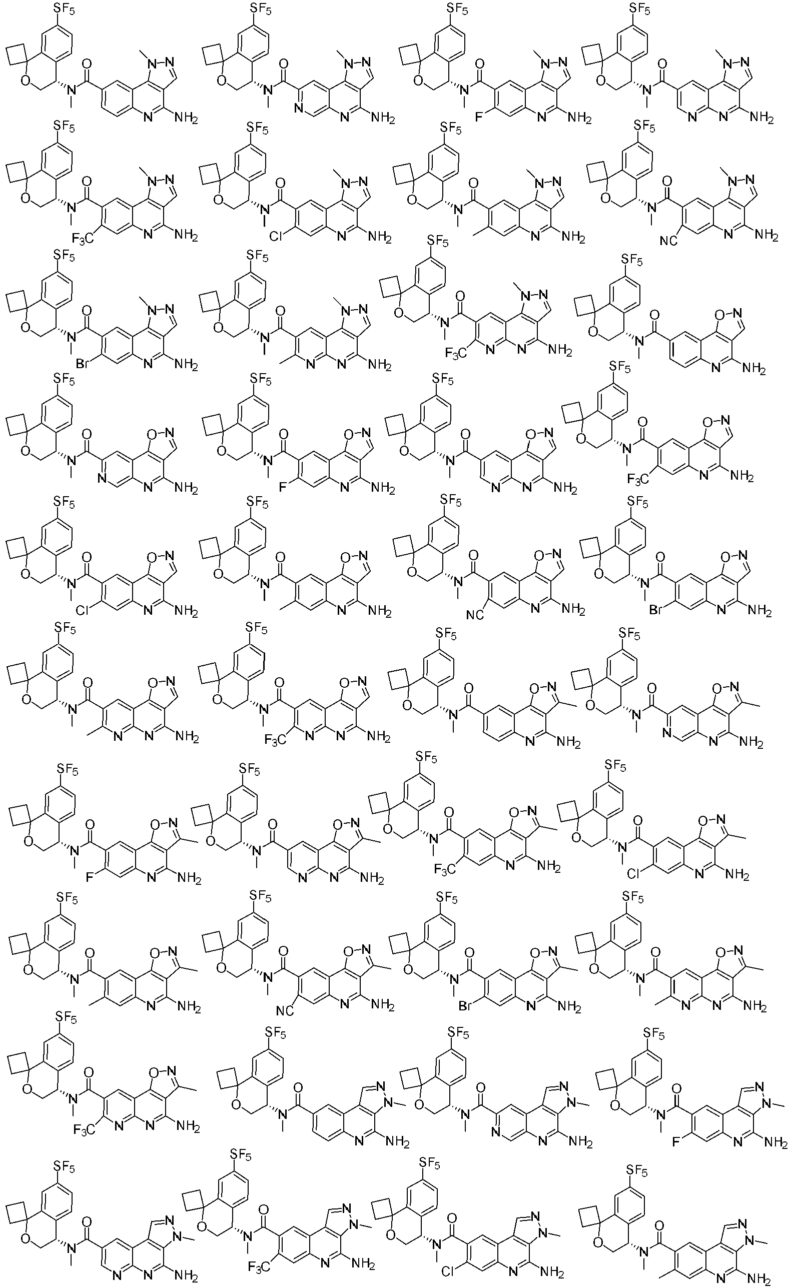

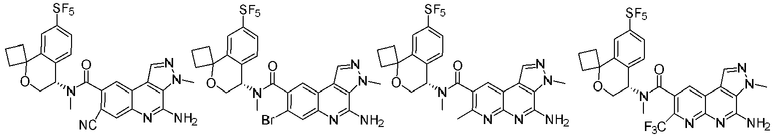

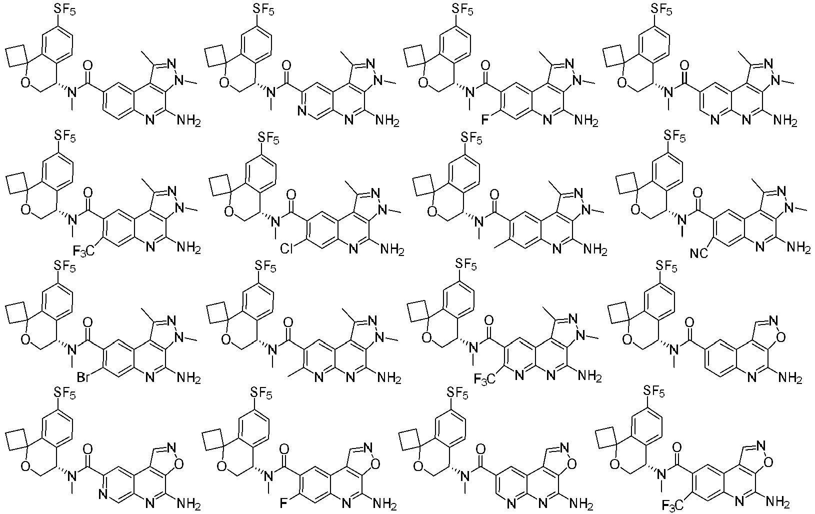

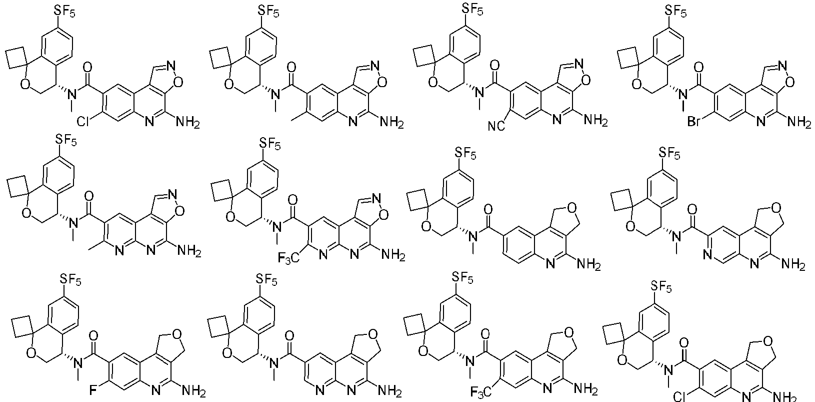

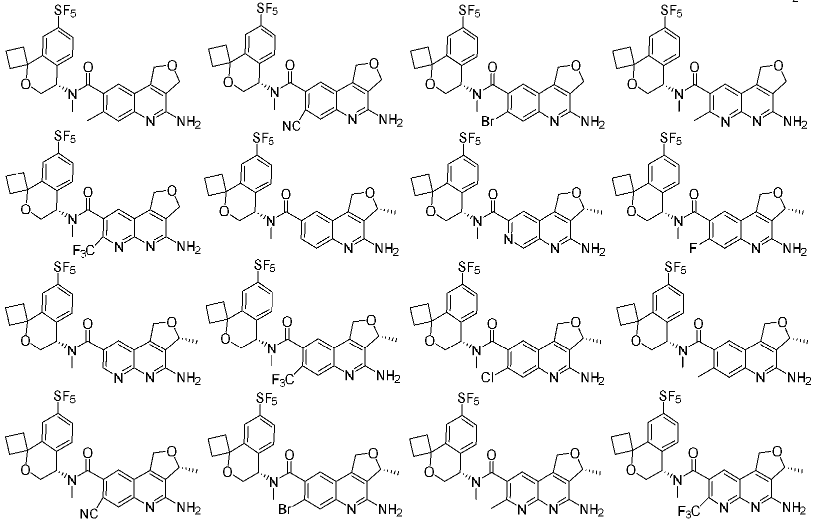

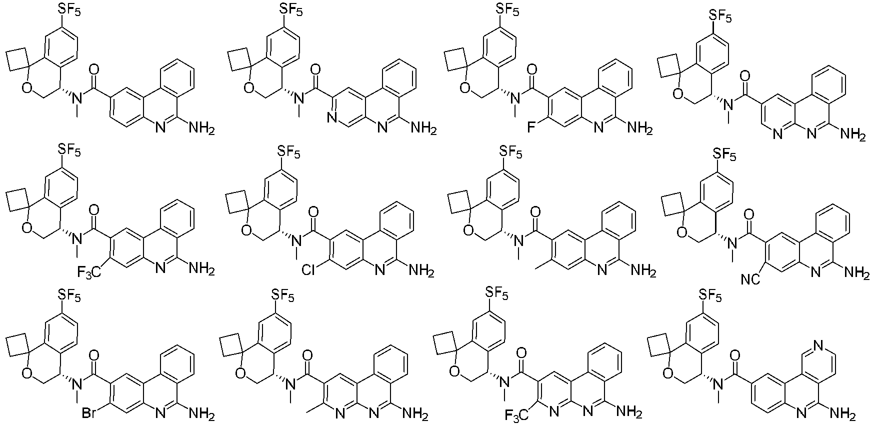

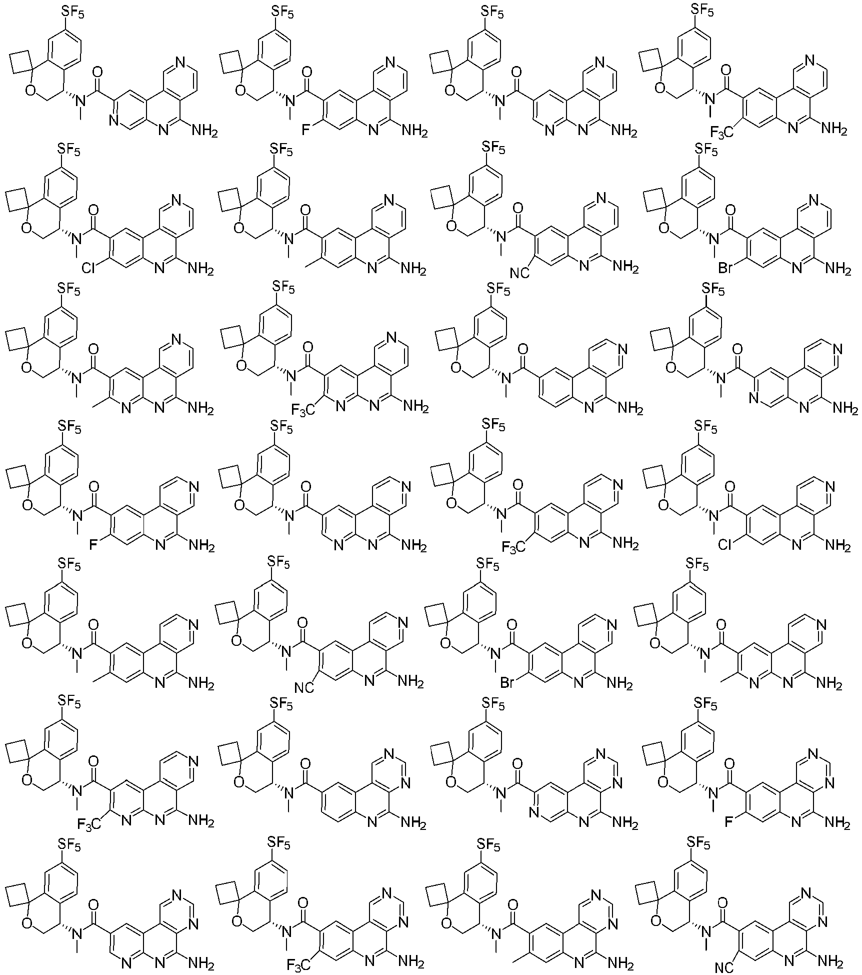

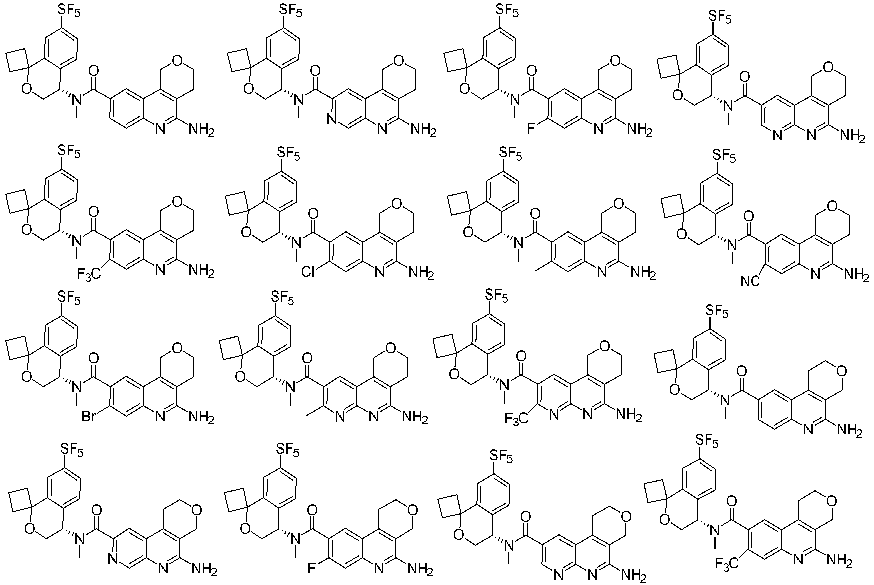

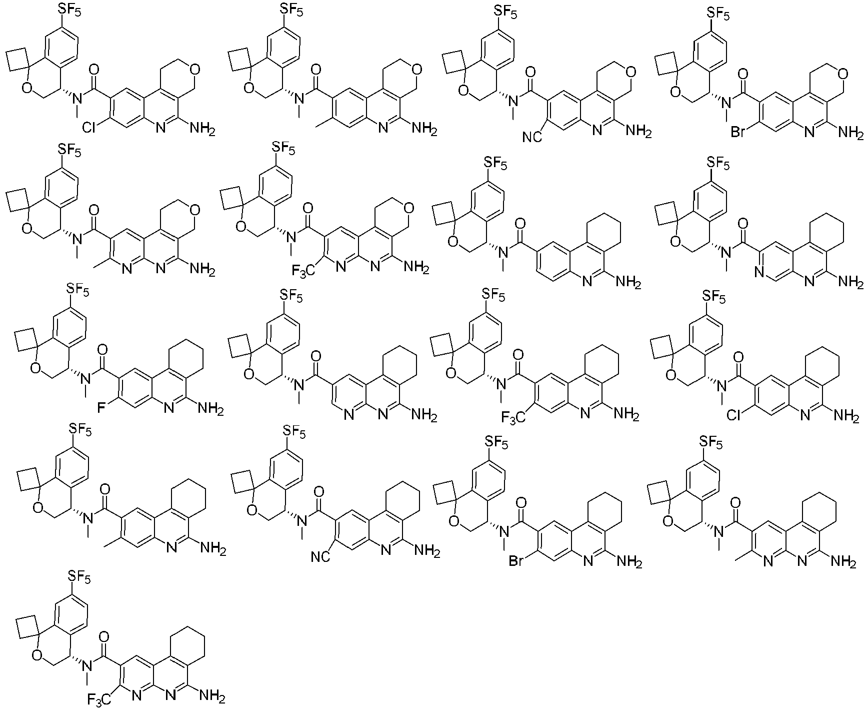

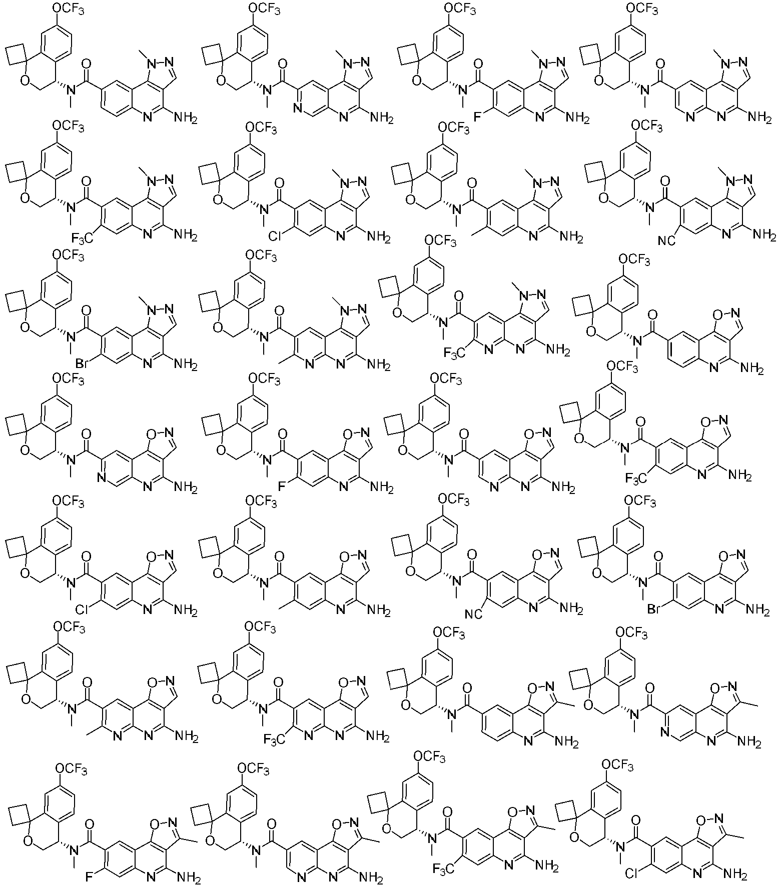

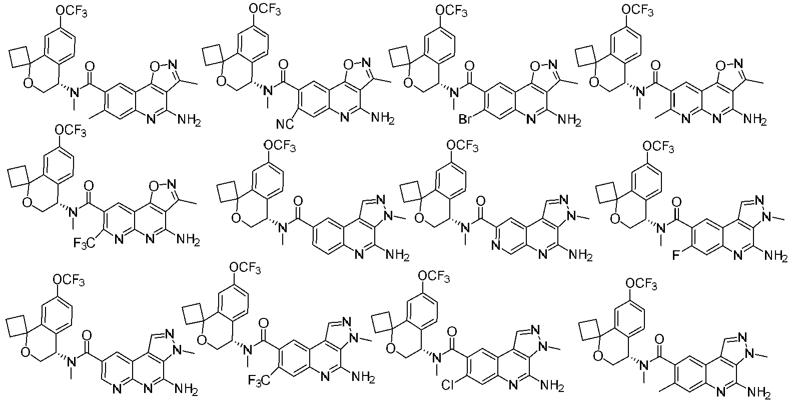

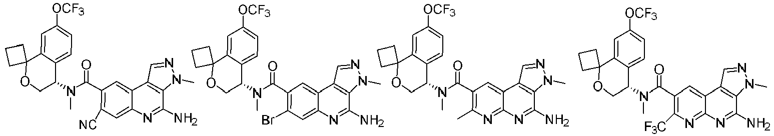

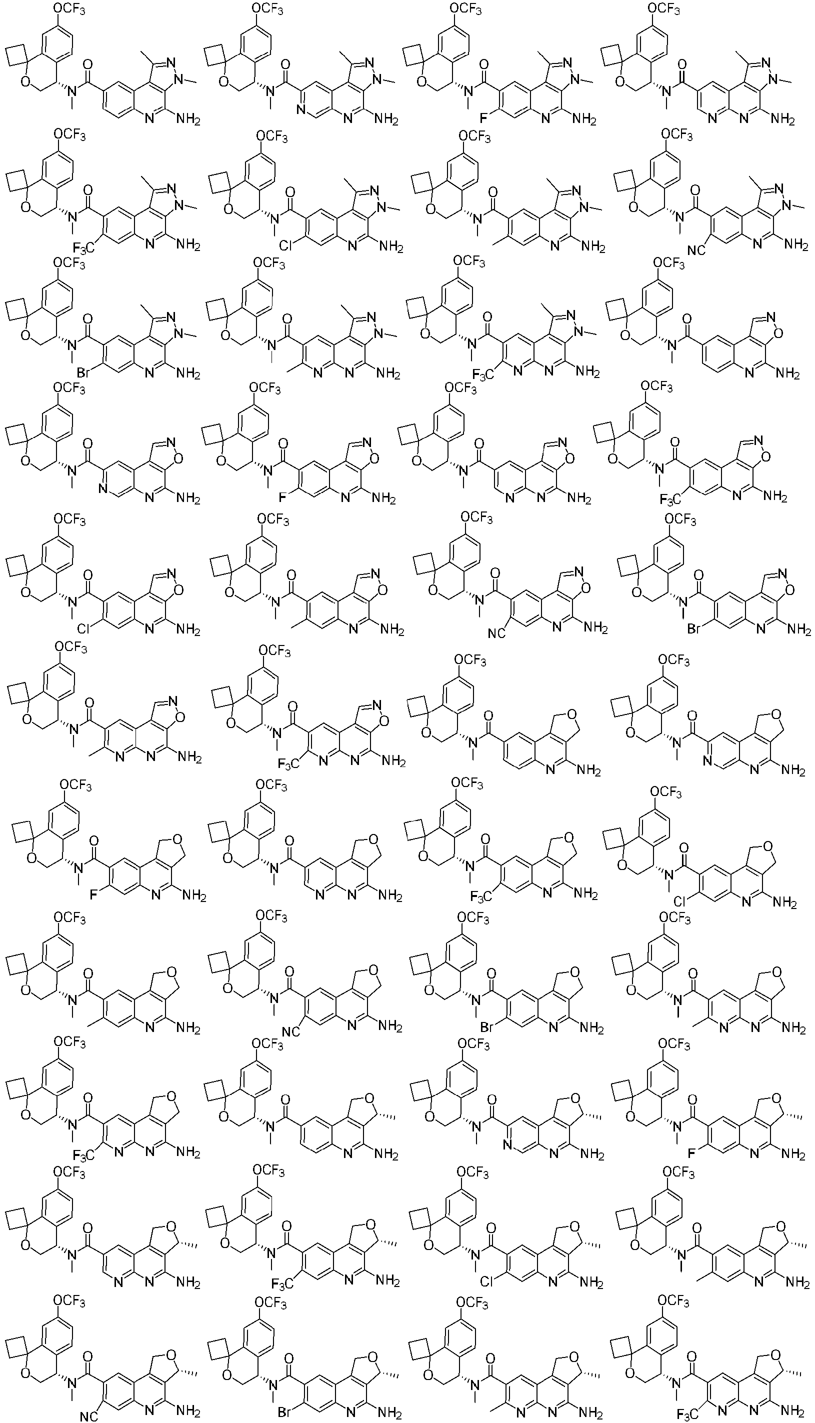

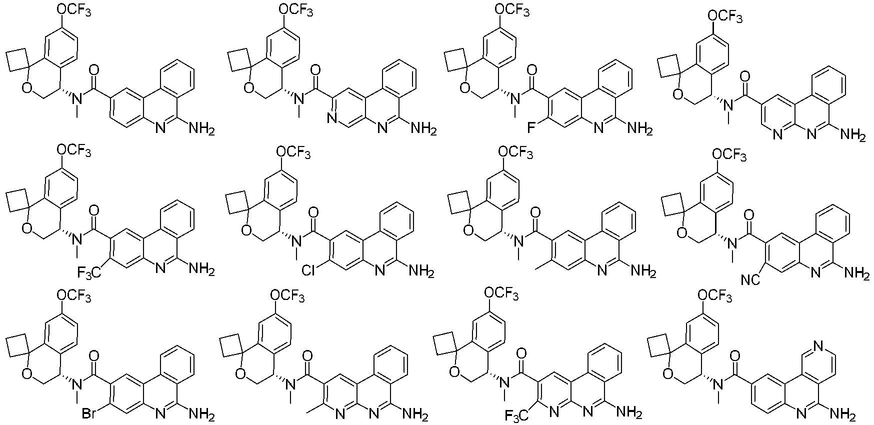

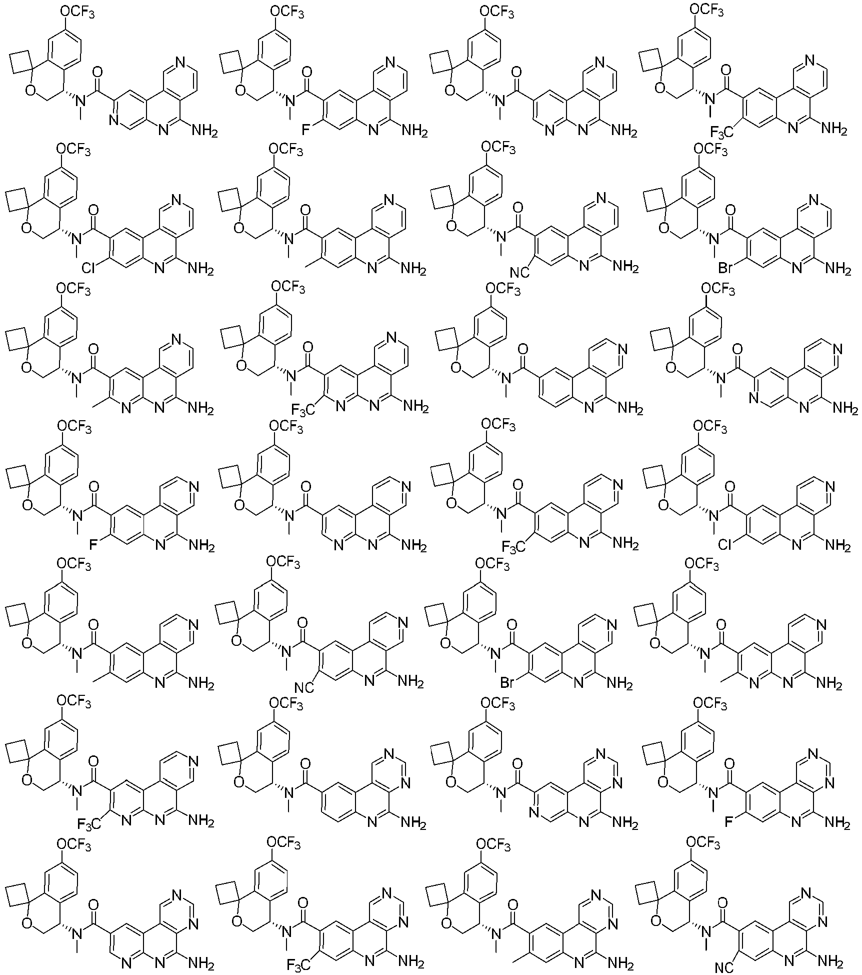

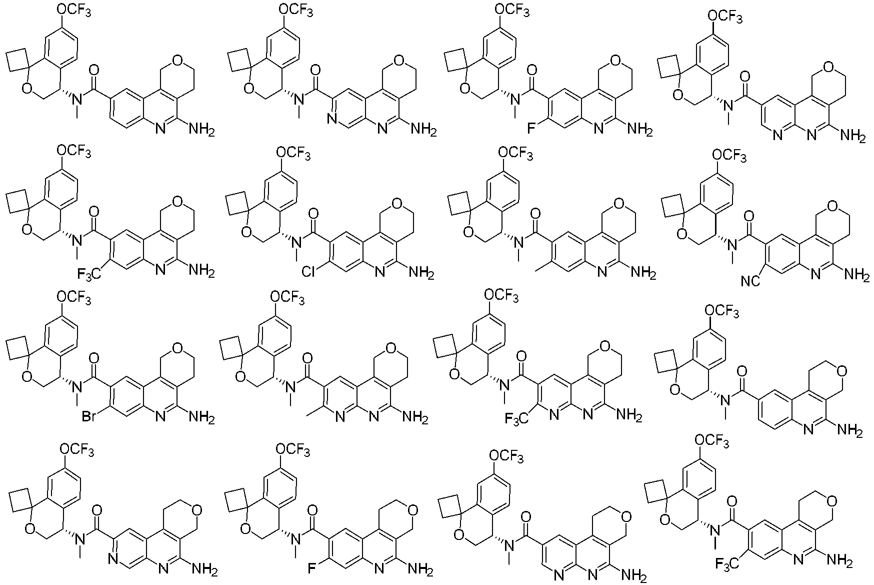

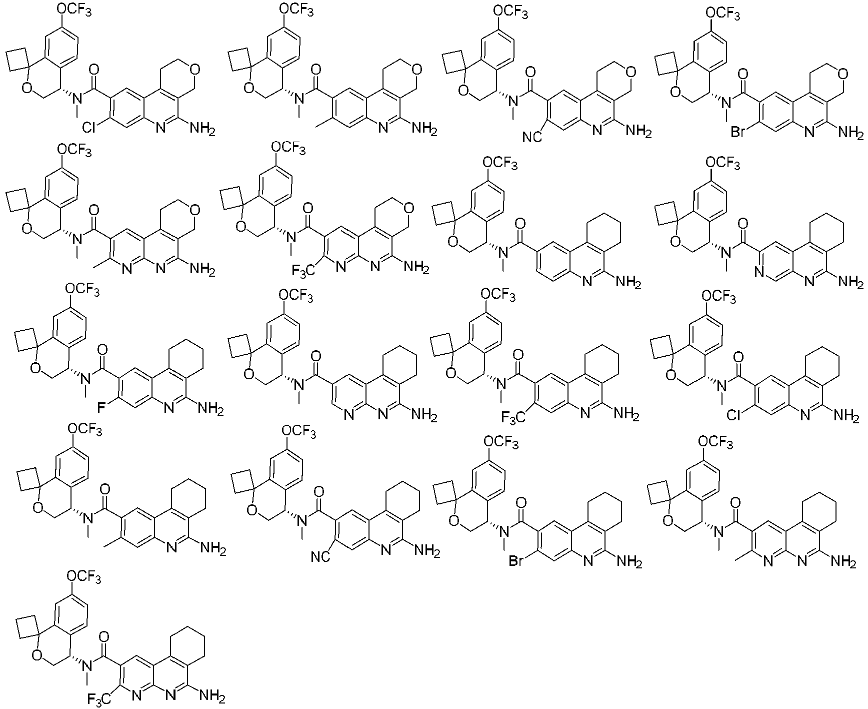

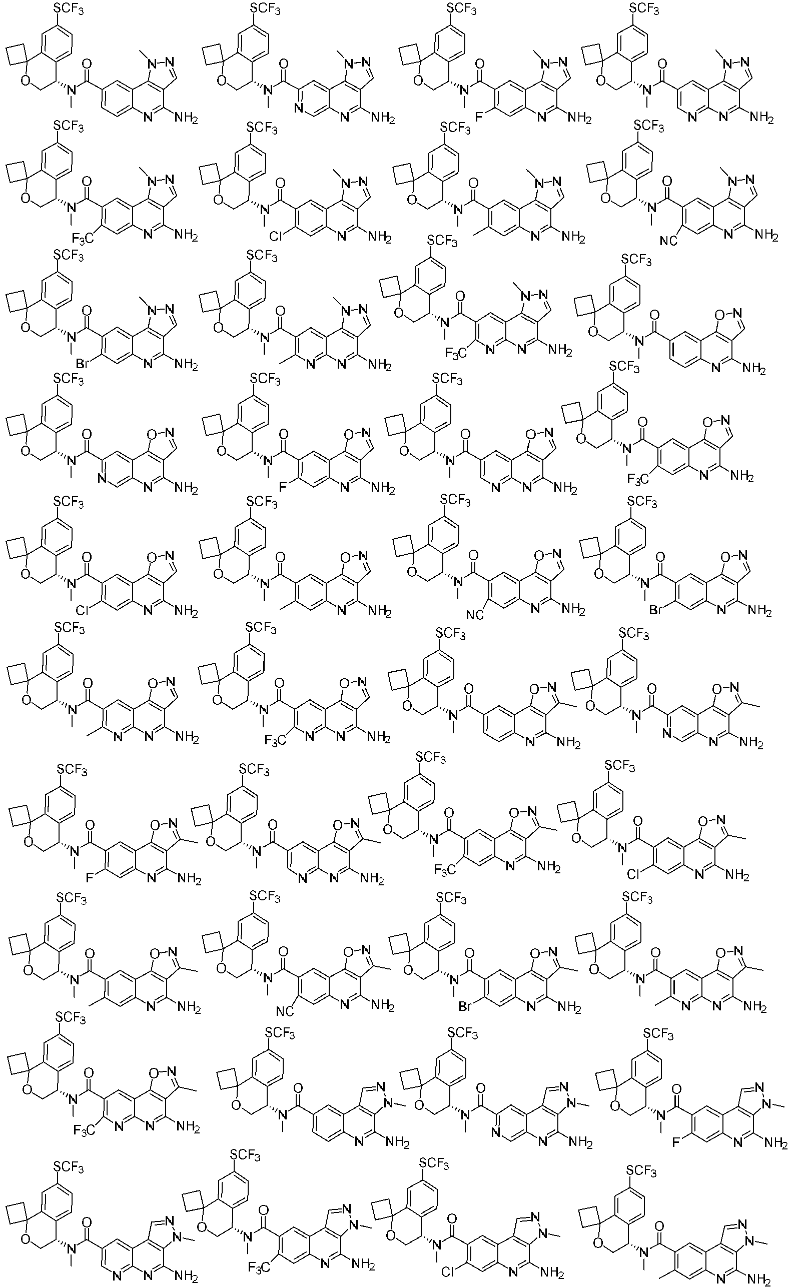

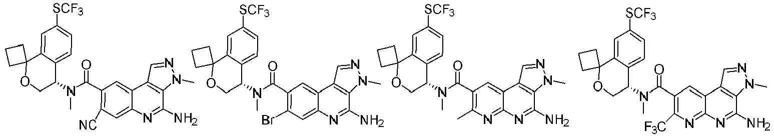

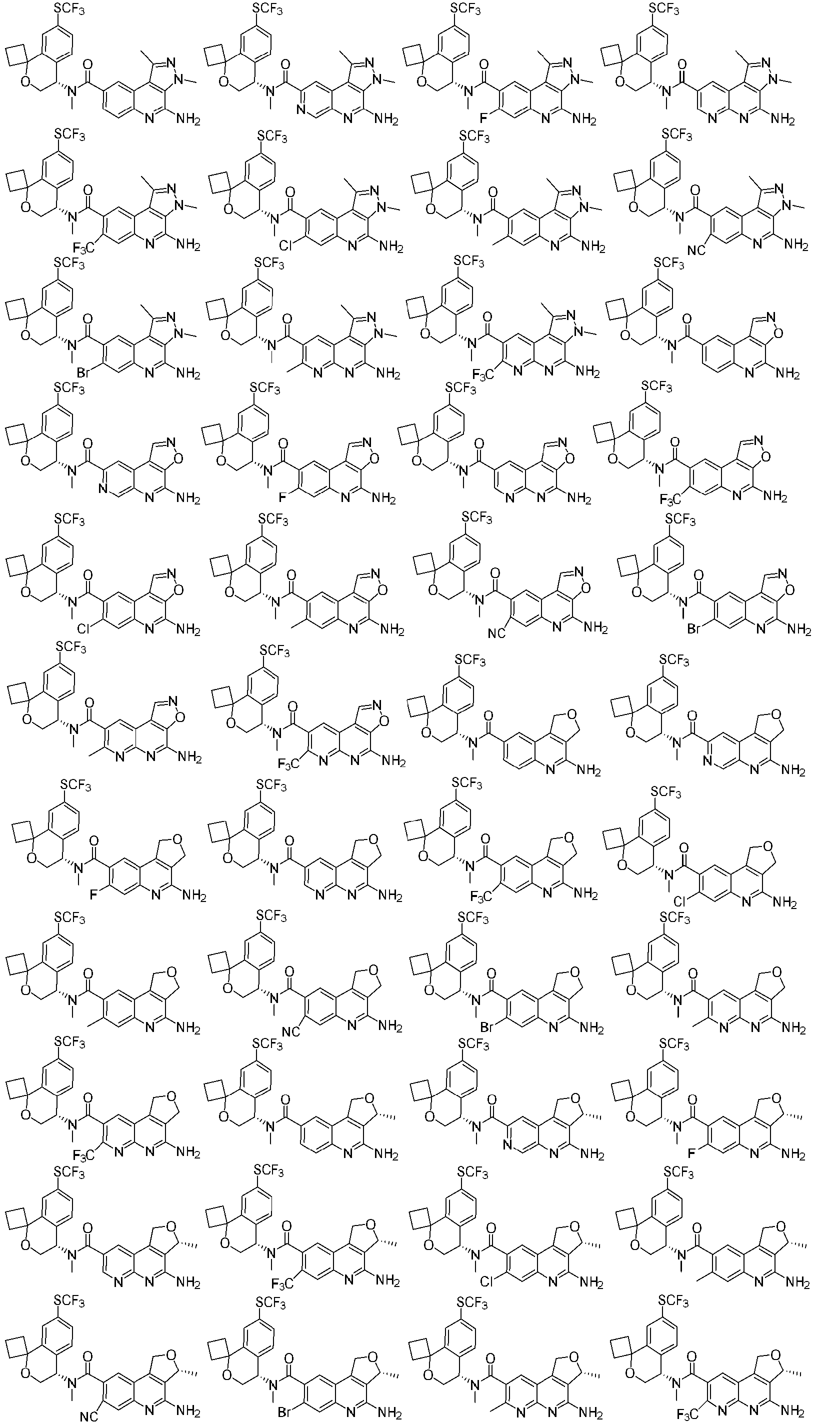

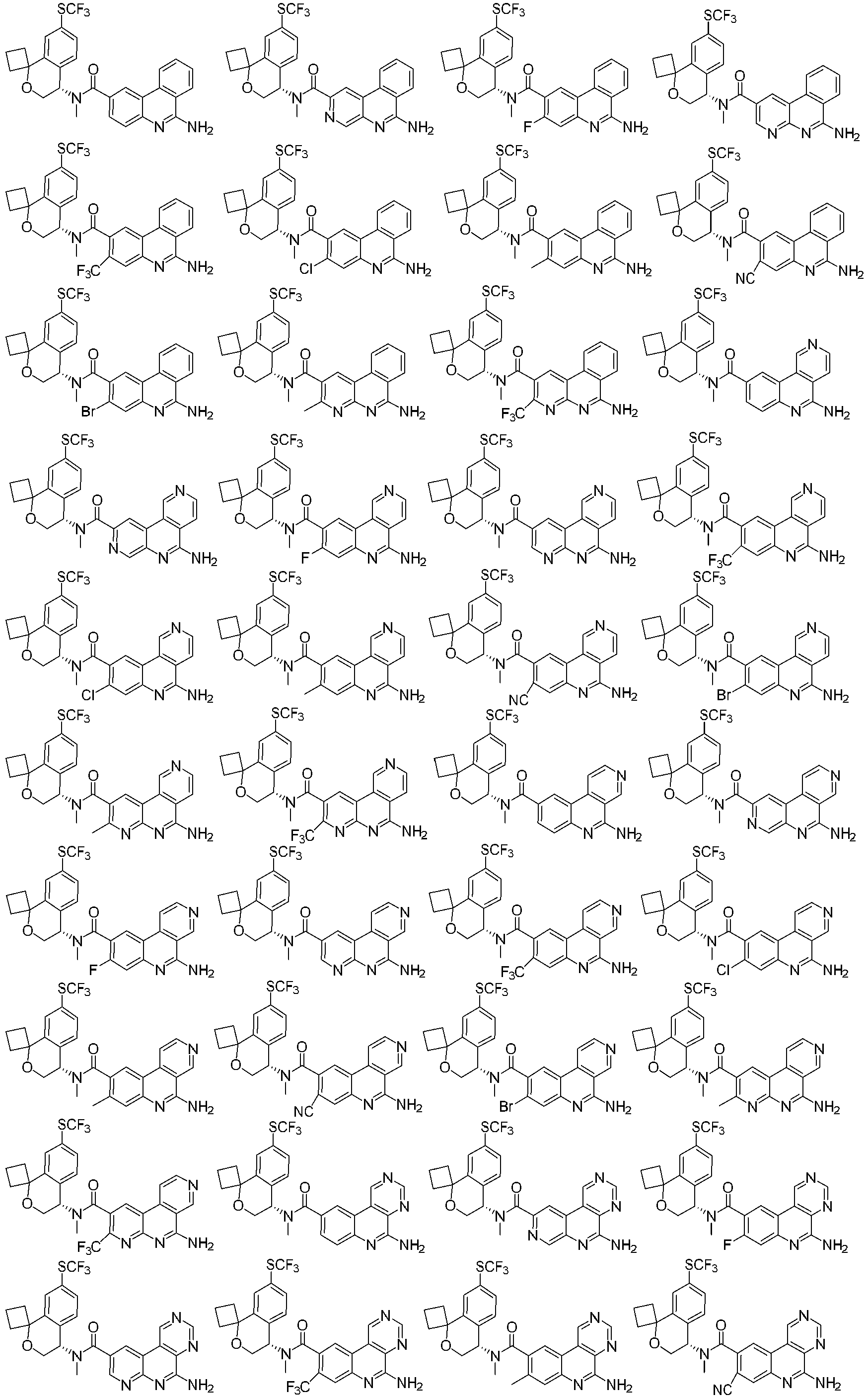

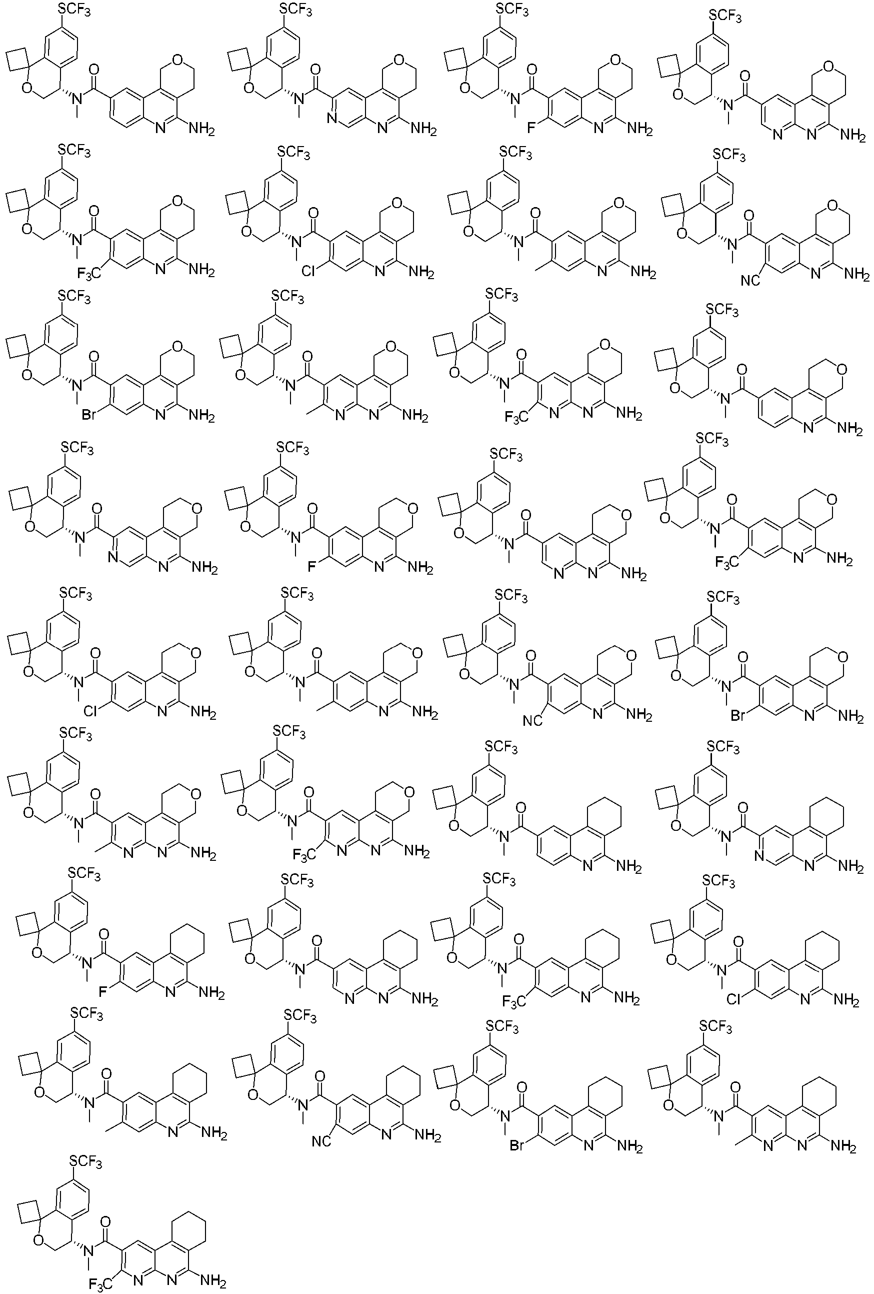

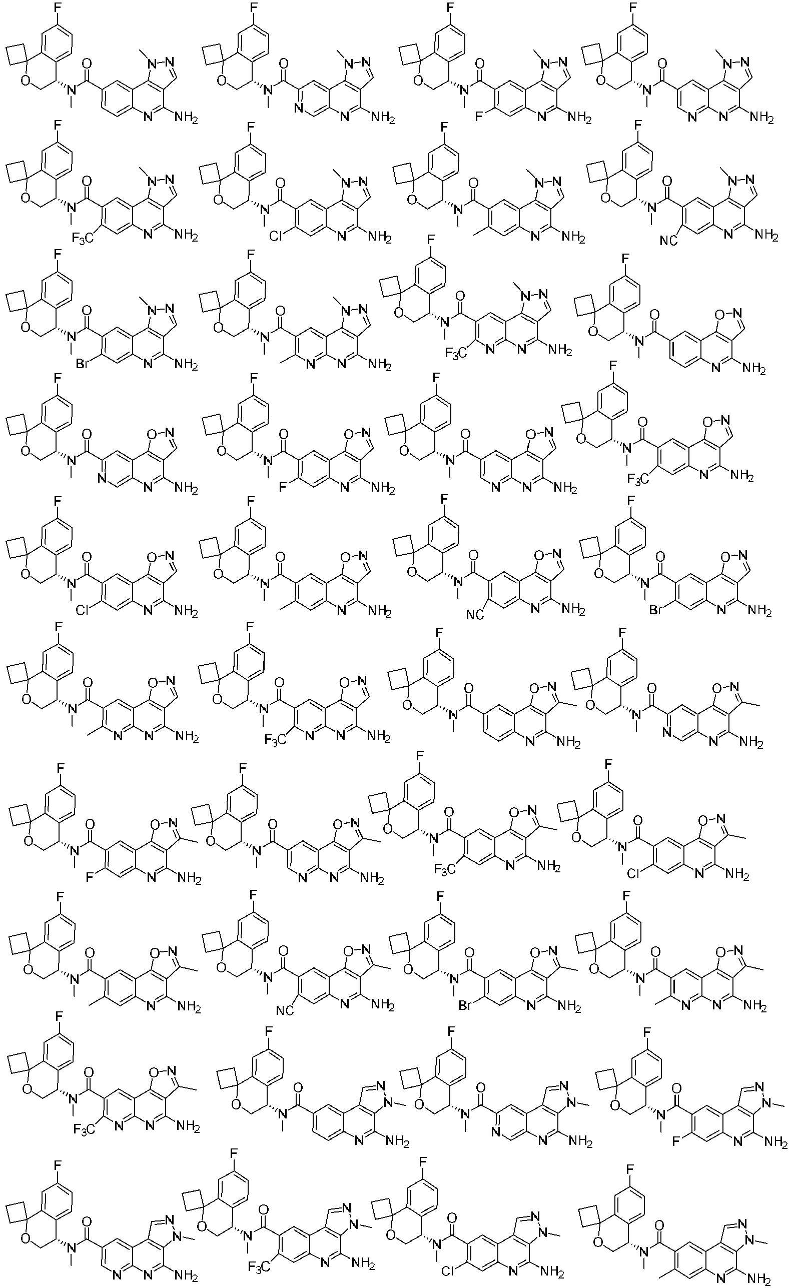

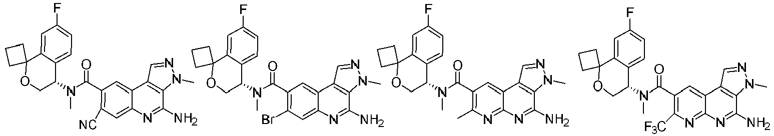

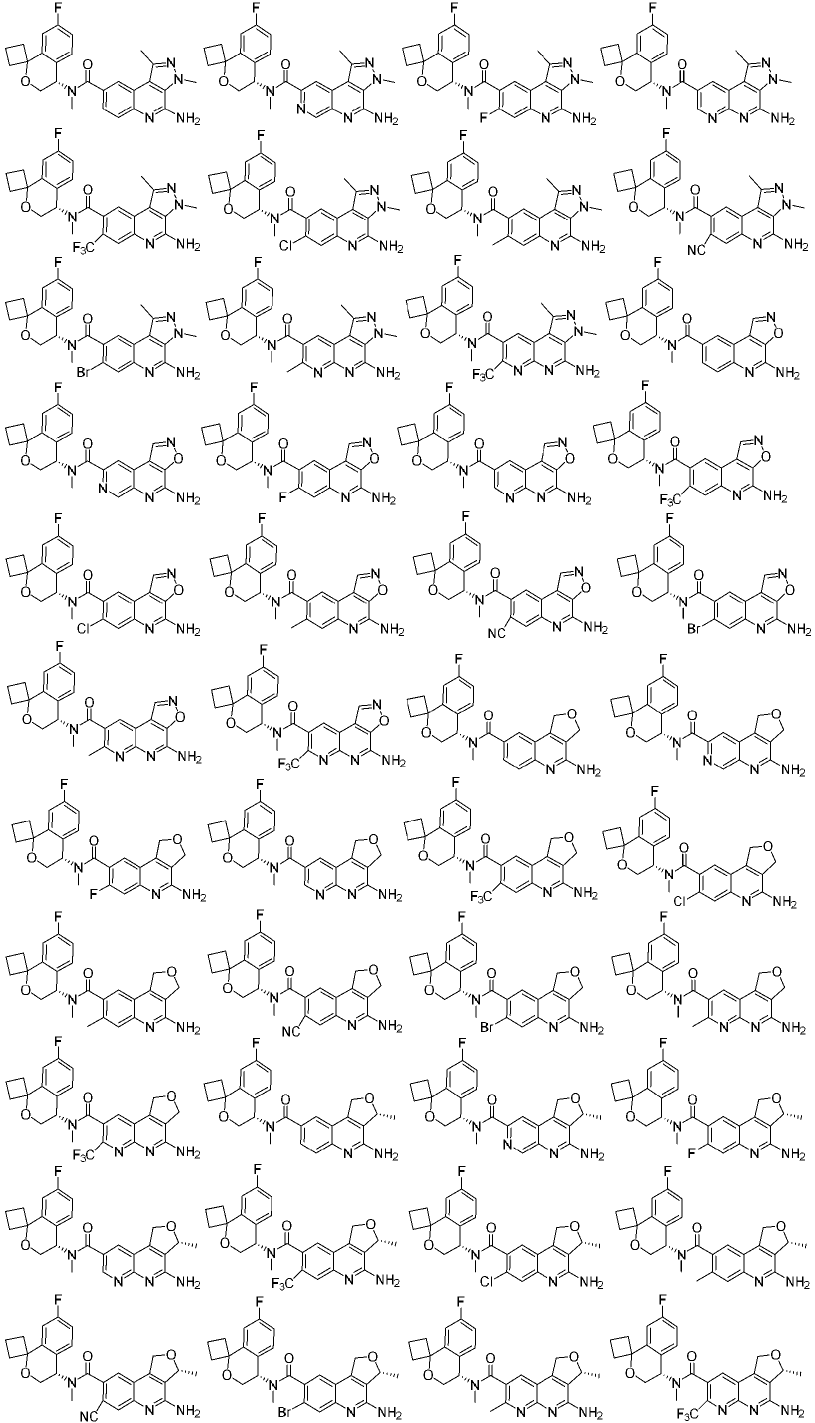

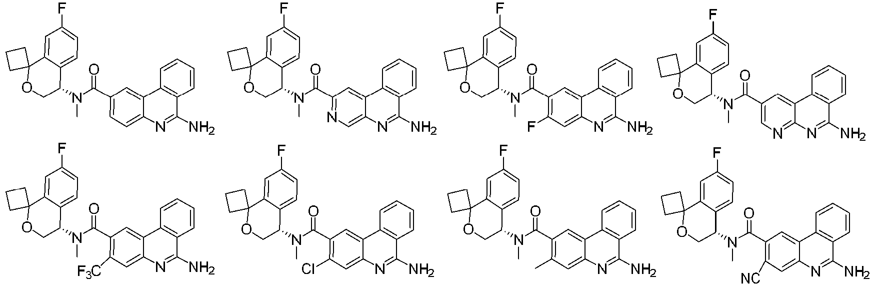

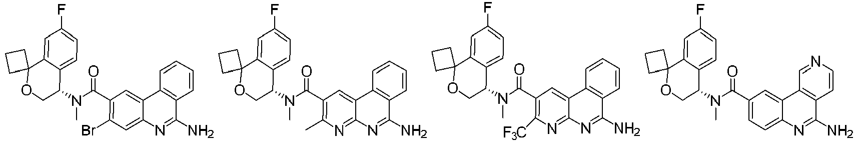

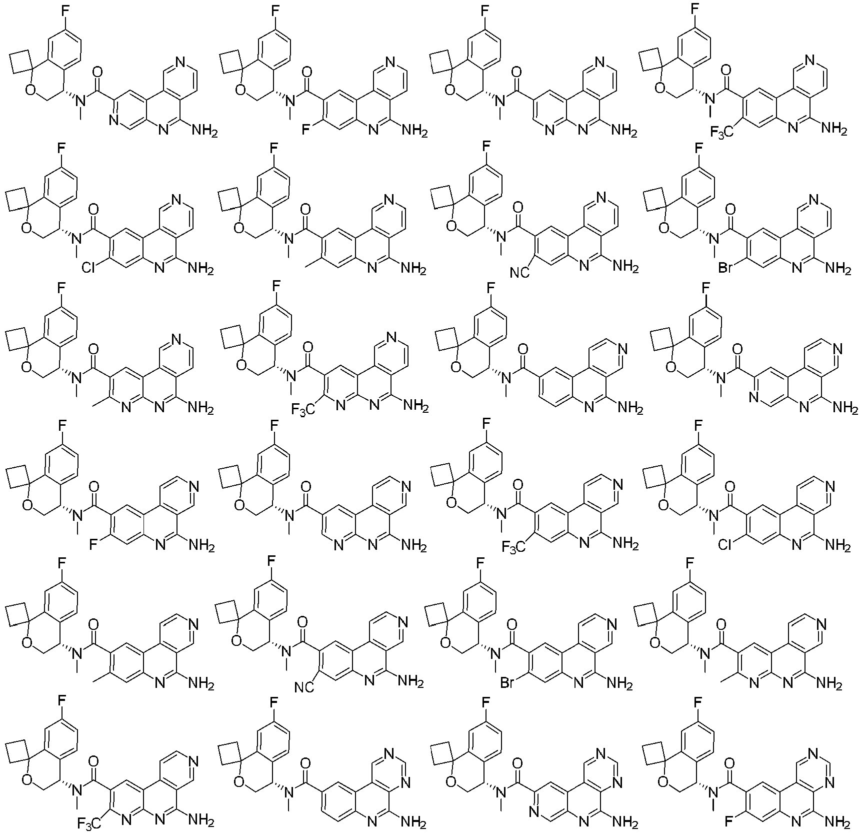

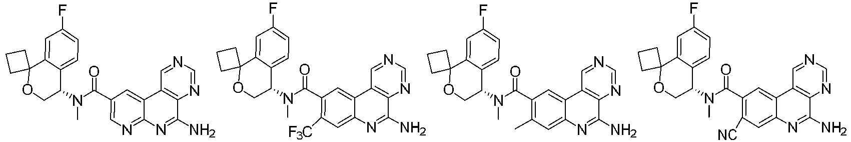

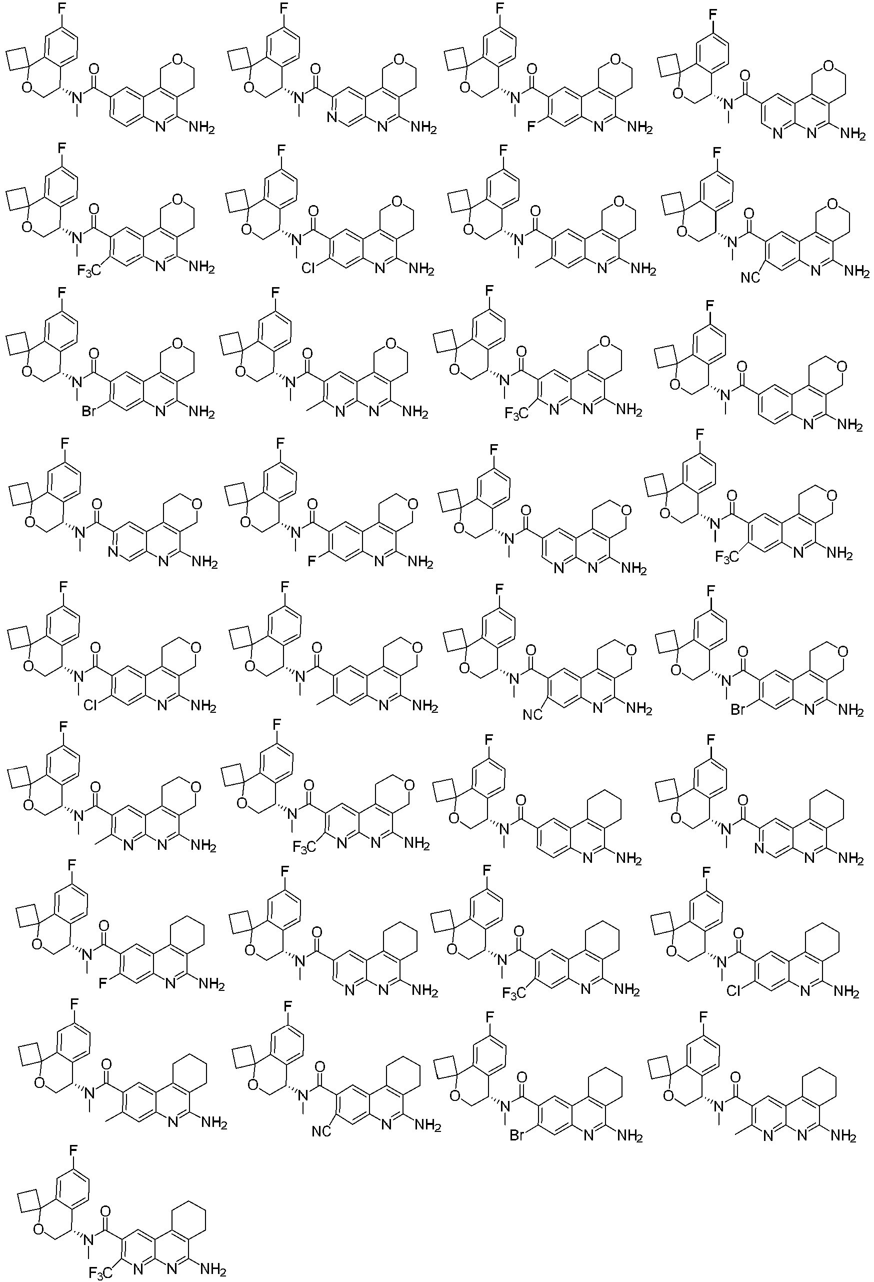

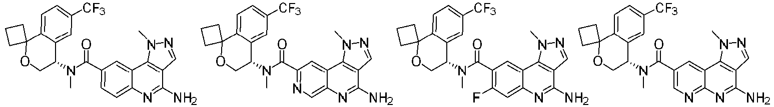

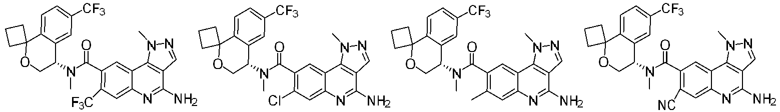

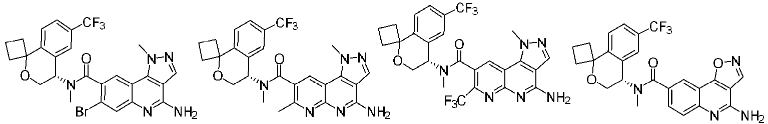

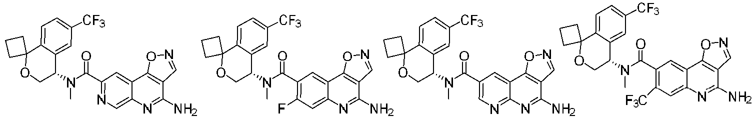

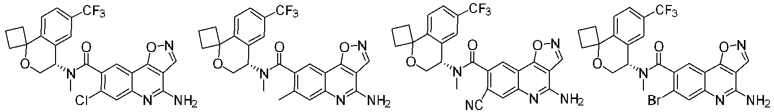

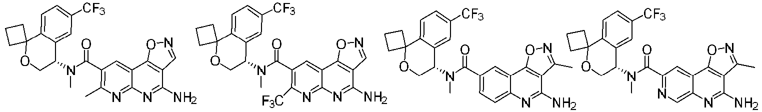

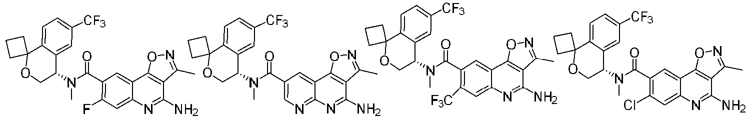

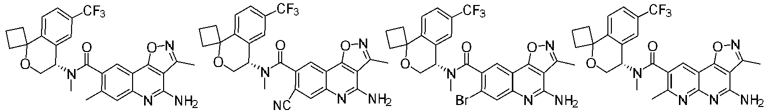

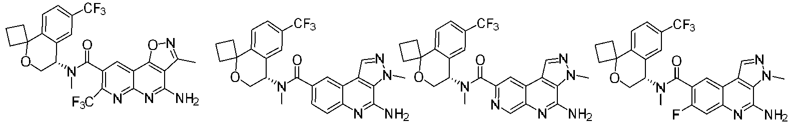

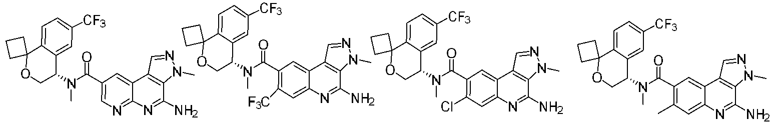

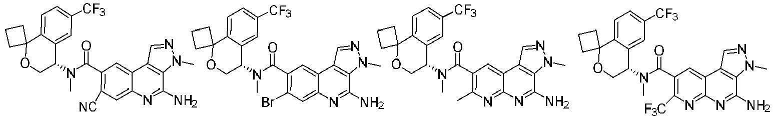

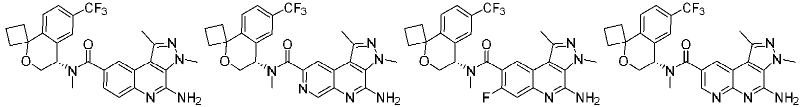

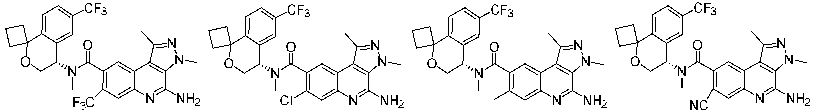

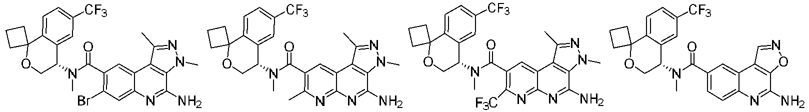

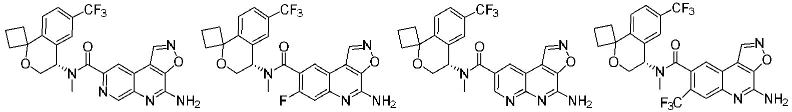

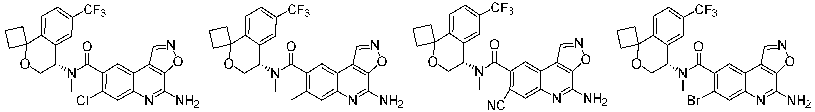

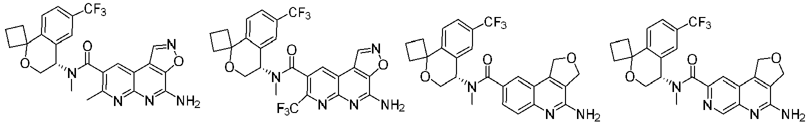

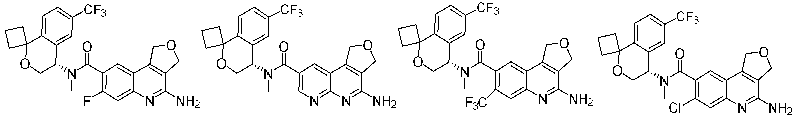

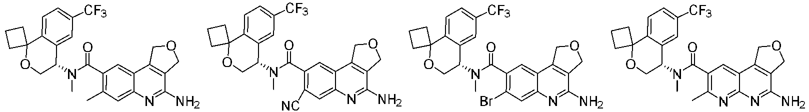

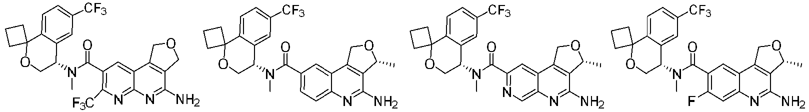

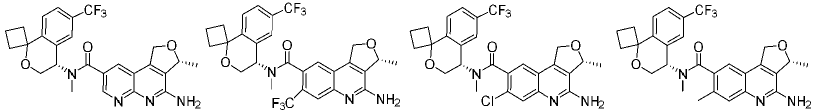

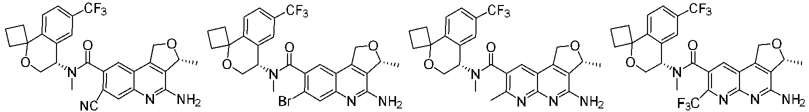

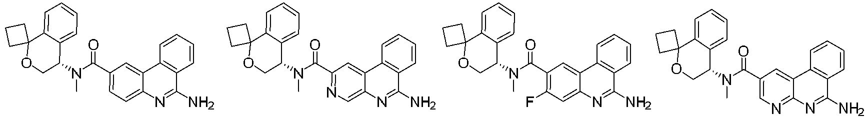

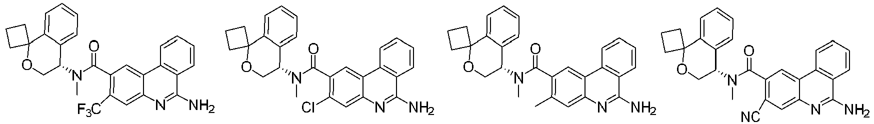

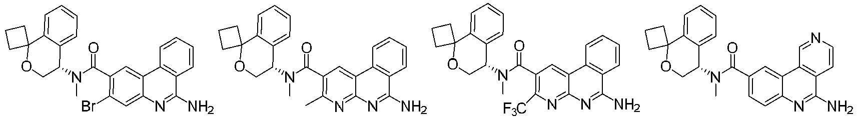

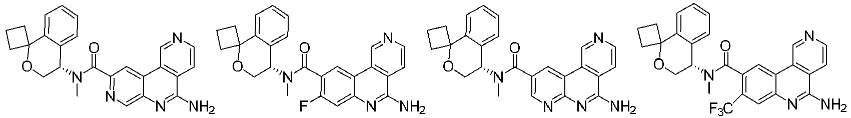

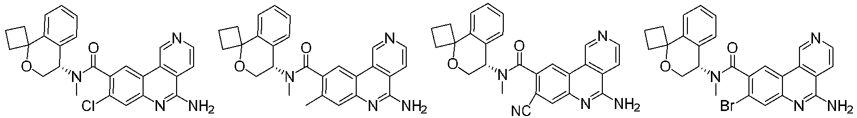

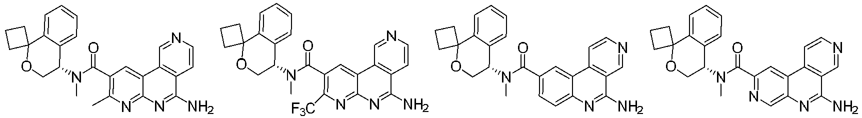

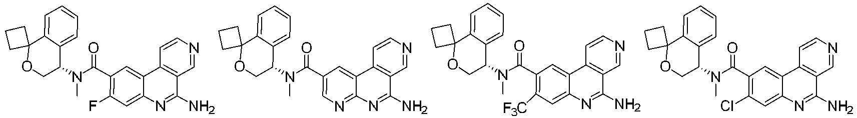

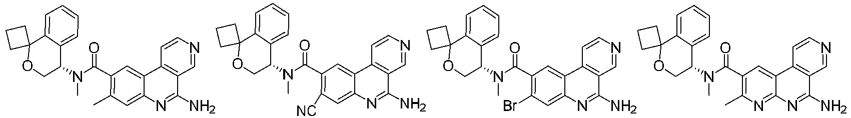

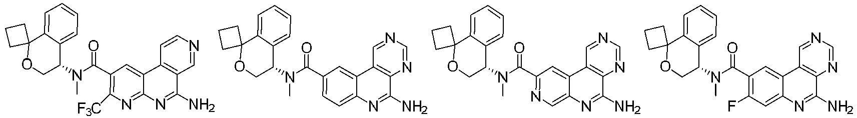

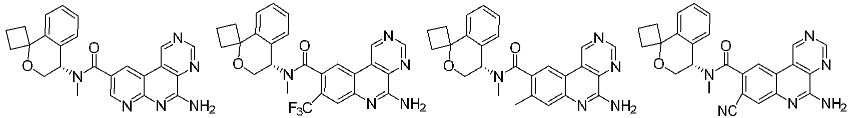

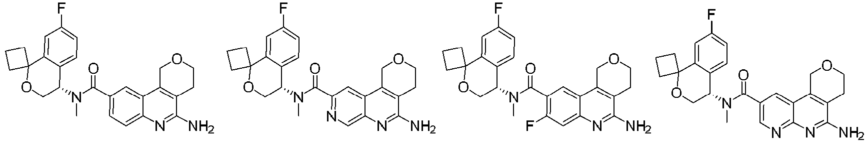

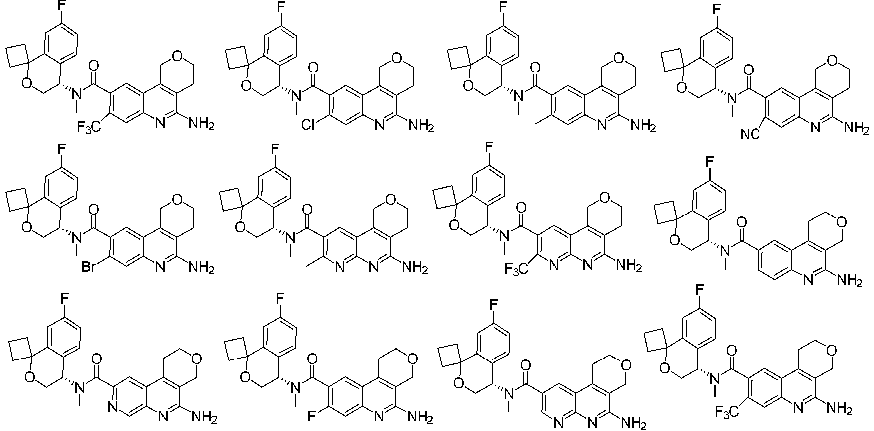

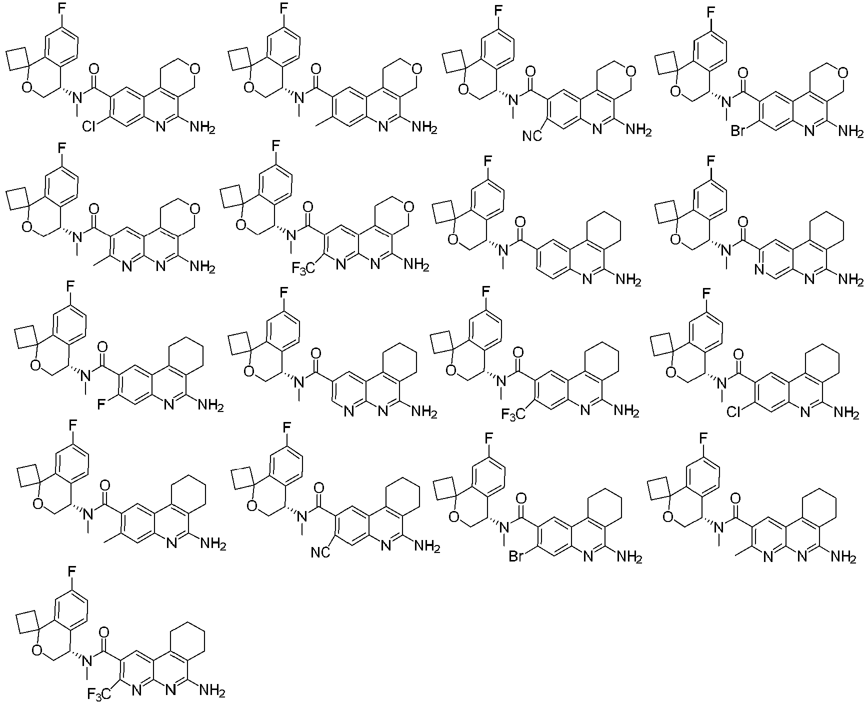

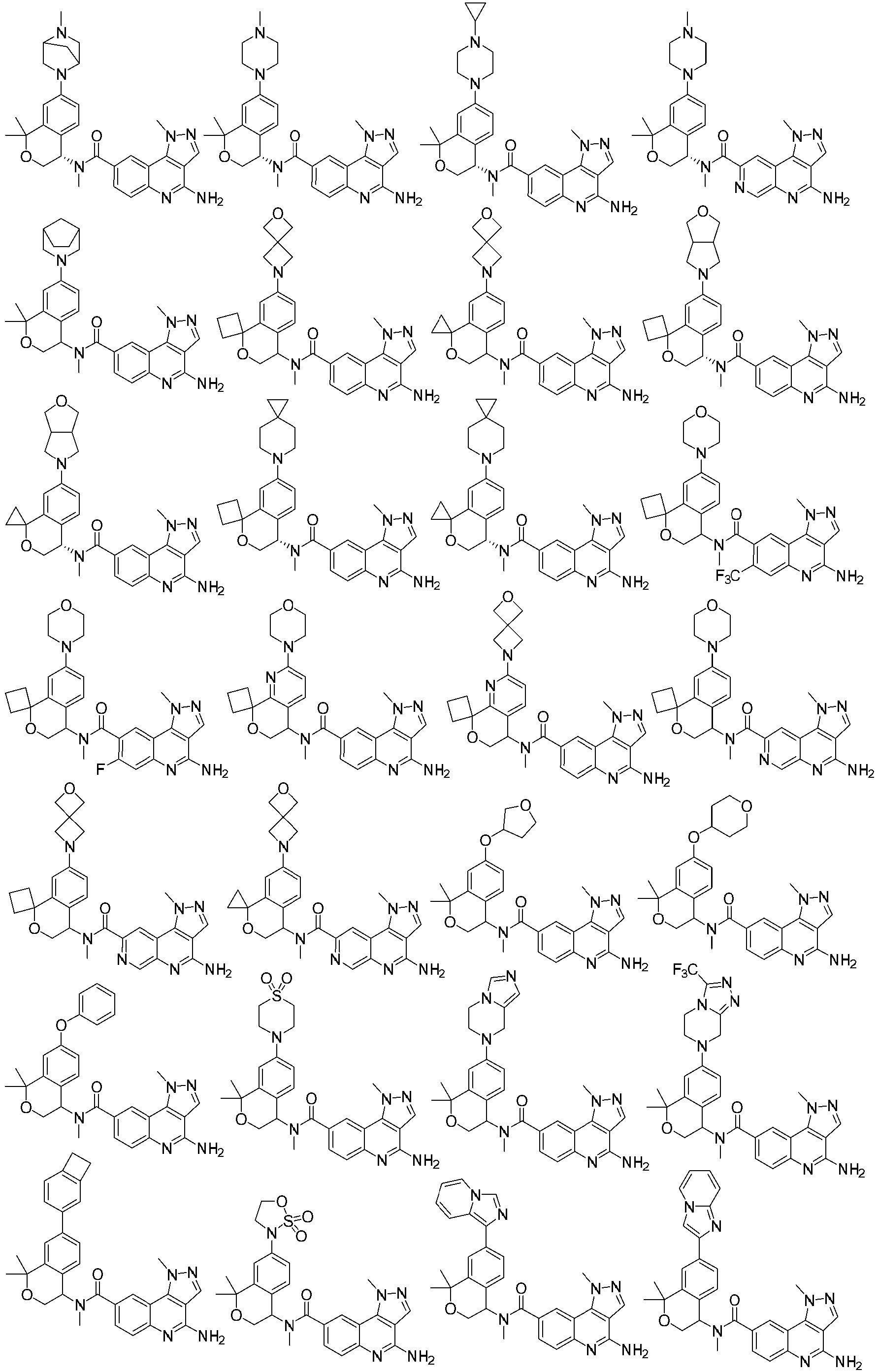

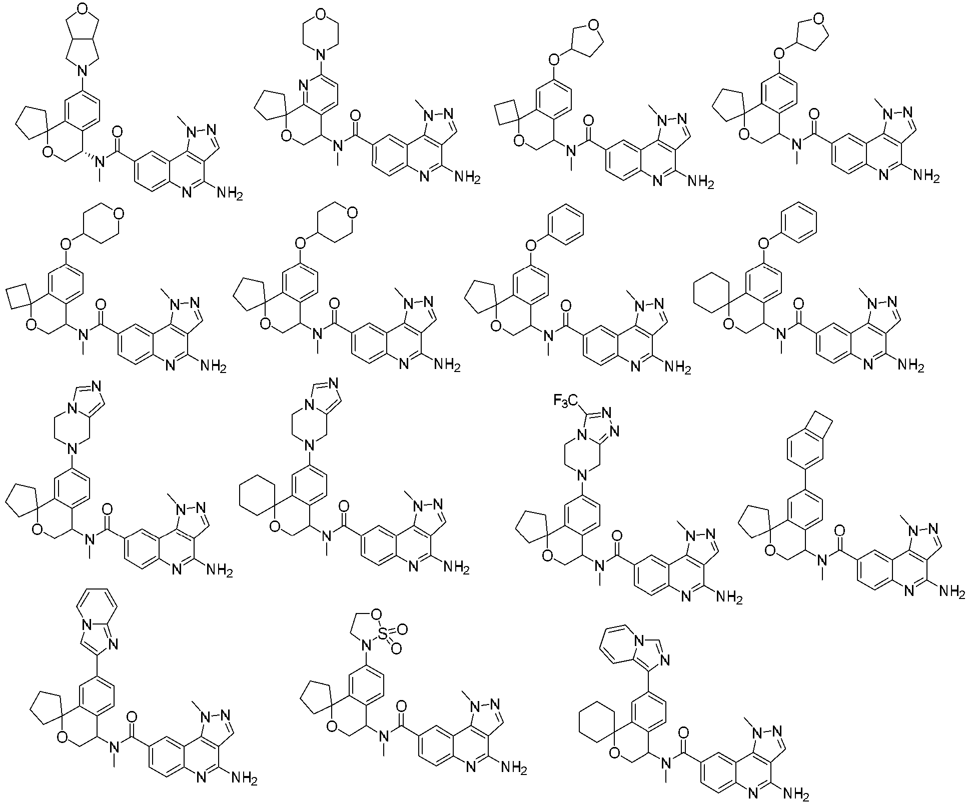

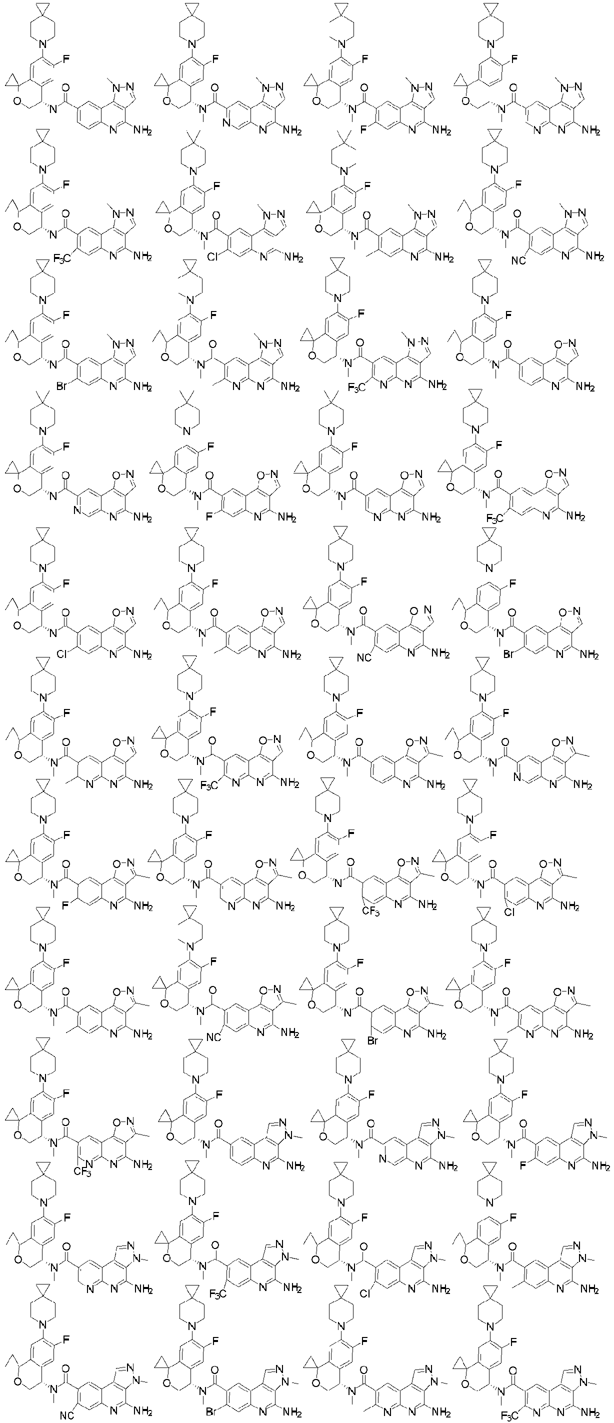

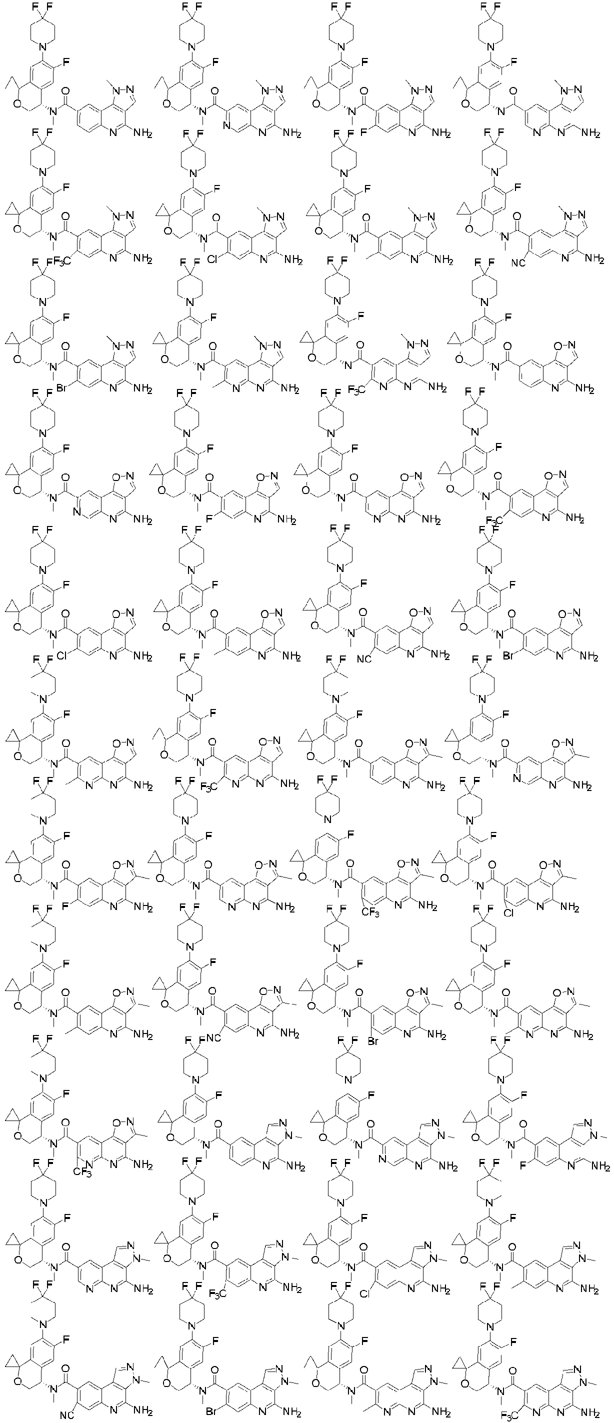

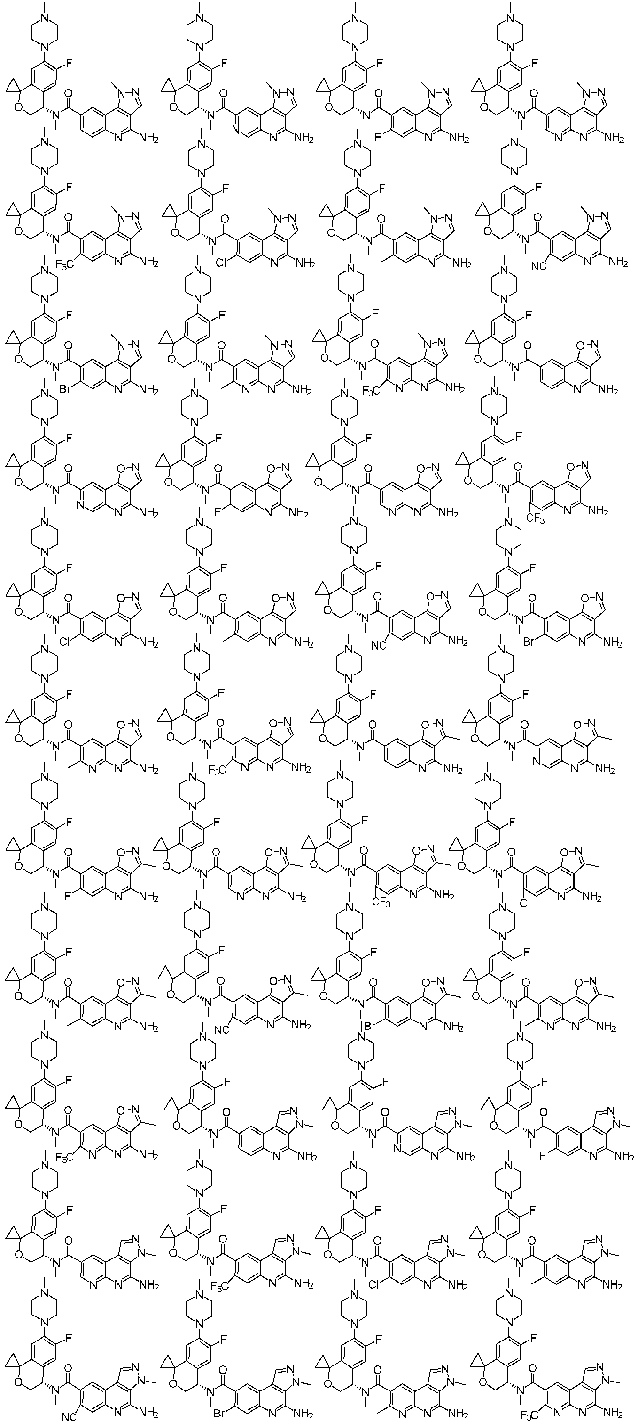

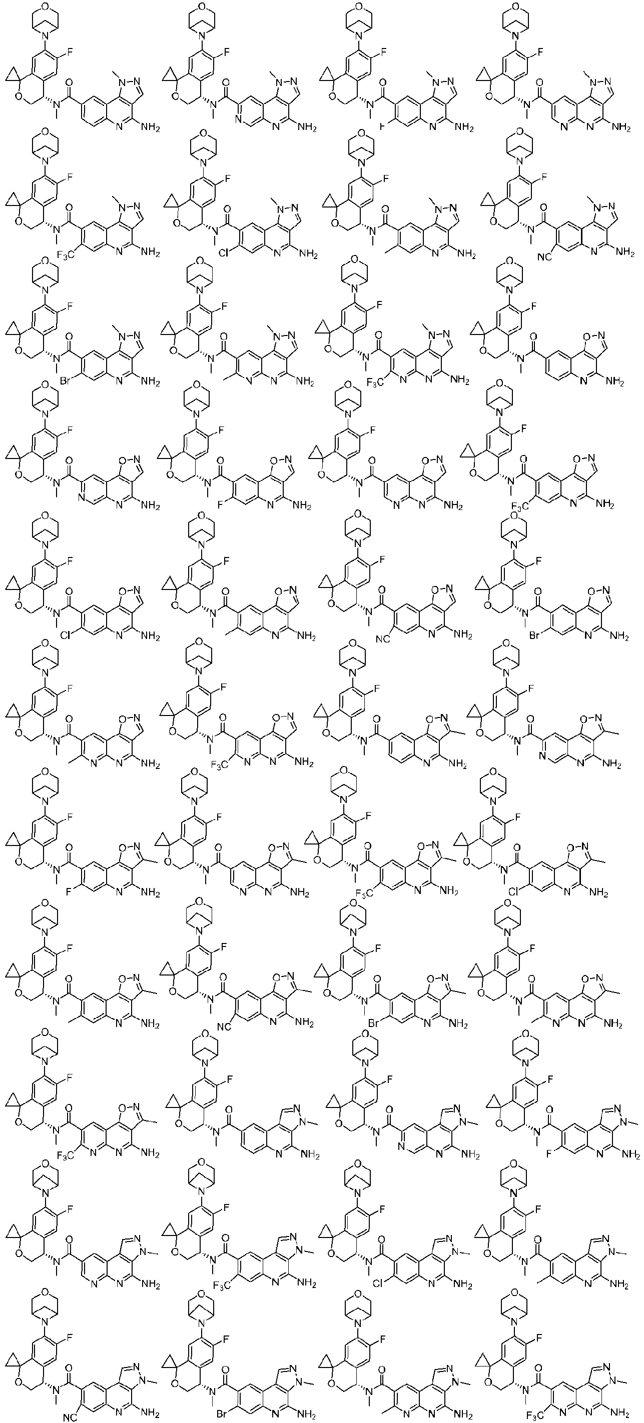

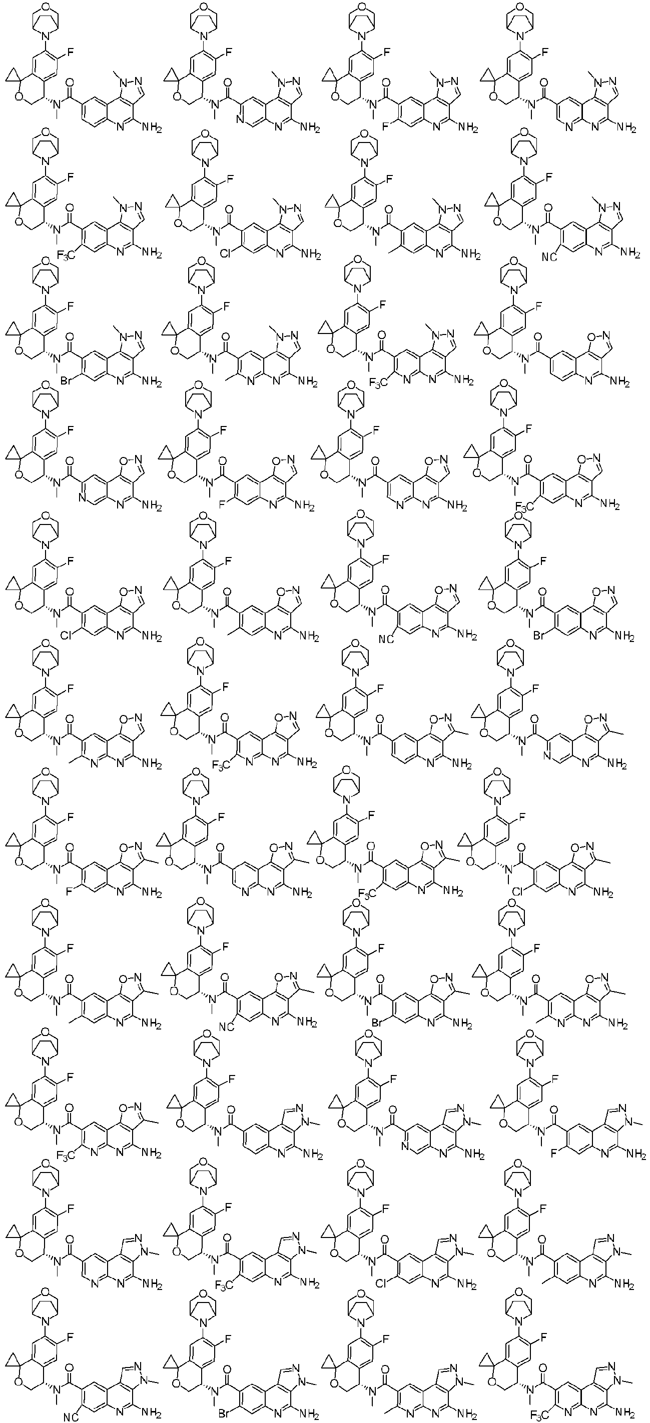

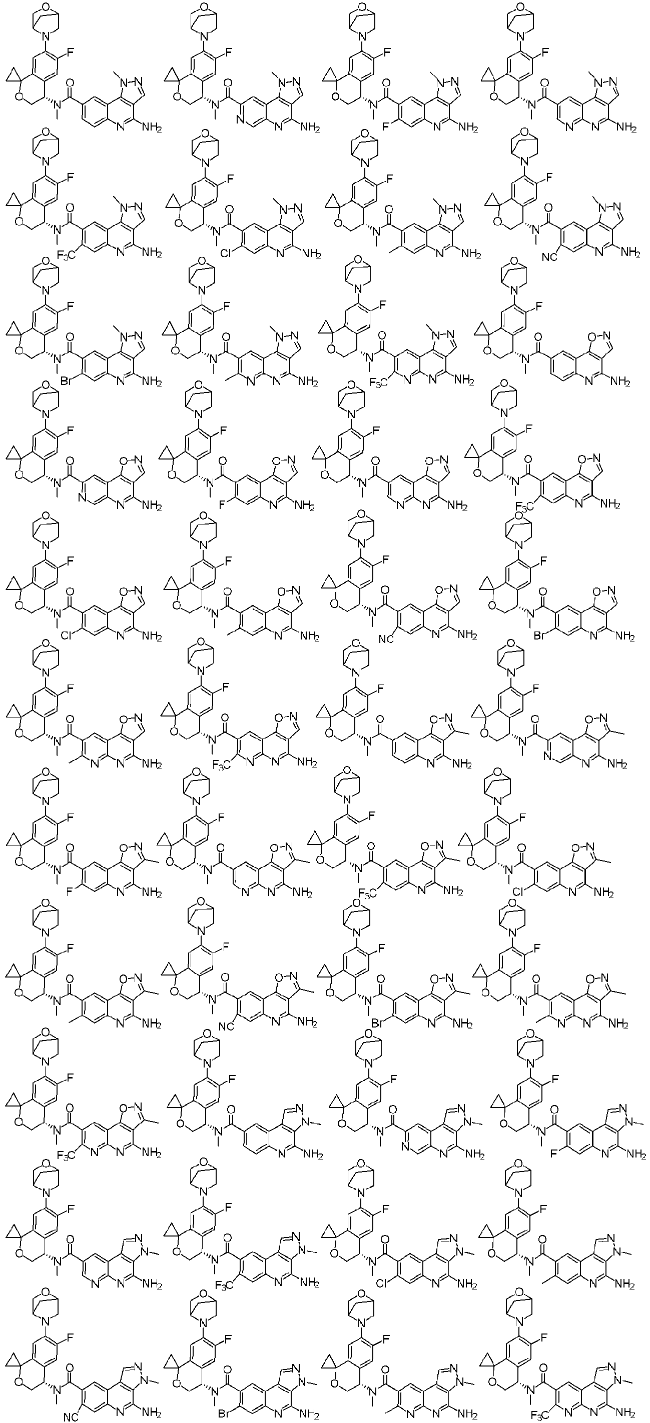

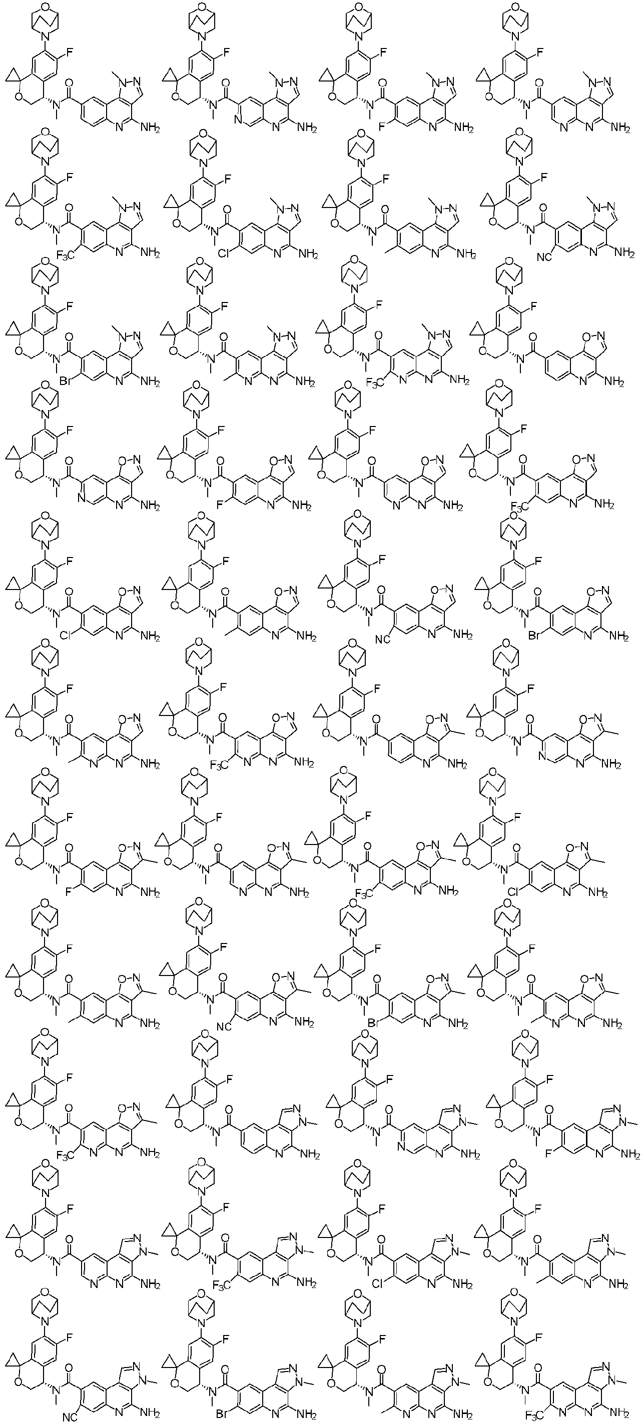

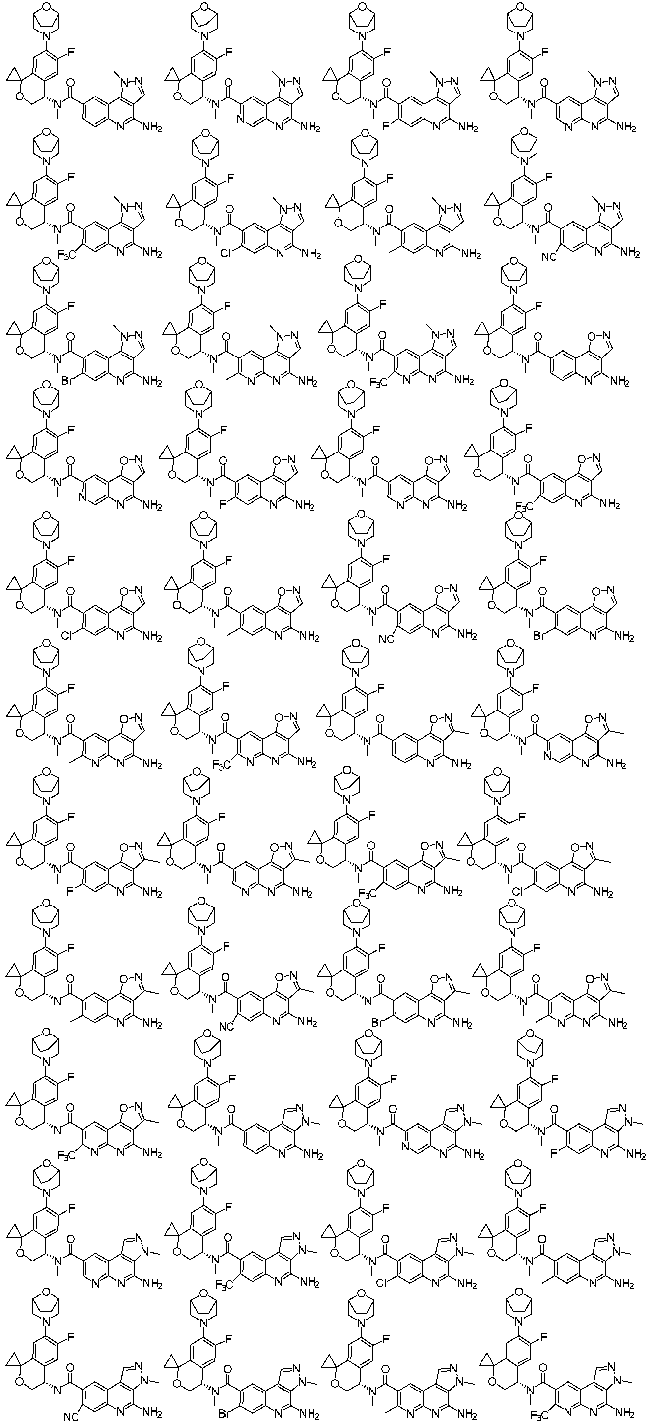

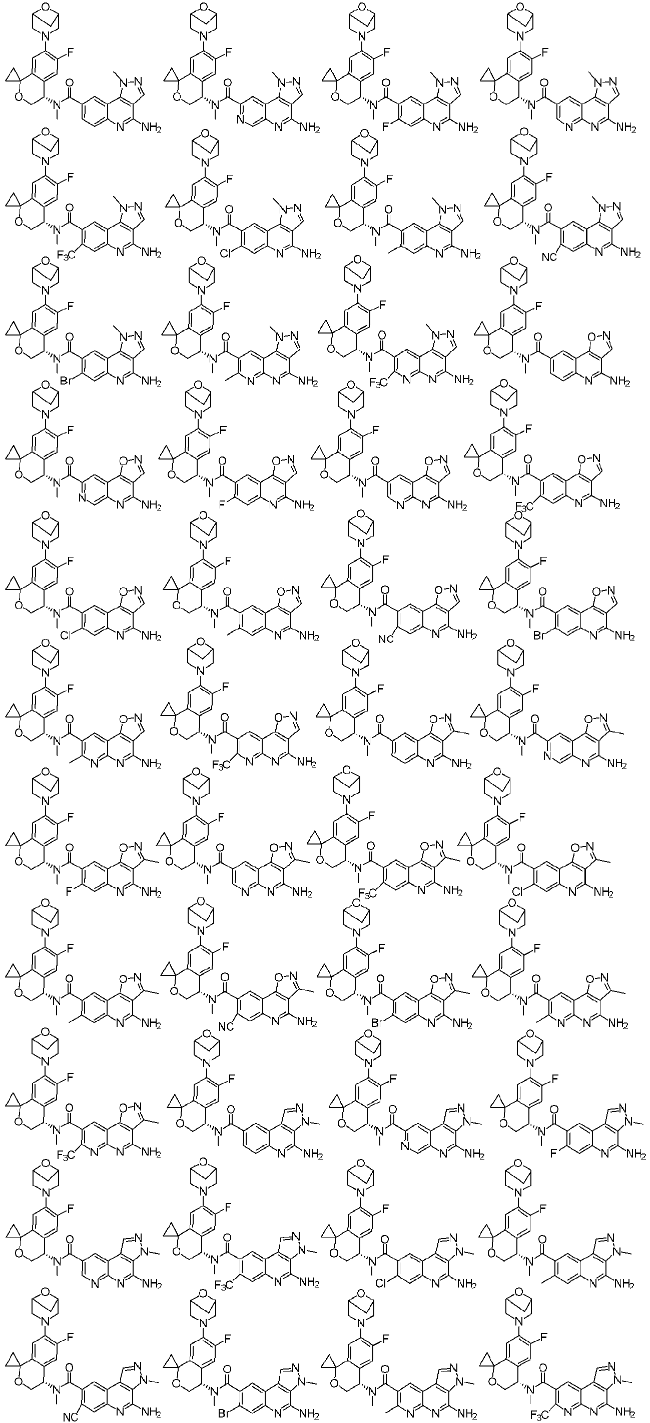

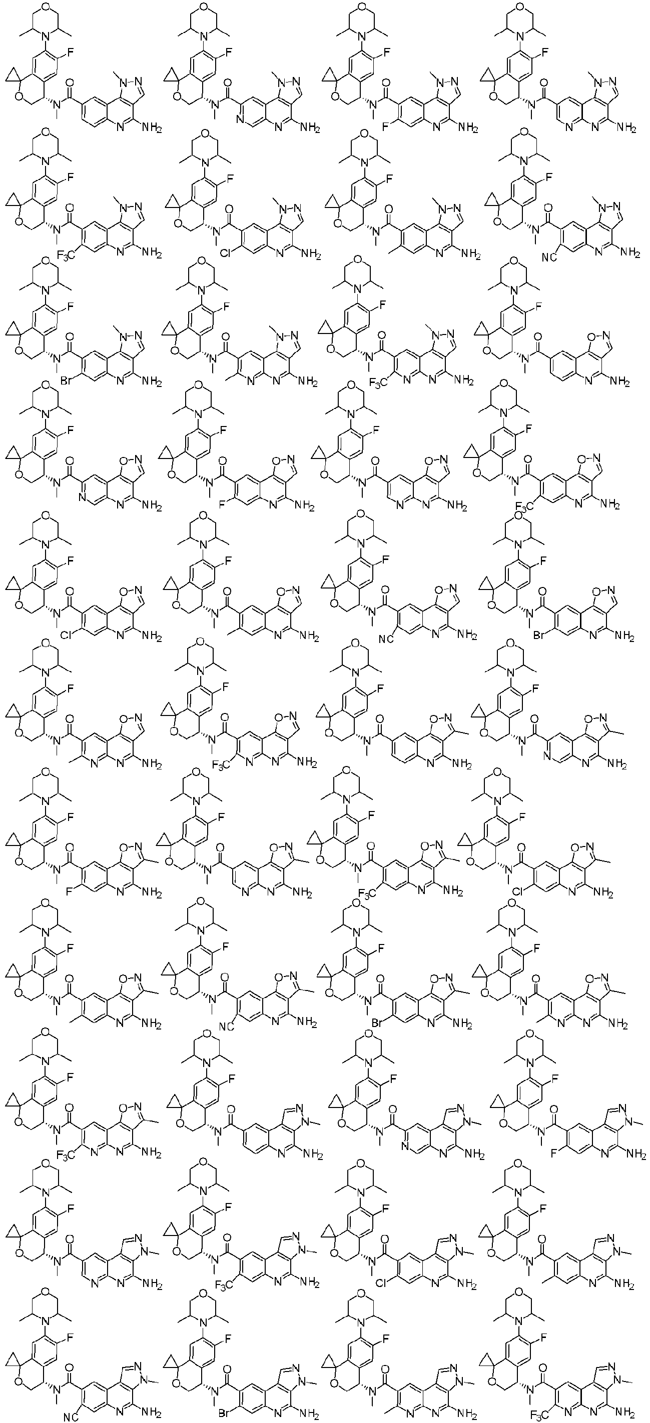

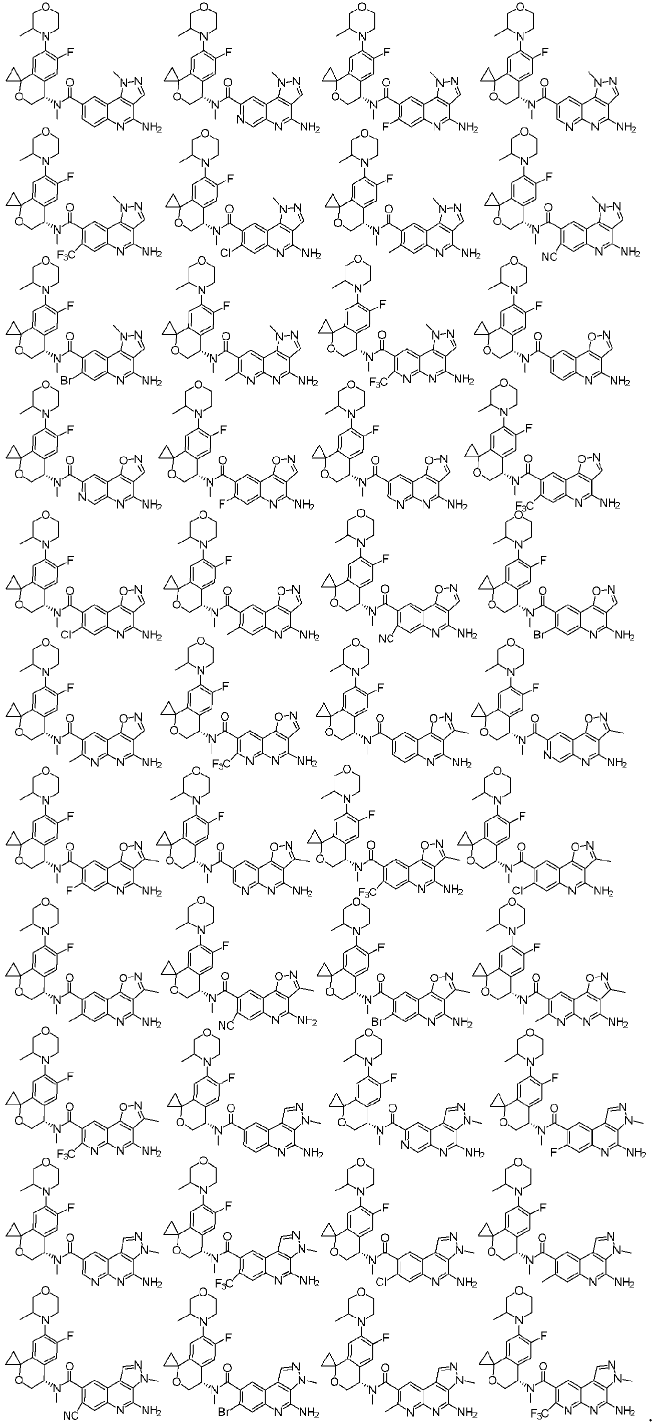

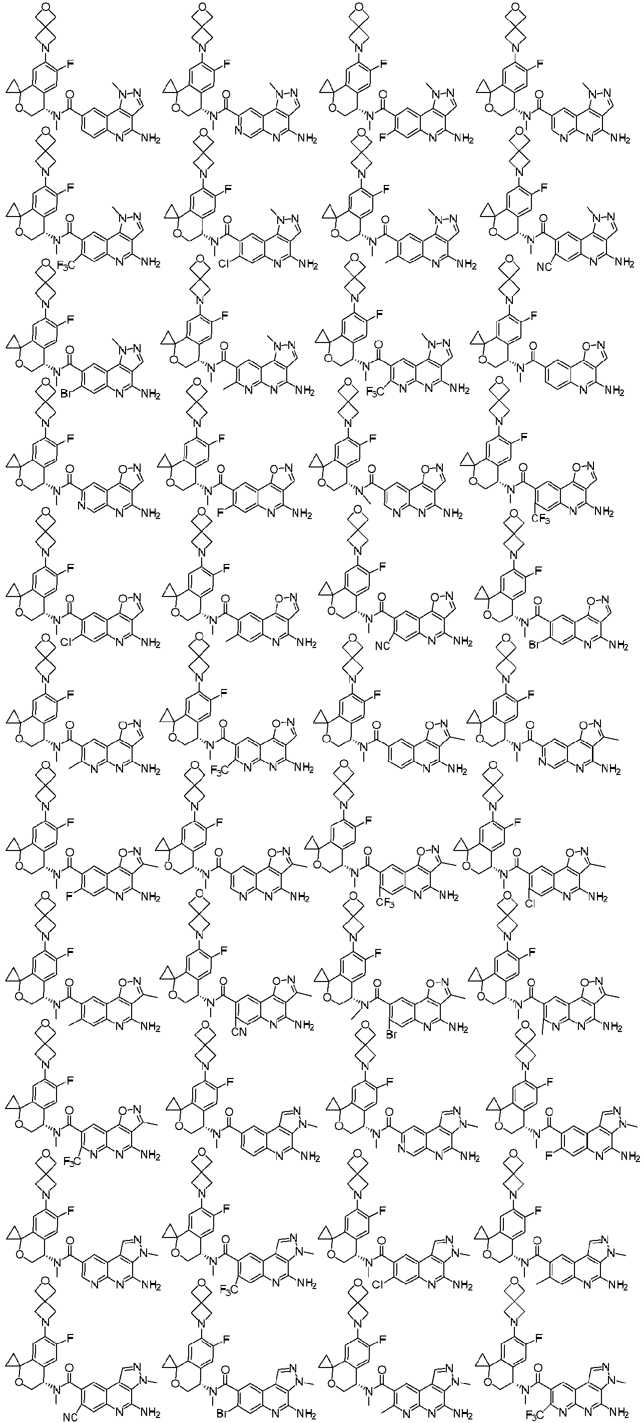

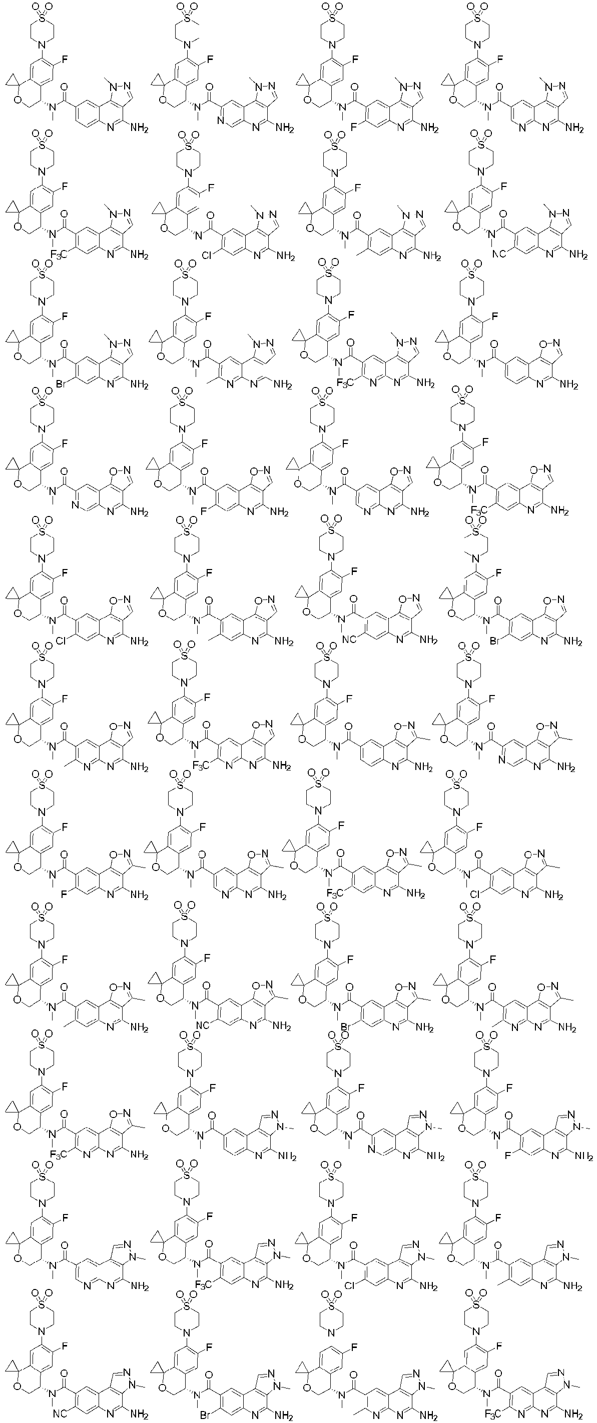

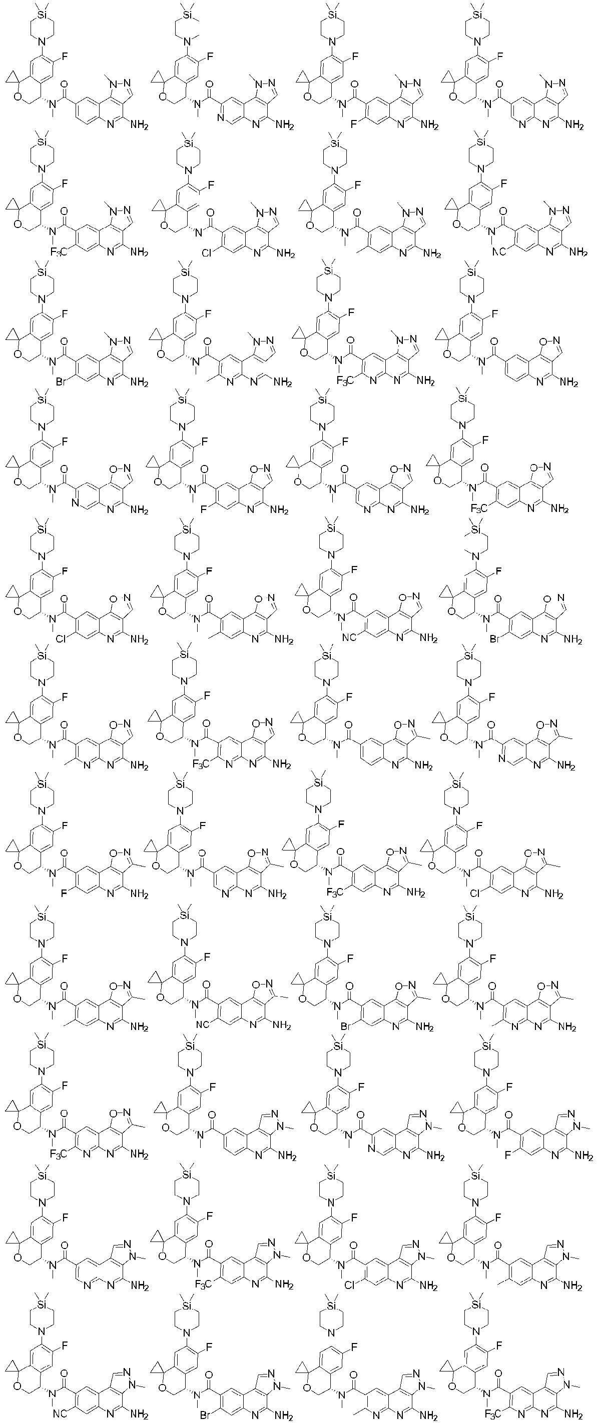

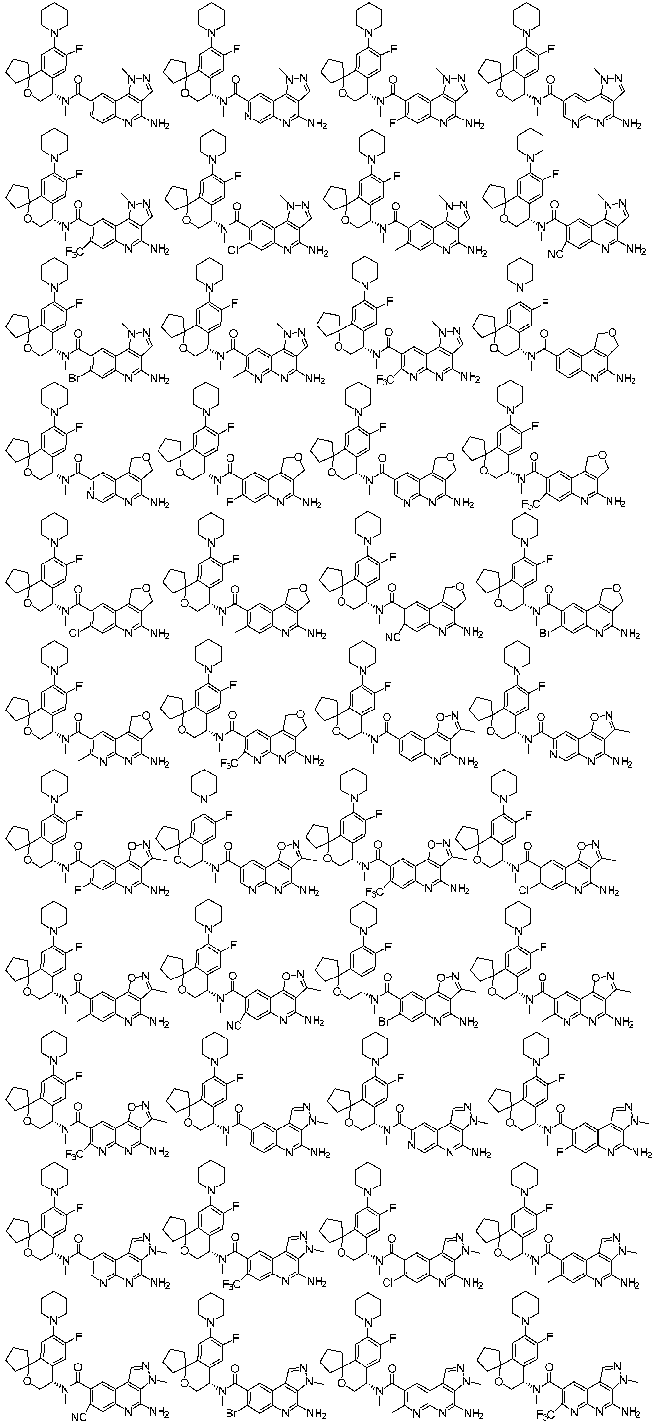

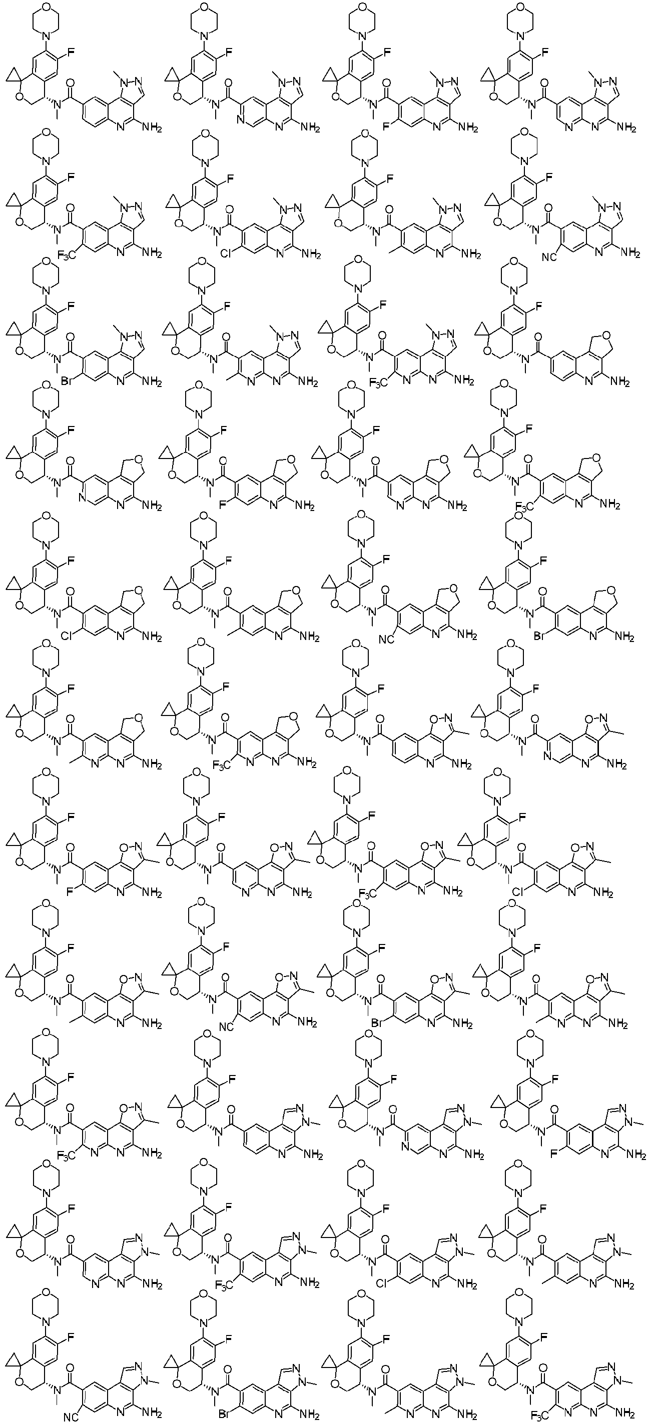

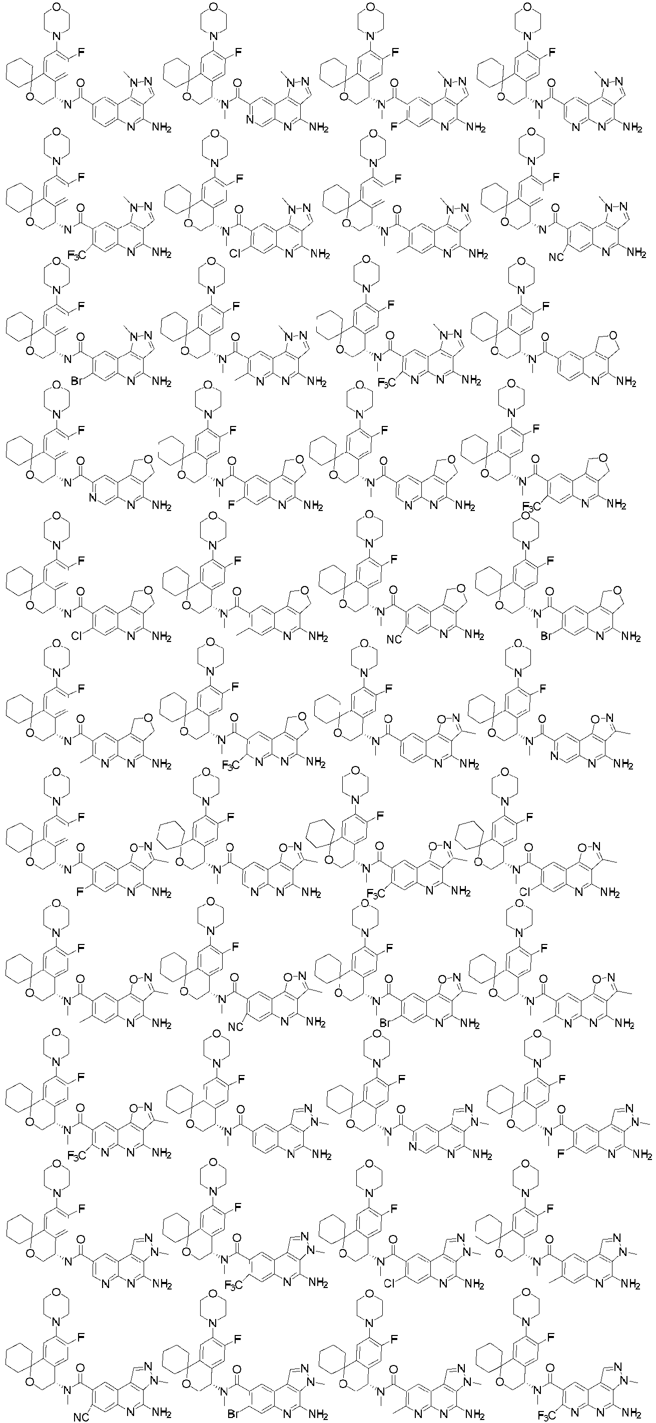

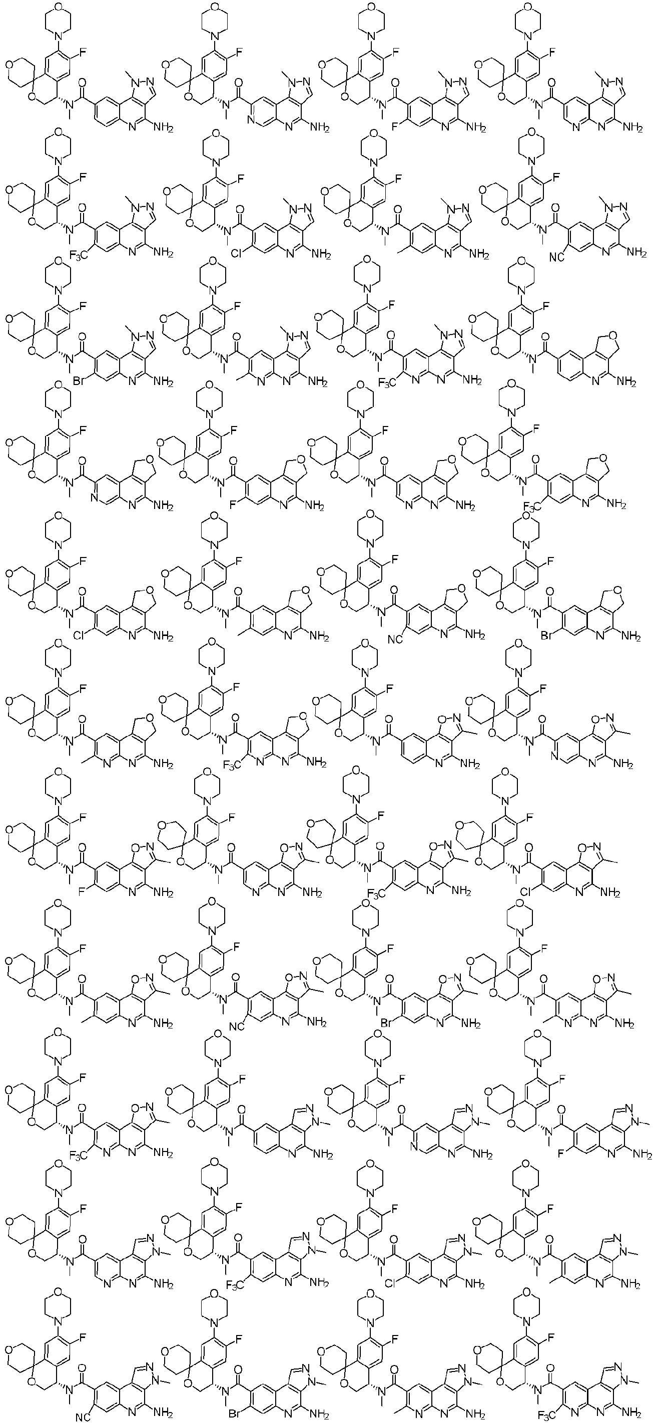

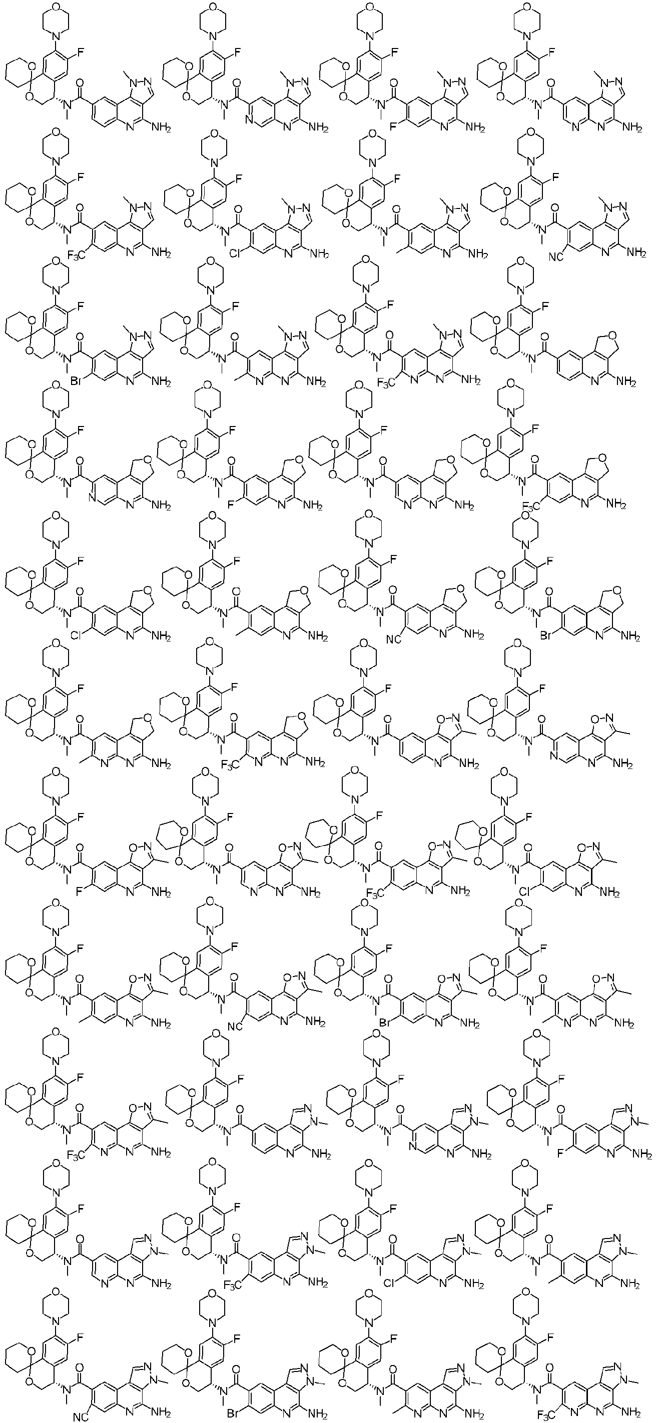

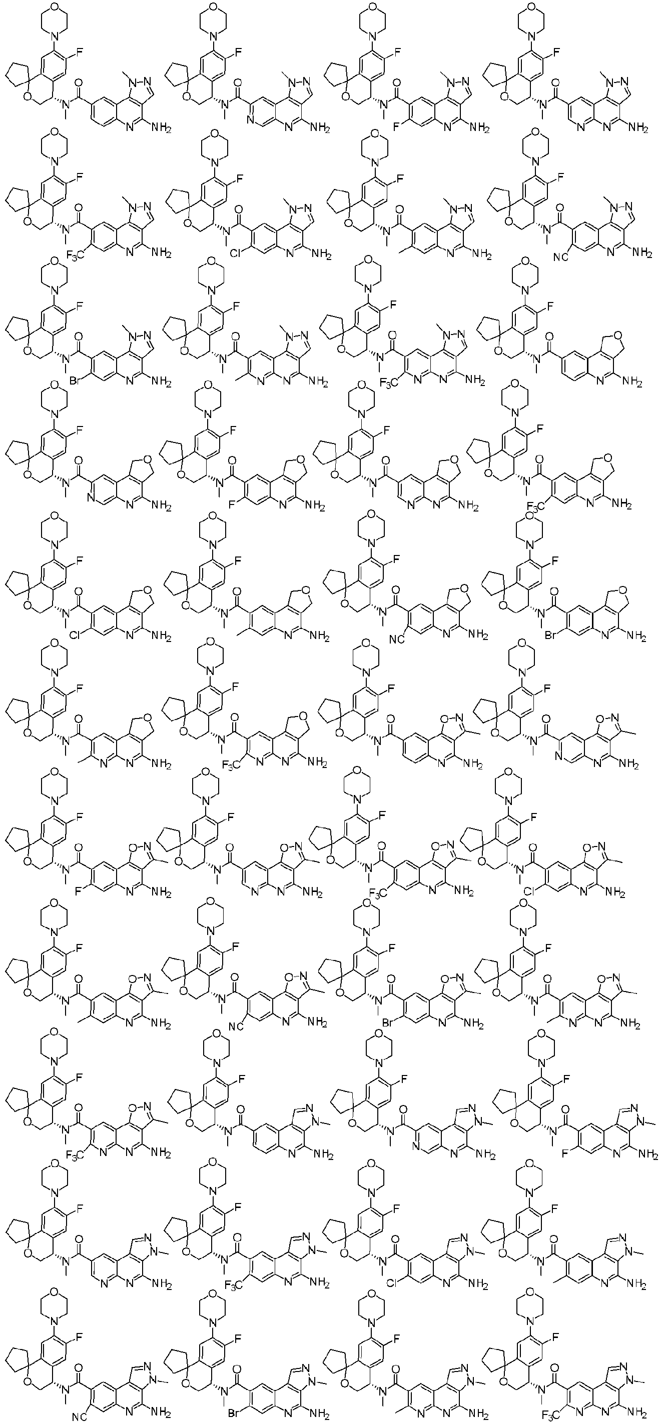

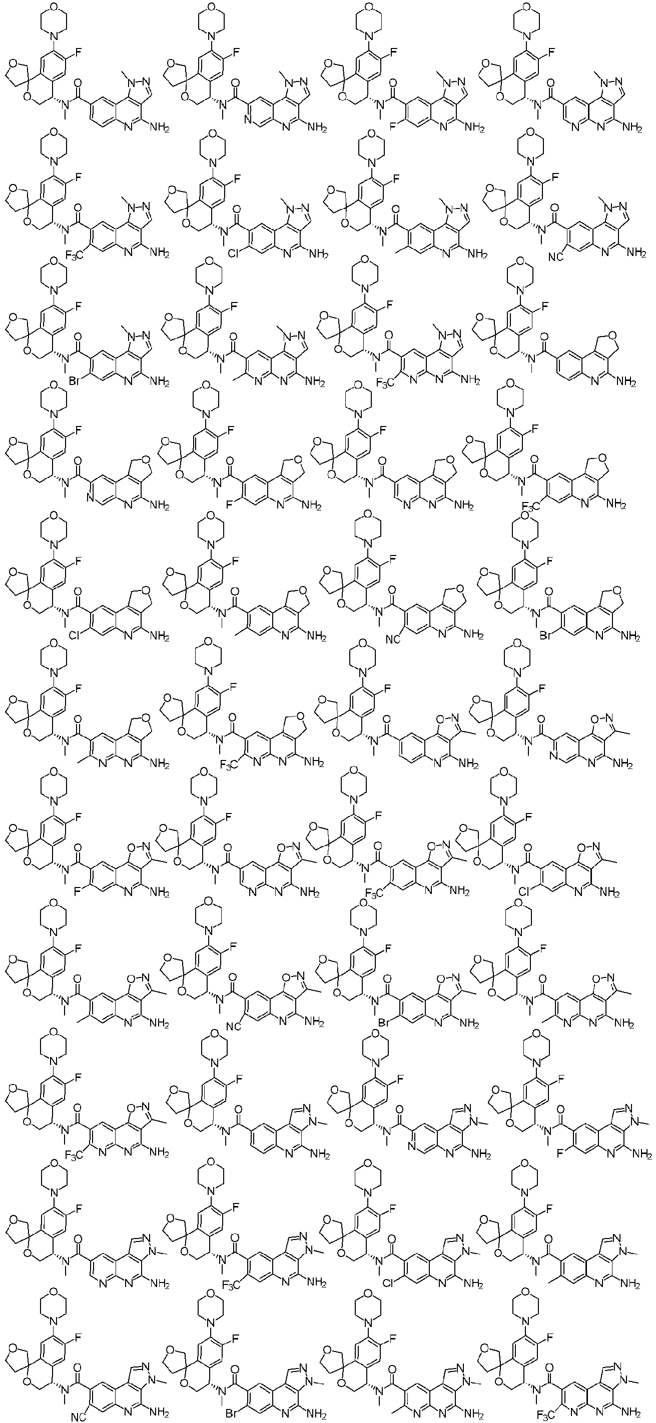

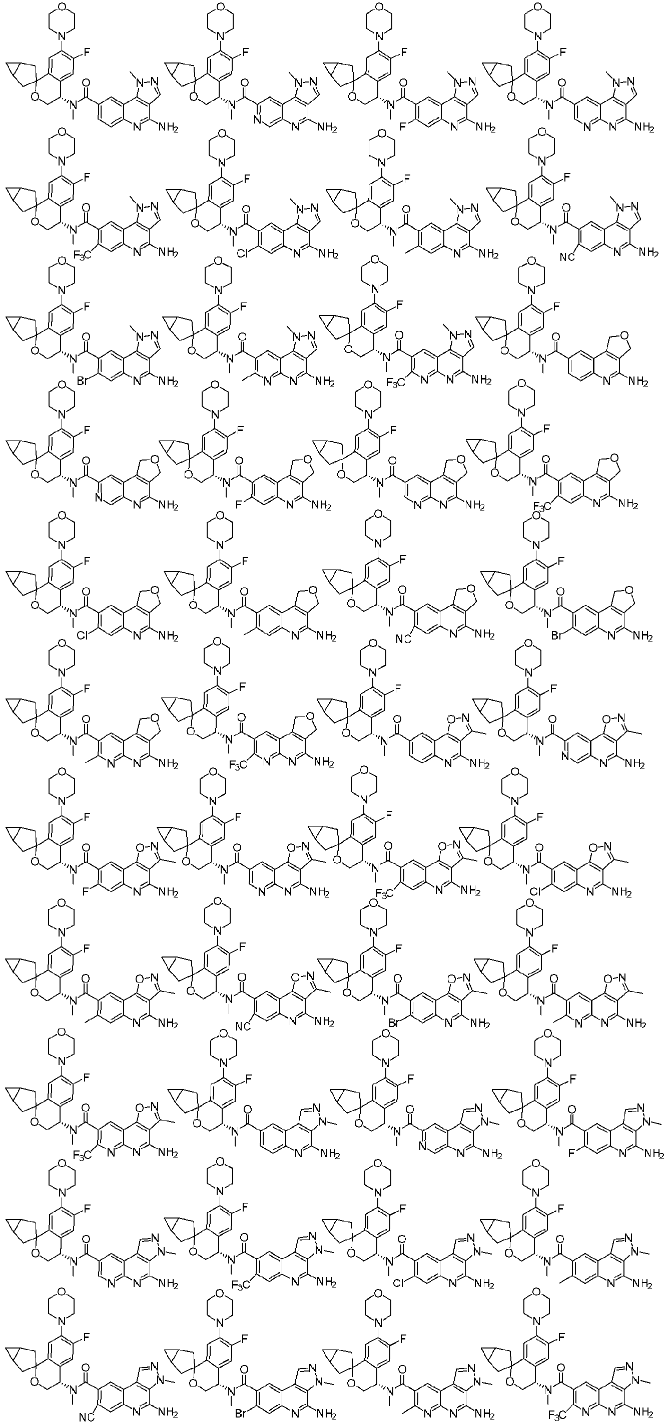

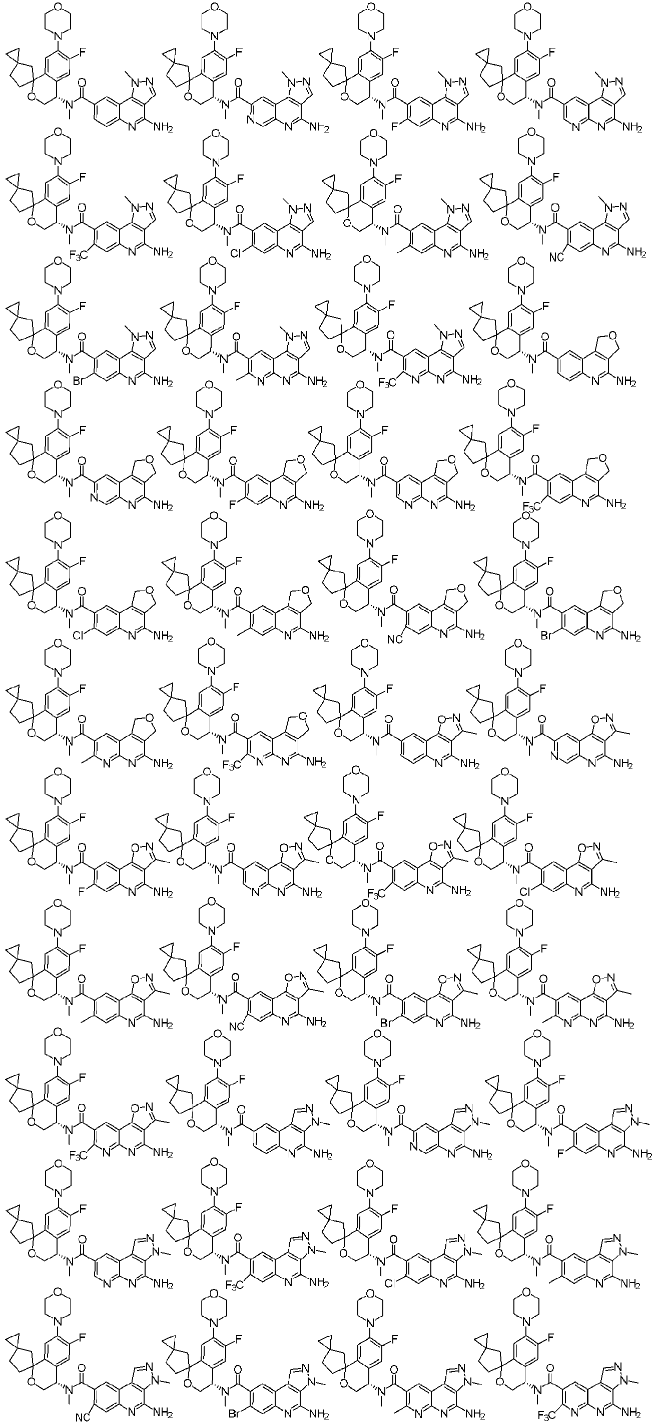

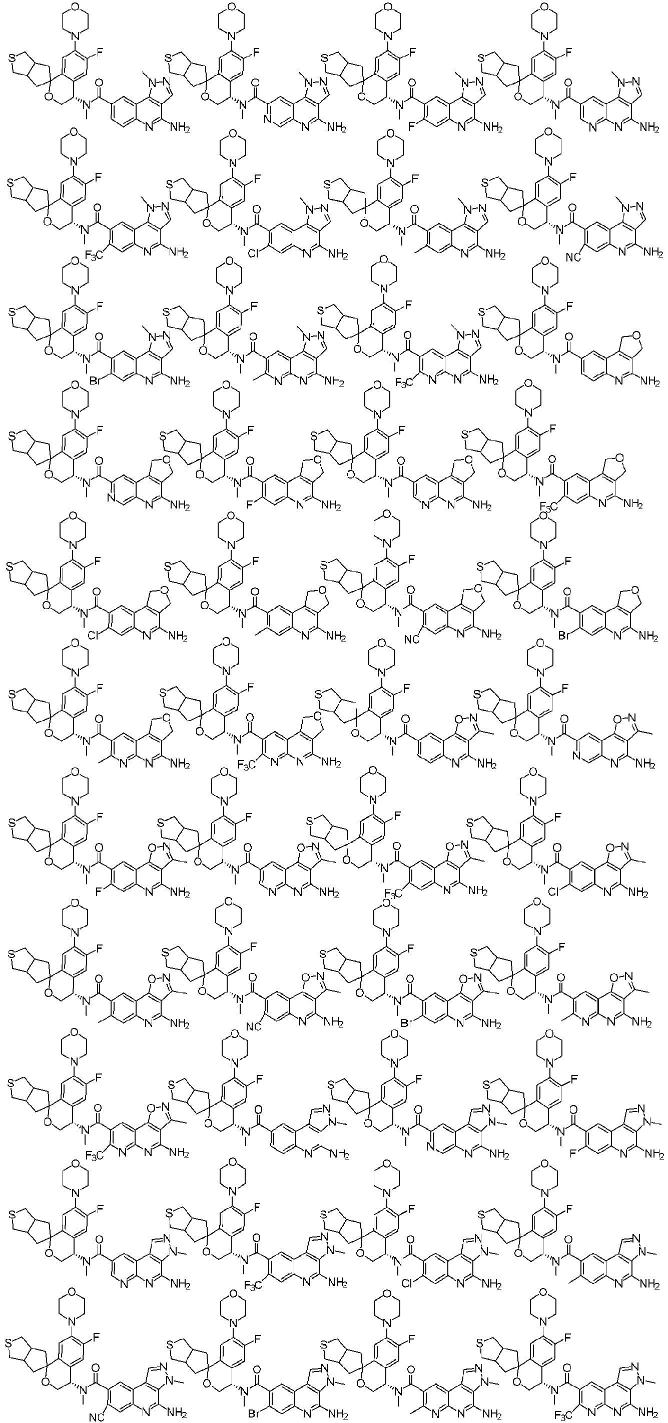

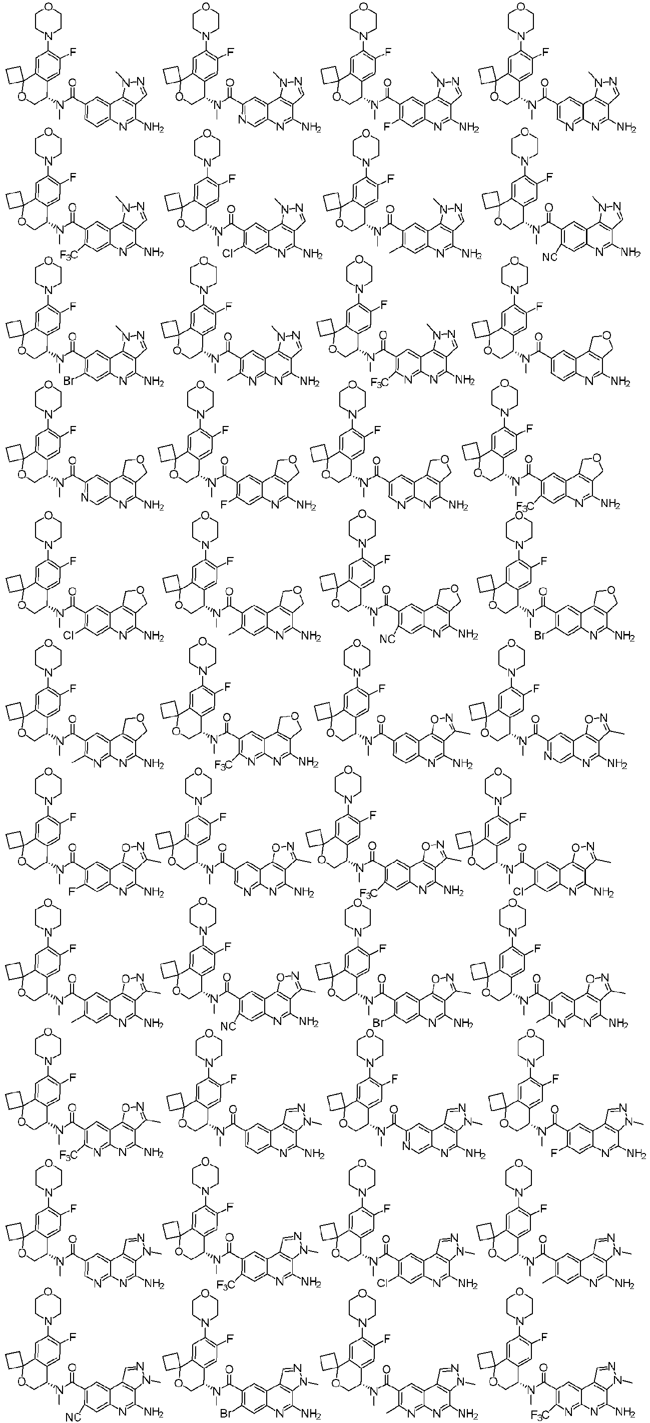

Unless otherwise specified, the compounds of the present disclosure may encompass, in addition to their specific structures, pharmaceutically acceptable salts, stereoisomers, isotope isomers (e.g., deuterates), solvates, hydrates, prodrugs and metabolites thereof. Thus, the pharmaceutically acceptable salts, stereoisomers, isotope isomers, solvates, hydrates, prodrugs, and metabolites of these compounds are also considered within the scope of protection.

Preferably, the pharmaceutical composition of the present disclosure may further comprise a second active substance, wherein the second active substance is an anti-tumor drug selected from one or more of a chemotherapeutic drug, a targeted anti-tumor therapy drug, or an anti-tumor antibody drug.

Furthermore, the present disclosure provides a compound of the disclosure, its pharmaceutically acceptable salts, esters, prodrugs, stereoisomers, or isotopic derivatives, and methods of treating diseases, preferably tumors, by inhibiting the action of PRMT5.

### Definition:

Unless otherwise specified, the term "alkyl" alone or as part of another substituent, refers to linear (i.e. unbranched) or branched, or cyclic hydrocarbyl, or combinations thereof, which may be saturated, mono- or polyunsaturated, and may include divalent or multivalent groups, having a defined number of carbon atoms (i.e., C₁-C₁₀ refers to one to ten carbon atoms). Examples of saturated hydrocarbyl include, but are not limited to, groups such as methyl, ethyl, n-propyl, isopropyl, n-butyl, t-butyl, isobutyl, sec-butyl, cyclohexyl, cyclohexylmethyl, and cyclopropylmethyl, homologs and isomers such as n-pentyl, n-hexyl, n-heptyl, and n-octyl. An unsaturated alkyl has one or more double or triple bonds. Examples of unsaturated alkyl include, but are not limited to, vinyl, 2-propenyl, crotonyl, 2-isopentenyl, 2-(butadienyl), 2,4-pentadienyl, 3-(1,4-pentadienyl), ethynyl, 1- and 3-propynyl, 3-butynyl, as well as higher homologs and isomers. The alkyl defined as the hydrocarbyl is called "homoalkyl". The alkyl is optionally substituted with one or more halogen atoms.

The term "haloalkyl" refers to alkyl as defined above, wherein one or more hydrogen atoms are substituted with halogen atoms.

The term "alkylene" itself or as part of another substituent refers to a divalent group derived from alkyl, such as, but not limited to, -CH₂CH₂CH₂CH₂-, -CH₂CH=CHCH₂-, -CH₂C≡CCH₂- and -CH₂CH₂CH(CH₂CH₂CH₃)CH₂-. The alkyl (or the alkylene) usually has 1-24 carbon atoms, and groups having 10 or fewer carbon atoms are preferred in the present disclosure. "Lower alkyl" or "lower alkylene" refers to shorter chain alkyl or alkylene, usually having eight or fewer carbon atoms. The alkylene is optionally substituted with one or more halogen atoms.

The term "alkynyl" refers to a carbon chain containing at least one carbon-carbon triple bond, which may be linear or branched, or a combination thereof. Examples of the alkynyl include ethynyl, propargyl, 3-methyl-1-pentynyl, 2-heptynyl. The alkynyl is optionally substituted with one or more halogen atoms.

The term "cycloalkyl" refers to a monocyclic or bicyclic saturated carbon ring, each having 3-10 carbon atoms. The "fused analogues" of cycloalkyl groups refer to a monocyclic ring fused to an aryl or heteroaryl, where the connecting point is in the non-aromatic portion. Examples of cycloalkyl and fused analogues thereof include cyclopropyl, cyclobutyl, cyclopentyl, cyclohexyl, tetrahydronaphthyl, decahydronaphthyl, and dihydroindenyl. The cycloalkyl is optionally substituted with one or more halogen atoms. Further, the term "cycloalkyl" in the present disclosure includes both bridged ring systems and spiro ring systems.

The term "alkoxy" refers to linear or branched alkoxy groups indicating the number of carbon atoms. C₁₋₆ alkoxy, for example, includes methoxy, ethoxy, propoxy, isopropoxy.

Unless otherwise specified, the term "heteroalkyl" itself or combination with another term refers to a stable linear or branched, or cyclic hydrocarbon consisting of at least one carbon atom and at least one heteroatom selected from O, N, P, Si, S, or a combination thereof, wherein nitrogen atoms, phosphorus atoms or sulfur atoms may optionally be oxidized and the nitrogen atoms may optionally be quaternized. The heteroatoms O, N, P, S and Si may be placed at any position within the heteroalkyl or at positions where the alkyl is linked to the rest of the molecule. Examples include, but are not limited to, - CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, -O-CH₂-CH₃, and -CN. At most two or three heteroatoms may be contiguous. For instance, -CH₂-NH-OCH₃ and -CH₂-O-Si(CH₃)₃. Similarly, the term "heteroalkylene" itself or combination with another term refers to a divalent group derived from heteroalkyl such as, but not limited to, -CH₂-CH₂-S-CH₂-CH₂- and -CH₂-S-CH₂-CH₂-NH-CH₂-. For heteroalkylene, the heteroatom may be at either or both ends of the chain (e.g., alkylene oxy, alkylene dioxy, alkylene amino, alkylene diamino). Furthermore, for alkylene and heteroalkylene linking groups, the direction in the molecular formula of the linking groups does not indicate the orientation of the linking groups. For example, the molecular formula -C(O)OR'- represents -C(O)OR'- and -R'OC(O)-. As mentioned above, the heteroalkyl as used herein includes those groups that are linked to the rest of the molecule via a heteroatom, for example, -C(O)R', -C(O)NR', -NR'R", -OR', -SR' and/or -SO₂R'. Where reference is made to "heteroalkyl" followed by specific heteroalkyl such as -NR'R", it should be understood that the terms heteroalkyl and -NR'R" do not repeat and are not mutually exclusive. Instead, these specific heteroalkyl groups are referenced for greater clarity. Therefore, the term "heteroalkyl" should not be interpreted herein to exclude specific heteroalkyl such as -NR'R".

The term "cycloalkoxyl" refers to cycloalkyl as defined above that is bonded to an oxygen atom, such as cyclopropoxy.

The term "haloalkoxy" refers to alkoxy as defined above in which one or more hydrogen atoms are substituted with halogen.

The term "aryl" refers to a monocyclic or bicyclic aryl consisting exclusively of carbon atoms. The "fused analogues" of aryl are those in which an aryl is fused to a monocyclic cycloalkyl or monocyclic heterocyclyl, with the connection point located in the aryl portion. Examples of the aryl and fused ring analogues thereof include phenyl, naphthyl, indanyl, indenyl, tetrahydronaphthyl, 2,3-dihydrobenzofuryl, dihydrobenzopyranyl, 1,4-benzodioxanyl.

The term "heteroaryl" refers to a monocyclic or bicyclic aryl containing at least one heteroatom selected from N, O and S. The "fused analogues" of heteroaryl groups are those in which a heteroaryl is fused to a monocyclic cycloalkyl or monocyclic heterocyclyl, with the connection point located in the aryl portion. Examples of the heteroaryl include pyrrolyl, isozolyl, isothiazolyl, pyrazolyl, pyridinyl, oxazolyl, oxadiazolyl, thiadiazolyl, thiazolyl, imidazolyl, triazolyl, tetrazolyl, furyl, triazinyl, thienyl, pyrimidinyl, pyridazinyl, pyrazinyl, benzoxazolyl, benzothiazolyl, benzimidazolyl, benzofuranyl, benzothiopheneyl, furano(2,3-b)pyridinyl, quinolinyl, indolyl, isoquinolinyl.

"Substituted or unsubstituted": the defined alkyl, aryl and heteroaryl are unsubstituted or substituted with at least one substituent selected from the group consisting of substituents. The substituents are selected from the group consisting of: halogen atoms; alkyl having 1 to 6 carbon atoms; alkoxy having 1 to 6 carbon atoms; haloalkyl having 1 to 6 carbon atoms; haloalkoxy having 1 to 6 carbon atoms; -CN; alkynyl having 2 to 6 carbon atoms; alkanoyl having 1 to 6 carbon atoms; cycloalkyl having 3 to 7 ring atoms; heteroaryl; aryl; aralkyloxy having 7 to 10 carbon atoms; arylcarbonyl; aminocarbonyl; alkenyl having 2 to 5 carbon atoms; alkylthio having 1 to 6 carbon atoms; aminosulfinyl; aminosulfonyl; hydroxy; -SF₅; hydroxyalkyl having 1 to 4 carbon atoms; nitro; amino; carboxyl; alkoxycarbonyl having 2 to 5 carbon atoms; alkoxyalkyl having 1 to 4 carbon atoms; alkylsulfonyl having 1 to 4 carbon atoms; alkanoylamino having 1 to 4 carbon atoms; alkanoyl(alkyl)amino having 1 to 6 carbon atoms; alkanoylamidoalkyl having 1 to 6 carbon atoms in each of the alkanoyl and alkyl moieties; alkanoyl(alkyl)aminoalkyl having 1 to 6 carbon atoms in each of the alkanoyl and alkyl portions; alkylsulfonamido having 1 to 4 carbon atoms; monoalkylaminocarbonyl or dialkylaminocarbonyl having 1 to 6 carbon atoms; monoalkylaminosulfinyl or dialkylaminosulfinyl having 1 to 6 carbon atoms; monoalkylaminosulfonyl or dialkylaminosulfonyl having 1 to 6 carbon atoms; aminoalkyl having 1 to 4 carbon atoms; monoalkylamino or dialkylamino having 1 to 6 carbon atoms; monoalkylaminoalkyl or dialkylaminoalkyl having 1 to 6 carbon atoms in each alkyl moiety; aralkyl having 7 to 10 carbon atoms; heteroaralkyl having 1 to 4 carbon atoms in the alkyl portion; heteroarylalkoxy having 1 to 4 carbon atoms in the alkoxy portion; and alkylsulfonamido having 1 to 4 carbon atoms.

As used herein, the term "heterocycle" or "heterocyclic" or "heterocycloalkyl" or "heterocyclyl" refers to a saturated, partially saturated or unsaturated group (but not aromatic) having a monocyclic or condensed ring (including bridged and spiro systems in which there are 1 to 10 carbon atoms and 1 to 4 heteroatoms selected from nitrogen, sulfur, or oxygen, where one or more of the rings may be cycloalkyl, aryl, or heteroaryl, as long as the connection point passes through a nonaromatic ring. In an Example, nitrogen atoms and/or sulfur atoms of the heterocyclic group are optionally oxidized to provide N-oxide, sulfinyl and sulfonyl portions. Examples of "heterocyclyl" and fused analogues thereof include pyrrolidinyl, piperidinyl, piperazinyl, imidazolidinyl, 2,3-dihydrofuran(2,3-b)pyridyl, benzoxazinyl, tetrahydroquinolinyl, tetrahydroisoquinolinyl, and dihydroindolyl. The term also includes non-aromatic, partially unsaturated monocyclic rings such as 2- or 4-pyridones linked via a nitrogen atom or N-substituted -(1H,3H)-pyrimidine-2,4-diones (N-substituted uracils).

As used herein, the term "substituted heterocyclic" or "substituted heterocycloalkyl" or "substituted heterocyclyl" refers to a heterocyclic group substituted with 1 to 5 (e.g., 1 to 3) substituents that are identical to those defined by substituted cycloalkyl.

Unless otherwise specified, the term "halo" or "halogen" itself or as part of another substituent refers to a fluorine, chlorine, bromine or iodine atom. In addition, the term "haloalkyl" includes mono-haloalkyl and poly-haloalkyl. For example, the term "(C₁-C₆) haloalkyl" refers to including, but not limited to, trifluoromethyl, 2,2,2-trifluoroethyl, pentafluoroethyl, 4-chlorobutyl, 3-bromopropyl, and so on.

"Prodrug" refers to a substance that, when administered into the body, undergoes a conversion to the parent drug. In some cases, the prodrugs are often used because they are easier to administer than the parent drugs. For example, the prodrugs may be bioavailable by oral administration while the parent drugs are not. In drug combinations, prodrugs can also exhibit higher solubility than the parent drug. An example of the prodrug, but not limited to, may be that any one of the compounds of Formula I is administered in the form of an ester (prodrug) to facilitate transport across cell membranes where water solubility is detrimental to migration and which subsequently metabolically hydrolyzes into the active substance (carboxylic acid) once within cells where water solubility is beneficial. Another example of a prodrug can be short peptide (polyamino acid) conjugated with an acid moiety, where the peptide undergoes metabolism to release the active portion.

Optical isomers-diastereomers-geometrical isomers-tautomers:

The compounds of Formula (I) contain one or more asymmetric centers and may therefore exist as racemates and racemic mixtures, single enantiomers, mixtures of diastereomers and single diastereomers. The present disclosure should comprise all these isomeric forms of the compounds of formula (1).

Some of the compounds described herein contain an olefinic double bond, which refers to including both E and Z geometrical isomers unless otherwise specified.

Some compounds of the present disclosure may comprise one or more ring systems, and thus cis- and trans-isomers may be present. The present disclosure aims to contain all of these cis- and trans-isomers.

Some of the compounds described herein may have different sites linked to a hydrogen atom, referred to as tautomers. Such examples may be ketones and enol forms thereof known as keto-enol tautomers. Individual tautomers and their mixtures are included in the compounds of the present disclosure.

The compounds of the present disclosure may be isolated into diastereoisomeric pairs, for example by fractional crystallization from a suitable solvent such as methanol or ethyl acetate or mixtures thereof. A pair of such enantiomers obtained can be separated into individual stereoisomers by conventional methods, for example using optically active amines or acids as resolution reagents or in a chiral HPLC column.

Alternatively, any enantiomers of the compounds of the present disclosure may be obtained by stereospecific synthesis using optically pure starting materials or reagents of known configuration.

Stable isotope-labeled analogues: one or more protons in the compounds of the present disclosure may be replaced with deuterium atoms, thereby providing deuterated analogues having improved pharmacological activity.

### Salt and Dosage Form

It should be understood that, as used herein, reference to compounds of the present disclosure also includes pharmaceutically acceptable salts.

### Application

The compounds of the present disclosure can be used for treating PRMT5-associated diseases.

The compounds of the present disclosure can be prepared through the following reaction scheme:

### Method A:

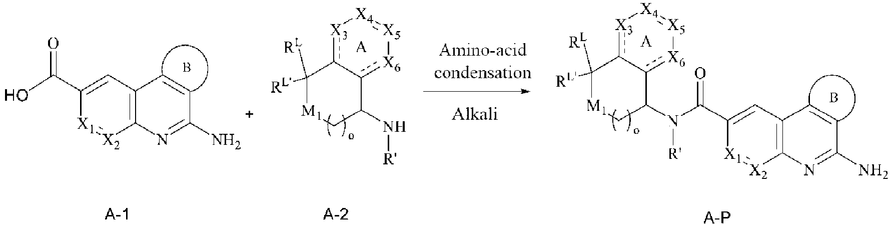

### Method A-SFC

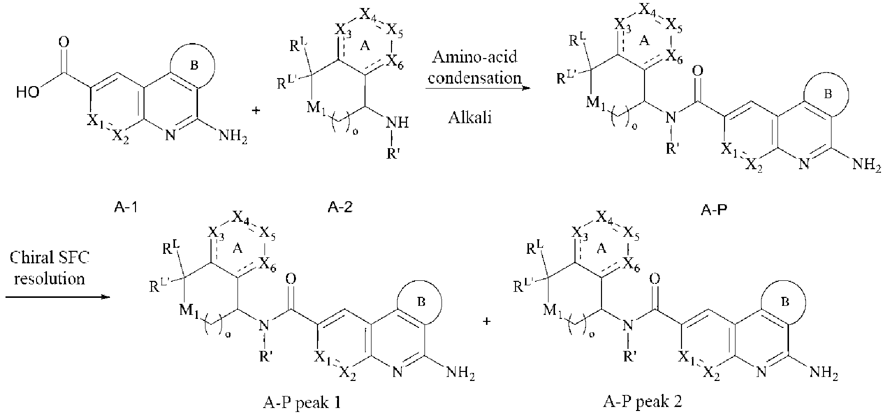

Wherein, R^{L}, R^{L'}, R', ring A, ring B, o, M₁, X₁, X₂, X₃, X₄, X₅, X₆ and -̅ -̅ -̅ are as defined in claim 1.

Method A: Compounds A-P can be obtained by the amino-acid condensation reaction of carboxylic acid A-1 with amine A-2. The condensing agent may be HATU or PyBrOP, the base may be DIPEA or TEA, and the solvent may be DMF or DMAc. If the amine used is a racemate, it will be resolved by chiral SFC and the stereochemistry of the obtained isomer will be assigned arbitrarily to R or S.

### Analytical HPLC

Equipment: Agilent 1260; column specification: Agilent Poroshell HPH-C18 (3.0×50 mm, 2.7 µm); binary solvent system, mobile phase A: water (0.1% v/v ammonium bicarbonate), mobile phase B: acetonitrile; flow rate: 1 mL/min; gradient: from 10% B to 90% B; duration: 12 min; detector: DAD detector; wavelength: 254/220 nm;

### Preparation of HPLC-MS

HPLC equipment: Waters 2489; column specification: Ultimate µ XB-C18(130A, 5 µm, 30 mm×150 mm); binary solvent system, mobile phase A: water (0.1% v/v ammonium bicarbonate), mobile phase B: acetonitrile; flow rate: 60-100 mL/min; gradient: from 10 % B to 90 % B; detector: DAD detector; wavelength: 254/220 nm;

### Mass spectrometer: Agilent G6125B.

The compound of the present disclosure may be prepared by chemical synthesis, examples of which are shown below. It will be understood that the sequence of steps in the process described may vary, those specifically mentioned reagents, solvents and reaction conditions may be substituted, and readily reactable sites may be protected and deprotected if necessary.

The following abbreviations have the meanings shown below. ACN refers to acetonitrile; EA refers to ethyl acetate; CDI refers to N, N'-carbonyldiimidazole; DBU refers to 1,8-diazabicyclo[5.4.0]undec-7-ene; DIBAL-H represents diisobutylaluminium hydride; DIEA refers to diisopropylethylamine; DMAP refers to N,N-dimethylaminopyridine; DME refers to 1,2-dimethoxyethane; DMF refers to N,N-dimethylformamide; DMA and DMAc refers to N,N-dimethylacetamide; DMPE refers to 1,2-bis(dimethylphosphino)ethane; DMSO means dimethylsulfoxide; DPPB means 1,4-bis(diphenylphosphino)butane; dppe refers to 1,2-bis(diphenylphosphino)ethane; dppf means 1,1'-bis(diphenylphosphino)ferrocene; dppm refers to 1,1'-bis(diphenylphosphino)methane; DIAD means diisopropyl azodicarboxylate; EDCI represents 1-(3-dimethylaminopropyl)-3-ethylcarbodiimide; HATU represents 2-(7-aza-1H-benzotriazol-1-yl)-1,1,3,3-tetramethylurea hexafluorophosphate; HMPA refers to hexamethylphosphoramide; IPA refers to isopropyl alcohol; LDA refers to lithium diisopropylamide; LHMDS refers to lithium di(trimethylsilyl)amide; LAH represents lithium aluminium hydride; NCS refers to N-chlorosuccinimide; NaHMDS refers to sodium di(trimethylsilyl)amide; PyBOP refers to benzotriazol-1-yl-oxytripyrrolidinophosphonium hexafluorophosphate; PyBrOP refers to bromo-tris-pyrrolidino-phosphonium hexafluorophosphate; TDA-I refers to tris(2-(2-methoxyethoxy)ethyl)amine; DCM refers to dichloromethane; TEA refers to triethylamine and TFA means trifluoroacetic acid; THF refers to tetrahydrofuran; NCS refers to N-chlorosuccinimide; NMM means N-methylmorpholine; NMP refers to N-methylpyrrolidone; PPh₃ refers to triphenylphosphine; r.t. refers to room temperature; PMB means p-methoxybenzyl; Tosmic means p-Toluenesulfonylmethyl isocyanide; (Boc)₂O refers to di-tert-butyl dicarbonate; PE refers to petroleum ether; o/n refers to overnight reaction.

The following preparations and examples illustrate the present disclosure, but do not limit the present disclosure in any way.

The features and advantages of the subject matter of the present disclosure will become more apparent from a detailed description of selected examples. As will be appreciated, the disclosed and claimed subject matter can be modified in various aspects, and all the modifications will not depart from the scope of the claims. Therefore, the description should be treated as illustrative in nature rather than restrictive. The entire scope of the subject matter of the present disclosure is set forth in the claims.

The present disclosure may be more easily understood with reference to the following examples, which are only used for illustrating the present disclosure instead of limiting the scope of the present disclosure.

The following intermediates were prepared with reference to Amgen's patents WO2022/115377 A1, WO2022/132914 A1, WO2022/169948A1 and WO2023/034786A1

The present disclosure may be more easily understood with reference to the following examples, which are only used for illustrating the present disclosure instead of limiting the scope of the present disclosure.

The following intermediates were prepared with reference to Amgen's patents WO2022/115377 A1, WO2022/132914 A1, WO2022/169948A1 and WO2023/034786A1

| Intermediate | Structure | Name |
|---|---|---|
| 1 | 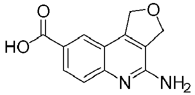 | 4-Amino-1,3-dihydrofuro[3,4-c]quinoline-8-carboxylic acid |
| 2 | 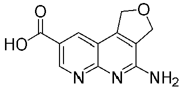 | 4-Amino-1,3-dihydrofuro[3,4-c][1,8]naphthyridine-8-carboxylic acid |
| 3 | 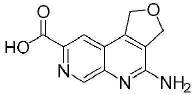 | 4-Amino-1,3-dihydrofuro[3,4-c][1,7]naphthyridine-8-carboxylic acid |
| 4 | 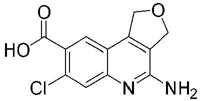 | 4-Amino-7-chloro-1,3-dihydrofuro[3,4-c]quinoline-8-carboxylic acid |
| 5 | 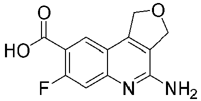 | 4-Amino-7-fluoro-1,3-dihydrofuro[3,4-c]quinoline-8-carboxylic acid |
| 6 | 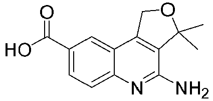 | 4-Amino-3,3-dimethyl-1,3-dihydrofuro[3,4-c]quinoline-8-carboxylic acid |
| 7 | 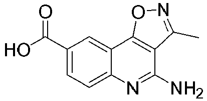 | 4-Amino-3-methylisoxazolo[4,5-c]quinoline-8-carboxylic acid |
| 8 | 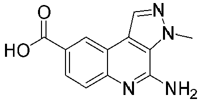 | 4-Amino-3-methyl-3H-pyrazolo[3,4-*c*]quinoline-8-carboxylic acid |
| 9 | 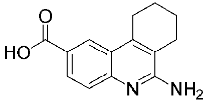 | 6-Amino-7,8,9,10-tetrahydrophenanthridine-2-carboxylic acid |
| 10 | 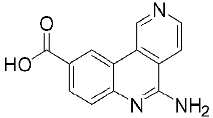 | 5-Aminobenzo[*c*][2,6]naphthyridine-9-carboxylic acid |
| 11 | 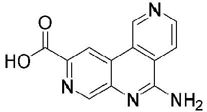 | 5-Aminopyrido[4,3-*c*][1,7]naphthyridine-9-carboxylic acid |
| 12 | 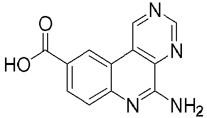 | 5-Aminopyrimido[4,5-*c*]quinoline-9-carboxylic acid |
| 13 | 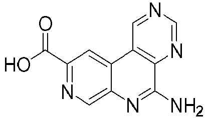 | 5-Aminopyrimido[4,3-c][1,7]naphthyridine-9-carboxylic acid |
| 14 | 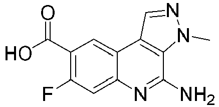 | 4-Amino-7-fluoro-3-methyl-3H-pyrazolo[3,4-*c*]quinoline-8-carboxylic acid |
| 15 | 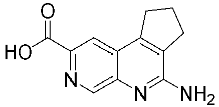 | 6-Amino-8,9-dihydro-7*H*-cyclopenta[*c*][1,7]naphthyridine-2-carboxylic acid |
| 16 | 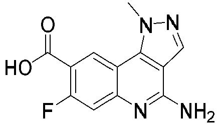 | 4-Amino-7-fluoro-1-methyl-1H-pyrazolo[4,3-*c*]quinoline-8-carboxylic acid |
| 17 | 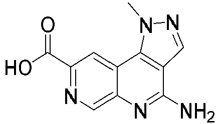 | 4-Amino-1-methyl-1*H*-pyrazolo[4,3-*c*][1,7]naphthyridine-9-carboxylic acid |
| 18 | 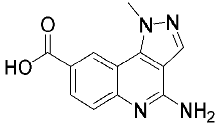 | 4-Amino-1-methyl-1H-pyrazolo[4,3-c]quinoline-8-carboxylic acid |
| 19 | 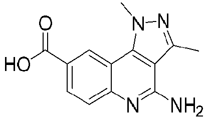 | 4-Amino-1,3-dimethyl-1*H*-pyrazolo[4,3-*c*]quinoline-8-carboxylic acid |
| 20 | 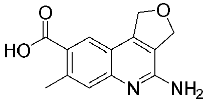 | 4-Amino-7-methyl-1,3-dihydrofuro[3,4-c]quinoline-8-carboxylic acid |
| 21 | 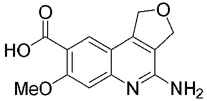 | 4-Amino-7-methoxy-1,3-dihydrofuro[3,4-c]quinoline-8-carboxylic acid |
| 22 | 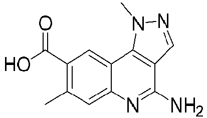 | 4-Amino-1,7-dimethyl-1H-pyrazolo[4,3-c]quinoline-8-carboxylic acid |
| 23 | 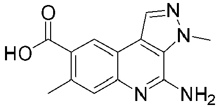 | 4-Amino-3,7-dimethyl-3H-pyrazolo[3,4-c]quinoline-8-carboxylic acid |
| 24 | 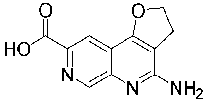 | 4-Amino-2,3-dihydrofuro[3,2-c][1,7]naphthyridine-8-carboxylic acid |
| 25 | 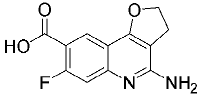 | 4-Amino-7-fluoro-2,3-dihydrofuro[3,2-c]quinoline-8-carboxylic acid |
| 26 | 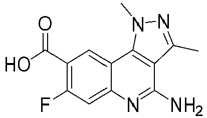 | 4-Amino-7-fluoro-1,3-dimethyl-1H-pyrazolo[4,3-c]quinoline-8-carboxylic acid |
| 27 | 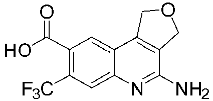 | 4-Amino-7-trifluoromethyl-1,3-dihydrofuro[3,4-*c*]quinoline-8-carboxylic acid |
| 28 | 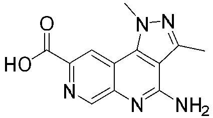 | 4-Amino-1,3-dimethyl-1*H*-pyrazolo[4,3-*c*]quinoline-8-carboxylic acid |
| 29 | 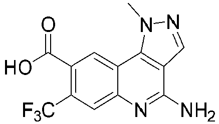 | 4-Amino-1,3-dimethyl-1*H*-pyrazolo[4,3-*c*][1,7]naphthyridine-8-carboxylic acid |
| 30 | 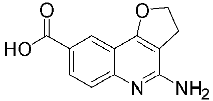 | 4-Amino-2,3-dihydrofuro[3,2-c]quinoline-8-carboxylic acid |
| 31 | 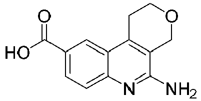 | 5-Amino-1,4-dihydro-2*H*-pyrano[3,4-*c*]quinoline-9-carboxylic acid |
| 32 | 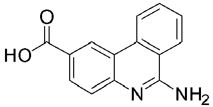 | 6-Aminophenanthridine-2-carboxylic acid |
| 33 | 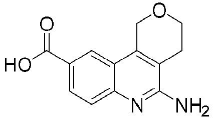 | 5-Amino-3,4-dihydro-1H-pyrano[4,3-c]quinoline-9-carboxylic acid |
| 34 | 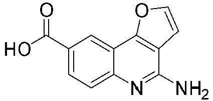 | 4-Aminofuro[3,2-c]quinoline-8-carboxylic acid |
| 35 | 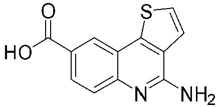 | 4-Aminothieno[3,2-c]quinoline-8-carboxylic acid |
| 36 | 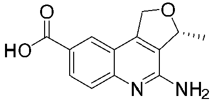 | (*R*)-4-amino-3-methyl-1,3-dihydrofuro[3,4-*c*]quinoline-8-carboxylic acid |
| 37 | 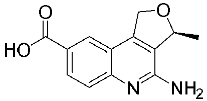 | (*S*)-4-amino-3-methyl-1,3-dihydrofuro[*3,4-*c]quinoline-8-carboxylic acid |
| 38 | 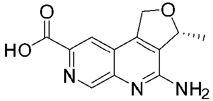 | (*R*)-4-amino-3-methyl-1,3-dihydrofuro[3,4-*c*][1,7]naphthyridine-8-carboxylic acid |
| 39 | 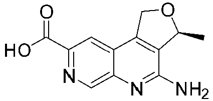 | (*S*)-4-amino-3-methyl-1,3-dihydrofuro[3,*4-c*][1,7]naphthyridine-8-carboxylic acid |
| 40 | 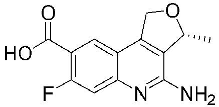 | (R)-4-Amino-7-fluoro-3-methyl-1,3-dihydrofuro[3,4-c]quinoline-8-carboxylic acid |
| 41 | 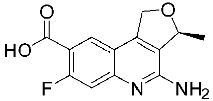 | (S)-4-Amino-7-fluoro-3-methyl-1,3-dihydrofuro[3,4-c]quinoline-8-carboxylic acid |
| 42 | 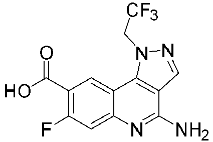 | 4-Amino-7-fluoro-1-(2,2,2-trifluoroethyl)-1*H*-pyrazolo[4,3-c]quinoline-8-carboxylic acid |
| 43 | 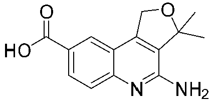 | 4-Amino-3,3-dimethyl-1,3-dihydrofuro[3,4-c]quinoline-8-carboxylic acid |
| 44 | 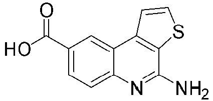 | 4-Aminothieno[2,3-c]quinoline-8-carboxylic acid |
| 45 | 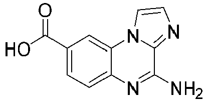 | 4-Aminoimidazo[1,2-*a*]quinoxaline-8-carboxylic acid |
| 46 | 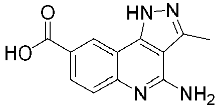 | 4-Amino-3-methyl-1H-pyrazolo[4,3-c]quinoline-8-carboxylic acid |

### Intermediate 47: Synthesis of N,1,1-trimethyl-7-(trifluoromethyl)isochroman-4-amine

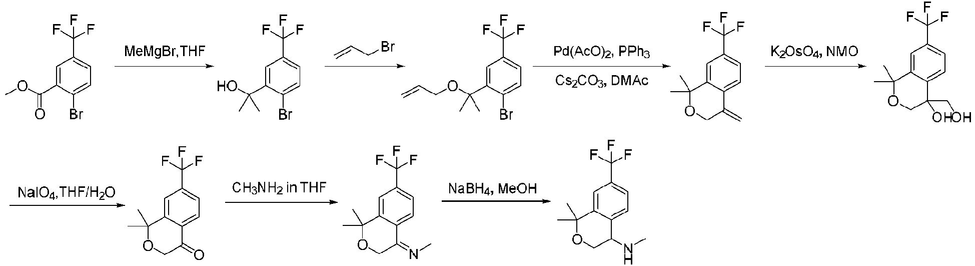

Step 1: At 0°C, methylmagnesium bromide (9.36 mL, 28.09 mmol) was added to a solution of 1-acetyl-2-bromo-5-(trifluoromethyl)benzene (5.00 g, 18.72 mmol) in tetrahydrofuran (10.00 mL). The mixture was heated to 25°C and stirred for 16 hours under nitrogen atmosphere. After the reaction was complete, the mixture was poured into water (150 mL), extracted with ethyl acetate (100 mL), and dried over anhydrous sodium sulfate. After filtration, the filtrate was concentrated under reduced pressure. The crude product was purified by silica gel column chromatography (petroleum ether: ethyl acetate = 2:1) to give 2-(2-bromo-5-(trifluoromethyl)phenyl)propan-2-ol (3.1 g, yield: 46.7%).
LCMS (ESI): 283.1 [M+H]⁺

Step 2: 2-(2-Bromo-5-(trifluoromethyl)phenyl)propan-2-ol (3.00 g, 10.60 mmol) was dissolved in tetrahydrofuran (30.00 mL). 3-bromopropene (2.62 g, 21.20 mmol) and tetrabutylammonium hydrogen sulfate (0.55 g, 1.59 mmol) were added, then potassium hydroxide (1.20 g, 21.20 mmol) was added, and the mixture was reacted overnight at room temperature. After the reaction was complete, the reaction mixture was poured into water (50 mL), extracted with ethyl acetate (30 mL×3), washed with brine (50 mL), dried over anhydrous sodium sulfate, filtered and concentrated to dryness to give a crude product. The crude product was purified by silica gel column chromatography (petroleum ether: ethyl acetate = 1:3) elution to give 2-(2-(allyloxy)propan-2-yl)-1-bromo-4-(trifluoromethyl)benzene (2.40 g, yield: 70%).
LCMS (ESI): 323.1 [M+H]⁺

Step 3: Under nitrogen atmosphere, palladium (II) acetate (1.39 g, 6.19 mmol) was added to a solution of 2-(2-(allyloxy)propan-2-yl)-1-bromo-4-(trifluoromethyl)benzene (20.00 g, 61.89 mmol), triphenylphosphine (8.11 g, 30.95 mmol) and cesium carbonate (24.14 g, 74.27 mmol) in dimethylformamide (200 mL). The reaction mixture was stirred at 100°C for 2 hours. The mixture was poured into water (100 mL) and the aqueous layer was extracted with ethyl acetate (2×100 mL). The combined organic layer was washed with saturated brine (100 mL), dried over anhydrous sodium sulfate and concentrated to give a crude product. The crude product was purified by silica gel column chromatography (petroleum ether: ethyl acetate = 4:1) to give 1,1-dimethyl-4-methylene-7-(trifluoromethyl)isochroman (10.00 g, yield: 53%).
LCMS (ESI): 243.2 [M+H]⁺

Step 4: A mixture of 1,1-dimethyl-4-methylene-7-(trifluoromethyl)isochroman (10.00 g, 41.28 mmol), potassium osmate (1.21 g, 4.13 mmol) and N-methylmorpholine (16.90 g, 144.48 mmol) in tetrahydrofuran (100.00 mL) was stirred overnight at room temperature. After the reaction was complete, the mixture was poured into water (100 mL) and the aqueous layer was extracted with ethyl acetate (2×100 mL). The combined organic layer was washed with saturated brine (100 mL), dried over anhydrous sodium sulfate and concentrated to give 4-(hydroxymethyl)-1,1-dimethyl-7-(trifluoromethyl)isochroman-4-ol (6.00 g crude, yield: 42%).
LCMS (ESI):277.2 [M+H]⁺

Step 5: A mixture of 4-(hydroxymethyl)-1,1-dimethyl-7-(trifluoromethyl)isochroman-4-ol (6.00 g, 21.72 mmol) and sodium periodate (14.07 g, 65.16 mmol) in tetrahydrofuran (50.00 mL) and water (10.00 mL) was stirred at room temperature for 2 hours. The mixture was diluted with water (100 mL) and the aqueous layer was extracted with ethyl acetate (2×100 mL). The combined organic layer was washed with saturated brine (100 mL), dried over anhydrous sodium sulfate and concentrated to give a crude product. The crude product was purified by silica gel column chromatography (petroleum ether: ethyl acetate = 2:1) to give 1,1-dimethyl-7-(trifluoromethyl)isochroman-4-one (5.00 g, yield: 75%).
LCMS (ESI): 245.2[M+H]⁺

Step 6: A mixture of 1,1-dimethyl-7-(trifluoromethyl)isochroman-4-one (5.00 g, 20.47 mmol), methylamine (1.91 g, 61.42 mmol) and molecular sieves (17.81 g, 61.42 mmol) in methanol (50.00 mL) was stirred overnight at room temperature. The mixture was poured into water (100 mL). The aqueous layer was extracted with ethyl acetate (2×100 mL). The combined organic layer was washed with saturated brine (100 mL), dried over anhydrous sodium sulfate and concentrated to give a crude product. The crude product was purified by silica gel column chromatography (petroleum ether: ethyl acetate = 1:1) to give *N*,1,1-trimethyl-7-(trifluoromethyl)isochroman-4-imine (3.00 g, 57%)
LCMS (ESI):257 [M+H]⁺

Step 7: Sodium borohydride (294 mg, 7.78 mmol) was added to a mixture of N,1,1-trimethyl-7-(trifluoromethyl)isochroman-4-amine (1.00 g, 3.89 mmol) in methanol (10.00 mL). The mixture was stirred at room temperature for 1 hour. The mixture was poured into water (10 mL) and the aqueous layer was extracted with ethyl acetate (2×10 mL). The combined organic layer was washed with saturated brine (30 mL), dried over anhydrous sodium sulfate and concentrated to give a crude product. The crude product was purified by silica gel column chromatography (petroleum ether: ethyl acetate = 1:1.5) to give N,1,1-trimethyl-7-(trifluoromethyl)isochroman-4-amine (0.60 g, yield: 60%).

Employing the preparation method and steps of intermediate 47, with the sole replacement of the corresponding raw material intermediate, the following intermediate was obtained:

| Intermediate No. | Chemical Structure | Chemical Name | m/z(ESI): (M+H)⁺ |
|---|---|---|---|
| 48 | 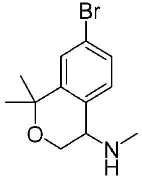 | 7-Bromo-*N*,1,1-trimethylisochroman-4-amine | 270.2 |

### Intermediate 49: Synthesis of N,1,1-trimethyl-7-(1-methyl-1H-pyrazol-4-yl)isochroman-4-amine

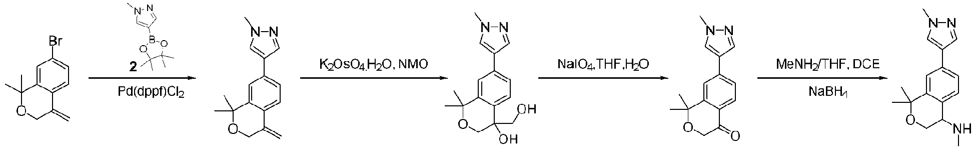

Step 1: Under nitrogen atmosphere, 7-bromo-1,1-dimethyl-4-methyleneisochroman (500 mg, 1.976 mmol) was dissolved in 1,4-dioxane and water (4:1, 10 mL). 1-Methyl-4-(4,4,5,5-tetramethyl-1,3,2-dioxaborolan-2-yl)pyrazole(455 mg, 2.173 mmol), potassium carbonate (819 mg, 5.928 mmol) and [1,1'-bis(diphenylphosphino)ferrocene]dichloropalladium(II) (148 mg, 0.20 mmol) were added. The mixture was reacted at 100°C for 16 hours. The system was poured into water (30 mL) and extracted with ethyl acetate (40 mL×3). The combined organic layer was dried over anhydrous sodium sulfate, and filtered to remove the anhydrous sodium sulfate. The filtrate was concentrated under reduced pressure to give a crude product. The crude product was purified by silica gel column chromatography (petroleum ether: ethyl acetate = 10:1) to give 4-(1,1-dimethyl-4-methyleneisochroman-7-yl)-1-methylpyrazole (360 mg, yield: 71.7%) as a brown solid.
LCMS (ESI) m/z: 255.2 [M+H]⁺

Step 2: At room temperature, 4-(1,1-dimethyl-4-methyleneisochroman-7-yl)-1-methylpyrazole (360 mg, 1.417 mmol) was dissolved in a mixed solvent of acetone (5 mL) and water (1 mL). N-methylmorpholine-N-oxide (613 mg, 4.534 mmol) and potassium osmate (44 mg, 0.142 mmol) were added. Under nitrogen atmosphere, the mixture was stirred for reaction at 25°C for 16 hours. After the reaction was complete, sodium sulfite solid (350 mg) was added to the reaction mixture, then stirred for 10 minutes, concentrated under reduced pressure to remove a certain amount of acetone, poured into water (20 mL), and extracted with ethyl acetate (20 mL×3). The combined organic layer was dried over anhydrous sodium sulfate and concentrated under reduced pressure to give 4-(hydroxymethyl)-1,1-dimethyl-7-(1-methyl-1H-pyrazol-4-yl)isochroman-4-ol (310 mg, crude).
LCMS (ESI) m/z: 289.1 [M+H]⁺

Step 3: At room temperature, 4-(hydroxymethyl)-1,1-dimethyl-7-(1-methyl-1H-pyrazol-4-yl)isochroman-4-ol (310 mg, 1.076 mmol) was dissolved in a mixed solvent of tetrahydrofuran (3 mL) and water (0.3 mL). Sodium periodate (698mg, 3.228 mmol) was added. Under nitrogen atmosphere, the mixture was stirred for reaction at 25°C for 4 hours. After the reaction was complete, the reaction mixture was diluted with ethyl acetate, filtered and washed. The combined filtrate was dried over anhydrous sodium sulfate and filtered. The filtrate was concentrated under reduced pressure to give a crude product. The crude product was purified by silica gel column chromatography (petroleum ether: ethyl acetate = 1:1) to give 1,1-dimethyl-7-(1-methyl-1H-pyrazol-4-yl)isochroman-4-one (200 mg, yield: 72.5%).
LCMS (ESI) m/z:257.1 [M+H]⁺

Step 4: At room temperature, compound 1,1-dimethyl-7-(1-methyl-1H-pyrazol-4-yl)isochroman-4-one (200 mg, 0.781 mmol) was dissolved in 1,2-dichloroethane (5 mL). Titanium tetraethoxide (446 mg, 1.952 mmol) and methylamine tetrahydrofuran solution (1 mL, 30%) were added. The mixture was stirred for reaction at room temperature for 16 hours, then sodium borohydride (46 mg, 1.171 mmol) was added, and the mixture was further stirred for reaction at room temperature for 8 hours. After LCMS indicated the reaction was complete, water (10 mL) was added, and the mixture was extracted with ethyl acetate (20 mL×2). The combined organic layer was dried over anhydrous sodium sulfate and filtered. The filtrate was concentrated under reduced pressure and purified by column chromatography (50-100% ethyl acetate/petroleum ether) to give *N*,1,1-trimethyl-7-(1-methyl-1*H*-pyrazol-4-yl)isochroman-4-amine (130 mg, yield: 61.4%).
LCMS (ESI) m/z: 272.1 [M+H]⁺

Employing the preparation method and steps of intermediate 49, with the sole replacement of the corresponding raw material intermediate, the following intermediates were obtained:

| Intermediat e No. | Chemical Structure | Chemical Name | m/z(ESI): (M+H)⁺ |
|---|---|---|---|
| 50 | 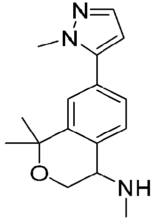 | *N*,1,1-trimethyl-7-(1-methyl-1H-pyrazol-5-yl)isochroman-4-amine | 272.1 |
| 52 | 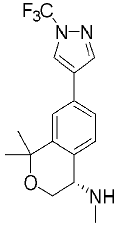 | *(S)-N,*1,1-trimethyl-7-(1-(trifluoromethyl)-1H-pyrazol-4-yl)isochroman-4-amine | 325.9 |
| 53 | 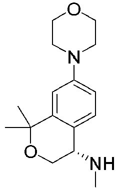 | *(S)-N,1,1*-trimethyl-7-morpholinochroman-4-amine | 276.9 |
| 54 | 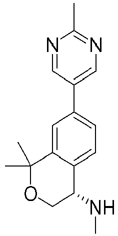 | (S)-N,1,1-trimethyl-7-(2-methylpyrimidin-5-yl)isochroman-4-amine | 284.0 |

### Intermediate 51: Synthesis of N-methyl-7'-(trifluoromethyl)spiro[cyclobutane-1,1'-isochroman]-4'-amine

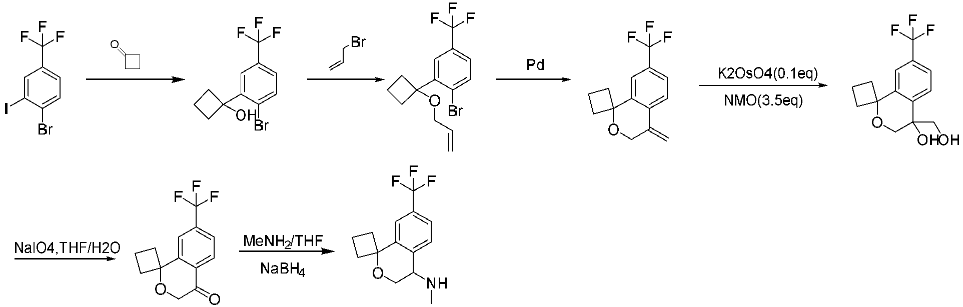

### Step 1: Synthesis of 1-(2-bromo-5-(trifluoromethyl)phenyl)cyclobutan-1-ol

At room temperature, 2-bromo-5-(trifluoromethyl)benzaldehyde (10 g, 39.52 mmol) was dissolved in tetrahydrofuran (100 mL). At 0°C, a 3 M methylmagnesium chloride tetrahydrofuran solution (20 mL, 59.28 mmol) was slowly added dropwise into the system. The mixture was stirred for reaction at 0°C for 2 hours. After the reaction was complete, it was quenched with ammonium chloride solution. The reaction mixture was poured into water (200 mL) and extracted with ethyl acetate (100 mL×3). The combined organic layer was dried over anhydrous sodium sulfate and filtered. The filtrate was concentrated under reduced pressure to give 1-(2-bromo-5-(trifluoromethyl)phenyl)cyclobutan-1-ol (10 g, crude), which was used directly in the next step without purification.
LCMS (ESI) m/z:295.1[M-OH]⁺

### Step 2: Synthesis of 2-[1-(allyloxy)cyclobutyl]-1-bromo-4-(trifluoromethyl)benzene

At room temperature, 1-(2-bromo-5-(trifluoromethyl)phenyl)cyclobutan-1-ol (7 g, 23.80 mmol) was dissolved in tetrahydrofuran (100 mL). Potassium hydroxide (2.69 g, 47.60 mmol), tetrabutylammonium hydrogen sulfate (1.67 g, 4.760 mmol) and 3-bromopropene (4.3 g, 35.70mmol) were added. The mixture was reacted at 25°C for 16 hours. After the reaction was complete, the reaction mixture was concentrated under reduced pressure, then poured into water (100 mL), and extracted with ethyl acetate (100 mL×3). The combined organic layer was dried over anhydrous sodium sulfate and filtered. The filtrate was concentrated under reduced pressure to give a crude product. The crude product was purified by column chromatography (petroleum ether: ethyl acetate = 10:1) to give 2-[1-(allyloxy)cyclobutyl]-1-bromo-4-(trifluoromethyl)benzene (4.8 g, yield: 60.3%).
LCMS (ESI) m/z: 335.1 [M+H]⁺

### Step 3: Synthesis of 4'-methylene-7'-(trifluoromethyl)spiro[cyclobutane-1,1'-isochromane]

At room temperature, N,N-diisopropylethylamine (5.57 g, 43.11 mmol), 4,5-bis(diphenylphosphino)-9,9-dimethylxanthene (1.67 g, 2.874 mmol) and palladium (II) acetate (0.325 g, 1.437mmol) were added to a solution of 2-[1-(allyloxy)cyclobutyl]-1-bromo-4-(trifluoromethyl)benzene (4.8 g, 14.37 mmol) in N,N-dimethylformamide (100 mL). The mixture was stirred at 100°C for 16 hours under nitrogen atmosphere. The reaction mixture was poured into water (400 mL) and extracted with ethyl acetate (100 mL×3). The combined organic layer was dried over anhydrous sodium sulfate and concentrated under reduced pressure to give a crude product. The crude product was purified by column chromatography (petroleum ether: ethyl acetate = 10:1) to give 4'-methylene-7'-(trifluoromethyl)spiro[cyclobutane-1,1'-isochromane] (2.7 g, yield: 73.9%).
LCMS (ESI) m/z: 255.1 [M+H]⁺

### Step 4: Synthesis of 4'-(hydroxymethyl)-7'-(trifluoromethyl)spiro[cyclobutane-1,1'-isochroman]-4'-ol

At room temperature, 4'-methylene-7'-(trifluoromethyl)spiro[cyclobutane-1,1'-isochromane] (2.7 g, 10.62 mmol) was dissolved in a mixed solvent of acetone (60 mL) and water (20 mL). N-methylmorpholine-N-oxide (3.9 g, 31.86 mmol) and potassium osmate (0.41 g, 1.062 mmol) were added. Under nitrogen atmosphere, the mixture was stirred for reaction at 25°C for 16 hours. After the reaction was complete, sodium sulfite solid (5 g) was added to the reaction mixture, then stirred for 10 minutes, concentrated under reduced pressure to remove a certain amount of acetone, poured into water (200 mL), and extracted with ethyl acetate (100 mL×3). The combined organic layer was dried over anhydrous sodium sulfate and concentrated under reduced pressure to give 4'-(hydroxymethyl)-7'-(trifluoromethyl)spiro[cyclobutane-1,1'-isochroman]-4'-ol (2.1 g crude, yield: 68.67%).
LCMS (ESI) m/z: 289.0 [M+H]⁺

### Step 5: Synthesis of 7'-(trifluoromethyl)spiro[cyclobutane-1,1'-isochroman]-4'-one

At room temperature, 4'-(hydroxymethyl)-7'-(trifluoromethyl)spiro[cyclobutane-1,1'-isochroman]-4'-ol (2.1 g, 7.29 mmol) was dissolved in a mixed solvent of tetrahydrofuran (50 mL) and water (2 mL). Sodium periodate (6.37 g, 29.16 mmol) was added. Under nitrogen atmosphere, the mixture was stirred for reaction at 25°C for 4 hours. After the reaction was complete, the reaction mixture was diluted with ethyl acetate, filtered and washed. The combined filtrate was dried over anhydrous sodium sulfate and filtered. The filtrate was concentrated under reduced pressure to give a crude product. The crude product was purified by column chromatography (petroleum ether: ethyl acetate = 5:1) to give 7'-(trifluoromethyl)spiro[cyclobutane-1,1'-isochroman]-4'-one (0.9 g, yield: 48.23%).
LCMS (ESI) m/z:257.1 [M+H]⁺

### Step 6: Preparation of N-methyl-7'-(trifluoromethyl)spiro[cyclobutane-1,1'-isochroman]-4'-amine

At room temperature, compound 7'-(trifluoromethyl)spiro[cyclobutane-1,1'-isochroman]-4'-one (500 mg, 1.953 mmol) was dissolved in 1,2-dichloroethane (5 mL). Titanium tetraethoxide (1115 mg, 4.88 mmol) and methylamine tetrahydrofuran solution (2 mL, 30%) were added. The mixture was stirred for reaction at room temperature for 16 hours, then sodium borohydride (115 mg, 2.928 mmol) was added, and the mixture was further stirred for reaction at room temperature for 8 hours. After LCMS indicated the reaction was complete, water (10 mL) was added, and the mixture was extracted with ethyl acetate (20 mL×2). The combined organic layer was dried over anhydrous sodium sulfate and filtered. The filtrate was concentrated under reduced pressure and purified by column chromatography (50-100% ethyl acetate/petroleum ether) to give N-methyl-7'-(trifluoromethyl)spiro[cyclobutane-1,1'-isochroman]-4'-amine (150 mg, yield: 28.33%).
LCMS (ESI) m/z: 272.1 [M+H]⁺

Employing the preparation method and steps of intermediate 51, with the sole replacement of the corresponding raw material intermediate, the following intermediates were obtained:

| Intermediate No. | Chemical Structure | Chemical Name | m/z(ESI) : (M+H)⁺ |
|---|---|---|---|
| 65 | 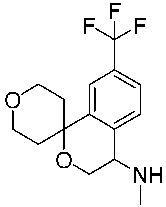 | *N*-methyl-7-trifluoromethyl-2',3',5',6'-tetrahydrospiro[isochroman-1,4'-pyran]-4-amine | 302.1 |
| 66 | 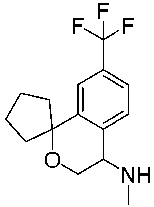 | *N*-methyl-7'-(trifluoromethyl)spiro[cyclopentane-1,1'-isochroman]-4'-amine | 286.1 |

### Intermediate 55: Synthesis of (S)-N-methyl-7'-(trifluoromethyl)spiro[cyclobutane-1,1'-isochroman]-4'-amine

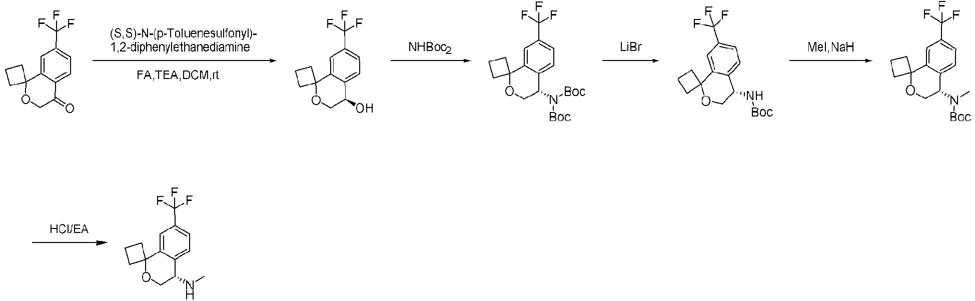

### Step 1: Synthesis of (R)-7'-(trifluoromethyl)spiro[cyclobutane-1,1'-isochroman]-4'-ol

At room temperature, 7'-(trifluoromethyl)spiro[cyclobutane-1,1'-isochroman]-4'-one (2.5 g, 9.76 mmol) was dissolved in dichloromethane (30 mL). (*S,S*)-N-(p-toluenesulfonyl)-1,2-diphenylethanediamine(chloro)(p-cymene)ruthenium(II) (605 mg, 1.17 mmol) and formic acid (1.34 g, 29.28 mmol) were added and triethylamine (2.54 g, 25.09 mmol) was slowly added dropwise. The mixture was reacted at 25°C for 4 hours. After the reaction was complete, the reaction mixture was poured into water (50 mL) and extracted with ethyl acetate (50 mL×3). The combined organic layer was dried over anhydrous sodium sulfate and concentrated under reduced pressure to give a crude product. The crude product was purified by column chromatography (petroleum ether: ethyl acetate = 5:1) to give (R)-1'-(trifluoromethyl)spiro[cyclobutane-1,1'-isochroman]-4'-ol (1.8 g, yield: 71.4%).
LCMS (ESI) m/z:259.2 [M+H]⁺

### Step 2: Synthesis of tert-butyl (S)-(tert-butoxycarbonyl)(7'-(trifluoromethyl)spiro[cyclobutane-1,1'-isochroman]-4'-yl)carbamate

At room temperature, (R)-7'-(trifluoromethyl)spiro[cyclobutane-1,1'-isochroman]-4'-ol (1.8 g, 6.97 mmol) was dissolved in tetrahydrofuran (20 mL). Triphenylphosphine (2.2 g, 8.37 mmol) and di-tert-butyl iminodicarboxylate (1.81 g, 8.37 mmol) were added. Under nitrogen atmosphere, the mixture was reacted at 0°C for 10 minutes. Diisopropyl azodicarboxylate (1.69 g, 8.37 mmol) was slowly added dropwise to the reaction system. The mixture was reacted at 25°C for 16 hours. After the reaction was complete, the reaction mixture was poured into water (40 mL) and extracted with ethyl acetate (20 mL×3). The combined organic layer was dried over anhydrous sodium sulfate and filtered. The filtrate was concentrated under reduced pressure to give a crude product. The crude product was purified by silica gel column chromatography (petroleum ether: ethyl acetate = 10:1) to give tert-butyl (S)-(tert-butoxycarbonyl)(7'-(trifluoromethyl)spiro[cyclobutane-1,1'-isochroman]-4'-yl)carbamate (1.3 g, yield: 40.7%) as a yellow solid.
LCMS (ESI) m/z: 458.1 [M+H]⁺

### Step 3: Synthesis of tert-butyl (S)-(7'-(trifluoromethyl)spiro[cyclobutane-1,1'-isochroman]-4'-yl)carbamate

At room temperature, tert-butyl (S)-(tert-butoxycarbonyl)(7'-(trifluoromethyl)spiro[cyclobutane-1,1'-isochroman]-4'-yl)carbamate (1.3 g, 2.84 mmol) was dissolved in acetonitrile (20 mL). Lithium bromide (1.23 g, 14.22 mmol) was added. The mixture was reacted at 60°C for 16 hours. After the reaction was complete, the reaction mixture was concentrated under reduced pressure, poured into water (50 mL) and extracted with ethyl acetate (50 mL×3). The combined organic layer was dried over anhydrous sodium sulfate and filtered. The filtrate was concentrated under reduced pressure to give tert-butyl (S)-(7'-(trifluoromethyl)spiro[cyclobutane-1,1'-isochroman]-4'-yl)carbamate (0.7 g crude, yield: 89.7%).
LCMS (ESI) m/z: 358.1 [M+H]⁺

### Step 4: Synthesis of tert-butyl (S)-methyl(7'-(trifluoromethyl)spiro[cyclobutane-1,1'-isochroman]-4'-yl)carbamate

At room temperature, tert-butyl (S)-(7'-(trifluoromethyl)spiro[cyclobutane-1,1'-isochroman]-4'-yl)carbamate (0.7 g, 1.96 mmol) was dissolved in N,N-dimethylformamide (20 mL). At 0°C, 60 wt.% sodium hydrogen (158 mg, 3.92mmol) was added. After stirring for 30 minutes, iodomethane (418 mg, 2.94 mmol) was added. The mixture was reacted at 25°C for 4 hours. After the reaction was complete, the reaction mixture was poured into water (40 mL) and extracted with ethyl acetate (30 mL×3). The combined organic layer was dried over anhydrous sodium sulfate and filtered. The filtrate was concentrated under reduced pressure to give tert-butyl (S)-methyl(7'-(trifluoromethyl)spiro[cyclobutane-1,1'-isochroman]-4'-yl)carbamate (0.6 g crude, yield: 82.5%).
LCMS (ESI) m/z: 372.1 [M+H]⁺

### Step 5: Synthesis of (S)-N-methyl-1'-(trifluoromethyl)spiro[cyclobutane-1,1'-isochroman]-4'-amine hydrochloride

At room temperature, tert-butyl (S)-methyl(7'-(trifluoromethyl)spiro[cyclobutane-1,1'-isochroman]-4'-yl)carbamate (0.6 g, 1.61 mmol) was dissolved in 4 M ethyl acetate hydrochloride (4 mL, 12.93 mmol). After stirring for 1 hour, the reaction mixture was concentrated under reduced pressure to give (S)-N-methyl-7'-(trifluoromethyl)spiro[cyclobutane-1,1'-isochroman]-4'-amine hydrochloride (430 mg crude, yield: 98.1%).
LCMS (ESI) m/z: 272.1 [M+H]⁺

### Intermediate 56: Synthesis of N,1,1-trimethyl-7-(4-(trifluoromethyl)-1H-pyrazol-1-yl)isochroman-4-amine

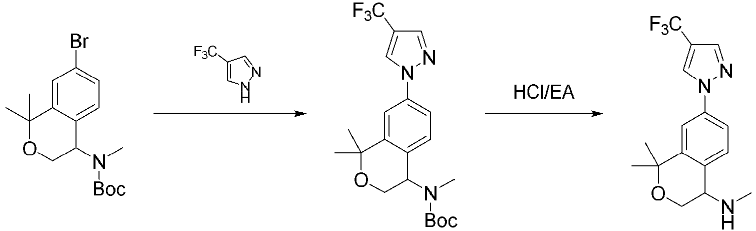

### Step 1: Synthesis of tert-butyl (1,1-dimethyl-7-(4-(trifluoromethyl)-1H-pyrazol-1-yl)isochroman-4-yl)(methyl)carbamate

Under nitrogen atmosphere, tert-butyl (7-bromo-1,1-dimethylisochroman-4-yl)(methyl)carbamate (150 mg, 0.405 mmol) was dissolved in N,N-dimethylformamide (2 mL). 4-(Trifluoromethyl)-1H-pyrazole (66 mg, 0.486 mmol), potassium phosphate (343 mg, 1.62 mmol), trans-(1R,2R)N,N'-dimethyl-cyclohexane-1,2-diamine (116 mg, 0.810 mmol) and cuprous oxide (78.0 mg, 0.405 mmol) were added. The mixture was reacted at 100°C for 16 hours. The system was poured into water (20 mL) and extracted with ethyl acetate (10 mL×3). The combined organic layer was dried over anhydrous sodium sulfate and filtered to remove anhydrous sodium sulfate. The filtrate was concentrated under reduced pressure to give a crude product. The crude product was purified by silica gel column chromatography (petroleum ether: ethyl acetate = 3:1) to give tert-butyl (1,1-dimethyl-7-(4-(trifluoromethyl)-1H-pyrazol-1-yl)isochroman-4-yl)(methyl)carbamate (90 mg, 0.212 mmol, yield: 52.3%) as a brown oil.
LCMS (ESI) m/z: 426.1 [M+H]⁺

### Step 2: Synthesis of N,1,1-trimethyl-7-(4-(trifluoromethyl)-1H-pyrazol-1-yl)isochroman-4-amine

At room temperature, tert-butyl (1,1-dimethyl-7-(4-(trifluoromethyl)-1H-pyrazol-1-yl)isochroman-4-yl)(methyl)carbamate (90 mg, 0.308 mmol) was dissolved in 4 M ethyl acetate hydrochloride (2 mL, 2.46 mmol). After stirring for 1 hour, the reaction mixture was concentrated under reduced pressure to give *N*,1,1-trimethyl-7-(4-(trifluoromethyl)-1H-pyrazol-1-yl)isochroman-4-amine hydrochloride (90 mg, crude), which was directly used in the next step without purification.
LCMS (ESI) m/z: 326.2 [M+H]⁺

### Intermediate 57: Synthesis of N-methyl-7'-morpholino-3',4'-dihydrospiro[cyclobutane-1,1'-pyrano[4,3-c]pyridin]4'-amine

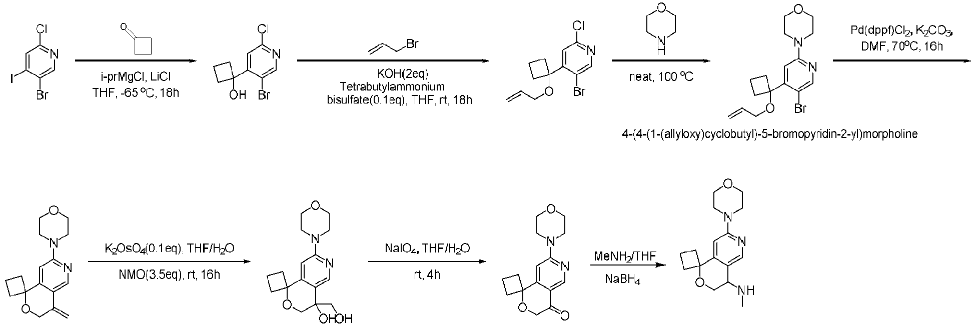

### Step 1: Synthesis of 1-(5-bromo-2-chloropyridin-4-yl)cyclobutan-1-ol

At room temperature, 5-bromo-2-chloro-4-iodopyridine (25 g, 78.6 mmol) was dissolved in tetrahydrofuran (250 mL). The mixture was purged with nitrogen three times. At -78 °C, isopropylmagnesium chloride lithium chloride complex (60.4 mL, 1.3 M, 78.5 mmol) was slowly added dropwise to the system. The mixture was stirred for reaction at -78°C for 2 hours. Then, a solution of cyclobutanone (5.5 g, 78.5 mmol) in tetrahydrofuran (30 mL) was added to the reaction mixture. The mixture was slowly warmed to room temperature and reacted for 16 hours. After the reaction was complete, it was quenched with ammonium chloride solution (150 mL). The mixture was poured into water (200 mL) and extracted with ethyl acetate (200 mL×3). The combined organic layer was dried over anhydrous sodium sulfate and filtered. The filtrate was concentrated under reduced pressure to give a crude product. The crude product was purified by column chromatography (petroleum ether: ethyl acetate = 1:0-10:1) to give 1-(5-bromo-2-chloropyridin-4-yl)cyclobutan-1-ol (10.9 g, yield: 53%) as a pale yellow oil.

¹H NMR (400 MHz, DMSO-*d*₆) δ 8.53 (s, 1H), 7.49 (s, 1H), 5.78 (s, 1H), 2.65 - 2.56 (m, 2H), 2.36 - 2.27 (m, 2H), 2.03 - 1.99 (m, 1H), 1.63 - 1.55 (m, 1H).

### Step 2: Synthesis of 2-[1-(allyloxy)cyclobutyl]-1-bromo-4-(trifluoromethyl)benzene

At room temperature, 1-(5-bromo-2-chloropyridin-4-yl)cyclobutan-1-ol (10.9 g, 41.5 mmol) was dissolved in tetrahydrofuran (100 mL). Potassium hydroxide (4.28 g, 83.0 mmol), tetrabutylammonium hydrogen sulfate (2.59 g, 8.3 mmol) and 3-bromopropene (4.6 g, 41.5mmol) were added. The mixture was reacted at 25°C for 16 hours. After the reaction was complete, the reaction mixture was poured into water (200 mL) and extracted with ethyl acetate (200 mL×3). The combined organic layer was dried over anhydrous sodium sulfate and filtered. The filtrate was concentrated under reduced pressure to give a crude product. The crude product was purified by column chromatography (petroleum ether: ethyl acetate = 10:1) to give 2-[1-(allyloxy)cyclobutyl]-1-bromo-4-(trifluoromethyl)benzene (6.6 g, yield: 57.4%) as a pale yellow oil.

LCMS (ESI): [M+H]⁺ 301.9/303.9.

### Step 3: Synthesis of 4-(4-(1-(allyloxy)cyclobutyl]-5-bromopyridin-2-yl)morpholine

At room temperature, 2-[1-(allyloxy)cyclobutyl]-1-bromo-4-(trifluoromethyl)benzene (6.6 g, 21.9 mmol) was dissolved in morpholine (10 mL). The mixture was reacted at 100°C for 16 hours. After the reaction was complete, the mixture was concentrated under reduced pressure to give a crude product. The crude product was purified by column chromatography (petroleum ether: ethyl acetate = 5:1) to give 4-(4-(1-(allyloxy)cyclobutyl]-5-bromopyridin-2-yl)morpholine (4.5 g, yield: 58.4%) as a pale yellow oil.

LCMS (ESI): [M+H]⁺ 353.1/355.1.

### Step 4: Synthesis of 4'-methylene-7'-morpholino-3'-,4'-dihydrospiro[cyclobutane-1,1'-pyrano[4,3-c]pyridine]

At room temperature, 4-(4-(1-(allyloxy)cyclobutyl)-5-bromopyridin-2-yl)morpholine (1.0 g, 2.8 mmol) was dissolved in N,N-dimethylformamide (20 mL). Potassium carbonate (1.2 g, 8.5 mmol) and [1,1'-bis(diphenylphosphino)ferrocene]dichloropalladium(II) (0.2 g, 0.28 mmol) were added. Under nitrogen atmosphere, the mixture was stirred at 70°C for 16 hours. The reaction mixture was poured into water (200 mL) and extracted with ethyl acetate (80 mL×3). The combined organic layer was dried over anhydrous sodium sulfate and concentrated under reduced pressure to give a crude product. The crude product was purified by column chromatography (petroleum ether: ethyl acetate = 10:1) to give 4'-methylene-7'-morpholino-3'-,4'-dihydrospiro[cyclobutane-1,1'-pyrano[4,3-c]pyridine] (0.2 g pure, yield: 25.94%) as a colorless oil.

¹H NMR (400 MHz, DMSO-*d*₆) δ 8.53 (s, 1H), 6.79 (s, 1H), 5.54 (s, 1H), 4.85 (s, 1H), 4.25 (s, 2H), 3.73 - 3.70 (m, 4H), 3.53 - 3.51 (m, 4H), 2.45 - 2.37 (m, 2H), 2.33 - 2.26 (m, 2H), 2.05 - 1.97 (m, 2H).

### Step 5: Synthesis of 4'-(hydroxymethyl)-1'-morpholino-3'-,4'-dihydrospiro[cyclobutane-1,1'-pyrano[4,3-c]pyridin]-4'-ol

At room temperature, 4'-methylene-7'-morpholino-3'-,4'-dihydrospiro[cyclobutane-1,1'-pyrano[4,3-c]pyridine] (0.8 g, 2.9 mmol) was dissolved in a mixed solvent of tetrahydrofuran (30 mL) and water (10 mL). *N*-methylmorpholine-N-oxide (1.0 g, 8.8 mmol) and potassium osmate (0.09 g, 0.3 mmol) were added. Under nitrogen atmosphere, the mixture was stirred for reaction at 25°C for 16 hours. After the reaction was complete, sodium sulfite solid (1.5 g) was added to the reaction mixture, stirred for 10 minutes, concentrated under reduced pressure to remove a certain amount of acetone, poured into water (60 mL), and extracted with ethyl acetate (80 mL×4). The combined organic layer was dried over anhydrous sodium sulfate and concentrated under reduced pressure to give 4'-(hydroxymethyl)-7'-morpholino-3'-,4'-dihydrospiro[cyclobutane-1,1'-pyrano[4,3-c]pyridin]-4'-ol (0.55 g, crude).

LCMS (ESI): [M+H]⁺ 307.2.

### Step 6: Synthesis of 7'-morpholinospiro[cyclobutane-1,1'-pyrano[4,3-c]pyridin]-4'(3'H)-one

At room temperature, 4'-(hydroxymethyl)-7'-morpholino-3'-,4'-dihydrospiro[cyclobutane-1,1'-pyrano[4,3-c]pyridin]-4'-ol (0.55 g, 1.8 mmol) was dissolved in a mixed solvent of tetrahydrofuran (20 mL) and water (0.8 mL). Sodium periodate (1.2 g, 5.6 mmol) was added. Under nitrogen atmosphere, the mixture was stirred for reaction at 25°C for 4 hours. After the reaction was complete, the reaction mixture was diluted with ethyl acetate, filtered and washed. The combined filtrate was dried over anhydrous sodium sulfate and filtered. The filtrate was concentrated under reduced pressure to give a crude product. The crude product was purified by column chromatography (petroleum ether: ethyl acetate = 2:1) to give a 7'-morpholinospiro[cyclobutane-1,1'-pyrano[4,3-c]pyridin]-4'(3'H)-one (0.3 g, yield: 39% (2 steps)) as a white solid.

LCMS (ESI): [M+H]⁺ 275.2.

¹H NMR (400 MHz, DMSO-*d*₆) δ 8.59 (s, 1H), 6.79 (s, 1H), 4.23 (s, 2H), 3.75 - 3.69 (m, 8H), 2.44 - 2.40 (m, 4H), 2.03 - 1.96 (m, 2H).

### Step 7: Synthesis of N-methyl-7'-morpholino-3',4'-dihydrospiro[cyclobutane-1,1'-pyrano[4,3-c]pyridin]4'-amine

At room temperature, compound 7'-morpholinospiro[cyclobutane-1,1'-pyrano[4,3-c]pyridin]-4'(3'H)-one (300 mg, 1.09 mmol) was dissolved in 1,2-dichloroethane (5 mL). Titanium tetraethoxide (750 mg, 3.27 mmol) and methylamine tetrahydrofuran solution (2 mL, 30%) were added. The mixture was stirred for reaction at room temperature for 16 hours, then sodium borohydride (62 mg, 1.64 mmol) was added, and the mixture was further stirred for reaction at room temperature for 8 hours. After LCMS indicated the reaction was complete, water (10 mL) was added and the mixture was extracted with ethyl acetate (20 mL×2). The combined organic layer was dried over anhydrous sodium sulfate and filtered. The filtrate was concentrated under reduced pressure and purified by column chromatography (50-100% ethyl acetate/petroleum ether) to give N-methyl-7'-morpholino-3',4'-dihydrospiro[cyclobutane-1,1'-pyrano[4,3-c]pyridin]4'-amine (100 mg, yield: 32%).
LCMS (ESI): [M+H]⁺ 290.2

Employing the preparation method and steps of intermediate 57, with the sole replacement of the corresponding raw material intermediate, the following intermediate was obtained:

| Intermediate No. | Chemical Structure | Chemical Name | m/z(ESI): (M+H)⁺ |
|---|---|---|---|
| 58 | 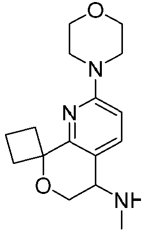 | *N*-methyl-2'-morpholino-5',6'-dihydrospiro[cyclobutane-1,8'-pyrano[*4,3-b*]pyridin]5'-amine | 290.2 |

### Intermediate 59: Synthesis of N,1,1-trimethyl-7-morpholino-3,4-dihydro-1H-pyrano[4,3-c]pyridin-4-amine

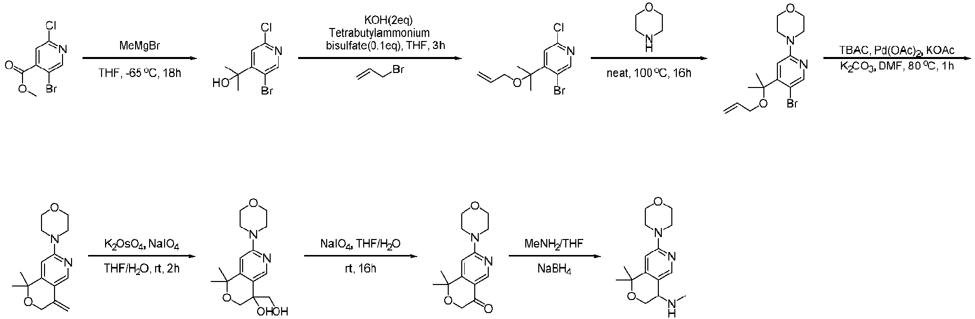

### Step 1: Synthesis of 2-(5-bromo-2-chloropyridin-4-yl)propan-2-ol

Under nitrogen atmosphere, at -65°C, methylmagnesium chloride solution (19.96 mL, 59.89 mmol) was slowly added to a solution of methyl 2-bromo-5-chloropyridine-4-carboxylate (10.00 g, 39.92 mmol) in tetrahydrofuran (50.00 mL). The mixture was then slowly warmed to room temperature and stirred overnight. The reaction was monitored by TLC (petroleum ether:ethyl acetate = 10:1), a small amount of raw materials remained and a more polar product was mainly observed. The reaction mixture was poured into water and 1N sodium hydroxide aqueous solution was added until no more precipitate was formed. The mixture was filtered and the filtrate was extracted with ethyl acetate. The combined organic layer was dried and concentrated. The residue was purified by silica gel column chromatography, eluting with 0%-10% petroleum ether/ethyl acetate (about 20 minutes), to give 2-(5-bromo-2-chloropyridin-4-yl)propan-2-ol (5.00 g, yield: 50%) as a pale yellow oil.

¹H NMR (400 MHz, CDCl₃) δ 8.44 (s, 1H), 7.74 (s, 1H), 1.74 (s, 6H).

### Step 2: Synthesis of 4-(2-(allyloxy)propan-2-yl)-5-bromo-2-chloropyridine

3-bromoprop-1-ene (3.70 g, 29.94 mmol), potassium hydroxide (2.26 g, 39.92 mmol) and tetrabutylammonium hydrogen sulfate (1.37 g, 3.99 mmol) were added to a solution of 2-(5-bromo-2-chloro-4-pyridyl)propan-2-ol (5.00 g, 19.96 mmol) in tetrahydrofuran (20.00 mL). The mixture was stirred at 70°C for 4 hours. The mixture was filtered and the filtrate was concentrated to give a crude product. The crude product was purified by silica gel column chromatography, eluting with 0%-10% petroleum ether/ethyl acetate (30 minutes), to give 4-(2-(allyloxy)propan-2-yl)-5-bromo-2-chloropyridine (4.12 g, yield: 71%) as a pale yellow oil.

¹H NMR (400 MHz, CDCl₃) δ 8.48 (s, 1H), 7.45 (s, 1H), 6.02-5.92 (m, 1H), 5.35-5.30 (m, 1H), 5.21-5.18 (m, 1H), 3.81-3.79 (m, 2H), 1.69 (s, 6H).

### Step 3: Synthesis of 4-(4-(2-(allyloxy)propan-2-yl)-5-bromopyridin-2-yl)morpholine

4-(2-(Allyloxy)propan-2-yl)-5-bromo-2-chloropyridine (3.40 g, 11.70 mmol) was dissolved in morpholine (10.00 mL). The mixture was stirred overnight at 100°C without solvent. The solvent was removed under vacuum by an oil pump. The residue was purified by silica gel column chromatography, eluting with 0%-15% petroleum ether/ethyl acetate for 15-20 minutes, to give 4-(4-(2-(allyloxy)propan-2-yl)-5-bromopyridin-2-yl)morpholine (3.15 g, yield: 79%) as a pale yellow oil.

LCMS (ESI): [M+H]⁺ 341.2/343.2.

### Step 4: Synthesis of 1,1-dimethyl-4-methyl-7-morpholino-3,4-dihydro-1H-pyrano[4,3-c]pyridine

Tetrabutyl ammonium chloride (1.43 g, 5.13 mmol), palladium (II) acetate (0.23 g, 1.03 mmol), potassium carbonate (1.45 g, 10.26 mmol) and potassium acetate (1.02 g, 10.2 mmol) were added to a solution of 1-[1-(5-bromo-2-morpholin-4-yl (4-pyridyl))-isopropoxy]prop-2-ene (1.75 g, 5.13 mmol) in N,N-dimethylformamide (20.00 mL). The mixture was stirred at 80°C under nitrogen atmosphere for 1 hour. After the reaction was complete, the mixture was diluted with water (100 mL) and extracted with ethyl acetate (2×100 mL). The combined organic layer was washed with saturated brine (100 mL), dried over anhydrous sodium sulfate and concentrated. The residue was purified by silica gel column chromatography, eluting with 0%-20% petroleum ether/ethyl acetate (30 minutes), to give 1,1-dimethyl-4-methyl-7-morpholino-3,4-dihydro-1H-pyrano[4,3-c]pyridine (0.45 g, yield: 34%) as a pale yellow oil that solidified upon standing to a pale yellow solid.

LCMS (ESI): [M+H]⁺ 261.01.

¹H NMR (400 MHz, CDCl₃) δ 8.51 (s, 1H), 6.29 (s, 1H), 5.47 (s, 1H), 4.88 (s, 1H), 4.37 (s, 2H), 3.84 - 3.81 (m, 4H), 3.53 - 3.49 (m, 4H), 1.52 (s, 6H).

### Step 5: Synthesis of 4-(hydroxymethyl)-1,1-dimethyl-7-morpholino-3,4-dihydro-1H-pyrano[4,3-c]pyridin-4-ol

1,1-Dimethyl-4-methyl-7-morpholino-4-yl-3-hydro-1H-pyrano[4,3-c]pyridine (300.00 mg, 1.15 mmol) was dissolved in tetrahydrofuran (3.00 mL) and water (0.50 mL). Potassium osmate(VI) dihydrate (43.33 mg, 0.12 mmol) and sodium periodate (377.27 mg, 1.73 mmol) were added. The mixture was stirred at room temperature for 2 hours. After the reaction was complete, the reaction mixture was poured into water (50 mL), extracted with ethyl acetate (50 mL×2), dried, and concentrated under reduced pressure to give 4-(hydroxymethyl)-1,1-dimethyl-7-morpholino-3,4-dihydro-1H-pyrano[4,3-c]pyridin-4-ol (400 mg crude, yield: 94%), which was directly used in the next step without purification.

LCMS (ESI): [M+H]⁺ 295.28.

### Step 6: Synthesis of 1,1-dimethyl-7-morpholino-1H-pyrano[4,3-c]pyridin-4(3H)-one

4-(Hydroxymethyl)-1,1-dimethyl-7-morpholino-4-yl-3,4-dihydro-1H-pyrano[4,3-c]pyridin-4-ol (400.00 mg, 1.09 mmol) was dissolved in tetrahydrofuran (4.00 mL) and water (2.00 mL). Sodium periodate (711.82 mg, 3.26 mmol) was added. The mixture was stirred overnight at room temperature. After the reaction was complete, the reaction mixture was poured into water (50 mL), extracted with ethyl acetate (50 mL×2), dried and concentrated under reduced pressure. The residue was purified by silica gel column chromatography, eluting with 0%-20% petroleum ether/ethyl acetate, to give 1,1-dimethyl-7-morpholino-1H-pyrano[4,3-c]pyridin-4(3H)-one (230 mg, yield: 81%) as a white solid.

LCMS (ESI): [M+H]⁺ 263.2.

¹H NMR (400 MHz, DMSO-*d*₆) δ 8.60 (s, 1H), 6.68 (s, 1H), 4.26 (s, 2H), 3.69 (s, 8H), 1.54 (s, 6H).

### Step 7: Synthesis of N,1,1-trimethyl-7-morpholino-3,4-dihydro-1H-pyrano[4,3-c]pyridin-4-amine

At room temperature, compound 1,1-dimethyl-7-morpholino-1H-pyrano[4,3-c]pyridin-4(3H)-one (230 mg, 0.878 mmol) was dissolved in 1,2-dichloroethane (5 mL). Titanium tetraethoxide (600 mg, 2.63 mmol) and methylamine tetrahydrofuran solution (2 mL, 30%) were added. The mixture was stirred for reaction at room temperature for 16 hours, then sodium borohydride (50 mg, 1.32 mmol) was added, and the mixture was further stirred for reaction at room temperature for 8 hours. After LCMS indicated the reaction was complete, water (10 mL) was added, and the mixture was extracted with ethyl acetate (20 mL×2). The combined organic layer was dried over anhydrous sodium sulfate and filtered. The filtrate was concentrated under reduced pressure and purified by column chromatography (50-100% ethyl acetate/petroleum ether) to give N,1,1-trimethyl-7-morpholino-3,4-dihydro-1H-pyrano[4,3-c]pyridin-4-amine (78 mg, yield: 32%).
LCMS (ESI): [M+H]⁺ 278.2

Employing the preparation method and steps of intermediate 59, with the sole replacement of the corresponding raw material intermediate, the following intermediate was obtained:

| Intermediate No. | Chemical Structure | Chemical Name | m/z(ESI) :(M+H)⁺ |
|---|---|---|---|
| 60 | 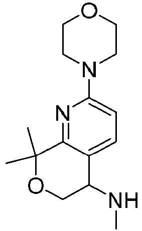 | *N*,8,8-trimethyl-2-morpholino-5,8-dihydro-6H-pyrano[*3,4-b*]pyridin-5-amine | 278.2 |

### Intermediate 61: Synthesis of (S)-N-1,1-trimethyl-7-morpholinoisochroman-4-amine

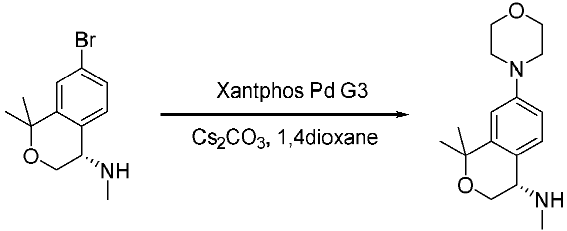

Under nitrogen atmosphere, (S)-7-bromo-N,1,1-trimethylisochroman-4-amine (150 mg, 0.56 mmol) was dissolved in 1,4-dioxane (5 mL). Morpholine (97 mg, 1.11 mmol), cesium carbonate (725 mg, 2.23 mmol), and methanesulfonato(9,9-dimethyl-4,5-bis(diphenylphosphino)xanthene)(2'-amino-1,1'-biphenyl-2-yl)palladium(II) (53 mg, 0.056mmol) were added. The reaction mixture was stirred at 100°C for 16 hours. The system was poured into water (20mL) and extracted with ethyl acetate (10 mL×3). The combined organic layer was dried over anhydrous sodium sulfate and filtered to remove the anhydrous sodium sulfate. The filtrate was concentrated under reduced pressure to give a crude product. The crude product was purified by silica gel column chromatography (petroleum ether: ethyl acetate = 2:1) to give (S)-N-1,1-trimethyl-7-morpholinoisochroman-4-amine (80 mg, yield: 52%) as an oily liquid.

Employing the preparation method and steps of intermediate 61, with the sole replacement of the corresponding raw material intermediate, the following intermediate was obtained:

| Intermedia te No. | Chemical Structure | Chemical Name | m/z(ESI): (M+H)⁺ |
|---|---|---|---|
| 62 | 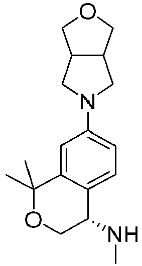 | *(4S)-N-*1,1-trimethyl-7-(tetrahydro-1H-furo[3,4-c]pyrrol-5(3H)-yl)isochroman-4-amine | 303.2 |

### Intermediate 63: Synthesis of (S)-7-(3,6-dihydro-2H-pyran-4-yl) N,1,1-trimethylisochroman-4-amine

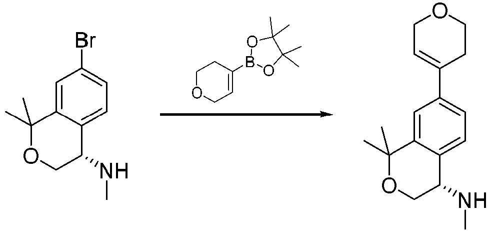

Under nitrogen atmosphere, (S)-7-bromo-N,1,1-trimethylisochroman-4-amine (150 mg, 0.56 mmol) and 2-(3,6-dihydro-2H-pyran-4-yl)-4,4,5,5-tetramethyl-1,3,2-dioxaborolane (175 mg, 0.84 mmol) were dissolved in 1,4-dioxane (5 mL) and water (1 mL). [1,1'-Bis(diphenylphosphino)ferrocene]dichloropalladium(II) (40 mg, 0.056 mmol) and potassium carbonate (230 mg, 1.68 mmol) were added to the reaction mixture. The reaction mixture was stirred at 80°C for 16 hours, poured into water (15 mL) and extracted with ethyl acetate (15 mL×3). The combined organic layer was dried over anhydrous sodium sulfate and filtered to remove anhydrous sodium sulfate. The filtrate was concentrated under reduced pressure to give a crude product. The crude product was purified by silica gel column chromatography (petroleum ether: ethyl acetate = 3:1) to give (S)-7-(3,6-dihydro-2H-pyran-4-yl) N,1,1-trimethylisochroman-4-amine (130 mg, yield: 85%) as an oily liquid.
LCMS (ESI): *m*/*z* 274.2 [M+H]⁺

### Intermediate 64: Synthesis of 4-amino-[1,2,5]thiadiazolo[3,4-C]quinoline-8-carboxylic acid

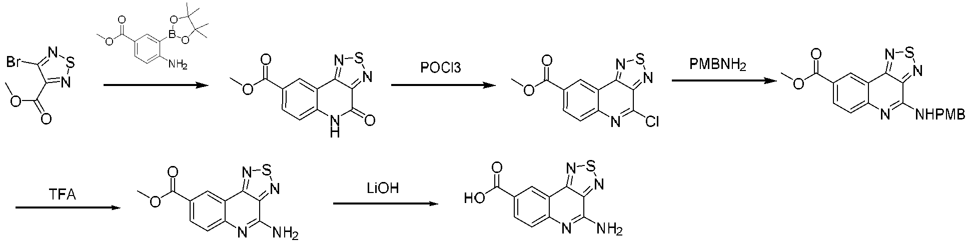

### Step 1: Synthesis of methyl 4-oxo-4,5-dihydro-[1,2,5]thiadiazolo[3,4-C]quinoline-8-carboxylate

At room temperature, methyl 4-amino-3-(4,4,5,5-tetramethyl-1,3,2-dioxaborolan-2-yl)benzoate (2 g, 7.217 mmol) and methyl 4-bromo-1,2,5-thiadiazole-3-carboxylate (1.9 g, 8.66 mmol) were dissolved in a mixed solvent of 1,4-dioxane and water (25 mL, 4:1). Tetrakis(triphenylphosphine)palladium (851 mg, 0.722 mmol) and potassium carbonate (3 g, 21.6 mmol) were added. The reaction mixture was purged with nitrogen three times and stirred at 100°C for 16 hours. After the reaction was complete, the reaction mixture was concentrated under reduced pressure, poured into water and filtered. The filter cake was washed with water and dried under vacuum to give a crude product (1.3 g, yield: 68%), which was directly used in the next step without purification.
LCMS (ESI) m/z: 262.1 [M+H]⁺

### Step 2: Synthesis of methyl 4-chloro-[1,2,5]thiadiazolo[3,4-C]quinoline-8-carboxylate

At room temperature, methyl 4-oxo-4,5-dihydro-[1,2,5]thiadiazolo[3,4-C]quinoline-8-carboxylate (1.4 g, 5.359 mmol) was dissolved in phosphorus oxychloride (30 mL). The mixture was stirred for reaction at 100°C for 16 hours. After the reaction was complete, the reaction mixture was concentrated under reduced pressure, diluted with a small amount of acetonitrile, poured into water (80 mL) and filtered. The filter cake was washed with water and dried under vacuum to give methyl 4-chloro-[1,2,5]thiadiazolo[3,4-C]quinoline-8-carboxylate (1.4 g, crude) as a brown solid.
LCMS (ESI) m/z: 280.1 [M+H]⁺

### Step 3: Synthesis of methyl 4-((4-methoxybenzyl)amino)-[1,2,5]thiadiazolo[3,4-C]quinoline-8-carboxylate

At room temperature, methyl 4-chloro-[1,2,5]thiadiazolo[3,4-C]quinoline-8-carboxylate (1 g, 3.575 mmol) was dissolved in dimethyl sulfoxide (30 mL). 4-Methoxybenzylamine (735 mg, 5.363 mmol) and N,N-diisopropylethylamine (943 mg, 7.15 mmol) were added. The mixture was reacted at 90°C for 3 hours. After the reaction was complete, the reaction mixture was poured into water (200 mL) and extracted with ethyl acetate (50 mL×3). The combined organic layer was dried over anhydrous sodium sulfate and filtered. The filtrate was concentrated under reduced pressure to give a crude product. The crude product was purified by silica gel column chromatography (petroleum ether: ethyl acetate=1:1) to give methyl 4-((4-methoxybenzyl)amino)-[1,2,5]thiadiazolo[3,4-C]quinoline-8-carboxylate (1.25 g, yield: 91%) as a yellow solid.
LCMS (ESI) m/z: 381 [M+H]⁺

### Step 4: Synthesis of methyl 4-amino-[1,2,5]thiadiazolo[3,4-C]quinoline-8-carboxylate

At room temperature, methyl 4-((4-methoxybenzyl)amino)-[1,2,5]thiadiazolo[3,4-C]quinoline-8-carboxylate (800 mg, 2.103 mmol) was dissolved in trifluoroacetic acid (10 mL). The mixture was reacted at 80°C for 16 hours. After the reaction was complete, the reaction mixture was concentrated under reduced pressure, poured into water (10 mL) and filtered. The filter cake was dried under vacuum to give a crude product (800 mg, crude), which was used directly in the next step without purification.
LCMS (ESI) m/z:261 [M+H]⁺

### Step 5: Synthesis of 4-amino-[1,2,5]thiadiazolo[3,4-C]quinoline-8-carboxylic acid

Methyl 4-amino-[1,2,5]thiadiazolo[3,4-C]quinoline-8-carboxylate (600 mg, 2.305 mmol) was dissolved in tetrahydrofuran/methanol/water (25 mL, 2:2:1). Lithium hydroxide (167 mg, 6.916 mmol) was added. The mixture was reacted at 50°C for 4 hours. After the reaction was complete, the reaction mixture was concentrated under reduced pressure to give a crude product of 4-amino-[1,2,5]thiadiazolo[3,4-C]quinoline-8-carboxylic acid (0.7 g, crude ), which was directly used in the next step without purification.
LCMS (ESI) m/z: 247 [M+H]⁺

### Example 1

### Example 1: Synthesis of 4-amino-N-(1,1-dimethyl-7-(trifluoromethyl)isochroman-4-yl)-N,1-dimethyl-1H-pyrazolo [4,3-c] quinoline-8-carboxamide

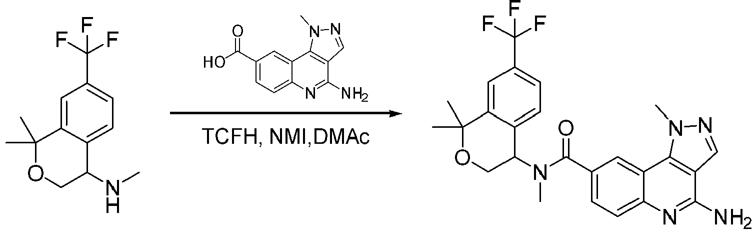

A mixture of [1,1-dimethyl-7-(trifluoromethyl)isochroman-4-yl]methanamine (60 mg, 0.23 mmol), 4-amino-1-methylpyrazolo[4,5-c]quinoline-8-carboxylic acid (56 mg, 0.23 mmol), *N,N,N'*,N'-tetramethylchloroformamidine hexafluorophosphate (194 mg, 0.69 mmol), N-methylimidazole (57 mg, 0.69 mmol) and N, N-dimethylacetamide (1.00 mL) was stirred at room temperature for 2 hours. After the reaction was complete, the reaction mixture was poured into water (10 mL) and the aqueous layer was extracted with ethyl acetate (2×10 mL). The combined organic layer was washed with saturated brine (10 mL), dried over anhydrous sodium sulfate and concentrated to give a crude product. The crude product was purified by preparative high performance liquid chromatography (column: Sunfire C18 5 m, 30 mm×150 mm; mobile phase A: water (0.1% formic acid), mobile phase B: acetonitrile; flow rate: 60 mL/min; gradient: from 18% B to 35% B over 8 minutes; wavelength: 254 nm/220 nm; retention time (minutes): 7.35) to give 4-amino-N-(1,1-dimethyl-7-(trifluoromethyl)isochroman-4-yl)-N,1-dimethyl-1H-pyrazolo[4,3-c]quinoline-8-carboxamide(9.18 mg, 8%) as a white solid.
LCMS (ESI):484.05 [M+H]⁺
¹H NMR (400 MHz, DMSO-*d₆*) δ 8.36 - 8.31 (m, 2H), 8.25 (s, 1H), 7.71(m,1H), 7.66-7.65 (m, 1H), 7.62-7.61(m, 2H), 7.15 (s, 2H), 5.81- 4.95(m,1H), 4.39 (s, 3H), 4.13 (s, 2H), 2.79 (s, 3H), 1.57 (m, 6H)

The Examples in the following table may be prepared using the synthesis step described for **Example 1,** only replacing the corresponding starting materials:

| Examp le No. | Chemical Structure | Chemical Name | ¹HNMR&¹⁹F NMR& LCMS |
|---|---|---|---|
| 2 | 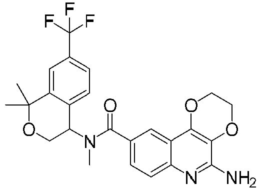 | 5-Amino-N-(1,1-dimethyl-7-(trifluoromethyl)isochro man-4-yl)-N-methyl-2,3-dihydro-[1,4]dioxa[2,3-c]quinoline-9-carboxamide | ¹H NMR (400 MHz, DMSO-*d₆*) δ7.94-7.93 (m, 2H), 7.74 - 7.64 (m, 6H), 5.70-4.60 (m, 1H), 4.62 (s, 2H), 4.50 (s, 2H), 4.18 - 4.07 (m, 2H), 2.72 (s, 3H), 1.57 - 1.53 (m, 6H). |
| | | | LCMS (ESI):487.80 [M+H]⁺ |
| 3 | 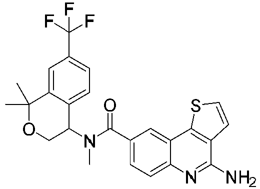 | 4-Amino-N-(1,1-dimethyl-7-(trifluoromethyl)isochro man-4-yl)-N-methylthieno[*3,2-*c]quinoline-8-carboxamide | ¹H NMR (400 MHz, DMSO-*d₆*) δ 8.04 (s, 1H), 7.87 (s, 2H), 7.73 (s, 1H), 7.66 (d, *J =* 8.3 Hz, 1H), 7.61 (d, *J* = 1.2 Hz, 3H), 7.16 (s, 2H), 5.73 (s, 1H), 4.11 (s, 2H), 2.77 (s, 3H), 1.58 - 1.39 (m, 6H). |
| | | | LCMS (ESI):485.80 [M+H]⁺ |
| 4 | 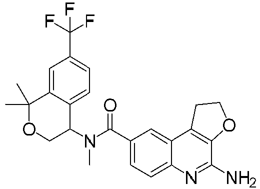 | 4-Amino-N-(1,1-dimethyl-7-(trifluoromethyl)isochro man-4-yl)-N-methyl-1,2-dihydrofuro[2,3-c]quinoline-8-carboxamide | ¹H NMR (400 MHz, DMSO-*d₆*) δ 7.76 (m, 2H), 7.61 (m, 4H), 5.72 - 4.87 (m, 3H), 4.19 - 4.09 (m, 2H), 3.54 (s, 2H), 2.74 (s, 3H), 1.56 - 1.39 (m, 6H). |
| | | | LCMS (ESI):471.80 [M+H]⁺ |
| 5 | 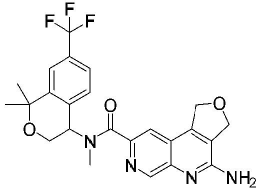 | 4-Amino-N-(1,1-dimethyl-7-(trifluoromethyl)isochro man-4-yl)-*N*-methyl-1,3-dihydrofuro[*3,4-c*][1,7]naphthyridine-8-carboxamide | ¹H NMR (400 MHz, DMSO-*d₆*) δ 8.95 (s, 1H), 7.98 (s, 1H), 7.94 (s, 1H), 7.72 - 7.53 (m, 2H),5.73-5.42 (m, 3H), 5.08 (s, 2H), 4.04 (m, 4H), 2.76 (d, *J* = 4.2 Hz, 3H), 1.60-1.42 (m, 6H). |
| | | | LCMS (ESI):473.00 [M+H]⁺ |
| 6 | 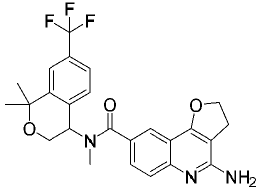 | 4-Amino-N-(1,1-dimethyl-7-(trifluoromethyl)isochro man-4-yl)-N-methyl-2,3-dihydrofuro[*3,2-c*]quinoline-8-carboxamide | ¹H NMR (400 MHz, DMSO-*d₆*) δ7.73 (d, *J* = 2.0 Hz, 2H), 7.65 (d, *J* = 8.4 Hz, 1H), 7.56-7.47 (m, 3H), 6.52 (s, *J* = 15.6 Hz, 2H), 5.68 (s, 1H), 4.82 (t, *J* = 9.0 Hz, 2H), 4.06 (dd, *J* = 12.0, 5.9 Hz, 2H), 3.12 (dd, *J* = 9.2 Hz, 2H), 2.73 (s, 3H), 1.56 - 1.51 (m, 6H). |
| | | | LCMS (ESI):472.05 [M+H]⁺ |
| 9 | 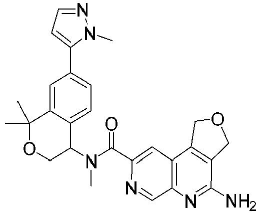 | (4-Amino(1,3-dihydrofuro[*3,4-d*]pyrido[5,4-b]pyridin-8-yl))-*N*-[1,1-dimethyl-7-(1-methylpyrazol-5-yl)isochroman-4-yl]-*N-*methylformamide | ¹H NMR (400 MHz, DMSO-*d₆*) δ 8.88 (s, 1H), 7.82 (s, 1H), 7.44 (s, 4H), 7.03 (s, 2H), 6.43(s,1H), 5.45-5.05 (m, 1H), 5.39 (s, 2H), 5.01-5.00(m, 2H), 4.05 (s, 2H), 3.88 (s, 3H), 2.80 (s, 3H), 1.58-1.42 (m, 6H). |
| | | | LCMS (ESI):484.90 [M+H]⁺ |
| 10 | 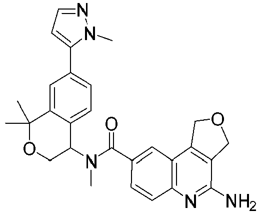 | 4-Amino-N-(1,1-dimethyl-7-(1-methyl-1H-pyrazol-5-yl)isochroman-4-yl)-N-methyl-1,3-dihydrofuro[*3,4-*c]quinoline-8-carboxamide | ¹H NMR (400 MHz, DMSO-*d₆*) δ 7.72 (d, 2H), 7.65 (s, 1H), 7.45 (d, *J* = 16.7 Hz, 2H), 6.73 (s, 1H), 6.44 (s, 1H), 5.71-4.82 (m,1H), 5.38 (s, 2H), 5.03 (s, 2H), 4.07 (s, 2H), 3.86 (d, *J* = 7.6 Hz, 3H), 2.76 (s, 3H), 1.55 (d, *J* = 12.0 Hz, 6H). |
| | | | LCMS (ESI): 484.10 [M+H]⁺ |
| 13 | 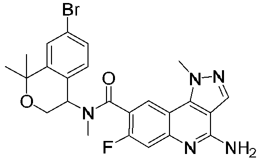 | (4-Amino-7-fluoro-1-methylpyrazolo[*4,5-c*]quinolin-8-yl)-N-(7-bromo-1,1-dimethylisochroman-4-yl)-N-methylformamide | ¹H NMR (400 MHz, DMSO-*d₆*) δ 8.41 - 8.11 (m, 2H), 7.56 - 7.18 (m, 7H), 6.44 (d, *J* = 10.7 Hz, 1H), 5.75-4.73 (t, *J* = 4.4 Hz, 1H), 4.40 (d, *J* = 5.8 Hz, 3H), 4.15 - 3.98 (m, 2H), 3.86 (d, *J =* 17.0 Hz, 3H), 2.78 (d, *J* = 53.0 Hz, 3H), 1.66 - 1.31 (m, 6H). |
| | | | LCMS (ESI): m/z 513.90 [M+H]⁺ |
| 15 | 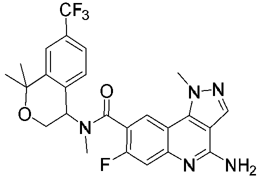 | 4-Amino-N-(1,1-dimethyl-7-(trifluoromethyl)isochro man-4-yl)-7-fluoro-N,1-dimethyl-1H-pyrazolo[*4*,*3-c*]quinoline-8-carboxamide | ¹H NMR (400 MHz, DMSO-*d₆*) δ 8.47-8.33 (m, 2H), 8.30 - 8.22 (m, 2H), 7.80-7.73 (m, 1H), 7.61 - 7.50 (m, 1H), 7.39 - 7.23 (m, 1H), 6.68 - 6.66 (m, 1H), 5.81 - 4.74 (m, 1H), 4.43 - 4.29 (m, 3H), 4.13 - 3.96 (m, 2H), 2.72 - 2.62 (m, 3H), 1.66 - 1.33 (m, 6H). |
| | | | LCMS (ESI): m/z 501.85 [M+H]⁺ |
| 21 | 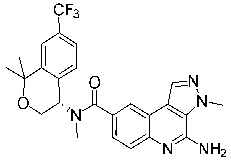 | (S)-4-amino-N-(1,1-dimethyl-7-(trifluoromethyl)isochro man-4-yl)-*N*,3-dimethyl-3H-pyrazolo[*3,4-c*]quinoline-8-carboxamide | ¹H NMR (400 MHz, DMSO-*d₆*) δ 8.55 (s, 1H), 8.27 (s, 1H), 7.79 - 7.64 (m, 2H), 7.63 - 7.49 (m, 3H), 6.88 (s, 2H), 5.76 - 4.86 (m, 1H), 4.36 (s, 3H), 4.20 - 4.05 (m, 2H), 2.77 (s, 3H), 1.61 - 1.49 (m, 6H). |
| | | | LCMS (ESI):483.95 [M+H]⁺ |
| 23 | 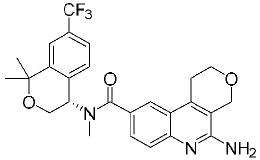 | (S)-4-amino-N-(1,1-dimethyl-7-(trifluoromethyl)isochro man-4-yl)-*N*,1-dimethyl-1H-pyrazolo[*4,3-c*]quinoline-8-carboxamide | ¹H NMR (400 MHz, DMSO-*d₆*) δ 7.85 (d, *J* = 2.0 Hz, 1H), 7.75 - 7.67 (m, 1H), 7.64 (d, *J* = 8.1 Hz, 1H), 7.64 - 7.53 (m, 2H), 7.51 (d, *J* = 8.4 Hz, 1H), 6.39 (s, 2H), 5.71, 4.87 (s, 1H), 4.54 (s, 2H), 4.21 - 4.05 (m, 2H), 3.98 (t, *J* = 5.5 Hz, 2H), 3.02 (s, 2H), 2.74 (s, 3H), 1.63 - 1.29 (m, 6H). |
| | | | LCMS (ESI):485.95 [M+H]⁺ |
| 32 | 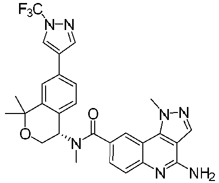 | (S)-4-amino-N-(1,1-dimethyl-7-(1-(trifluoromethyl)-1H-pyrazol-4-yl)isochroman-4-yl)-N,1-dimethyl-1H-pyrazolo[4,3-c]quinoline-8-carboxamide | ¹H NMR (400 MHz, DMSO-d₆) δ 9.06 (s, 1H), 8.54 (s, 1H), 8.34 (s, 1H), 8.25 (s, 1H), 7.80 - 7.59 (m, 4H), 7.45 - 7.32 (m, 1H), 7.14 (s, 2H), 5.67-4.94 (m, 1H), 4.40 (s, 3H), 4.13 - 4.02 (m, 2H), 2.80 (s, 3H), 1.59 (s, 3H), 1.58 - 1.37 (m, 3H). |
| | | | LCMS (ESI):550.00 [M+H]⁺ |
| 33 | 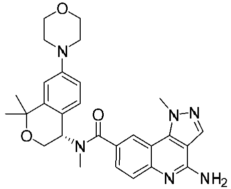 | (S)-4-amino-N-(1,1-dimethyl-7-morpholinoisochroman-4-yl)-N,1-dimethyl-1H-pyrazolo[4,3-c]quinoline-8-carboxamide | ¹H NMR (400 MHz, DMSO-d₆)δ 8.29 (s, 1H), 8.25 (s, 1H), 7.61 (s, 2H), 7.24 - 7.16 (m, 1H), 7.15 - 7.04 (m, 2H), 6.93 - 6.86 (m, 1H), 6.82 - 6.74 (m, 1H), 5.55-4.84 (m, 1H), 4.38 (s, 3H), 4.12 - 3.98 (m, 2H), 3.73 (s, 4H), 3.12 (s, 4H), 2.76 (s, 3H), 1.51 (s, 3H), 1.48 - 1.30 (m, 3H). |
| | | | LCMS (ESI):501.05 [M+H]⁺ |
| 34 | 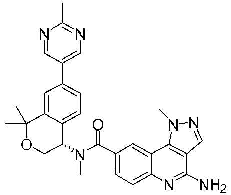 | (S)-4-amino-N-(1,1-dimethyl-7-(2-methylpyrimidin-5-yl)isochroman-4-yl)-N,1-dimethyl-1H-pyrazolo[4,3-c]quinoline-8-carboxamide | ¹H NMR (400 MHz, DMSO-d₆) δ 9.07 (s, 2H), 8.34 (s, 1H), 8.25 (s, 1H), 7.78 - 7.68 (m, 2H), 7.67 - 7.60 (m,2H), 7.47 (s, 1H), 7.15 (s, 2H), 5.71 - 4.99 (m, 1H), 4.40 (s, 3H), 4.22 - 4.07 (m, 2H), 2.82 (s, 3H), 2.67 (s, 3H), 1.60 (s, 3H), 1.57 - 1.39 (m, 3H). |
| | | | LCMS (ESI):507.95 [M+H]⁺ |
| 35 | 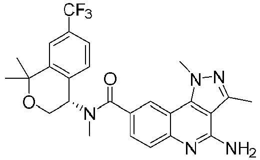 | (S)-4-amino-N-(1,1-dimethyl-7-(trifluoromethyl)isochro man-4-yl)-*N*,1,3-trimethyl-1H-pyrazolo[*4,3-c*]quinoline-8-carboxamide | ¹H NMR (400 MHz, DMSO-*d6*) δ 8.31 (s, 1H), 7.80-7.55 (m, 5H), 6.63 (s, 2H), 5.72, 5.00 (m, 1H), 4.31 (s, 3H), 4.13 (s, 2H), 2.78 (s, 3H), 2.60 (s, 3H), 1.67 - 1.35 (m, 6H). |
| | | | LCMS (ESI):497.90 [M+H]⁺ |
| 36 | 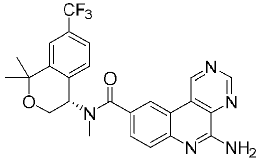 | *N*-[(4S)-1,1-dimethyl-7-(trifluoromethyl)isochro man-4-yl](5-aminopyrimido[*4,5-c*]quinolin-9-yl)-N-methylformamide | ¹H NMR (400 MHz, DMSO-*d₆*) δ 10.40 (d, *J* = 23.2 Hz, 1H), 9.52 (s, 1H), 8.90 - 8.75 (m, 1H), 7.87 - 7.46 (m, 7H), 5.76 (s, 1H), 4.25-4.07 (m, 2H), 2.79 (s, 3H), 1.72 - 1.37 (m, 6H). |
| | | | LCMS (ESI):481.95 [M+H]⁺ |
| 37 | 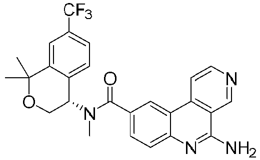 | (*S*)-5-amino-N-(1,1-dimethyl-7-(trifluoromethyl)isochro man-4-yl)-N-methylbenzo[*c*][2,7]naph thyridine-9-carboxamide | ¹H NMR (400 MHz, DMSO-*d₆*) δ 9.66 (s, 1H), 8.89 (d, *J* = 5.6 Hz, 1H), 8.75 - 8.58 (m, 2H), 7.79 - 7.72 (m, 2H), 7.69 - 7.62 (m, 2H), 7.60 (s, 1H), 7.59 - 7.51 (m, 2H), 5.75-4.86 (m, 1H), 4.26 - 4.05 (m, 2H), 2.78 (s, 3H), 1.65 - 1.31 (m, 6H). |
| | | | LCMS (ESI):480.95 [M+H]⁺ |
| 38 | 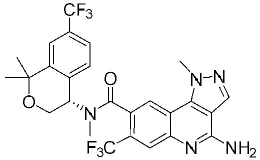 | *N*-[(4S)-1,1-dimethyl-7-(trifluoromethyl)isochro man-4-yl] [4-amino-1-methyl-7-(trifluoromethyl)pyrazol o[*4,5-c*]quinolin-8-yl]-N-methylformamide | ¹H NMR (400 MHz, DMSO-*d₆*) δ 10.40 (d, *J* = 23.2 Hz, 1H), 9.52 (s, 1H), 8.90 - 8.75 (m, 1H), 7.87 - 7.46 (m, 7H), 5.76 (s, 1H), 4.25 - 4.07 (m, 2H), 2.79 (s, 3H), 1.72 - 1.37 (m, 6H). |
| | | | LCMS (ESI):551.95 [M+H]⁺ |
| 41 | 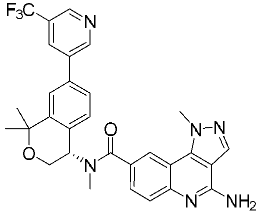 | (S)-4-amino-N-(1,1-dimethyl-7-(5-(trifluoromethyl)pyridin-3-yl)isochroman-4-yl)-N,1-dimethyl-1H-pyrazolo[4,3-c]quinoline-8-carboxamide | ¹H NMR (400 MHz, DMSO-*d₆*)δ 9.26 (s, 1H), 9.00 - 8.95 (m, 1H), 8.53 (s, 1H), 8.35 (s, 1H), 8.26 (s, 1H), 7.89 - 7.74 (m, 2H), 7.71 - 7.59 (m, 2H), 7.55 - 7.46 (m, 1H), 7.15 (s, 2H), 5.73, 5.00 (m, 1H), 4.40 (s, 3H), 4.26 - 4.06 (m, 2H), 2.83 (s, 3H), 1.75 - 1.41 (m, 6H). |
| | | | LCMS (ESI):560.95 [M+H]⁺ |
| 42 | 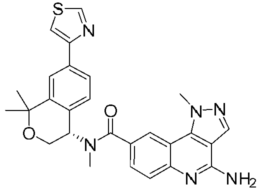 | *N*-((4S)-1,1-dimethyl-7-(1,3-thiazol-4-yl)isochroman-4-yl)(4-amino-1-methylpyrazolo[*4,5-c*]quinolin-8-yl)-N-methylformamide | ¹H NMR (400 MHz, DMSO-*d₆*) δ 9.14 (s, 1H), 8.44 (s, 1H), 8.36 (d, *J* = 17.4 Hz, 1H), 8.21 (s, 1H), 7.83-7.72 (m,4H),7.38-7.36(m,1H), 5.63-4.79 (m, 1H), 4.40-4.37 (m, 3H), 4.14-4.05 (m, 2H), 2.74-2.71 (m, 3H), 1.51-1.33 (m, 6H). |
| | | | LCMS (ESI):498.95 [M+H]⁺ |

### Example 7: Synthesis of 4-amino-N-(1,1-dimethyl-7-(1-methyl-1H-pyrazol-4-yl)isochroman-4-yl)-N,1-dimethyl-1H-pyrazolo[4,3-c]quinoline-8-carboxamide

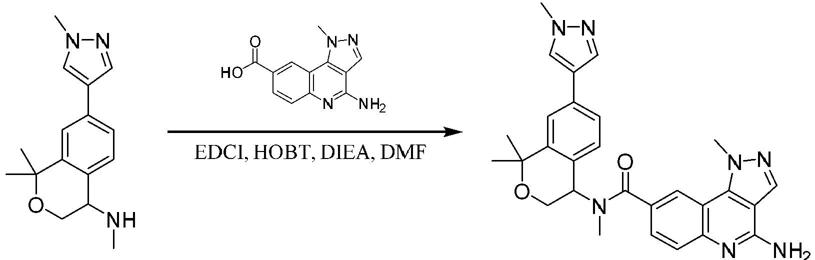

At room temperature, compound 4-amino-1-methylpyrazolo[4,3-c]quinoline-8-carboxylic acid (140 mg, 0.575 mmol) was dissolved in N,N-dimethylformamide (3 mL). 1-(3-Dimethylaminopropyl)-3-ethylcarbodiimide hydrochloride (110 mg, 0.575 mmol) and 1-hydroxybenzotriazole (79 mg, 0.575 mmol) were added, then N,N-diisopropylethylamine (186 mg, 1.437 mmol) was added and the mixture was stirred for reaction at room temperature for 0.5 hours. *N*,1,1-trimethyl-7-(1-methyl-1*H*-pyrazol-4-yl)isochroman-4-amine (130 mg, 0.479 mmol) was added. The mixture was reacted at room temperature for 16 hours. After LCMS indicated the reaction was complete, the mixture was poured into water (20 mL) and extracted with ethyl acetate (30 mL). The organic layer was washed with saturated saline (20 mL), dried over anhydrous sodium sulfate and filtered. The filtrate was concentrated under reduced pressure to give a crude product. The crude product was separated and purified by column chromatography (0-5% methanol/dichloromethane) to give 4-amino-N-(1,1-dimethyl-7-(1-methyl-1H-pyrazol-4-yl)isochroman-4-yl)-N,1-dimethyl-1H-pyrazolo[*4,3-c*]quinoline-8-carboxamide (25 mg, yield: 8.78%) as a white solid.
LCMS (ESI) m/z: 496.2 [M+H]⁺
¹H NMR (400 MHz, DMSO-*d₆*) δ 8.34 (s, 1H), 8.27 (s, 1H), 8.20 (s, 1H), 7.93 (s, 1H), 7.71 - 7.59 (m, 2H), 7.48 (s, 2H), 7.28 (s, 1H), 7.15 (s, 2H), 5.29 (d, *J* = 286.9 Hz, 1H), 4.41 (s, 3H), 4.13 (d, *J* = 30.6 Hz, 2H), 3.87 (s, 3H), 2.81 (s, 3H), 1.49 (d, *J* = 71.8 Hz, 6H).

The Examples in the following table may be prepared using the synthesis step described for **Example 7**, only replacing the corresponding starting materials:

| Example No. | Chemical Structure | Chemical Name | ¹HNMR&¹⁹F NMR& LCMS |
|---|---|---|---|
| 8 | 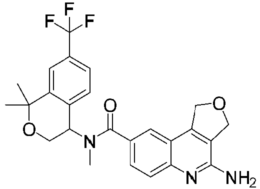 | 4-Amino-N-(1,1-dimethyl-7-(trifluoromethyl)isochro man-4-yl)-N-methyl-2,3-dihydrofuro[*3,4-c*]quinoline-8-carboxamide | ¹H NMR (400 MHz, DMSO-*d₆*) δ 7.78 - 7.54 (m, 6H), 6.73 (s, 2H), 5.82 - 5.52 (m, 1H), 5.39 (d, *J* = 20.2 Hz, 2H), 5.10 - 5.00 (m, 2H), 4.20 - 4.01 (m, 2H), 2.75 (s, 3H), 1.55 (d, *J* = 16.7 Hz, 6H). |
| | | | LCMS (ESI):472.2 [M+H]⁺ |
| 11 | 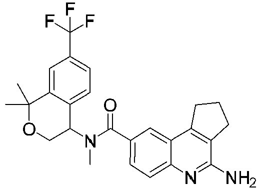 | 4-Amino-N-(1,1-dimethyl-7-(trifluoromethyl)isochro man-4-yl)-N-methyl-2,3-dihydro-1H-cyclopenta[*c*]quinoline-8-carboxamide | ¹H NMR (400 MHz, DMSO-*d₆*) δ 7.80 - 7.68 (m, 2H), 7.67 - 7.61 (m, 1H), 7.59 - 7.46 (m, 3H), 6.53 (s, 2H), 5.74 - 4.83 (m, 1H), 4.34 - 3.89 (m, 2H), 3.15 (s, 2H), 2.83 (t, J = 7.4 Hz, 2H), 2.74 (s, 3H), 2.25 - 2.11 (m, 2H), 1.63 - 1.35 (m, 6H). |
| | | | LCMS (ESI) m/z: 470.1 [M+H]⁺ |
| 12 | 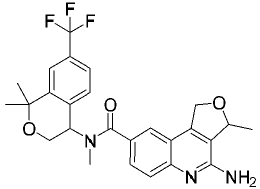 | 4-Amino-N-(1,1-dimethyl-7-(trifluoromethyl)isochro man-4-yl)-*N*,3-dimethyl-1,3-dihydrofuro[*3,4-c*]quinoline-8-carboxamide | ¹H NMR (400 MHz, DMSO-*d₆*) δ 7.77 - 7.44 (m, 6H), 6.61 (s, 2H), 5.70 (s, 0.5H), 5.46 - 5.22 (m, 3H), 4.84 (s, 0.5H), 4.21 - 4.02 (m, 2H), 2.74 (s, 3H), 1.63 - 1.47 (m, 5H), 1.44 - 1.35 (m, 4H). |
| | | | LCMS (ESI) m/z: 486.1 [M+H]⁺ |
| 18 | 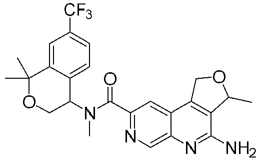 | 4-Amino-N-(1,1-dimethyl-7-(trifluoromethyl)isochro man-4-yl)-*N*,3-dimethyl-1,3-dihydrofuro[*3,4-c*][1,7]naphthyridine-8-carboxamide | ¹H NMR (400 MHz, DMSO-*d₆*) δ 8.90 -8.83 (m, 1H), 7.92 -7.80 (m, 1H), 7.76 -7.67 (m, 1H), 7.67 -7.46 (m, 2H), 6.99 (d, *J* = 11.6 Hz, 2H), 5.47 (d, *J* = 5.4 Hz, 1H), 5.43 -5.31 (m, 2H), 5.31 -5.20 (m, 1H), 4.19 - 4.00 (m, 2H), 2.76 (d, *J* = 14.1 Hz, 3H), 1.57 (d, *J* = 3.1 Hz, 3H), 1.53 (s, 1H), 1.41 (td, *J* = 3.7, 1.9 Hz, 5H). |
| | | | LCMS (ESI) m/z: 487.1 [M+H]⁺ |
| 19 | 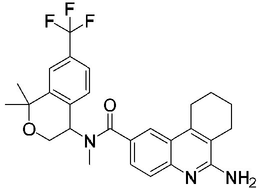 | 6-Amino-N-(1,1-dimethyl-7-(trifluoromethyl)isochro man-4-yl)-N-methyl-7,8,9,10-tetrahydrophenanthridine -2-carboxamide | ¹H NMR (400 MHz, DMSO-*d₆*) δ 7.87 (d, J = 2.0 Hz, 1H), 7.79 - 7.35 (m, 5H), 6.36 (s, 2H), 5.71 (s, 1H), 4.26 - 3.94 (m, 2H), 2.96 (s, 2H), 2.74 (s, 3H), 1.82 (s, 4H), 1.67 -1.24 (m, 8H). |
| | | | LCMS (ESI) m/z: 484.2 [M+H]⁺ |
| 20 | 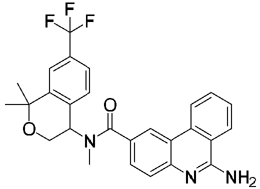 | 6-Amino-N-(1,1-dimethyl-7-(trifluoromethyl)isochro man-4-yl)-N-methylphenanthridine-2-carboxamide | ¹H NMR (400 MHz, DMSO-*d₆*) δ 8.75 (s, 1H), 8.63 (s, 1H), 8.36 (d, J = 8.2 Hz, 1H), 7.86 (ddd, J = 8.3, 7.1, 1.2 Hz, 1H), 7.80 - 7.45 (m, 6H), 7.24 (s, 2H), 5.33 (d, J = 341.9 Hz, 1H), 4.29 - 4.06 (m, 2H), 2.79 (s, 3H), 1.68 - 1.29 (m, 6H). |
| | | | LCMS (ESI) m/z: 480.2 [M+H]⁺ |
| 22 | 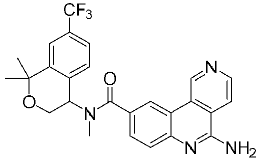 | 5-amino-N-(1,1-dimethyl-7-(trifluoromethyl)isochro man-4-yl)-N-methylbenzo[c][2,6]naph thyridine-9-carboxamide | ¹H NMR (400 MHz, DMSO-*d₆*) δ 10.15 (d, *J* = 21.3 Hz, 1H), 8.94 - 8.78 (m, 2H), 8.24 (d, *J* = 5.6 Hz, 1H), 7.83 - 7.57 (m, 5H),7.52 (s, 2H), 5.32 (d, *J* = 355.2 Hz, 1H), 4.18 (d, *J* = 19.3 Hz, 2H), 2.80 (s, 3H), 1.69 - 1.31 (m, 6H). |
| | | | LCMS (ESI) m/z:481.0[M+H]⁺ |
| 24 | 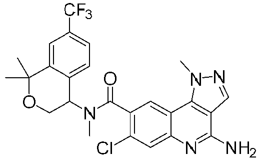 | 4-amino-7-chloro-N-(1,1-dimethyl-7-(trifluoromethyl)isochro man-4-yl)-*N*,1-dimethyl-1H-pyrazolo[*4,3-c*]quinoline-8-carboxamide | ¹H NMR (400 MHz, DMSO-*d₆*) δ 8.41 - 8.20 (m, 2H), 7.78 (d, *J =* 15.4 Hz, 1H), 7.71 - 7.60 (m, 3H), 7.29 (d, *J* = 4.6 Hz, 2H),5.83 - 5.30 (m, 1H), 4.43 - 4.34 (m, 3H), 4.13 - 4.02 (m, 1H), 2.81 (d, *J* = 8.1 Hz, 1H), 2.61 (s, 2H),1.99 (dt, *J* = 14.2, 7.1 Hz, 1H), 1.63 - 1.48 (m, 6H). |
| | | | LCMS (ESI) m/z:519.0[M+H]⁺ |
| 29 | 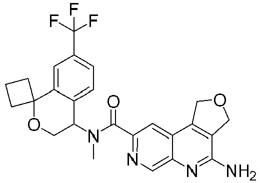 | 4-Amino-N-methyl-N-(7'-(trifluoromethyl)spiro[cy clobutane-1,1'-isochroman]-4'-yl)-1,3-dihydrofuro[*3,4-c*][1,7]naphthyridine-8-carboxamide | ¹H NMR (400 MHz, DMSO-*d₆*) δ 8.87 (d, *J* = 13.5 Hz, 1H), 8.01 -7.87 (m, 1H), 7.83 (d, *J* = 2.7 Hz, 1H), 7.76 -7.67 (m, 1H), 7.64 -7.43 (m, 1H), 7.11 (d, *J* = 10.1 Hz, 2H), 5.80 -5.33 (m, 3H), 5.05 (t, *J* = 3.7 Hz, 2H), 4.19 -3.95 (m, 2H), 2.72 (d, *J* = 18.8 Hz, 3H), 2.48 -2.35 (m, 3H), 2.33 -1.94 (m, 3H). |
| | | | LCMS (ESI) m/z: 485.2 [M+H]⁺ |
| 30 | 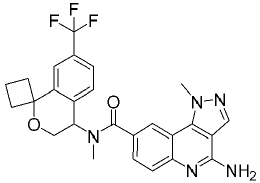 | 4-Amino-N-methyl-N-(7'-(trifluoromethyl)spiro[cy clobutane-1,1'-isochroman]-4'-yl)-1H-pyrazolo[*3,4-c*][1,7]quinoline-8-carboxamide | ¹H NMR (400 MHz, DMSO-*d₆*) δ 8.32 (d, *J* = 20.0 Hz, 2H), 7.86 (s, 1H), 7.65 (dt, *J* = 20.7, 10.2 Hz, 4H), 7.36 (s, 2H), 5.37 (d, *J* = 298.6 Hz, 1H), 4.41 (s, 3H), 4.10 (d, *J* = 8.5 Hz, 2H), 2.74 (s, 3H), 2.47 -2.33 (m, 3H), 2.13 (d, *J=* 75.0 Hz, 3H). |
| | | | LCMS (ESI) m/z: 496.2 [M+H]⁺ |
| 31 | 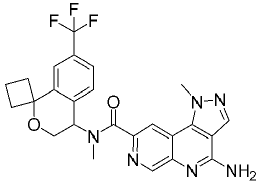 | 4-Amino-N-methyl-N-(7'-(trifluoromethyl)spiro[cy clobutane-1,1'-isochroman]-4'-yl)-1H-pyrazolo[*3,4-c*][1,7]naphthyridine-8-carboxamide | ¹H NMR (400 MHz, DMSO-*d₆*) δ 8.85 (d, *J* = 13.4 Hz, 1H), 8.52 - 8.34 (m, 2H), 7.85 (d, *J* = 23.0 Hz,1H), 7.78 - 7.52 (m, 2H), 7.48 (d, *J* = 7.4 Hz, 2H), 5.59 (d, *J =* 164.5 Hz, 1H), 4.46 (s, 3H), 4.21 - 3.95(m, 2H), 2.76 (d, *J* = 24.1 Hz, 3H), 2.48 - 1.96 (m, 6H). |
| | | | LCMS (ESI) m/z: 497.2 [M+H]⁺ |
| 39 | 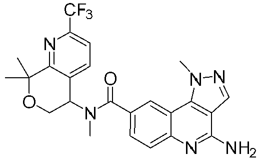 | 4-Amino-1-methylpyrazolo[*4,5-c*]quinolin-8-yl)-N-[8,8-dimethyl-2-(trifluoromethyl)(5,6-dihydro-8H-pyrano[*3,4-b*]pyridin-5-yl)]-N-methylformamide | ¹H NMR (400 MHz, DMSO-*d₆*) δ 8.35 (d, *J* = 1.8 Hz, 1H), 8.26 (s, 1H), 8.13 (d, *J* = 8.0 Hz, 1H), 7.83 (d, *J* = 8.1 Hz, 1H), 7.71 - 7.59(m, 2H), 7.16 (s, 2H), 5.77 (s, 1H), 4.40 (s, 3H), 4.25 - 4.12 (m, 2H), 2.83 (s, 3H), 1.62 - 1.39 (m, 6H). |
| | | | LCMS (ESI) m/z: 485.2 [M+H]⁺ |
| 40 | 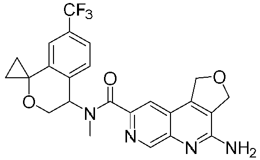 | 4-Amino-N-methyl-N-(7'-(trifluoromethyl)spiro[cy clopropane-1,1'-isochroman]-4'-yl)-1,3-dihydrofuro[*3,4-c*][1,7]naphthyridine-8-carboxamide | ¹H NMR (400 MHz, DMSO-*d₆*) δ 8.87 (d, J = 12.2 Hz, 1H), 7.87 (d, J = 31.0 Hz, 1H), 7.67 - 7.50 (m, 2H), 7.10 (dd, J = 28.3, 16.1 Hz, 3H), 5.96 - 5.48 (m, 1H), 5.38 (t, J = 3.6 Hz, 2H), 5.05 (t, J = 3.6 Hz, 2H), 4.14 (dq, J = 12.3, 6.4, 5.7 Hz, 2H),2.78 (d, J = 15.8 Hz, 3H), 1.30 - 1.20 (m, 4H). |
| | | | LCMS (ESI) m/z: 471.2 [M+H]⁺ |
| 43 | 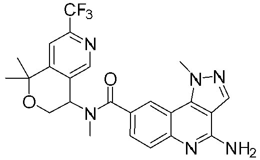 | (4-Amino-1-methylpyrazolo[*4,5-c*]quinolin-8-yl)-N-[1,1-dimethyl-7-(trifluoromethyl)(1,4-dihydro-3H-pyrano[*4,5-c*]pyridin-4-yl)]-N-methylformamide | ¹H NMR (400 MHz, DMSO-*d₆*) δ 8.79 (s, 1H), 8.37 (d, *J* = 1.8 Hz, 1H), 8.28 (s, 1H), 7.95 (s, 1H), 7.70 (dd, *J* = 8.5, 1.8 Hz, 1H), 7.63(d, *J* = 8.6 Hz, 1H), 7.24 (s, 2H), 5.73 (s, 1H), 4.41 (s, 3H), 4.31 - 4.10 (m, 2H), 2.84 (s, 3H), 1.59 (s, 6H). |
| | | | LCMS (ESI) m/z: 485.2 [M+H]⁺ |
| 46 | 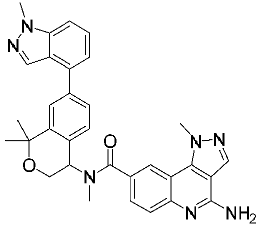 | 4-amino-N-(1,1-dimethyl-7-(1-methyl-1H-indazol-4-yl)isochroman-4-yl)-N,1-dimethyl-1H-pyrazolo[4,3-c]quinoline-8-carboxamide | ¹H NMR (400 MHz, DMSO-*d₆*) δ 8.36 (d, J = 1.9 Hz, 1H), 8.28 (s, 1H), 8.07 (s, 1H), 7.74 - 7.55 (m, 5H), 7.50 (t, J = 7.7 Hz, 2H), 7.38 - 7.12 (m, 3H), 5.76 (s, 1H), 4.41 (s, 3H), 4.16 (s, 2H), 4.10 (s, 3H), 2.86 (s, 3H), 1.62 (s, 3H), 1.44 (s, 3H). |
| | | | LCMS (ESI) m/z: 546.1 [M+H]⁺ |
| 47 | 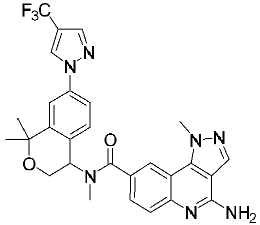 | 4-amino-N-(1,1-dimethyl-7-(4-(trifluoromethyl)-1H-pyrazol-1-yl)isochroman-4-yl)-N,1-dimethyl-1H-pyrazolo[4,3-c]quinoline-8-carboxamide | ¹H NMR (400 MHz, DMSO-*d₆*) δ 9.28 (s, 1H), 8.35 (s, 1H), 8.28 (s, 1H), 8.22 (s, 1H), 7.84 (d, J = 9.0 Hz, 2H), 7.73-7.59 (m, 2H), 7.58-7.46 (m, 1H), 7.25 (d, 2H), 5.76-5.00 (m, 1H), 4.41 (s, 3H), 4.30-4.02 (m, 2H), 2.81 (s, 3H), 1.69-1.40 (m, 6H). |
| | | | LCMS (ESI) m/z: 550.1 [M+H]⁺ |
| 48 | 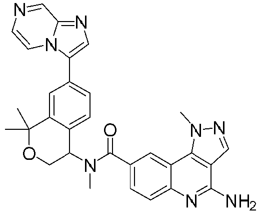 | 4-amino-N-(1,1-dimethyl-7-(1-methyl-1H-indazol-4-yl)isochroman-4-yl)-N,1-dimethyl-1H-pyrazolo[4,3-c]quinoline-8-carboxamide | ¹H NMR (400 MHz, DMSO-*d₆*) δ 9.15 (d, *J* = 1.4 Hz, 1H), 8.54 (s, 1H), 8.37 (s, 1H), 8.31 (s, 1H), 8.11 (s, 1H), 7.97 (d, *J* = 4.7 Hz, 1H),7.69 (q, *J* = 7.6 Hz, 4H), 7.36 (s, 2H), 5.77 - 4.92 (m, 1H), 4.42 (s, 3H), 4.16 (s, 2H), 2.85 (s, 3H), 1.66 - 1.40 (m, 6H). |
| | | | LCMS (ESI) m/z: 533.6 [M+H]⁺ |
| 52 | 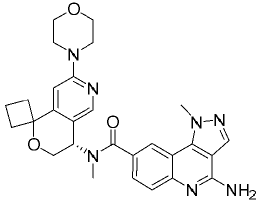 | (S)-4-amino-N,1-dimethyl-N-(7'-morpholino-3',4'-dihydrospiro[cyclobutanen -1,1'-pyrano[*4,3-c*]pyridin]-4'-yl)-1H-pyrazolo[*4,3-c*]quinoxaline-8-carboxamide | LCMS (ESI) m/z: 514.2 [M+H]⁺ |
| 53 | 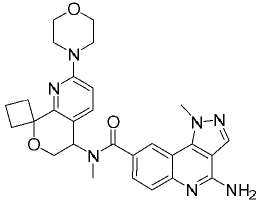 | 4-amino-N,1-dimethyl-N-(2'-morpholino-5',6'-dihydrospiro[cyclobutan e-1,8'-pyrano[*3,4-b*]pyridin]-5'-yl)-1H-pyrazolo[*4,3-c*]quinoxaline-8-carboxamide | ¹H NMR (400 MHz, DMSO-*d₆*) δ 8.33 (s, 1H), 8.24 (s, 1H), 7.82 - 7.50 (m, 3H), 7.12 (s, 2H), 6.85 (d, *J* = 8.8 Hz, 1H), 4.90 (s, 1H), 4.34 (d, *J* = 30.5 Hz, 3H), 4.04 (d, *J* = 6.3 Hz, 2H), 3.72 (t, *J* = 4.9 Hz, 4H), 3.46 (q, *J =* 5.5, 5.1 Hz, 4H), 2.70 (d, *J* = 11.3 Hz, 3H), 2.38 - 2.10 (m, 4H), 2.04 - 1.80 (m, 2H). |
| | | | LCMS (ESI) m/z: 514.2 [M+H]⁺ |
| 54 | 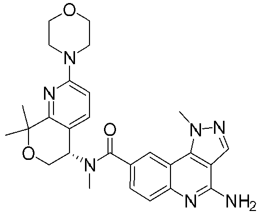 | (S)-4-amino-N-(8,8-dimethyl-2-morpholino-5,8-dihydro-6H-pyrano)[3,4-b]pyridin]-5-yl)-N,1-dimethyl-1H-pyrazolo[*4,3-c*]quinoxaline-8-carboxamide | ¹H NMR (400 MHz, DMSO-*d₆*) δ 8.40 - 8.22 (m, 2H), 7.63 (s, 3H), 7.02 (d, *J* = 189.2 Hz, 3H), 5.22 (d, *J* = 289.4 Hz, 1H), 4.40 (s, 3H), 4.03 (q, *J* = 7.0 Hz, 2H), 3.70 (t, *J =* 4.8 Hz, 4H), 3.45 (s, 4H), 2.79 (s, 3H), 1.49 (s, 4H), 1.31 (s, 2H). |
| | | | LCMS (ESI) m/z: 502.2 [M+H]⁺ |
| 55 | 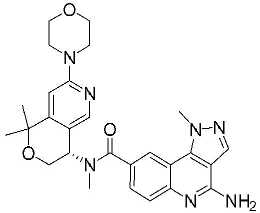 | (S)-4-amino-N-(1,1-dimethyl-7-morpholino-3,4-dihydro-1H-pyrano)[4,3-c]pyridin-4-yl)-N,1-dimethyl-1H-pyrazolo[*1,5-a*]quinoxaline-8-carboxamide | ¹H NMR (400 MHz, DMSO-*d₆*) δ 8.36 -8.25 (m, 2H), 8.06 (s, 1H), 7.71 -7.57 (m, 2H), 7.24 (s, 2H), 6.71 (s, 1H), 5.22 (d, *J* = 281.8 Hz, 1H), 4.40 (s, 3H), 4.05 (d, *J* = 8.3 Hz, 2H), 3.70 (t, *J* = 4.7 Hz, 4H), 3.46 (d, *J* = 5.0 Hz, 4H), 2.79 (s, 3H), 1.58 -1.29 (m, 6H). |
| | | | LCMS (ESI) m/z: 502.2 [M+H]⁺ |
| 56 | 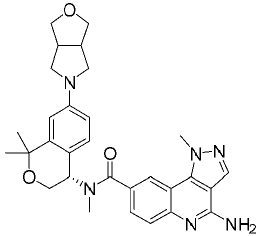 | 4-Amino-N-((4S)-1,1-dimethyl-7-(tetrahydro-1H-furo[3,4-c]pyrrol-5(3H)-yl)isochroman-4-yl)-*N*,1-dimethyl-1H-pyrazolo[*4,3-c*]quinoxaline-8-carboxamide | ¹H NMR (400 MHz, DMSO-*d₆*) δ 8.27 (d, *J* = 12.4 Hz, 2H), 7.61 (s, 2H), 7.14 (s, 3H), 6.43 (t, *J* = 62.7 Hz, 2H), 5.17 (d, *J* = 285.7 Hz, 1H), 4.39 (s, 3H), 4.03 (s, 2H), 3.87 (s, 2H), 3.48 (dt, *J* = 43.7, 7.8 Hz, 4H), 3.09 (dd, *J* = 46.0, 38.0 Hz, 4H), 2.76 (s, 3H), 1.51 (d, *J* = 2.5 Hz, 3H), 1.40 (d, *J =* 54.4 Hz, 3H). |
| | | | LCMS (ESI) m/z: 527.2 [M+H]⁺ |
| 57 | 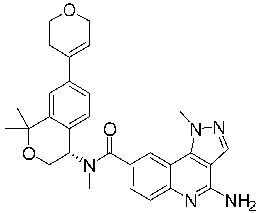 | (*S*)-4-amino-N-(7-(3,6-dihydro-2H-pyran-4-yl)-1,1-dimethylisochroman-4-yl)-*N*,1-dimethyl-1H-pyrazolo[*4,3-c*]quinoxaline-8-carboxamide | ¹H NMR (400 MHz, DMSO-*d₆*) δ 8.78 - 8.19 (m, 4H), 7.78 (d, *J =* 15.8 Hz, 2H), 7.33 (d, *J* = 18.2 Hz,3H), 6.30 (s, 1H), 5.24 (d, *J* = 328.3 Hz, 1H), 4.55 - 4.37 (m, 3H), 4.23 (s, 2H), 4.08 (s, 2H), 3.82 (s,2H), 2.77 (d, *J* = 10.7 Hz, 3H), 2.49 - 2.41 (m, 2H), 1.64 - 1.35 (m, 6H). |
| | | | LCMS (ESI) m/z: 498.2 [M+H]⁺ |
| 59 | 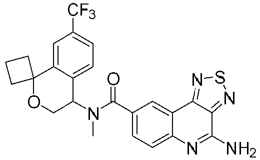 | 4-Amino-N-methyl-N-(7'-(trifluoromethyl)spiro[cy clobutane-1,1'-isochroman]-4'-yl)-[1,2,5]thiadiazolo[*3,4-c*]quinoline-8-carboxamide | ¹H NMR (400 MHz, DMSO-*d₆*) δ 8.40 (d, *J* = 2.0 Hz, 1H), 7.94 - 7.67 (m, 5H), 7.61 (t, *J* = 10.3 Hz, 2H), 5.76 (s, 1H), 4.30 - 3.95 (m, 2H), 2.95 (s, 1H), 2.79 (s, 1H), 2.73 (s, 3H), 2.41 (t, *J* = 8.2 Hz, 3H), 1.96 (s, 1H). |
| | | | LCMS (ESI) m/z: 500 [M+H]⁺ |
| 60 | 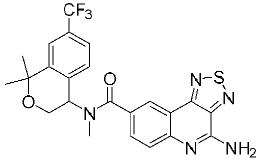 | 4-Amino-N-(1,1-dimethyl-7-(trifluoromethyl)isochro man-4-yl)-N-methyl-[1,2,5]thiadiazolo[3,4-c]quinoline-8-carboxamide | ¹H NMR (400 MHz, DMSO-*d₆*) δ 8.40 (d, *J* = 2.0 Hz, 1H), 7.85 - 7.50 (m, 8H), 5.34 (d, *J=* 315.5 Hz, 1H), 4.12 (dd, *J* = 12.1, 5.7 Hz, 2H), 2.79 (d, *J =* 3.3 Hz, 3H), 1.56 (d, *J* = 16.1 Hz, 6H). |
| | | | LCMS (ESI) m/z: 488 [M+H]⁺ |
| 61 | 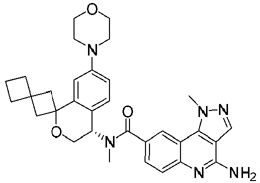 | (*S*)-4-amino-N,1-dimethyl-N-(7"-morpholinodispiro[cyclo butane-1,1'-cyclobutane-3',1"-isochroman]-4"-yl)-1H-pyrazolo[*4,3-c*]quinoxaline-8-carboxamide | LCMS (ESI) m/z: 553.3 [M+H]⁺ |
| 62 | 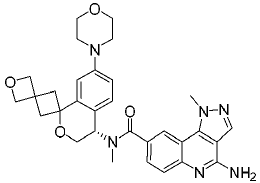 | (*S*)-4-amino-N,1-dimethyl-N-(7-morpholinodispiro[isochr oman-1,1'-cyclobutane-3',3"-oxetane]-4-yl)-1H-pyrazolo[*4,3-c*]quinoxaline-8-carboxamide | LCMS (ESI) m/z: 555.3 [M+H]⁺ |
| 63 | 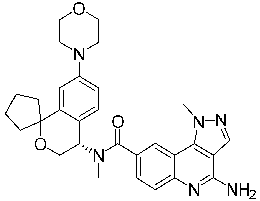 | (*S*)-4-Amino-N,1-Dimethyl-N-(7'-morpholinodispiro[cyclo pentane-1,1'-isochroman]-4'-yl)-1H-pyrazolo[*4,3-c*]quinoxaline-8-carboxamide | LCMS (ESI) m/z: 527.3 [M+H]⁺ |
| 64 | 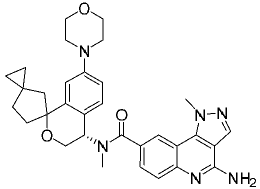 | 4-Amino-N,1-dimethyl-N-((4"S)-7"-morpholinodispiro[cyclo propane-1,1'-cyclopentane-3',1"-isochroman]-4"-yl)-1H-pyrazolo[*4,3-c*]quinoxaline-8-carboxamide | LCMS (ESI) m/z: 553.3 [M+H]⁺ |
| 65 | 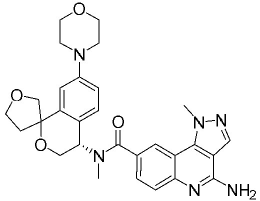 | 4-Amino-N,1-dimethyl-N-((4'S)-7'-morpholino-4,5-dihydro-2H-spiro[furan-3,1'-isochroman]-4'-yl)-1H-pyrazolo[*4,3-c*]quinoxaline-8-carboxamide | LCMS (ESI) m/z: 529.3 [M+H]⁺ |
| 66 | 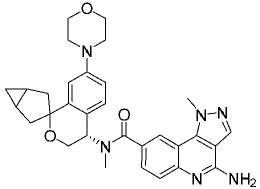 | 4-Amino-N,1-dimethyl-N-((4'S)-7'-morpholinospiro[bicyclo [3.1.0]hexane-3,1'-isochroman]-4'-yl)-1H-pyrazolo[*4,3-c*]quinoxaline-8-carboxamide | LCMS (ESI) m/z: 539.3 [M+H]⁺ |
| 67 | 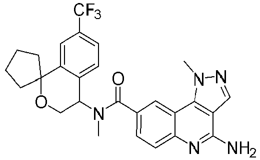 | 4-Amino-*N,1*-dimethyl-N-(7'-(trifluoromethyl)spiro[cy clopentane-1,1'-isochroman]-4'-yl)-1H-pyrazolo[*4,3-c*]quinoline-8-carboxamide | ¹H NMR (400 MHz, DMSO-*d₆*) δ 8.41 - 8.16 (m, 2H), 7.74 - 7.48 (m, 5H), 7.20 (s, 2H), 5.36 (d, *J =* 297.0 Hz, 1H), 4.40 (s, 3H), 4.08 (s, 2H), 2.78 (s, 3H), 1.84 (s, 8H). |
| | | | LCMS (ESI) m/z: 510.2[M+H]⁺ |
| 68 | 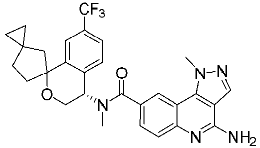 | 4-Amino-N,1-dimethyl-N-((4"S)-7"-(trifluoromethyl)dispiro[ cyclopropane-1,1'-cyclopentane-3',1"-isochroman]-4"-yl)-1H-pyrazolo[*4,3-c*]quinoxaline-8-carboxamide | LCMS (ESI) m/z: 536.2[M+H]⁺ |
| 69 | 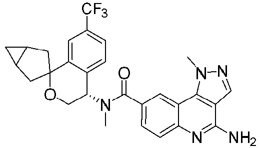 | 4-Amino-N,1-dimethyl-N-((4'S)-7'-(trifluoromethyl)dispiro[ bicyclo[3.1.0]hexane-3,1'-isochroman]-4'-yl)-1H-pyrazolo[*4,3-c*]quinoxaline-8-carboxamide | LCMS (ESI) m/z: 522.2[M+H]⁺ |
| 70 | 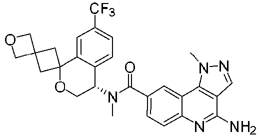 | (*S*)-4-amino-N,1-dimethyl-N-(7-(trifluoromethyl)dispiro[i sochroman-1,1'-cyclobutane-3',3"-oxetane]-4-yl)-1H-pyrazolo[*4,3-c*]quinoxaline-8-carboxamide | LCMS (ESI) m/z: 538.2[M+H]⁺ |
| 71 | 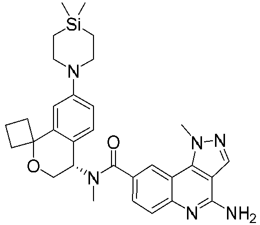 | (S)-4-amino-N-(7'-(4,4-dimethyl-1,4-azasilinane-1-yl)spiro[cyclobutane-1,1'-isochroman]-4'-yl)-N,1-dimethyl-1H-pyrazolo[*4,3-c*]quinoxaline-8-carboxamide | LCMS (ESI) m/z: 555.3M+H]⁺ |
| 72 | 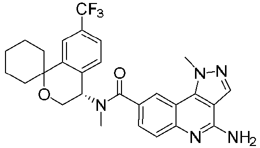 | (S)-4-amino-*N,1-*dimethyl-N-(7'-(trifluoromethyl)spiro[cy clohexane-1,1'-isochroman]-4'-yl)-1H-pyrazolo[*4,3-c*]quinoxaline-8-carboxamide | LCMS (ESI) m/z: 524.2M+H]⁺ |
| 73 | 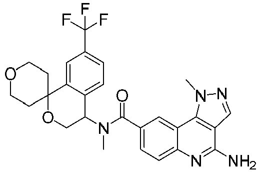 | 4-Amino-*N,1*-dimethyl-N-(7'-(trifluoromethyl)-2',3',5',6'-tetrahydrospiro[isochrom an-1,4'-pyran]-4-yl)-1H-pyrazolo[*4,3-c*]quinoxaline-8-carboxamide | ¹H NMR (400 MHz, DMSO-*d₆*) δ 8.40 - 8.20 (m, 2H), 7.81 - 7.57 (m, 5H), 7.21 (s, 2H), 5.39 (d, *J =* 293.1 Hz, 1H), 4.40 (s, 3H), 4.16 (s, 2H), 3.75 (s, 4H), 2.79 (s, 3H), 2.09 - 1.56 (m, 4H). |
| | | | LCMS (ESI) m/z: 526.2[M+H]⁺ |
| 74 | 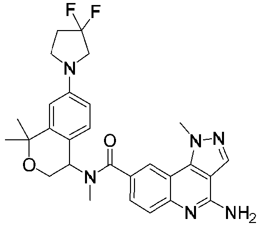 | 4-Amino-*N*-(7-(3,3-difluoropyrrolidin-1-yl)-1,1-dimethylisochroman-4-yl)-*N*,1-dimethyl-1H-pyrazolo[*4,3-c*]quinoxaline-8-carboxamide | ¹H NMR (400 MHz, DMSO-*d₆*) δ 8.28 (d, *J* = 12.2 Hz, 2H), 7.62 (s, 2H), 7.14 (d, *J* = 41.9 Hz, 3H), 6.67-6.37 (m, 2H), 5.18 (d, *J* = 288.5 Hz, 1H), 4.39 (s, 3H), 4.03 (s, 2H), 3.68 (d, *J* = 13.6 Hz, 2H), 3.48 (s, 2H), 2.76 (s, 3H), 1.50 (d, *J* = 18.7 Hz, 4H), 1.38 - 1.25 (m, 4H). |
| | | | LCMS (ESI) m/z: 521.2M+H]⁺ |
| 77 | 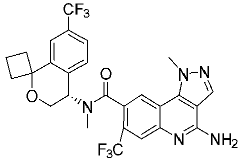 | (S)-4-amino-*N,1-*dimethyl-7-(trifluoromethyl)-N-7'-(trifluoromethyl)spiro[cy clobutane-1,1'-isochroman]-4'-yl)-1H-pyrazolo[4,3-c]quinoxaline-8-carboxamide | ¹H NMR (400 MHz, DMSO-*d₆*) δ 8.33 (d, *J* = 4.1 Hz, 1H), 8.27 (d, *J =* 4.8 Hz, 1H), 7.93 - 7.87 (m, 2H), 7.82 - 7.69 (m, 2H), 7.38 (s, 2H), 5.78 (s, 1H), 4.47 (d, *J* = 5.5 Hz, 3H), 4.12 (s, 2H), 2.55 (s, 3H), 2.39 (d, *J* = 10.0 Hz, 2H), 2.12 - 1.99 (m, 2H), 1.23 (s, 2H). |
| | | | LCMS (ESI) m/z: 564.2M+H]⁺ |
| 78 | 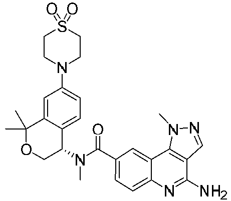 | (S)-4-amino-N-(7-(1,1-dioxidothiomorpholino)-1,1-dimethylisochroman-4-yl)-*N*,1-dimethyl-1H-pyrazolo[*4,3-c*]quinoxaline-8-carboxamide | ¹H NMR (400 MHz, DMSO-*d₆*) δ 8.28 (d, *J* = 17.3 Hz, 2H), 7.62 (d, *J* = 4.2 Hz, 2H), 7.14 (s, 2H), 7.03 - 6.84 (m, 2H),5.21 (d, *J* = 284.8 Hz, 1H), 4.38 (d, *J* = 5.9 Hz, 3H), 4.04 (s, 1H), 3.79 (s, 4H), 3.12 (s, 4H), 2.78 (s, 3H), 1.44 (d, *J* = 70.6 Hz, 6H). |
| | | | LCMS (ESI) m/z: 549.2M+H]⁺ |
| 79 | 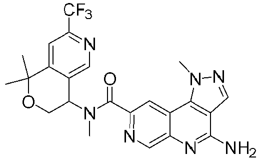 | 4-Amino-*N*-(1,1-dimethyl-7-(trifluoromethyl)-3,4-dihydro-1H-pyrano[4,3-c]pyridin-4-yl)-*N*,1-dimethyl-1H-pyrazolo[*4,3-c*][1,7]naphthyridine-8-carboxamide | ¹H NMR (400 MHz, DMSO-*d₆*) δ 8.94 - 8.69 (m, 2H), 8.52 - 8.35 (m, 2H), 7.96 (d, *J* = 20.2 Hz, 1H), 7.49 (d, *J* = 9.2 Hz, 2H), 5.79 - 5.32(m, 1H), 4.46 (d, *J* = 2.4 Hz, 3H), 4.25 - 4.06 (m, 2H), 2.86 (d, *J* = 22.7 Hz, 3H), 1.62 - 1.29 (m, 6H). |
| | | | LCMS (ESI) m/z: 486.2M+H]⁺ |
| 80 | 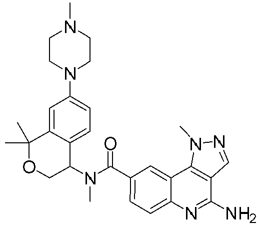 | 4-Amino-N-(1,1-dimethyl-7-(4-methylpiperazin-1-yl)isochroman-4-yl)-N,1-dimethyl-1H-pyrazolo[4,3-c]quinoxaline-8-carboxamide | ¹H NMR (400 MHz, DMSO-*d₆*) δ 8.28 (d, *J* = 12.7 Hz, 2H), 7.63 (s, 2H), 7.17 (s, 3H), 6.98 - 6.71 (m, 2H), 5.20 (d, *J* = 286.7 Hz, 1H), 4.48 - 4.32 (m, 3H), 4.07 (d, *J* = 30.6 Hz, 2H), 3.17 (s, 4H), 2.77 (s, 3H), 2.27 (s, 3H), 1.92 (s, 1H), 1.50 (d, *J* = 16.1 Hz, 4H), 1.41 - 1.20 (m, 5H). |
| | | | LCMS (ESI) m/z: 514.1M+H]⁺ |
| 81 | 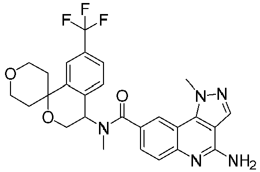 | 4-Amino-N,1-dimethyl-N-(2'-morpholino-5',6'-dihydrospiro[cyclobutane -1,8'-pyrano[*3,*4-b]pyridin]-5'-yl)-1H-pyrazolo[4,3-c]quinoline-8-carboxamide | ¹H NMR (400 MHz, DMSO-*d₆*) δ 8.40 - 8.20 (m, 2H), 7.81 - 7.57 (m, 5H), 7.21 (s, 2H), 5.39 (d, *J* = 293.1 Hz, 1H), 4.40 (s, 3H), 4.16 (s, 2H), 3.75 (s, 4H), 2.79 (s, 3H), 2.09- 1.56 (m, 4H). |
| | | | LCMS (ESI) m/z: 526.2[M+H]⁺ |
| 83 | 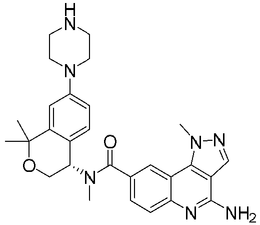 | (S)-4-amino-N-(1,1-dimethyl-7-piperazin-1-yl)isochroman-4-yl)-N,1-dimethyl-1H-pyrazolo[4,3-c]quinoxaline-8-carboxamide | LCMS (ESI) m/z:500.6[M+H]⁺ |
| 84 | 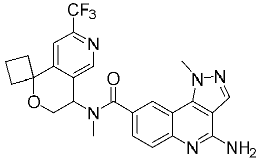 | N-[(4S)-7-(trifluoromethyl)spiro[1, 4-dihydro-3H-pyrano[*4,5-c*]pyridine-1,1'-cyclobutan]-4-yl](4-amino-1-methylpyrazolo[*4,5*- c]quinolin-8-yl)-N-methylformamide | ¹H NMR (400 MHz, DMSO-*d₆*) δ 8.79 (s, 1H), 8.38 (s, 1H), 8.31 (s, 1H), 8.03 (s, 1H), 7.73 (dd, *J* = 8.6, 1.8 Hz, 1H), 7.64 (d, *J* = 8.6 Hz, 1H), 7.42 (s, 2H), 5.72 - 5.09 (m, 1H), 4.42 (s, 3H), 4.19 (d, *J* = 39.9 Hz, 2H), 2.79 (s, 3H), 2.63 (d, *J* = 30.2 Hz, 2H), 2.47 - 2.34 (m, 2H), 2.06 (d, *J* = 13.2 Hz, 2H). |
| | | | LCMS (ESI) m/z:497.5[M+H]⁺ |
| 85 | 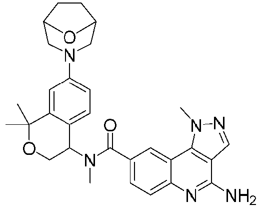 | *N*-[(4S)-1,1-dimethyl-7-(8-oxa-3-azabicyclo[3.2.1]octan-3-yl)isochroman-4-yl](4-amino-1-methylpyrazolo[*4,5-c*]quinolin-8-yl)-N-methylformamide | ¹H NMR (400 MHz, DMSO-*d₆*) δ 8.29 (s, 1H), 8.25 (s, 1H), 7.61 (s, 2H), 7.14 (s, 3H), 6.84 - 6.61 (m, 2H), 5.18 (d, *J* = 288.6 Hz, 1H),4.40 (d, *J* = 13.8 Hz, 5H), 4.05 (d, *J* = 27.6 Hz, 2H), 3.42 (d, *J* = 10.7 Hz, 2H), 2.76 (s, 4H), 1.82 (s, 4H), 1.42 (d, *J* = 71.9 Hz, 6H). |
| | | | LCMS (ESI) m/z:527.5[M+H]⁺ |
| 86 | 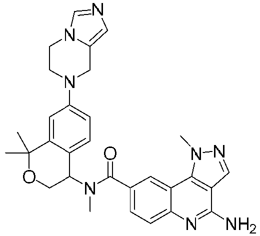 | *N*-((4S)-7-imidazo[*5,1-*c]piperazin-7-yl-1,1-dimethylisochroman-4-yl)(4-amino-1-methylpyrazolo[*4,5-c*]quinolin-8-yl)-N-methylformamide | ¹H NMR (400 MHz, DMSO-*d₆*) δ 8.29 (d, J = 14.3 Hz, 2H), 7.62 (d, *J* = 11.6 Hz, 3H), 7.17 (s, 3H), 7.05 - 6.86 (m, 2H), 6.78 (s, 1H),5.85 - 4.60 (m, 1H), 4.52 - 4.34 (m, 5H), 4.13 (t, *J =* 5.6 Hz, 2H), 4.04 (s, 2H), 3.70 (s, 2H), 2.78 (s, 3H), 1.52 (d, *J* = 18.1 Hz, 6H). |
| | | | LCMS (ESI) m/z:536.6[M+H]⁺ |
| 87 | 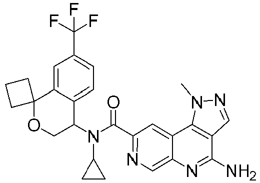 | 4-Amino-N-cyclopropyl-1-methyl-N-(7'-(trifluoromethyl)spiro[cy clobutane-1,1'-isochroman]-4'-yl)-1H-pyrazolo[*4,3-c*][1,7]naphthyridine-8-carboxamide | ¹H NMR (400 MHz, DMSO-*d₆*) δ 8.86 (s, 1H), 8.35 (s, 1H), 8.30 (s, 1H), 7.81 (s, 1H), 7.69-7.55 (m, 2H), 7.44 (s, 2H), 5.35 (s, 1H), 4.44 (s, 3H), 4.35-4.21 (m, 1H), 4.19- 4.05 (m, 1H), 2.99 (s, 1H), 2.47-2.00 (m, 6H), 0.45 - 0.09 (m, 4H). |
| | | | LCMS (ESI): [M+H]⁺ =523.3 |
| 89 | 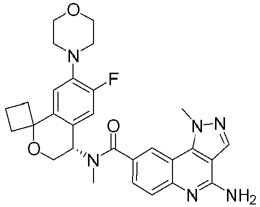 | (S)-4-amino-N-(6'-fluoro-7'-morpholinospiro[cyclobu tane-1,1'-isochroman]-4'-yl)-N,1-dimethyl-1H-pyrazolo[4,3-c]quinoline-8-carboxamide | LCMS (ESI): [M+H]⁺ =531.24 |
| 90 | 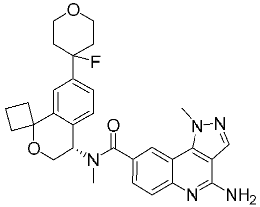 | (S)-4-amino-N-(7'-(4-fluorotetrahydro-2H-pyran-4-yl)spiro[cyclobutane-1,1'-isochroman]-4'-yl)-N,1-dimethyl-1H-pyrazolo[4,3-c]quinoline-8-carboxamide | LCMS (ESI): [M+H]⁺ =530.62 |
| 91 | 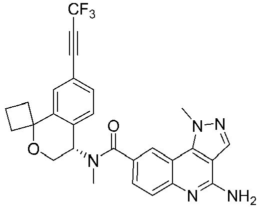 | (S)-4-amino-N,1-dimethyl-N-(7'-(3,3,3-trifluoroprop-1-yn-1-yl)spiro[cyclobutane-1,1'-isochroman]-4'-yl)-1H-pyrazolo[4,3-c]quinoline-8-carboxamide | LCMS (ESI): [M+H]⁺ =520.2 |

### Example 14: Synthesis of (4-amino-7-fluoro-1-methylpyrazolo[4,5-c]quinolin-8-yl)-N-[1,1-dimethyl-7-(1-methylpyrazol-5-yl)isochroman-4-yl]-N-methylformamide

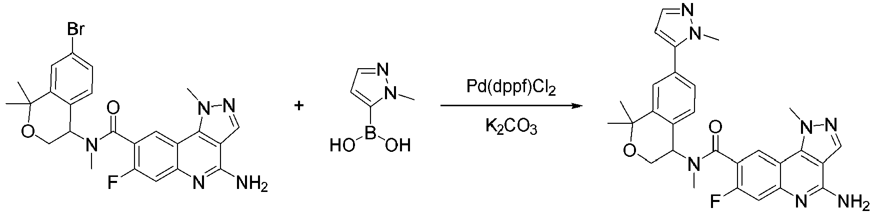

A mixture of (4-amino-7-fluoro-1-methylpyrazolo[*4,5-c*]quinolin-8-yl)-*N*-(7-bromo-1,1-dimethylisochroman-4-yl)-N-methylformamide (70 mg, 0.14 mmol) and (1-methyl-1H-pyrazol-5-yl)boronic acid (17.2 mg, 0.14 mmol) was dissolved in 1,4-dioxane/water (4 mL/1 mL). [1,1'-Bis(diphenylphosphino)ferrocene]dichloropalladium(II) (10 mg, 0.01 mmol) and potassium carbonate (38 mg, 0.27 mmol) were added to the reaction mixture. The mixture was stirred at 80°C for 2 hours, poured into water, extracted with ethyl acetate and purified by preparative high performance liquid chromatography (column: Sunfire C18 5 m, 30 mm × 150 mm; mobile phase A: water (0.1% formic acid), mobile phase B: acetonitrile; flow rate: 60 mL/min; gradient: from 11% B to 30% B over 8 minutes; wavelength: 254 nm/220 nm; retention time (min): 7.33) to give (4-amino-7-fluoro-1-methylpyrazolo[*4,5-c*]quinolin-8-yl)-N-[1,1-dimethyl-7-(1-methylpyrazol-5-yl)isochroman-4-yl]-N-methylformamide (7.90 mg, 11%).
LCMS (ESI): *m*/*z* 513.90 [M+H]⁺
¹H NMR (400 MHz, DMSO-*d₆*) *δ* 8.41 - 8.11 (m, 2H), 7.56 - 7.18 (m, 7H), 6.44 (d, *J* = 10.7 Hz, 1H), 5.75-4.73 (t, *J* = 4.4 Hz, 1H), 4.40 (d, *J* = 5.8 Hz, 3H), 4.15 - 3.98 (m, 2H), 3.86 (d, *J* = 17.0 Hz, 3H), 2.78 (d, *J* = 53.0 Hz, 3H), 1.66 - 1.31 (m, 6H).

### Example 49: Synthesis of (S)-4-amino-N-(1,1-dimethyl-7-(2-oxa-6-azaspiro[3.3]heptan-6-yl)isochroman-4-yl)-N,1-dimethyl-1H-pyrazolo[4,3-c]quinoline-8-carboxamide

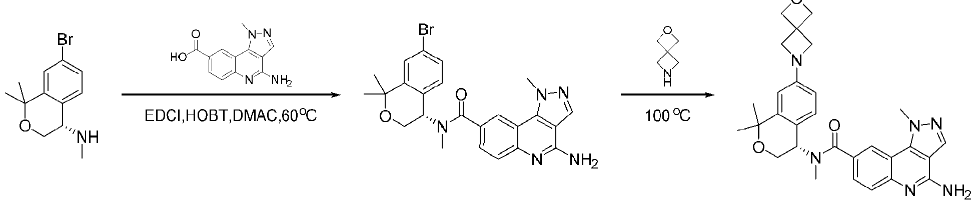

### Step 1: Synthesis of (S)-4-amino-N-(7-bromo-1,1-dimethylisochroman-4-yl)-N,1-dimethyl-1H-pyrazolo[4,3-c]quinoline-8-carboxamide

At room temperature, compound 4-amino-1-methyl-1H-pyrazolo[4,3-c]quinoline-8-carboxylic acid (97.0 mg, 0.390 mmol) was dissolved in N,N-dimethylformamide (3 mL). 1-(3-Dimethylaminopropyl)-3-ethylcarbodiimide hydrochloride (767 mg, 4.01 mmol) and 1-hydroxybenzotriazole (546 mg, 4.01 mmol) were added, then N,N-diisopropylethylamine (1293 mg, 10.02 mmol) was added and the mixture was stirred for reaction at room temperature for 0.5 hours. Then (S)-7-bromo-*N*,1,1-trimethylisochroman-4-amine hydrochloride (900.0 mg, 3.34 mmol) was added. The mixture was reacted at room temperature for 16 hours. After LCMS indicated the reaction was complete, the mixture was poured into water (100 mL) and extracted with ethyl acetate (50×3 mL). The organic layer was washed with saturated saline (50 mL), dried over anhydrous sodium sulfate and filtered. The filtrate was concentrated under reduced pressure to give a crude product. The crude product was purified by silica gel column chromatography (dichloromethane: methanol = 10:1) to give (S)-4-amino-N-(7-bromo-1,1-dimethylisochroman-4-yl)-*N,1*-dimethyl-1H-pyrazolo[4,3-c]quinoline-8-carboxamide (700 mg, 42.5%).
LCMS (ESI) m/z:494.1 [M+H]⁺

### Step 2: Synthesis of (S)-4-amino-N-(1,1-dimethyl-7-(2-oxa-6-azaspiro[3.3]heptan-6-yl)isochroman-4-yl)-N,1-dimethyl-1H-pyrazolo[4,3-c]quinoline-8-carboxamide

Under nitrogen atmosphere, (S)-4-amino-N-(7-bromo-1,1-dimethylisochroman-4-yl)-N,1-dimethyl-1H-pyrazolo[*4,3-*c]quinoline-8-carboxamide (110 mg, 0.223 mmol) was dissolved in 1,4-dioxane (2 mL). 2-Oxa-6-azaspiro[3.3]heptane (34 mg, 0.335 mmol), cesium carbonate (290 mg, 0.892 mmol) and methanesulfonato(9,9-dimethyl-4,5-bis(diphenylphosphino)xanthene)(2'-amino-1,1'-biphenyl-2-yl)palladium(II) (28.0 mg, 0.03 mmol) were added. The mixture was reacted at 100°C for 16 hours. The system was poured into water (20 mL) and extracted with ethyl acetate (10 mL×3). The combined organic layer was dried over anhydrous sodium sulfate and filtered to remove anhydrous sodium sulfate. The filtrate was concentrated under reduced pressure to give a crude product. The crude product was purified by silica gel column chromatography (petroleum ether: ethyl acetate = 3:1) to give (S)-4-amino-N-(1,1-dimethyl-7-(2-oxa-6-azaspiro[3.3]heptan-6-yl)isochroman-4-yl)-*N,1*-dimethyl-1H-pyrazolo[4,3-c]quinoline-8-carboxamide (20 mg, 0.039 mmol, yield: 17.5%) as a white solid.
LCMS (ESI) m/z: 513.1 [M+H]⁺

¹H NMR (400 MHz, DMSO-*d₆*) δ 8.28 (d, *J* = 4.0 Hz, 2H), 7.62 (s, 2H), 7.48 - 6.99 (m, 3H), 6.38 (s, 1H), 6.26 (d, *J* = 17.9 Hz, 1H), 4.80 (s, 1H), 4.70 (s, 4H), 4.38 (s, 3H), 4.06 (d, *J =* 32.1 Hz, 2H), 3.96 (s, 4H), 2.74 (s, 3H), 1.49 (s, 6H).

The Examples in the following table may be prepared using the synthesis step described for **Example 49,** only replacing the corresponding starting materials:

| Examp le No. | Chemical Structure | Chemical Name | ¹HNMR&¹⁹F NMR& LCMS |
|---|---|---|---|
| 75 | 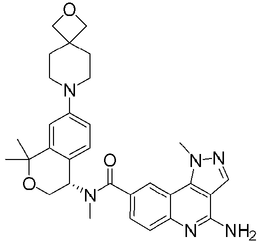 | (S)-4-amino-N-(1,1-dimethyl-7-(2-oxa-7-azaspiro[3.5]nonan-7-yl)isochroman-4-yl)-N,1-dimethyl-1H-pyrazolo[*4,3-c*]quinoxaline-8-carboxamide | ¹H NMR (400 MHz, DMSO-*d₆*) δ 8.28 (d, *J* = 6.8 Hz, 2H), 7.63 (s, 2H), 7.33 - 7.03 (m, 3H), 6.82 (d, *J* = 52.1 Hz, 2H), 5.17 (d, *J* = 289.6 Hz, 1H), 4.36 (d, *J* = 19.4 Hz, 6H), 4.06 (d, *J* = 31.3 Hz, 2H), 3.08 (s, 4H), 2.75 (s, 3H), 1.87 (s, 3H), 1.48 (d, *J =* 16.7 Hz, 4H), 1.34 (d, *J =* 10.5 Hz, 2H), 1.25 (d, *J* = 10.0 Hz, 2H). |
| | | | LCMS (ESI) m/z: 541.3M+H]⁺ |
| 76 | 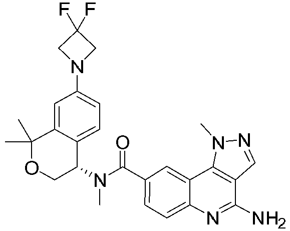 | (S)-4-amino-*N*-(7-(3,3-difluoroazetidin-1-yl)-1,1-dimethylisochroman-4-yl)-*N*,1-dimethyl-1H-pyrazolo[*4,3-c*]quinoxaline-8-carboxamide | ¹H NMR (400 MHz, DMSO-*d₆*) δ 8.29 (d, *J* = 8.3 Hz, 2H), 7.63 (s, 2H), 7.21 (d, *J =* 57.5 Hz, 3H), 6.61 - 6.29 (m, 2H), 5.19 (d, *J* = 292.6 Hz, 1H), 4.49 - 4.34 (m, 3H), 4.26 (t, *J* = 12.4 Hz, 4H), 4.08 (d, *J* = 29.5 Hz, 2H), 2.76 (s, 3H), 1.42 (d, *J* = 71.8 Hz, 6H). |
| | | | LCMS (ESI) m/z: 507.2M+H]⁺ |

### Example 58: (S)-4-amino-N-(1,1-dimethyl-7-(tetrahydro-2H-pyran-4-yl)-N,1-dimethyl-1H-pyrazolo[4,3-c]quinoxaline-8-carboxamide

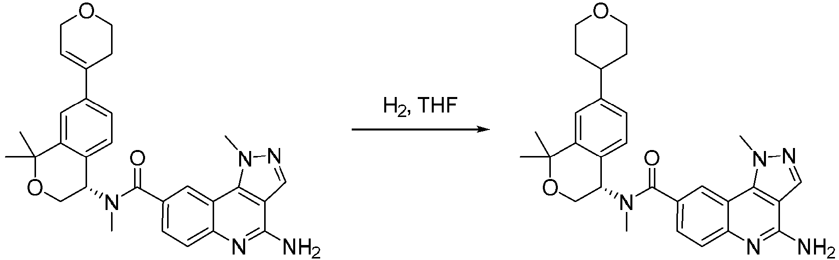

At room temperature, (S)-4-amino-N-(7-(3,6-dihydro-2H-pyran-4-yl)-1,1-dimethylisochroman-4-yl)-N,1-dimethyl-1H-pyrazolo[4,3-c]quinoline-8-carboxamide (50 mg, 0.100 mmol) was dissolved in methanol/tetrahydrofuran (1:1, 2 mL). Palladium on carbon (212 mg, 0.200 mmol) was added. The reaction mixture was purged with hydrogen three times and stirred at room temperature for 16 hours under hydrogen atmosphere. After the reaction was complete, the mixture was poured into water (20 mL) and extracted with ethyl acetate (20×3 mL). The organic layer was washed with saturated saline (20 mL), dried over anhydrous sodium sulfate and filtered. The filtrate was concentrated under reduced pressure to give a crude product. The crude product was purified by silica gel column chromatography (dichloromethane: methanol = 10:1) to give (S)-4-amino-N-(1,1-dimethyl-7-(tetrahydro-2H-pyran-4-yl)isochroman-4-yl)-N,1-dimethyl-1H-pyrazolo[4,3-c]quinoline-8-carboxamide (5 mg, yield: 10.0%).
LCMS (ESI) m/z: 500.2 [M+H]⁺
¹H NMR (400 MHz, DMSO-*d₆*) δ 8.28 (d, *J* = 22.2 Hz, 2H), 7.62 (s, 2H), 7.44 - 7.02 (m, 5H), 5.32 (s, 1H), 4.54 - 4.22 (m, 3H), 4.10 - 3.91 (m, 3H), 3.44 (s, 4H), 2.82 - 2.72 (m, 3H), 1.80 - 1.60 (m, 4H), 1.59 - 1.31 (m, 6H).

### Example 88: (S)-4-amino-N,1-dimethyl-N-(7'-(piperazin-1-yl)-3'-dihydrospiro[cyclobutane-1,1'-pyrano[4,3-c]pyridin]-4'-yl)-1H-pyrazolo[4,3-c]quinoline-8-carboxamide

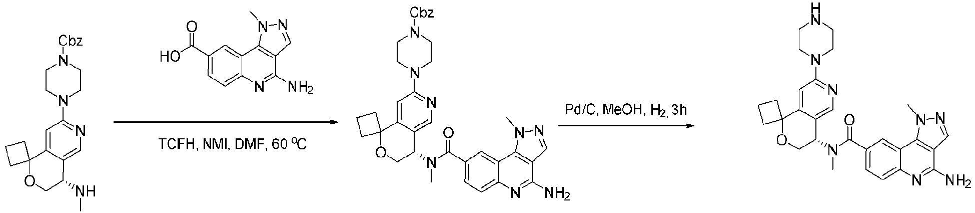

Step 1: 4-Amino-1-methyl-1*H*-pyrazolo[4,3-c]quinoline-8-carboxylic acid (270 mg, 1.11 mmol) was dissolved in N,N-dimethylformamide (5.0 mL). Chloro-*N*,*N*,*N*,N'-tetramethylformamidinium hexafluorophosphate (435 mg, 1.52mmol), N-methylimidazole (420 mg, 5.06 mmol) and N,N-diisopropylethylamine (400 mg, 3.04 mmol) were added. The mixture was stirred at room temperature for 20 minutes, then benzyl (S)-4-(4'-(tert-butoxycarbonyl)(methyl)amino)-3'-dihydrospiro[cyclobutane-1,1'-pyrano[*4,3-c*]pyridin]-7'-yl)piperazine-1-carboxylate (428 mg, 1.01 mmol) was added, and the mixture was stirred at 60°C for 16 hours. After the reaction was complete, the reaction mixture was poured into water (40 mL) and extracted with ethyl acetate (60 mL×3). The combined organic layer was dried over anhydrous sodium sulfate and filtered. The filtrate was concentrated under reduced pressure to give a crude product. The crude product was purified by reversed phase column chromatography (acetonitrile: water = 40:60) to give benzyl (S)-4-(4'-(4-amino-N,1-dimethyl-1H-pyrazolo[4,3-c]quinoline-8-carboxamido)-3'-dihydrospiro[cyclobutane-1,1'-pyrano[*4,3-c*]pyridin]-7'-yl)piperazine-1-carboxylate (280mg, yield: 42.7%).

LCMS (ESI) m/z: [M+H]⁺ = 647.38.

Step 2: Benzyl (S)-4-(4'-(4-amino-N,1-dimethyl-1H-pyrazolo[4,3-c]quinoline-8-carboxamido)-3'-dihydrospiro[cyclobutane-1,1'-pyrano[4,3-c]pyridin]-7'-yl)piperazine-1-carboxylate (20 mg, 0.03 mmol) was dissolved in methanol (3.0 mL). Palladium on carbon (20 mg) was added. The reaction mixture was purged with hydrogen three times and stirred at room temperature for 1 hour. After the reaction was complete, palladium on carbon was filtered out with Celite and the solvent was removed under vacuum. The residue was purified by preparative high performance liquid chromatography, eluting with 10%-90% acetonitrile/water (containing 0.1% ammonium bicarbonate), and freeze-dried to give (S)-4-amino-N-methyl-N-(7'-(piperazin-1-yl)-3'-,4'-dihydrospiro[cyclobutane-1,1'-pyrano[4,3-c]pyridin]-4'-yl)imidazo[*1,5-a*]quinoxaline-8-carboxamide (5.52 mg, yield: 34.57%) as a white solid.

LCMS (ESI) m/z: [M+H]⁺ = 513.4.

¹H NMR (400 MHz, DMSO-*d₆*) δ 8.30 (s, 1H), 8.25 (s, 1H), 8.05 (d, J = 36.2 Hz, 1H), 7.61 (s, 2H), 7.12 (s, 2H), 6.78 (s, 1H), 5.74 (s, 1H), 5.22 (d, J = 281 Hz, 1H), 4.39 (s, 3H), 4.01 (s, 2H), 3.44 (s, 4H), 2.76 (d, J = 20.6 Hz, 7H), 2.36-2.25 (m, 3H), 2.05 -1.95 (m, 2H).

### Example 16 & 17

### Example 16: (S)-4-amino-N-(1,1-dimethyl-7-(trifluoromethyl)isochroman-4-yl)-N,1-dimethyl-1H-pyrazolo[4,3-c]quinoline-8-carboxamide & Example 17: (R)-4-amino-N-(1,1-dimethyl-7-(trifluoromethyl)isochroman-4-yl)-N,1-dimethyl-1H-pyrazolo[4,3-c]quinoline-8-carboxamide

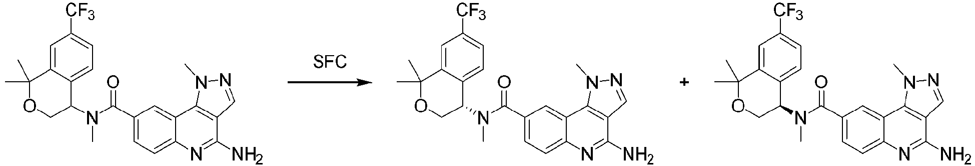

### Compound 4-amino-N-(1,1-dimethyl-7-(trifluoromethyl)isochroman-4-yl)-N,1-dimethyl-1H-pyrazolo[4,3-c]quinoline-8-carboxamide (70 mg) was purified by SFC (column: ChiralPak IBN, 250×40 mm I.D., 10 µm; conditions:

A for carbon dioxide and B for methanol (0.1% aqueous ammonia), 40%/40%, flow rate: 120 mL/min) to give (S)-4-amino-N-(1,1-dimethyl-7-(trifluoromethyl)isochroman-4-yl)-N,1-dimethyl-1H-pyrazolo[*4,3-c*]quinoline-8-carboxamide (20 mg) and (*R*)-4-amino-N-(1,1-dimethyl-7-(trifluoromethyl)isochroman-4-yl)-*N*,1-dimethyl-1H-pyrazolo[*4,3-c*]quinoline-8-carboxamide (22 mg).

### Example 16(S)-4-amino-N-(1,1-dimethyl-7-(trifluoromethyl)isochroman-4-yl)-N,1-dimethyl-1H-pyrazolo[4,3-c]quinoline-8-carboxamide

LCMS (ESI) m/z: 484.2 [M+H]⁺
¹H NMR (400 MHz, DMSO-*d₆*) δ 8.34 (d, *J* = 1.8 Hz, 1H), 8.27 (s, 1H), 7.76 - 7.69 (m, 1H), 7.69 - 7.56 (m, 4H), 7.16 (s, 2H), 5.38 (d, *J* = 286.9 Hz, 1H), 4.40 (s, 3H), 4.14 (s, 2H), 2.80 (s, 3H), 1.64 - 1.34 (m, 6H).

### Example 17 (R)-4-amino-N-(1,1-dimethyl-7-(trifluoromethyl)isochroman-4-yl)-N,1-dimethyl-1H-pyrazolo[4,3-c]quinoline-8-carboxamide

LCMS (ESI) m/z: 484.2 [M+H]⁺
¹H NMR (400 MHz, DMSO-*d₆*) δ 8.34 (d, *J =* 1.9 Hz, 1H), 8.27 (s, 1H), 7.73 (s, 1H), 7.69 - 7.55 (m, 4H), 7.16 (s, 2H), 5.38 (d, *J* = 286.9 Hz, 1H), 4.40 (s, 3H), 4.14 (s, 2H), 2.80 (s, 3H), 1.64 - 1.35 (m, 6H).

Using the procedures described in **Examples 16 and 17** and the corresponding conditions for chiral SFC resolution, compounds were obtained as follows:

| Example No. | Chemical Structure | Chemical Name | SFC condition |
|---|---|---|---|
| 25 | 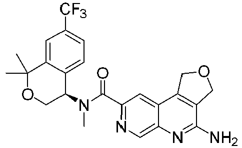 | (R)-4-Amino-N-(1,1-dimethyl-7-(trifluoromethyl)isochroma n-4-yl)-*N*-methyl-1,3-dihydrofuro[*3,4-c*][1,7]naphthyridine-8-carboxamide | Prepeak: SFC (column: ChiralPak IK (250×40 mm I.D., 10 µm), conditions: A for hexane and B for ethanol, 50%/50%, flow rate: 60 mL/min) |
| 26 | 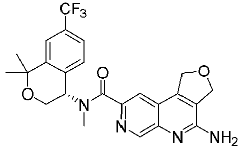 | (S)-4-Amino-N-(1,1-dimethyl-7-(trifluoromethyl)isochroma n-4-yl)-*N*-methyl-1,3-dihydrofuro[3,4-c][1,7]naphthyridine-8-carboxamide | Postpeak: SFC (column: ChiralPak IK (250×40 mm I.D., 10 µm), conditions: A for hexane and B for ethanol, 50%/50%, flow rate: 60 mL/min) |
| 27 | 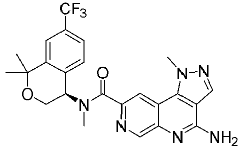 | (R)-4-amino-N-(1,1-dimethyl-7-(trifluoromethyl)isochroma n-4-yl)-*N*,1-dimethyl-1H-pyrazolo[*4,3-c*][1,7]naphthyridine-8-carboxamide | Prepeak: SFC (column: ChiralPak IK (250×40 mm I.D., 10 µm), conditions: A for carbon dioxide and B for methanol (0.1% aqueous ammonia), 35%/35%, flow rate: 140 mL/min) |
| 28 | 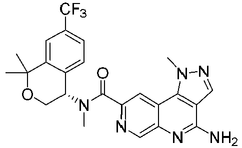 | (S)-4-amino-N-(1,1-dimethyl-7-(trifluoromethyl)isochroma n-4-yl)-*N*,1-dimethyl-1H-pyrazolo[4,3-c][1,7]naphthyridine-8-carboxamide | Prepeak: SFC (column: ChiralPak IK (250×40 mm I.D., 10 µm), conditions: A for carbon dioxide and B for methanol (0.1% aqueous ammonia), 35%/35%, flow rate: 140 mL/min) |
| 44 | 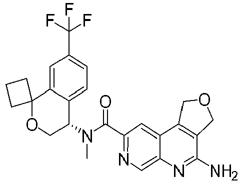 | (S)-4-amino-N-methyl-N-(7'-(trifluoromethyl)spiro[cycl obutane-1,1'-isochroman]-4'-yl)-1,3-dihydrofuro[*3,4-c*][1,7]naphthyridine-8-carboxamide | Prepeak: SFC (column: ChiralPak IG, ChiralPak IK (250×40 mm I.D., 10 µm), conditions: A for carbon dioxide and B for methanol (0.1% aqueous ammonia), 50%/50%, flow rate: 140 mL/min) |
| 45 | 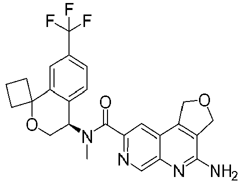 | (R)-4-amino-N-methyl-N-(7'-(trifluoromethyl)spiro[cycl obutane-1,1'-isochroman]-4'-yl)-1,3-dihydrofuro[*3,4-c*][1,7]naphthyridine-8-carboxamide | Post peak: SFC (column: ChiralPak IG, ChiralPak IK (250×40 mm 1.D., 10 µm), conditions: A for carbon dioxide and B for methanol (0.1% aqueous ammonia), 50%/50%, flow rate: 140 mL/min) |
| 50 | 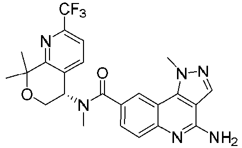 | (S)-4-amino-1-methylpyrazolo[*4,5-c*]quinolin-8-yl)-N-[8,8-dimethyl-2-(trifluoromethyl)(5,6-dihydro-8H-pyrano[*3,4-b*]pyridin-5-yl)]-N-methylformamide | Prepeak: SFC (column: Daicel ChiralPak IBN, (250×40 mm I.D., 10 µm), conditions: A for carbon dioxide and B for methanol (0.1% aqueous ammonia), 40%/40%, flow rate: 130 mL/min) |
| 51 | 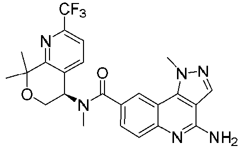 | (R)-4-amino-1-methylpyrazolo[*4,5-c*]quinolin-8-yl)-N-[8,8-dimethyl-2-(trifluoromethyl)(5,6-dihydro-8H-pyrano[*3,4-b*]pyridin-5-yl)]-N-methylformamide | Post peak: SFC (column: Daicel ChiralPak IBN, (250×40 mm I.D., 10 µm), conditions: A for carbon dioxide and B for methanol (0.1% aqueous ammonia), 40%/40%, flow rate: 130 mL/min) |
| 81 | 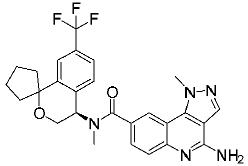 | (*R*)-4-*amino-N,1*-dimethyl-N-(7'-(trifluoromethyl)spiro[cycl opentane-1,1'-isochroman]-4'-yl)-1H-pyrazolo[*4,3-c*]quinoline-8-carboxamide | Prepeak: SFC (column: Daicel ChiralPak IBN, (250×40 mm I.D., 10 µm), conditions: A for carbon dioxide and B for 0.9 methanol (0.1% aqueous ammonia) + 0.1 acetonitrile (0.1% aqueous ammonia), 40%/40%, flow rate: 140 mL/min) |
| 82 | 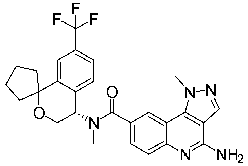 | (S)-4-amino-*N,1*-dimethyl-N-(7'-(trifluoromethyl)spiro[cycl opentane-1,1'-isochroman]-4'-yl)-1H-pyrazolo[*4,3-c*]quinoline-8-carboxamide | Postpeak: SFC (column: Daicel ChiralPak IBN, (250×40 mm I.D., 10 µm), conditions: A for carbon dioxide and B for 0.9 methanol (0.1% aqueous ammonia) + 0.1 acetonitrile (0.1% aqueous ammonia), 40%/40%, flow rate: 140 mL/min) |

### Biological Activity Evaluation

### Inhibitory Activity Test of the Compound on Tumor Cell Proliferation

### Test example 1: Inhibitory activity test of compounds on the proliferation of HCT-116 MTAP(-/-)deleted cells

Materials and cells: HCT-116 MTAP(-/-)deleted cells were purchased from Kyinno Biotechnology (China); RPMI-1640 medium, and fetal bovine serum were purchased from Thermo Fisher (United States); 384-well plates were purchased from PerkinElmer (United States); Cell-Titer Glo kit was purchased from Promega (United States).

Cell culture: HCT116 MTAP(-/-)deleted cells were cultured with RPMI 1640 medium containing 10% fetal bovine serum at 37°C in 5% carbon dioxide. Only cells in the logarithmic growth phase could be used for the test.

Test of inhibitory activity on cell proliferation: The Cell-Titer Glo kit was used to test the proliferation inhibitory activity of the compounds on HCT-116 MTAP(-/-)deleted cells. The cell concentration was adjusted, rendering that cells were inoculated into a 384-well plate at 40 µL per well and incubated overnight at 37°C and 5% carbon dioxide. 80 nL of the compound was added into each well to reach a final concentration of 0-1,000 nM (starting concentration 1,000 nM, 2.5-fold dilution, 10 points), and the cell plate with 0.2% DMSO content was incubated at 37°C and 5% carbon dioxide for 6 d. 40 µL of Cell-Titer Glo reagent was added to test cell viability. The test results are shown in Table 1.

### Test example 2: Inhibitory activity test of compounds on HCT-116 wild-type cells proliferation

Materials and cells: HCT-116 wild-type cells were purchased from Kyinno Biotechnology (China); RPMI-1640 medium, and fetal bovine serum were purchased from Thermo Fisher (United States); 384-well plates were purchased from PerkinElmer (United States); Cell-Titer Glo kit was purchased from Promega (United States).

Cell culture: HCT-116 wild-type cells were cultured in RPMI-1640 medium containing 10% fetal bovine serum at 37°C and 5% carbon dioxide. Only cells in the logarithmic growth phase could be used for the test.

Test of cell proliferation activity: Cell-Titer Glo kit was used to test the proliferation inhibitory activity of the compound on HCT-116 wild-type cells. The cell concentration was adjusted, rendering that cells were inoculated into a 384-well plate at 40 µL per well and incubated overnight at 37°C and 5% carbon dioxide. 80 nL of the compound was added to each well to reach a final concentration of 0-10,000 nM (starting concentration 10,000 nM, 2.5-fold dilution, 10 points), and the cell plate with 0.2% DMSO content was incubated at 37°C and 5% carbon dioxide for 6 d. 40 µL of Cell-Titer Glo reagent was added to test cell viability. The test results are shown in Table 1.

Table 1 below shows the inhibitory activities of the compounds of the Examples against the proliferation of HCT116 MTAP(-/-)deleted cells and HCT116 wild-type cells.

**Table 1**

| Examples | IC₅₀ (nM) | |
|---|---|---|
| | HCT-116 MTAP(-/-)deleted | HCT-116 wild-type |
| 1 | 5.2 | 1202 |
| 2 | >3000 | >10000 |
| 3 | 435 | 7537 |
| 4 | 1089 | 8142.7 |
| 5 | 28.2 | 7770 |
| 6 | 269 | 5947.3 |
| 7 | 5 | 2523.9 |
| 8 | 16 | 3511.1 |
| 9 | 45.4 | >10000 |
| 10 | 10.1 | 2062.1 |
| 11 | 146.6 | 3122.2 |
| 12 | 21.2 | 2613.6 |
| 13 | 10.9 | 720.4 |
| 14 | 4.1 | 285.9 |
| 15 | 13.6 | 445.6 |
| 16 | 3.7 | 486.9 |
| 17 | 139.6 | 5548.2 |
| 18 | 38.4 | 3084.1 |
| 19 | 317.6 | 7214.5 |
| 20 | 737.8 | 5867.1 |
| 21 | 20.8 | 4366.8 |
| 22 | 13.4 | 1251.5 |
| 23 | 99.4 | 4857.6 |
| 24 | 15.1 | 155.8 |
| 25 | 817 | >10000 |
| 26 | 11 | 2054.6 |
| 27 | 74 | >10000 |
| 28 | 10.3 | 226.5 |
| 29 | 17 | 1788.2 |
| 30 | 5.3 | 767 |
| 31 | 6.2 | 285 |
| 32 | 5.7 | 1526.6 |
| 33 | 5.3 | 1337.6 |
| 34 | 8.5 | 1624.7 |
| 35 | 15 | 2097.2 |
| 36 | 95.1 | 2839.2 |
| 37 | 33.4 | 4095.4 |
| 38 | 8.1 | 1472 |
| 39 | 5.2 | 1136.3 |
| 40 | 13 | 2198 |
| 41 | 4.6 | 1027.7 |
| 42 | 4.4 | 1760 |
| 43 | 9.4 | 2131.5 |
| 44 | 7.9 | 837.2 |
| 45 | 874.5 | >10000 |
| 46 | 4.3 | 1095.6 |
| 47 | 5.9 | 1596.6 |
| 48 | 7.9 | 435 |
| 49 | 7.9 | 3849.5 |
| 50 | 412.7 | >10000 |
| 51 | 3.1 | 447 |
| 53 | 312.4 | >10000 |
| 54 | 5.8 | 1807 |
| 55 | 20.2 | >10000 |
| 56 | 4.4 | 1332.8 |
| 57 | 5.2 | 880.2 |
| 74 | 10.5 | 2113.2 |
| 75 | 8.2 | 2606.6 |
| 76 | 6.1 | 2518.6 |
| 77 | 9.8 | 1025.1 |
| 80 | 9.4 | 3845.3 |
| 83 | 103 | 10545 |
| 84 | 6.7 | 1372 |
| 86 | 21.6 | 3787.2 |
| 87 | 11.2 | 763 |

As shown in Table 1, the compounds of the present disclosure exhibit excellent antiproliferative activity against HCT116 MTAP(-/-)cells and excellent selectivity relative to HCT MTAP WT cells.

### Test example 3: Inhibitory activity assay of compounds on proliferation of LU99 MTAP(-/-)deleted cells

Materials and cells: LU99 MTAP deleted cell line was purchased from Kyinno Biotechnology (China); cell culture medium and fetal bovine serum were purchased from Thermo Fisher Scientific (USA); 384-well plates were purchased from PerkinElmer (USA); Cell-Titer Glo kits were purchased from Promega (USA).

Cell culture: LU99 MTAP(-/-)deleted cells were cultured with medium containing 10% fetal bovine serum at 37°C in 5% carbon dioxide. Only cells in the logarithmic growth phase could be used for the test.

Test of inhibitory activity on cell proliferation: The Cell-Titer Glo kit was used to test the proliferation inhibitory activity of the compounds on LU99 MTAP(-/-)deleted cells. The cell concentration was adjusted, rendering that cells were inoculated into a 384-well plate at 40 µL per well and incubated overnight at 37°C and 5% carbon dioxide. 40 nL of the compound was added to each well to reach a final concentration of 0-1,000 nM (starting concentration 1,000 nM, 2.5-fold dilution, 10 points), and the cell plate with 0.2% DMSO content was incubated at 37°C and 5% carbon dioxide for 6 d. 40 µL of Cell-Titer Glo reagent was added to test cell viability. The test results are shown in Table 2.

### Test example 4: Inhibitory activity assay of compounds on proliferation of LU99 MTAP overexpressing cells

Materials and cells: LU99 MTAP overexpressing cell line was purchased from Kyinno Biotechnology (China); cell culture medium and fetal bovine serum were purchased from Thermo Fisher Scientific (USA); 384-well plates were purchased from PerkinElmer (USA); Cell-Titer Glo kits were purchased from Promega (USA).

Cell culture: LU99 MTAP overexpressing cells were cultured with medium containing 10% fetal bovine serum at 37°C in 5% carbon dioxide. Only cells in the logarithmic growth phase could be used for the test.

Test of inhibitory activity on cell proliferation: The Cell-Titer Glo kit was used to test the proliferation inhibitory activity of the compounds on LU99 MTAP overexpressing cells. The cell concentration was adjusted, rendering that cells were inoculated into a 384-well plate at 40 µL per well and incubated overnight at 37°C and 5% carbon dioxide. 40 nL of the compound was added to each well to reach a final concentration of 0-10,000 nM (starting concentration 10,000 nM, 2.5-fold dilution, 10 points), and the cell plate with 0.2% DMSO content was incubated at 37°C and 5% carbon dioxide for 6 d. 40 µL of Cell-Titer Glo reagent was added to test cell viability. The test results are shown in Table 2.

**Table 2**

| Examples | IC₅₀ (nM) | |
|---|---|---|
| | Lu99 MTAP(-/-)Deleted | Lu99 MTAP Overexpressing |
| 4 | 2265 | 3268.2 |
| 5 | 37.1 | 4979.2 |
| 6 | 354.8 | 5938.7 |
| 7 | 3.6 | 3096.3 |
| 8 | 25.2 | 3246 |
| 9 | 85 | 9791 |
| 10 | 18.3 | 3742.8 |
| 12 | 32.3 | 1988.6 |
| 13 | 18.9 | 1067 |
| 14 | 5.9 | 308.8 |
| 15 | 25.9 | 795.1 |
| 16 | 4.7 | 681.6 |
| 17 | 173.8 | 5224.4 |
| 18 | 62.9 | 3972.1 |
| 21 | 33.7 | 2815 |
| 22 | 25.5 | 770.7 |
| 23 | 79.5 | 3174 |
| 24 | 16.4 | 251.7 |
| 25 | 503.2 | >10000 |
| 26 | 14.9 | 1308.2 |
| 27 | 51.9 | 9588.9 |
| 28 | 10.7 | 376.5 |
| 29 | 23.7 | 1034.8 |
| 30 | 10.9 | 862.5 |
| 32 | 6.8 | 1888.9 |
| 33 | 8.6 | 2263.3 |
| 34 | 5.2 | 1656.4 |
| 35 | 24.2 | 858.2 |
| 36 | 116.2 | 668.2 |
| 37 | 36.4 | 809.7 |
| 38 | 15 | 2051 |
| 39 | 8.4 | 1290.4 |
| 40 | 21.2 | 2043.9 |
| 41 | 6.1 | 1856.9 |
| 42 | 6.1 | 1142.8 |
| 43 | 12.3 | 2567 |
| 44 | 12.2 | 673.1 |
| 45 | 943.8 | >10000 |
| 46 | 7.9 | 1053.8 |
| 47 | 7.5 | 1375.4 |
| 49 | 19.2 | 5888.6 |
| 50 | 524.8 | 7396.2 |
| 51 | 4.9 | 710.9 |
| 58 | 5.5 | 986.8 |
| 59 | 3143 | 4016.5 |
| 60 | >3000 | 2951.3 |
| 78 | 17 | 3740.4 |
| 79 | 41.9 | 4376.7 |
| 80 | 16.8 | 3658.8 |
| 81 | 196.5 | 3823 |
| 82 | 5.9 | 523 |
| 83 | 101.5 | 12745 |
| 84 | 10 | 1635.7 |
| 85 | 7.1 | 2136.3 |
| 86 | 31 | 7865.5 |

As shown in Table 2, the compounds of the present disclosure exhibit excellent antiproliferative activity against LU99 MTAP(-/-)cells and excellent selectivity relative to LU99 MTAP overexpressing cells.

Although preferred examples have been described hereinabove, it will be apparent to those skilled in the art that modifications may be made without departing from the present disclosure. Such modifications are considered to be possible variants encompassed within the scope of the present disclosure.

## Claims

1. A compound of Formula (I), a pharmaceutically acceptable salt, an ester, a prodrug, a stereoisomer or an isotopic derivative thereof; 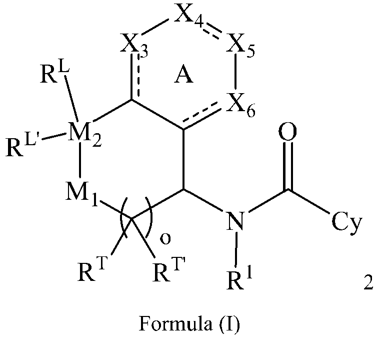
wherein, Cy represents the following structure: 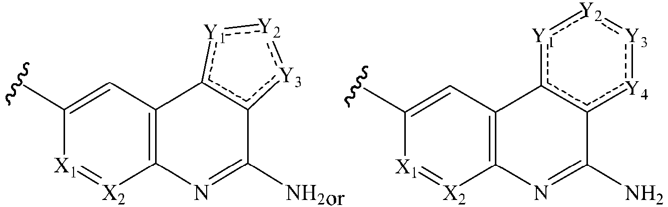
wherein, the dashed line represents a single bond or double bond;
wherein, Y₁ independently represents O, S, Se, N or CR^{Y1};
wherein, Y₂ independently represents O, S, Se, N or CR^{Y2};
wherein, Y₃ independently represents O, S, Se, N or CR^{Y3};
wherein, Y₄ independently represents O, S, Se, N or CR^{Y4};
wherein, X₁ represents N or CR^{X1};
wherein, X₂ represents N or CR^{X2};
wherein, X₃ represents N or CR^{X3};
wherein, X₄ represents N or CR^{X4};
wherein, X₅ represents N or CR^{X5};
wherein, X₆ represents N or CR^{X6};
wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, -OC(O)R^{a}, - OCONR^{a}R^{b}, halogen, -OSO₃R^{a}, -NR^{a}R^{b}, -SF₅, -C(O)OR^{a}, and -CO₂NR^{a}R^{b};
wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, -C(O)OR^{a}, -CO₂NR^{a}R^{b}, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, -NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
wherein, R^{X4} represents -L-R^{X4-1};
wherein, L represents absent or CR^{a}R^{b}, SiR^{a}R^{b}, O, S, Se, or NR^{a};
wherein, R^{X4-1} represents hydrogen, deuterium, C₁-C₆ alkyl, C₁-C₆ deuterated alkyl, C₂-C₆ alkenyl, C₂-C₆ alkynyl, halogen, - OR^{a}, -SR^{a}, -P(O)R^{a}R^{b}, -CN, -S(O)₂R^{a}, -S(O)R^{a}, -SF₅, -NR^{a}R^{b}, -C(O)OR^{a}, -OC(O)R^{a}, -OCONR^{a}R^{b}, -NR^{a}COR^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, - C(O)R^{a}, -C(O)OR^{a}, -OC(O)R^{a}, -OC(O)NR^{a}R^{b}, -NR^{a}COR^{b} and -CONR^{a}R^{b};
wherein R^{Y1}, R^{Y2}, R^{Y3} and R^{Y4} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, - OC(O)R^{a}, -OCONR^{a}R^{b}, halogen, -OSO₃R^{a}, -NR^{a}R^{b}, -SF₅, -C(O)OR^{a}, and -CO₂NR^{a}R^{b};
wherein, said ring A may also be optionally fused with a 5- to 6-membered saturated or unsaturated ring at the chemical bond between X₃ and X₄, which may contain 0-3 heteroatoms selected from O, N and S; wherein, the ring may also optionally be substituted with 0, 1, 2, or 3 substituents selected from the group consisting of: deuterium, C₁-C₆ alkyl, C₁-C₆ deuterated alkyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
wherein, the ring A may also optionally be fused with the chemical bond between X₄ and X₅ to form a 5-6-membered saturated or unsaturated ring which may contain 0-3 heteroatoms selected from O, N and S; wherein, the ring may also optionally be substituted with 0, 1, 2, or 3 substituents selected from the group consisting of: deuterium, C₁-C₆ alkyl, C₁-C₆ deuterated alkyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
wherein, R¹ represents hydrogen or C₁-C₆ alkyl, C₃-C₁₀ cycloalkyl, 4- to 10-membered heterocycloalkyl, C₆-C₁₀ aryl, and 5-to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
preferably, R¹ represents -CHR^{s}R^{t} or -CDR^{s}R^{t};
wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, - OC(O)R^{a}, -OC(O)NR^{a}R^{b}, -NR^{a}COR^{b} and -CONR^{a}R^{b};
wherein, M₁ represents CR^{a}R^{b}, -SiR^{a}R^{b}, NR^{a}, O, S or Se;
wherein, M₂ represents C or Si;
wherein, R^{L} and R^{L'} each independently represent C₁-C₆ alkyl; or R^{L} and R^{L'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -C(O)OR^{a}, -OC(O)R^{a}, -OCONR^{a}R^{b}, halogen, -OSO₃R^{a}, - NR^{a}R^{b}, and -SF₅;
wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -C(O)OR^{a}, -OC(O)R^{a}, -OCONR^{a}R^{b}, halogen, -OSO₃R^{a}, - NR^{a}R^{b}, and -SF₅;
wherein, o represents 0, 1 or 2;
wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S, Se, N and Si.

2. A compound of formula (1-1), a pharmaceutically acceptable salt, an ester, a prodrug, a stereoisomer or an isotopic derivative thereof. 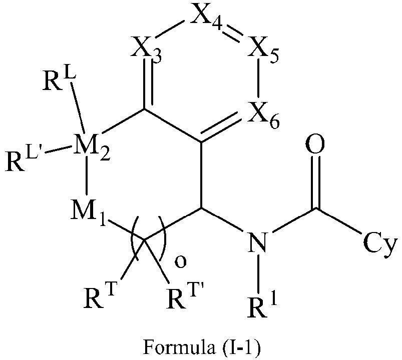
wherein, Cy represents the following structure: 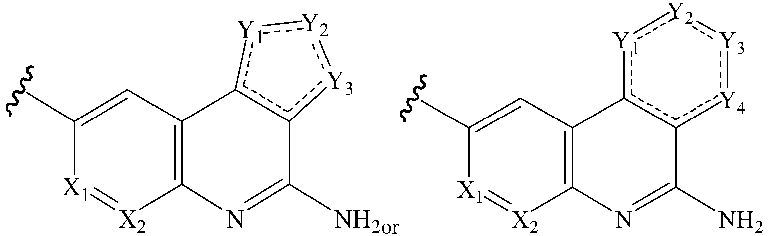
wherein, the dashed line represents a single bond or double bond;
wherein, Y₁ independently represents O, S, Se, N or CR^{Y1};
wherein, Y₂ independently represents O, S, Se, N or CR^{Y2};
wherein, Y₃ independently represents O, S, Se, N or CR^{Y3};
wherein, Y₄ independently represents O, S, Se, N or CR^{Y4};
wherein, X₁ represents N or CR^{X1};
wherein, X₂ represents N or CR^{X2};
wherein, X₃ represents N or CR^{X3};
wherein, X₄ represents N or CR^{X4};
wherein, X₅ represents N or CR^{X5};
wherein, X₆ represents N or CR^{X6};
wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, -OC(O)R^{a}, - OCONR^{a}R^{b}, halogen, -OSO₃R^{a}, -NR^{a}R^{b}, -SF₅, -C(O)OR^{a}, and -CO₂NR^{a}R^{b};
wherein, R^{X3}, R^{X5} and R^{X6} each independently represent hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, -C(O)OR^{a}, -CO₂NR^{a}R^{b}, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, -NR^{a}R^{b}, -CN, C₁-C₆ haloalkyl, C₁-C₆ haloalkoxy, -SO₃R^{a}, - SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b};
wherein, R^{X4} represents -L-R^{X4-1};
wherein, L represents absent or CR^{a}R^{b}, SiR^{a}R^{b}, O, S, Se, or NR^{a};
wherein, R^{X4-1} represents hydrogen, deuterium, C₁-C₆ alkyl, C₁-C₆ deuterated alkyl, C₂-C₆ alkenyl, C₂-C₆ alkynyl, halogen, - OR^{a}, -SR^{a}, -P(O)R^{a}R^{b}, -CN, -S(O)₂R^{a}, -S(O)R^{a}, -SF₅, -NR^{a}R^{b}, -C(O)OR^{a}, -OC(O)R^{a}, -OCONR^{a}R^{b}, -NR^{a}COR^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, - C(O)R^{a}, -C(O)OR^{a}, -OC(O)R^{a}, -OC(O)NR^{a}R^{b}, -NR^{a}COR^{b} and -CONR^{a}R^{b};
wherein R^{Y1}, R^{Y2}, R^{Y3} and R^{Y4} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, - OC(O)R^{a}, -OCONR^{a}R^{b}, halogen, -OSO₃R^{a}, -NR^{a}R^{b}, -SF₅, -C(O)OR^{a}, and -CO₂NR^{a}R^{b};
wherein, R¹ represents hydrogen or C₁-C₆ alkyl, C₃-C₁₀ cycloalkyl, 4- to 10-membered heterocycloalkyl, C₆-C₁₀ aryl, and 5-to 10-membered heteroaryl, wherein the said C₁-C₆ alkyl, C₃-C₁₀ cycloalkyl, 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
preferably, R¹ represents -CHR^{s}R^{t} or -CDR^{s}R^{t};
wherein, R^{s} and R^{t} each independently represent hydrogen, deuterium, -OR^{a}, halogen, -CN, C₁-C₆ alkyl, C₂-C₆ alkenyl, C₂-C₆ alkynyl, C₁-C₆ haloalkyl, C₁-C₆ haloalkoxy, hydroxy (C₁-C₆) alkyl, -C₃-C₁₀ cycloalkyl, or a group selected from 4-10-membered heterocycloalkyl, C₆-C₁₀ aryl or 5-10-membered heteroaryl substituted with 0-3 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, - OC(O)R^{a}, -OC(O)NR^{a}R^{b}, -NR^{a}COR^{b} and -CONR^{a}R^{b};
wherein, M₁ represents CR^{a}R^{b}, -SiR^{a}R^{b}, NR^{a}, O, S or Se;
wherein, M₂ represents C or Si;
wherein, R^{L} and R^{L'} each independently represent C₁-C₆ alkyl; or R^{L} and R^{L'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -C(O)OR^{a}, -OC(O)R^{a}, -OCONR^{a}R^{b}, halogen, -OSO₃R^{a}, - NR^{a}R^{b}, and -SF₅;
wherein, R^{T} and R^{T'} each independently represent hydrogen, deuterium, C₁-C₆ alkyl, halogen, hydroxyl, carboxyl, C₁-C₆ haloalkyl, hydroxy (C₁-C₆ alkyl) or mercapto (C₁-C₆) alkyl, or R^{T} and R^{T'}, together with the atom attached thereto, form a 3-10-membered saturated or unsaturated ring which may also optionally contain 0, 1 or 2 heteroatoms selected from O, N, S and Se; further, the ring may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -C(O)OR^{a}, -OC(O)R^{a}, -OCONR^{a}R^{b}, halogen, -OSO₃R^{a}, - NR^{a}R^{b}, and -SF₅;
wherein, o represents 0, 1 or 2;
wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S and N.

3. The compound according to claim 1 or 2, pharmaceutically acceptable salts, esters, prodrugs, stereoisomers or isotopic derivatives thereof, wherein Cy represents the following structure:

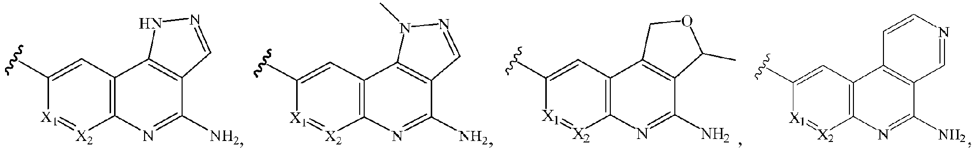

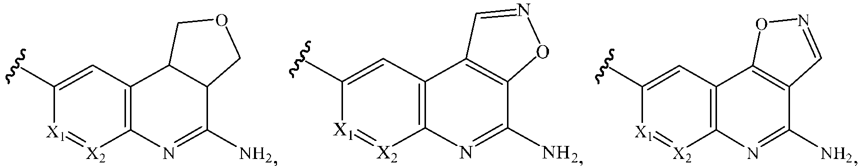

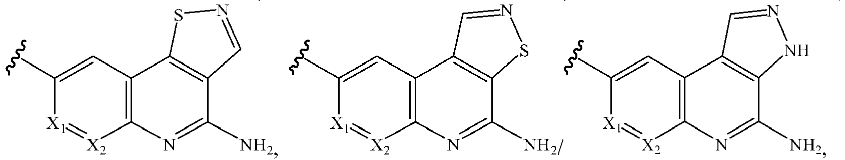

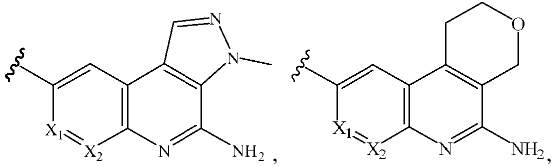

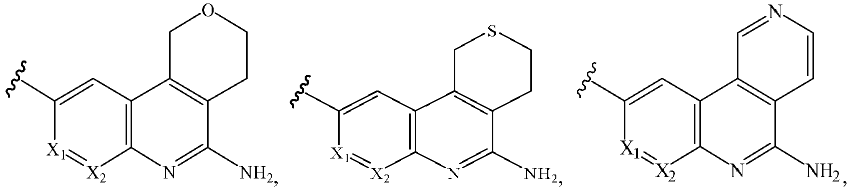

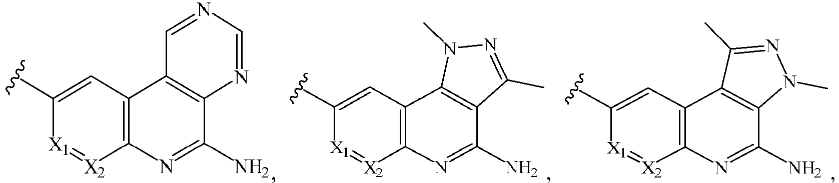

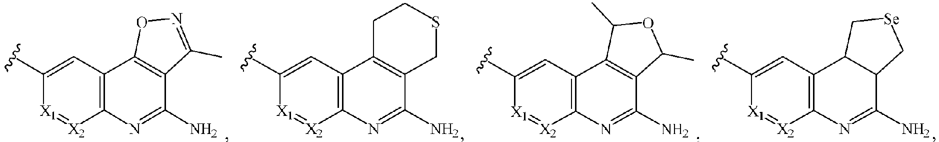

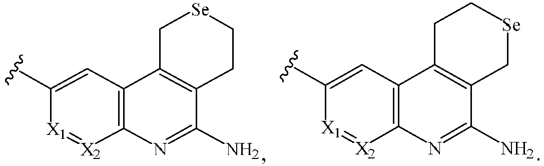

4. The compound according to claim 1 or 2, pharmaceutically acceptable salts, esters, prodrugs, stereoisomers or isotopic derivatives thereof, wherein X₁ represents CR^{X1} or N, and wherein R^{X1} represents hydrogen, deuterium, halogen, - CN, C₁-C₆ alkyl, C₁-C₆ deuterated alkyl, C₁-C₆ alkoxy, or C₁-C₆ haloalkyl.

5. The compound according to claim 1 or 2, pharmaceutically acceptable salts, esters, prodrugs, stereoisomers or isotopic derivatives thereof, wherein X₁ represents CH, CF, or N.

6. The compound according to claim 1 or 2, pharmaceutically acceptable salts, esters, prodrugs, stereoisomers or isotopic derivatives thereof, wherein X₂ represents CH or CD.

7. The compound according to claim 1 or 2, pharmaceutically acceptable salts, esters, prodrugs, stereoisomers or isotopic derivatives thereof, wherein X₂ represents CH.

8. The compound according to claim 1 or 2, pharmaceutically acceptable salts, esters, prodrugs, stereoisomers or isotopic derivatives thereof, wherein X₃ represents CH, CD, or N.

9. The compound according to claim 1 or 2, pharmaceutically acceptable salts, esters, prodrugs, stereoisomers or isotopic derivatives thereof, wherein X₃ represents CH.

10. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents hydrogen, deuterium, C₁-C₆ alkyl, C₁-C₆ deuterated alkyl, C₂-C₆ alkenyl, C₂-C₆ alkynyl, halogen, -OR^{a}, -SR^{a}, -P(O) R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered saturated or unsaturated heterocyclyl, C₆-C₁₀ aryl or 5-10-membered heteroaryl substituted with 0 to 4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b}.

11. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents hydrogen, deuterium, C₁-C₆ alkyl, halogen, C₁-C₆ haloalkyl, -CN, -NH₂, -NHCH₃, -N(CH₃)₂, -OH, -OCH₃, C₁-C₆ alkoxy, C₁-C₆ haloalkoxy, -SF₅, or -P(O)(CH₃)₂.

12. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents hydrogen, halogen (preferably F), -CF₃, -NH₂, -NHCH₃, -N(CH₃)₂, -OCH₃, -OCF₃, -SF₅, or -P(O)(CH₃)₂.

13. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, -NR^{a}COR^{b} and -CONR^{a}R^{b}.

14. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents the following groups: 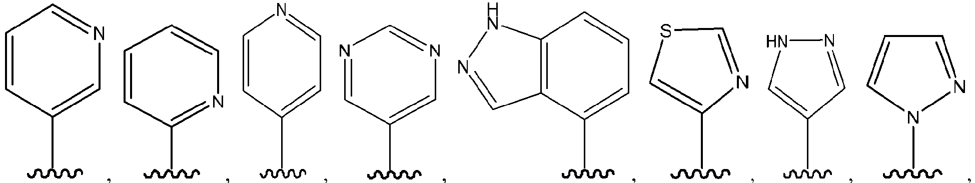 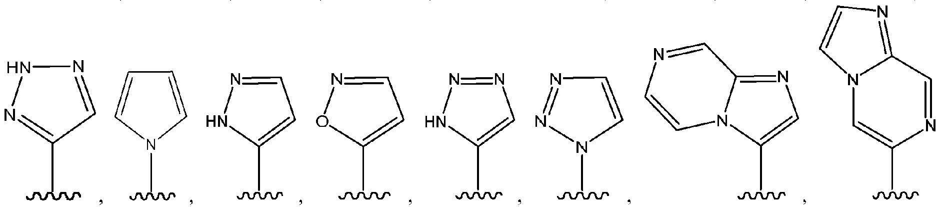 furthermore, R^{X4} may optionally be substituted with 0, 1 or 2 substituents selected from the group consisting of: halogen, hydroxyl, -CN, C₁-C₆ alkyl, C₁-C₆ haloalkyl, and C₁-C₆ haloalkoxy.

15. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents C₃-C₁₀ cycloalkyl, C₆-C₁₀ cycloalkenyl or 4-10-membered saturated or unsaturated heterocycle substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, - OC(O)R^{a}, -OC(O)NR^{a}R^{b}, -NR^{a}COR^{b} and -CONR^{a}R^{b}.

16. The compound according to claim 1 or 2, or a pharmaceutically acceptable salt, ester, prodrug, stereoisomer or isotopic derivative thereof, wherein X⁴ represents C-L-R^{X4-1}; wherein L represents O, S, NH, or N(CH₃), and R^{X4-1} represents the following groups: 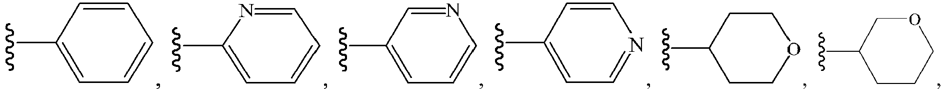 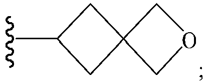 furthermore, R^{X4-1} may optionally be substituted with 0, 1 or 2 substituents selected from the group consisting of: halogen, hydroxyl, -CN, C₁-C₆ alkyl, C₁-C₆ haloalkyl, and C₁-C₆ haloalkoxy.

17. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents the following groups: 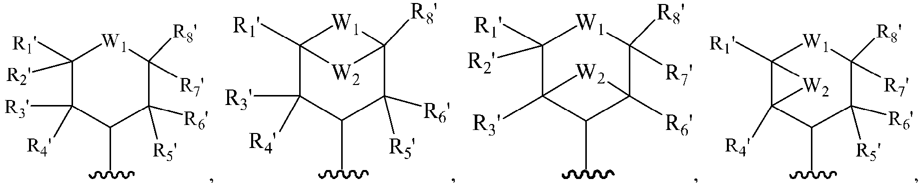 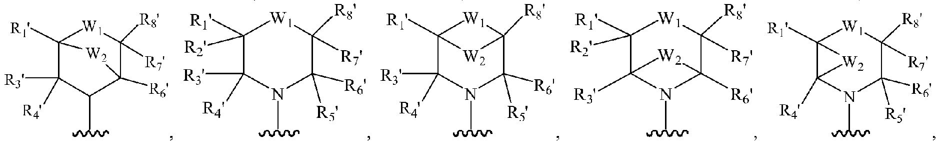 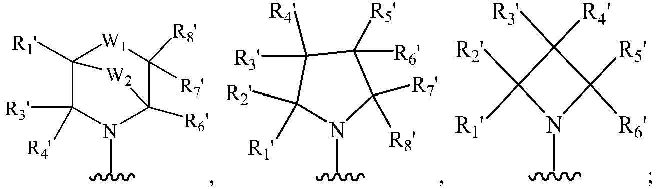
wherein, W₁ represents CR^{C}R^{D}, NR^{C}, O, S, or SiR^{C}R^{D};
wherein, W₂ represents -(CR^{M}R^{N})ᵢ-;
wherein, R₁', R₂', R₃', R₄', R₅'^{,} R₆', R₇' and R₈' each independently represent hydrogen, deuterium, halogen, C₁-C₆ alkyl or hydroxyl; or pairs R₁' and R₂', pairs R₃' and R₄', pairs R₅' and R₆' or pairs R₇' and R₈', together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl, and hydroxyl;
wherein, R^{C} and R^{D} each independently represent hydrogen, deuterium, halogen, C₁-C₆ alkyl or hydroxyl; or R^{C} and R^{D}, together with the atom attached thereto, form a 3-6-membered saturated or unsaturated ring which may also optionally contain 0, 1 or 2 heteroatoms selected from O, S and N; further, the ring may also optionally be substituted with 0, 1 or 2 substituents selected from the group consisting of: deuterium, halogen, C₁-C₆ alkyl, C₁-C₆ haloalkyl, and hydroxyl;
wherein, R^{M} and R^{N} each independently represent hydrogen or C₁-C₆ alkyl;
wherein, i represents an integer of 1 or 2;
furthermore, R^{X4-1} may optionally be substituted with 0, 1 or 2 substituents selected from the group consisting of: halogen, hydroxyl, -CN, C₁-C₆ alkyl, C₁-C₆ haloalkyl, and C₁-C₆ haloalkoxy.

18. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents the following groups: 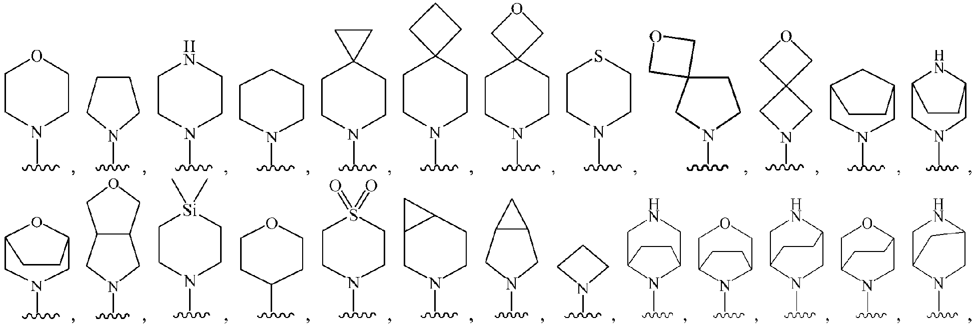 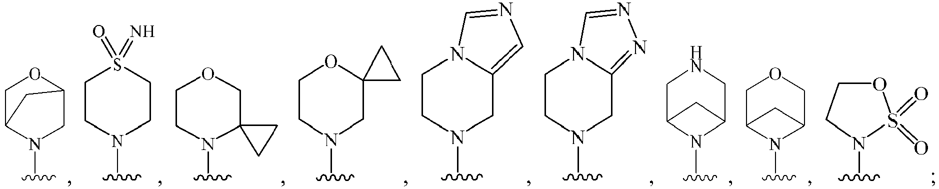 wherein, the R^{X4-1} may optionally be substituted with 0, 1, or 2 substituents selected from the group consisting of: halogen, hydroxyl, cyano, C₁-C₆ alkyl, and C₁-C₆ haloalkyl.

19. The compound according to claim 1 or 2, or a pharmaceutically acceptable salt, ester, prodrug, stereoisomer or isotopic derivative thereof, wherein X⁴ represents C-L-R^{X4-1}; wherein L represents absence, and R^{X4-1} represents the following groups:

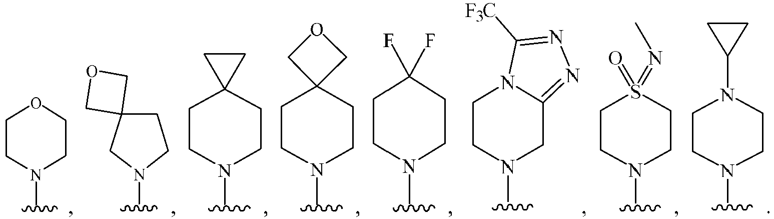

20. The compound according to claim 1 or 2, or a pharmaceutically acceptable salt, ester, prodrug, stereoisomer or isotopic derivative thereof, wherein X₄ represents CR^{X4}; wherein R^{X4} represents C₁-C₆ alkoxy, C₁-C₆ alkylthio, C₁-C₆ haloalkoxy, C₁-C₆ haloalkylthio, C₁-C₆ haloalkyl, or -SF₅.

21. The compound according to claim 1 or 2, or a pharmaceutically acceptable salt, ester, prodrug, stereoisomer or isotopic derivative thereof, wherein X₄ represents CR^{X4}; wherein R^{X4} represents C₁-C₆ haloalkoxy, C₁-C₆ haloalkylthio, C₁-C₆ haloalkyl, or -SF₅.

22. The compound according to claim 1 or 2, pharmaceutically acceptable salts, esters, prodrugs, stereoisomers or isotopic derivatives thereof, wherein X₅ represents CR^{X5} or N, and wherein R^{X5} represents hydrogen, deuterium, C₁-C₆ alkyl, C₁-C₆ alkoxy, C₁-C₆haloalkyl, C₁-C₆ haloalkoxy, halogen, SF₅, or cyano.

23. The compound according to claim 1 or 2, or a pharmaceutically acceptable salt, ester, prodrug, stereoisomer or isotopic derivative thereof, wherein X₅ represents CH.

24. The compound according to claim 1 or 2, or a pharmaceutically acceptable salt, ester, prodrug, stereoisomer or isotopic derivative thereof, wherein X₆ represents CH, CD, or N.

25. The compound according to claim 1 or 2, or a pharmaceutically acceptable salt, ester, prodrug, stereoisomer or isotopic derivative thereof, wherein X₆ represents CH.

26. The compound according to claim 1 or 2, pharmaceutically acceptable salts, esters, prodrugs, stereoisomers or isotopic derivatives thereof, wherein R¹ represents -CHR²R³ or -CDR²R³, wherein R² and R³ each independently represent hydrogen, deuterium, C₁-C₆ alkyl, or C₃-C₁₀ cycloalkyl, 4- to 10- membered heterocycloalkyl, C₆-C₁₀ aryl, or 5- to 10-membered heteroaryl, wherein the said C₃-C₁₀ cycloalkyl, 4- to 10- membered heterocycloalkyl, C₆-C₁₀ aryl, or 5- to 10-membered heteroaryl can be furtherly substituted with 0-3 substituents optionally selected from the group consisting of: halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, - NR^{a}COR^{b}, and -CONR^{a}R^{b}.

27. The compound according to claim 1 or 2, pharmaceutically acceptable salts, esters, prodrugs, stereoisomers or isotopic derivatives thereof, wherein R¹ represents -CHR²R³ or -CDR²R³, wherein R² represents hydrogen, deuterium, C₁-C₆ alkyl, and R³ represents hydrogen, deuterium, C₁-C₆ alkyl, or C₃-C₁₀ cycloalkyl, 4- to 10- membered heterocycloalkyl, C₆-C₁₀ aryl, or 5- to 10- membered heteroaryl, wherein the said C₃-C₁₀ cycloalkyl, 4- to 10- membered heterocycloalkyl, C₆-C₁₀ aryl, or 5- to 10- membered heteroaryl can be furtherly substituted with 0-3 substituents optionally selected from the group consisting of: halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, - OC(O)NR^{a}R^{b}, -NR^{a}COR^{b}, and -CONR^{a}R^{b}.

28. The compound according to claim 1 or 2, pharmaceutically acceptable salts, esters, prodrugs, stereoisomers or isotopic derivatives thereof, wherein R¹ represents C₃-C₁₀ cycloalkyl substituted with 0-3 substituents selected from the group consisting of: deuterated, halogen, C₁-C₆ alkyl, hydroxy (C₁-C₆) alkyl, -OR^{a}, -CN, NR^{a}R^{b}, C₁-C₆ haloalkyl, and C₁-C₆ haloalkoxy.

29. The compound according to claim 1 or 2, or a pharmaceutically acceptable salt, ester, prodrug, stereoisomer or isotopic derivative thereof, wherein R₁ represents C₁-C₆ alkyl (preferably methyl or ethyl) or C₁-C₆ deuterated alkyl (preferably deuterated methyl or deuterated ethyl) or C₃-C₆ cycloalkyl (preferably cyclopropyl).

30. The compound according to claim 1 or 2, pharmaceutically acceptable salts, esters, prodrugs, stereoisomers or isotopic derivatives thereof, wherein M₁ is O or S.

31. The compound according to claim 1 or 2, pharmaceutically acceptable salts, esters, prodrugs, stereoisomers or isotopic derivatives thereof, wherein o is 1 or 2.

32. The compound according to claim 1 or 2, pharmaceutically acceptable salts, esters, prodrugs, stereoisomers or isotopic derivatives thereof, wherein o is 1.

33. The compound according to claim 1 or 2, or a pharmaceutically acceptable salt, ester, prodrug, stereoisomer or isotopic derivative thereof, wherein R^{L} and R^{L'} each independently represent C₁-C₆ alkyl.

34. The compound according to claim 1 or 2, or a pharmaceutically acceptable salt, ester, prodrug, stereoisomer or isotopic derivative thereof, wherein R^{L} and R^{L'}, together with the atom attached thereto, form a 3-6-membered ring.

35. The compound according to claim 1 or 2, or a pharmaceutically acceptable salt, ester, prodrug, stereoisomer or isotopic derivative thereof, wherein R^{L} and R^{L'}, together with the atom attached thereto, form a 3- or 4-membered ring.

36. The compound according to claim 1 or 2, or a pharmaceutically acceptable salt, ester, prodrug, stereoisomer or isotopic derivative thereof, wherein R^{L} and R^{L'}, together with the atom attached thereto, form a ring of the following structure: 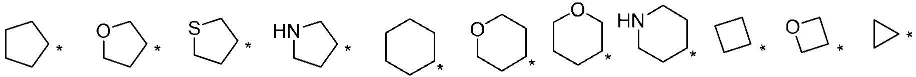 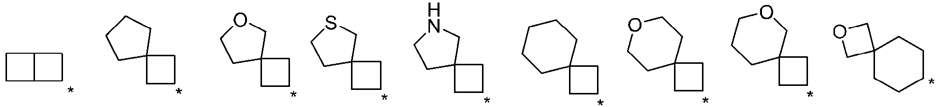 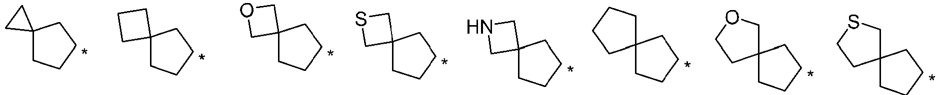 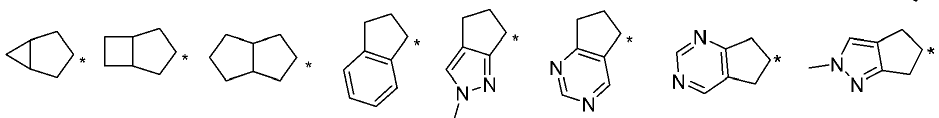 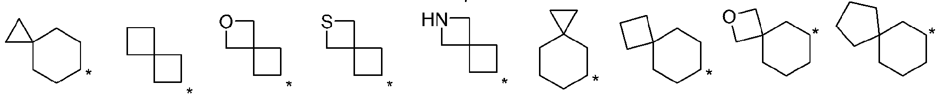 wherein, * represents an atomic site where R^{L} and R^{L'} are attached; further, the above cyclic structures may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, - S(O)₂R^{a}, -S(O)R^{a}, -CN, -C(O)OR^{a}, -OC(O)R^{a}, -OCONR^{a}R^{b}, halogen, -OSO₃R^{a}, -NR^{a}R^{b} and -SF₅.

37. The compound according to claim 1 or 2, or a pharmaceutically acceptable salt, ester, prodrug, stereoisomer or isotopic derivative thereof, wherein R^{T} and R^{T'}, together with the atom attached thereto, form a ring of the following structure: 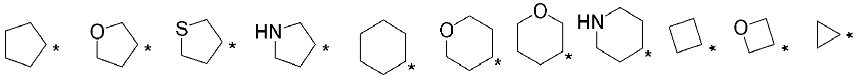 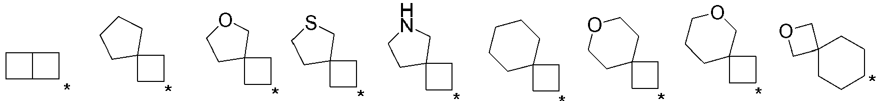 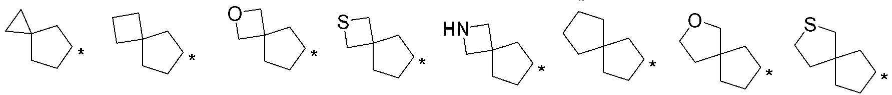 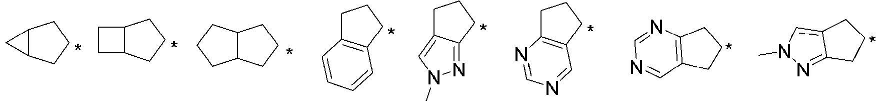 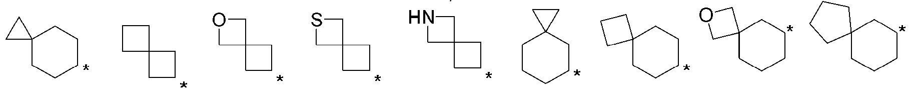 wherein, * represents an atomic site where R^{T} and R^{T'} are attached; further, the above cyclic structures may also optionally be substituted with 0, 1 or 2 substituents selected from the group consisting of: deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, - S(O)₂R^{a}, -S(O)R^{a}, -CN, -C(O)OR^{a}, -OC(O)R^{a}, -OCONR^{a}R^{b}, halogen, -OSO₃R^{a}, -NR^{a}R^{b} and -SF₅.

38. A compound of formula (I-2), or a pharmaceutically acceptable salt, ester, prodrug, stereoisomer or isotopic derivative thereof, 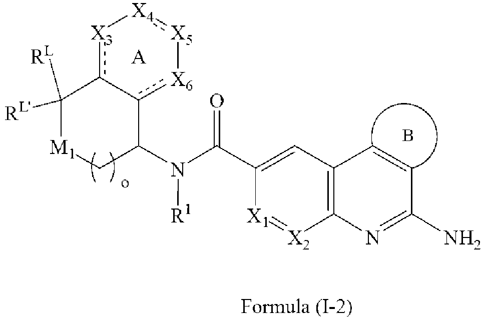
wherein, X₃ represents N or CR^{X3}; X₄ represents N or CR^{X4}; X₅ represents N or CR^{X5}; and X₆ represents N or CR^{X6};
wherein, when X₃ represents CR^{X3}, R^{X3} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, when X₄ represents CR^{X4}, R^{X4} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, when X₅ represents CR^{X5}, R^{X5} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, when X₆ represents CR^{X6}, R^{X6} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, said ring A may also be optionally fused with a 5- to 6-membered saturated or unsaturated ring at the chemical bond between X₃ and X₄, which may contain 0-3 heteroatoms selected from O, N and S;
wherein, the ring A may also optionally be fused with the chemical bond between X₄ and X₅ to form a 5-6-membered saturated or unsaturated ring which may contain 0-3 heteroatoms selected from O, N and S;
wherein, the ring A may also optionally be fused with the chemical bond between X₅ and X₆ to form a 5-6-membered saturated or unsaturated ring which may contain 0-3 heteroatoms selected from O, N and S;
wherein, the ring A may also optionally be substituted with 0, 1, 2 or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -CONR^{a}R^{b};
wherein, R¹ represents C₁-C₆ alkyl, C₃-C₁₀ cycloalkyl, 4- to 10-membered heterocycloalkyl, C₆-C₁₀ aryl, and 5- to 10-membered heteroaryl, wherein the said C₁-C₆ alkyl, C₃-C₁₀ cycloalkyl, 4- to 10-membered heterocycloalkyl, C₆-C₁₀ aryl, and 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy (C₁-C₆) alkyl, NR^{a}R^{b}, -CN, C₁-C₆ haloalkyl, C₁-C₆ haloalkoxy, -SO³R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
preferably, R¹ represents -CHR²R³ or -CDR²R³;
wherein, R² and R³ each independently represent hydrogen, deuterium, -OR^{a}, halogen, -CN, -C₁-C₆ alkyl, C₁-C₆ haloalkyl, C₁-C₆ haloalkoxy, hydroxy (C₁-C₆) alkyl, C₃-C₁₀ cycloalkyl, 4- to 10-membered heterocycloalkyl, C₆-C₁₀ aryl, or 5- to 10-membered heteroaryl, wherein the said 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO³R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
wherein, M¹ represents CR^{a}R^{b}, NR^{a}, O, S or Se;
wherein, R^{L} and R^{L'} each independently represent C₁-C₆ alkyl; or R^{L} and R^{L'}, together with the atom attached thereto, form a 3-6-membered ring;
wherein, o represents 0, 1 or 2;
wherein, ring B represents a 3-10-membered carbocycle, a 6-10-membered unsaturated carbocycle, a 4-10-membered heterocycle, a 6-10-membered unsaturated heterocycle, a C₆-C₁₀ aromatic ring, or a 5-10-membered heteroaromatic ring; the 4-10-membered heterocycle, the 6-10-membered unsaturated heterocycle, and the 5-10-membered heteroaromatic ring may contain 0-3 heteroatoms selected from O, S, and N.
wherein, the ring B may also optionally be substituted with 0, 1, 2 or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO³R^{a}, -SR^{a}, -SF₅, -C(O)R^{a}, -C(O)OR^{a}, -OC(O)R^{a}, -OC(O)NR^{a}R^{b}, - NR^{a}COR^{b} and -CONR^{a}R^{b};
wherein, X₁ represents N or CR^{X1};
wherein, X₂ represents N or CR^{X2};
wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, -OC(O)R^{a}, - OCONR^{a}R^{b}, halogen, -OSO₃R^{a}, -NR^{a}R^{b} and -SF₅;
wherein, -̅ -̅ -̅ represents a single bond or double bond;
wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S, Se, N and Si.

39. The compound according to claim 38, pharmaceutically acceptable salts, esters, prodrugs, stereoisomers or isotopic derivatives thereof, having the structure of formula (I-3) below: 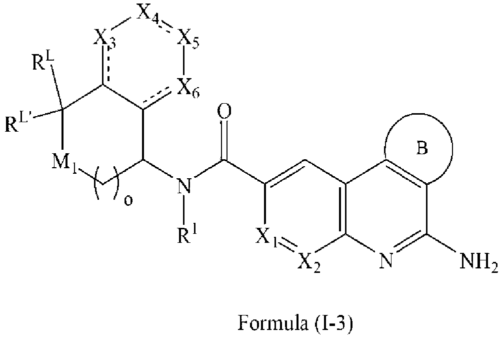
wherein, X₃ represents N or CR^{X3}; X₄ represents N or CR^{X4}; X₅ represents N or CR^{X5}; and X₆ represents N or CR^{X6};
wherein, when X₃ represents CR^{X3}, R^{X3} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, when X₄ represents CR^{X4}, R^{X4} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, when X₅ represents CR^{X5}, R^{X5} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, when X₆ represents CR^{X6}, R^{X6} represents hydrogen, deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, C₁-C₆ haloalkylthio, hydroxy (C₁-C₆) alkyl, or a group selected from C₃-C₁₀ cycloalkyl, C₆-C₁₀ cycloalkenyl, 4-10-membered heterocycloalkyl, 6-10-membered heterocycloalkenyl, C₆-C₁₀ aryl or 5-10-membered heteroaryl substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, - OC(O)NR^{a}R^{b}, -NR^{a}COR^{b} and -CONR^{a}R^{b};
wherein, said ring A may also be optionally fused with a 5- to 6-membered saturated or unsaturated ring at the chemical bond between X₃ and X₄, which may contain 0-3 heteroatoms selected from O, N and S;
wherein, the ring A may also optionally be fused with the chemical bond between X₄ and X₅ to form a 5-6-membered saturated or unsaturated ring which may contain 0-3 heteroatoms selected from O, N and S;
wherein, the ring A may also optionally be fused with the chemical bond between X₅ and X₆ to form a 5-6-membered saturated or unsaturated ring which may contain 0-3 heteroatoms selected from O, N and S;
wherein, the ring A may also optionally be substituted with 0, 1, 2 or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -CONR^{a}R^{b};
wherein, R¹ represents C₁-C₆ alkyl, C₃-C₁₀ cycloalkyl, 4- to 10-membered heterocycloalkyl, C₆-C₁₀ aryl, and 5- to 10-membered heteroaryl, wherein the said C₁-C₆ alkyl, C₃-C₁₀ cycloalkyl, 4- to 10-membered heterocycloalkyl, C₆-C₁₀ aryl, and 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, 3- to 6-membered saturated or unsaturated aliphatic monoheterocyclic group, -OR^{a}, oxo, hydroxy (C₁-C₆) alkyl, NR^{a}R^{b}, -CN, C₁-C₆ haloalkyl, C₁-C₆ haloalkoxy, -SO³R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
preferably, R¹ represents -CHR²R³ or -CDR²R³;
wherein, R² and R³ each independently represent hydrogen, deuterium, -OR^{a}, halogen, -CN, -C₁-C₆ alkyl, C₁-C₆ haloalkyl, C₁-C₆ haloalkoxy, hydroxy (C₁-C₆) alkyl, C₃-C₁₀ cycloalkyl, 4- to 10-membered heterocycloalkyl, C₆-C₁₀ aryl, or 5- to 10-membered heteroaryl, wherein the said 4- to 10-membered heterocycloalkyl, C₆-C₁₀ aryl, or 5- to 10-membered heteroaryl can be furtherly substituted with 0-3 substituents optionally selected from the group consisting of: deuterated, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO³R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b} or -CONR^{a}R^{b};
wherein, M¹ represents CR^{a}R^{b}, NR^{a}, O, S or Se;
wherein, R^{L} and R^{L'} each independently represent C₁-C₆ alkyl; or R^{L} and R^{L'}, together with the atom attached thereto, form a 3-6-membered ring;
wherein, o represents 0, 1 or 2;
wherein, ring B represents a 3-10-membered carbocycle, a 6-10-membered unsaturated carbocycle, a 4-10-membered heterocycle, a 6-10-membered unsaturated heterocycle, a C₆-C₁₀ aromatic ring, or a 5-10-membered heteroaromatic ring; the 4-10-membered heterocycle, the 6-10-membered unsaturated heterocycle, and the 5-10-membered heteroaromatic ring may contain 0-3 heteroatoms selected from O, S, and N.
wherein, the ring B may also optionally be substituted with 0, 1, 2 or 3 substituents selected from the group consisting of: deuterium, C₁-C₆ alkyl, C₁-C₆ deuterated alkyl, halogen, -OR^{a}, -SR^{a}, -P(O)R^{a}R^{b}, -CN, -S(O)₂R^{a}, -S(O)R^{a}, -SF₅, -NR^{a}R^{b}, C₁-C₆ haloalkyl, C₁-C₆ haloalkoxy, and hydroxy (C₁-C₆) alkyl, or substituted with 0-4 substituents selected from the group consisting of: deuterium, halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -SF₅, -C(O)R^{a}, -C(O)OR^{a}, -OC(O)R^{a}, -OC(O)NR^{a}R^{b}, - NR^{a}COR^{b} and -CONR^{a}R^{b};
wherein, X₁ represents N or CR^{X1};
wherein, X₂ represents N or CR^{X2};
wherein, R^{X1} and R^{X2} each independently represent hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, -OR^{a}, -SR^{a}, -S(O)₂R^{a}, -S(O)R^{a}, -CN, -OC(O)R^{a}, - OCONR^{a}R^{b}, halogen, -OSO₃R^{a}, -NR^{a}R^{b} and -SF₅;
wherein, -̅ -̅ -̅ represents a single bond or double bond;
wherein, R^{a} and R^{b} each independently represent hydrogen, deuterium, halogen, C₁-C₆ alkyl, C₃-C₆ cycloalkyl or C₁-C₆ haloalkyl, or R^{a} and R^{b}, together with the atom attached thereto, form a 3-14-membered saturated or unsaturated monocyclic ring, a 3-14-membered saturated or unsaturated spiro ring, or a 3-14-membered saturated or unsaturated fused ring, each of the rings may optionally contain 0-2 heteroatoms selected from O, S, Se, N and Si.

40. The compound according to claim 38 or 39, the pharmaceutically acceptable salt, the ester, prodrug, the stereoisomer or the isotopic derivative thereof, wherein ring B represents the following structure: 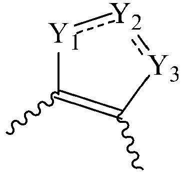
wherein, Y₁ represents CR^{Y1}R^{Y1'}, NR^{Y1}, O, S;
wherein, Y₂ represents CR^{Y2}R^{Y2}, NR^{Y2}, O or S;
wherein, Y₃ represents CR^{Y3}R^{Y3'}, NR^{Y3}, O or S;
wherein, R^{Y1}, R^{Y1'}, R^{Y2}, R^{Y2'}, R^{Y3}, and R^{Y3'} each independently represent absent, hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -OSO₃R^{a}, -NR^{a}R^{b}, -SF₅;
wherein, R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom attached thereto, form a 3- to 14-membered saturated or unsaturated ring which may optionally contain 0-2 heteroatoms selected from O, S, and N.

41. The compound according to claim 38 or 39, the pharmaceutically acceptable salt, the ester, prodrug, the stereoisomer or the isotopic derivative thereof, wherein ring B represents the following structure: 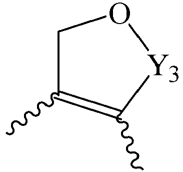 wherein, Y₃ represents CR^{Y3}R^{Y3'}, and R^{Y3} and R^{Y3'} each independently represent hydrogen, deuterium, or C₁-C₆ alkyl.

42. The compound according to claim 38 or 39, the pharmaceutically acceptable salt, the ester, prodrug, the stereoisomer or the isotopic derivative thereof, wherein ring B represents the following structure: 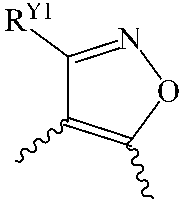 R^{Y1} represents hydrogen, deuterium, or C₁-C₆ alkyl.

43. The compound according to claim 38 or 39, the pharmaceutically acceptable salt, the ester, prodrug, the stereoisomer or the isotopic derivative thereof, wherein ring B represents the following structure: 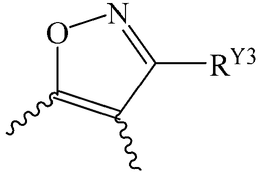 R^{Y3} represents hydrogen, deuterium, or C₁-C₆ alkyl.

44. The compound according to claim 38 or 39, the pharmaceutically acceptable salt, the ester, prodrug, the stereoisomer or the isotopic derivative thereof, wherein ring B represents the following structure: 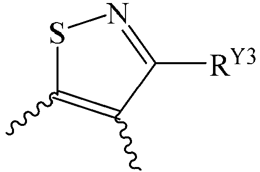 R^{Y3} represents hydrogen, deuterium, or C₁-C₆ alkyl.

45. The compound according to claim 38 or 39, the pharmaceutically acceptable salt, the ester, prodrug, the stereoisomer or the isotopic derivative thereof, wherein ring B represents the following structure: 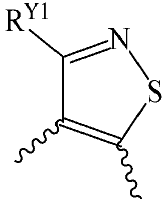 R^{Y1} represents hydrogen, deuterium, or C₁-C₆ alkyl.

46. The compound according to claim 38 or 39, the pharmaceutically acceptable salt, the ester, prodrug, the stereoisomer or the isotopic derivative thereof, wherein ring B represents the following structure: 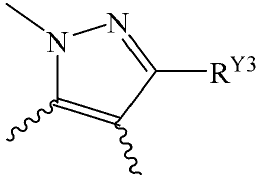 R^{Y3} represents hydrogen, deuterium, or C₁-C₆ alkyl.

47. The compound according to claim 38 or 39, the pharmaceutically acceptable salt, the ester, prodrug, the stereoisomer or the isotopic derivative thereof, wherein ring B represents the following structure: 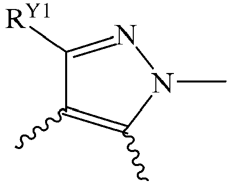 R^{Y1} represents hydrogen, deuterium, or C₁-C₆ alkyl.

48. The compound according to claim 38 or 39, the pharmaceutically acceptable salt, the ester, prodrug, the stereoisomer or the isotopic derivative thereof, wherein ring B represents the following structure: 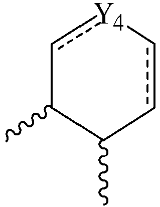
wherein, Y₄ represents CR^{Y4}R^{Y4'}, NR^{Y4}, O or S;
wherein, R^{Y4} and R^{Y4'} each independently represent absence, hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -OSO₃R^{a}, -NR^{a}R^{b}, or -SF₅; wherein, R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom attached thereto, form a 3- to 14-membered saturated or unsaturated ring which may optionally contain 0-2 heteroatoms selected from O, S, and N.

49. The compound according to claim 38 or 39, the pharmaceutically acceptable salt, the ester, prodrug, the stereoisomer or the isotopic derivative thereof, wherein ring B represents the following structure: 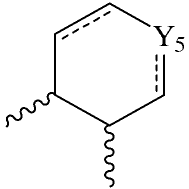
wherein, Y₅ represents CR^{Y5}R^{Y5'}, NR^{Y5}, O, or S;
wherein, R^{Y5} and R^{Y5'} each independently represent absence, hydrogen, deuterium, C₁-C₆ alkyl, C₁-C₆ deuterated alkyl, C₁-C₆ haloalkyl, C₁-C₆ haloalkoxy, hydroxy (C₁-C₆) alkyl, C₂-C₆ alkenyl, C₂-C₆ alkynyl, C₃-C₆ cycloalkyl, -OR^{a}, -SR^{a}, -S(O)₂R^{a}, -S(O)R^{a}, -CN, -OC(O)R^{a}, -OCONR^{a}R^{b}, halogen, -OSO₃R^{a}, -NR^{a}R^{b}, or -SF₅; wherein, R^{a} and R^{b} each independently represent hydrogen, deuterium, halogen, C₁-C₆ alkyl, C₃-C₆ cycloalkyl, C₁-C₆ haloalkyl, or R^{a} and R^{b}, together with the atom attached thereto, form a 3- to 14-membered saturated or unsaturated ring which may optionally contain 0-2 heteroatoms selected from O, S, and N.

50. The compound according to claim 38 or 39, the pharmaceutically acceptable salt, the ester, prodrug, the stereoisomer or the isotopic derivative thereof, wherein -̅ -̅ -̅ represents a double bond.

51. The compound according to claim 38 or 39, or a pharmaceutically acceptable salt, ester, prodrug, stereoisomer or isotopic derivative thereof, wherein X₁ represents CR^{X1} or N; wherein R^{X1} represents hydrogen, deuterium, halogen, -CN, C₁-C₆ alkyl, C₁-C₆ deuterated alkyl, C₁-C₆ alkoxy, or C₁-C₆ haloalkyl.

52. The compound according to claim 38 or 39, or a pharmaceutically acceptable salt, ester, prodrug, stereoisomer or isotopic derivative thereof, wherein X₁ represents CH, CF, or N.

53. The compound according to claim 38 or 39, or a pharmaceutically acceptable salt, ester, prodrug, stereoisomer or isotopic derivative thereof, wherein X₂ represents CH or CD.

54. The compound according to claim 38 or 39, or a pharmaceutically acceptable salt, ester, prodrug, stereoisomer or isotopic derivative thereof, wherein X₂ represents CH.

55. The compound according to claim 38 or 39, or a pharmaceutically acceptable salt, ester, prodrug, stereoisomer or isotopic derivative thereof, wherein X₃ represents CH, CD, or N.

56. The compound according to claim 38 or 39, or a pharmaceutically acceptable salt, ester, prodrug, stereoisomer or isotopic derivative thereof, wherein X₃ represents CH.

57. The compound according to claim 38 or 39, or a pharmaceutically acceptable salt, ester, prodrug, stereoisomer or isotopic derivative thereof, wherein X₄ represents CR^{X4}; wherein R^{X4} represents C₁-C₆ alkoxy, C₁-C₆ alkylthio, C₁-C₆ haloalkoxy, C₁-C₆ haloalkylthio, C₁-C₆ haloalkyl, or -SF₅.

58. The compound according to claim 38 or 39, or a pharmaceutically acceptable salt, ester, prodrug, stereoisomer or isotopic derivative thereof, wherein X₄ represents CR^{X4}; wherein R^{X4} represents C₁-C₆ haloalkoxy, C₁-C₆ haloalkylthio, C₁-C₆ haloalkyl, or -SF₅.

59. The compound according to claim 38 or 39, or a pharmaceutically acceptable salt, ester, prodrug, stereoisomer or isotopic derivative thereof, wherein X₅ represents CR^{X5} or N; wherein R^{X5} represents hydrogen, deuterium, C₁-C₆ alkyl, C₁-C₆ alkoxy, C₁-C₆ haloalkyl, C₁-C₆ haloalkoxy, halogen, SF₅, or cyano.

60. The compound according to claim 38 or 39, or a pharmaceutically acceptable salt, ester, prodrug, stereoisomer or isotopic derivative thereof, wherein X₅ represents CH.

61. The compound according to claim 38 or 39, or a pharmaceutically acceptable salt, ester, prodrug, stereoisomer or isotopic derivative thereof, wherein X₆ represents CH, CD, or N.

62. The compound according to claim 38 or 39, or a pharmaceutically acceptable salt, ester, prodrug, stereoisomer or isotopic derivative thereof, wherein X₆ represents CH.

63. The compound according to claim 38 or 39, or a pharmaceutically acceptable salt, ester, prodrug, stereoisomer or isotopic derivative thereof, wherein R¹ represents -CHR²R³ or -CDR²R³; wherein R² and R³ each independently represent hydrogen, deuterium, C₁-C₆ alkyl, or a group selected from C₃-C₁₀ cycloalkyl, 4-10-membered heterocycloalkyl, C₆-C₁₀ aryl or 5-10-membered heteroaryl substituted with 0-3 substituents selected from the group consisting of: halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO₃R^{a}, -SR^{a}, -S(O)₂R^{a}, -S(O)R^{a}, -SF₅, -C(O)R^{a}, -C(O)OR^{a}, -OC(O)R^{a}, -OC(O)NR^{a}R^{b}, -NR^{a}COR^{b}, and - CONR^{a}R^{b}.

64. The compound according to claim 38 or 39, or a pharmaceutically acceptable salt, ester, prodrug, stereoisomer or isotopic derivative thereof, wherein R¹ represents -CHR²R³ or -CDR²R³; wherein R² represents hydrogen, deuterium, C₁-C₆ alkyl, and R³ represents hydrogen, deuterium, C₁-C₆ alkyl, or a group selected from C₃-C₁₀ cycloalkyl, 4-10-membered heterocycloalkyl, C₆-C₁₀ aryl or 5-10-membered heteroaryl substituted with 0-3 substituents selected from the group consisting of: halogen, C₁-C₆ alkyl, C₂-C₆ alkenyl, C₂-C₆ alkynyl, C₃-C₆ cycloalkyl, -OR^{a}, oxo, hydroxy (C₁-C₆) alkyl, NR^{a}R^{b}, -CN, C₁-C₆ haloalkyl, C₁-C₆ haloalkoxy, -SO³R^{a}, -SR^{a}, -S(O)₂R^{a}, -S(O)R^{a}, -SF₅, -C(O)R^{a}, -C(O)OR^{a}, -OC(O)R^{a}, - OC(O)NR^{a}R^{b}, -NR^{a}COR^{b}, and -CONR^{a}R^{b}.

65. The compound according to claim 38 or 39, or a pharmaceutically acceptable salt, ester, prodrug, stereoisomer or isotopic derivative thereof, wherein R¹ represents C₃-C₁₀ cycloalkyl substituted with 0-3 substituents selected from the group consisting of: deuterium, halogen, C₁-C₆ alkyl, hydroxy (C₁-C₆) alkyl, -OR^{a}, -CN, NR^{a}R^{b}, C₁-C₆ haloalkyl, and C₁-C₆ haloalkoxy.

66. The compound according to claim 38 or 39, or a pharmaceutically acceptable salt, ester, prodrug, stereoisomer or isotopic derivative thereof, wherein R¹ represents C₁-C₆ alkyl (preferably methyl or ethyl) or C₁-C₆ deuterated alkyl (preferably deuterated methyl or deuterated ethyl) or C₃-C₆ cycloalkyl (preferably cyclopropyl).

67. The compound according to claim 38 or 39, or a pharmaceutically acceptable salt, ester, prodrug, stereoisomer or isotopic derivative thereof, wherein M₁ represents O or S.

68. The compound according to claim 38 or 39, or a pharmaceutically acceptable salt, ester, prodrug, stereoisomer or isotopic derivative thereof, wherein o represents 1 or 2.

69. The compound according to claim 38 or 39, or a pharmaceutically acceptable salt, ester, prodrug, stereoisomer or isotopic derivative thereof, wherein o represents 1.

70. The compound according to claim 38 or 39, or a pharmaceutically acceptable salt, ester, prodrug, stereoisomer or isotopic derivative thereof, wherein R^{L} and R^{L'} each independently represent C₁-C₆ alkyl.

71. The compound according to claim 38 or 39, or a pharmaceutically acceptable salt, ester, prodrug, stereoisomer or isotopic derivative thereof, wherein R^{L} and R^{L'}, together with the atom attached thereto, form a 3-6-membered ring.

72. The compound according to claim 38 or 39, or a pharmaceutically acceptable salt, ester, prodrug, stereoisomer or isotopic derivative thereof, wherein R^{L} and R^{L'}, together with the atom attached thereto, form a 3- or 4-membered ring.

73. A compound, or a pharmaceutically acceptable salt, ester, prodrug, stereoisomer or isotopic derivative thereof, having the following structure:
